(19) Europäisches Patentamt
European Patent Office
Office européen des brevets

(11) EP 4 036 085 A1

(12) # EUROPEAN PATENT APPLICATION

(43) Date of publication:
**03.08.2022 Bulletin 2022/31**

(21) Application number: **21153841.8**

(22) Date of filing: **27.01.2021**

(51) International Patent Classification (IPC):
***C07D 311/16*** *(2006.01)* ***A61P 27/02*** *(2006.01)*
***A61L 27/16*** *(2006.01)* ***C07D 311/42*** *(2006.01)*
***C08F 220/30*** *(2006.01)* ***C07F 7/18*** *(2006.01)*

(52) Cooperative Patent Classification (CPC):
(C-Sets available)
**C07D 311/16; A61L 27/16; A61P 27/02;**
**C07D 311/42; C07F 7/1804; C08F 220/30;**
A61L 2430/16 (Cont.)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **AMO Ireland**
**Dublin (IE)**

(72) Inventor: **The designation of the inventor has not yet been filed**

(74) Representative: **Carpmaels & Ransford LLP**
**One Southampton Row**
**London WC1B 5HA (GB)**

(54) **COMPOUNDS FOR OPTICALLY ACTIVE OPHTHALMIC DEVICES**

(57) The present invention relates to novel ophthalmic devices comprising polymerized compounds comprising a photoactive chromophore, special polymerized compounds, and special monomer compounds being particularly suitable for compositions and ophthalmic devices. The present invention is also directed to a process of changing the optical properties of said ophthalmic device or a precursor article for an ophthalmic device.

EP 4 036 085 A1

(52) Cooperative Patent Classification (CPC): (Cont.)

C-Sets
**A61L 27/16, C08L 33/08**

## Description

Field of the Invention

The present invention relates to novel ophthalmic devices comprising polymerized compounds comprising a photoactive chromophore, special polymerized compounds, and special monomer compounds being particularly suitable for compositions and ophthalmic devices. The present invention is also directed to a process of changing the optical properties of said ophthalmic device or a precursor article for an ophthalmic device.

Background of the Invention

Cataract is a general term for an affection of the eye that leads to a loss of vision and in the extreme to blindness by clouding of the normally clear lens of the eye. It is the major cause of blindness in the world, affecting more than 100 million people. Due to the fact that its major cause is age and the population's average age is increasing, it is expected that the number of cataracts will continue to increase substantially in the future.

Effective treatment of cataract is only possible by surgical intervention, whereby the natural lens of the eye is removed through an incision in the cornea and replaced with an ophthalmic device, often also referred to as "intraocular lens". In preparation of surgery current state-of-the-art surgical methods employ eye mapping so as to approximate the refractive power best suited to the respective patient.

Even though cataract surgery is one of the most widely used and safest surgical procedures it is not without specific post-surgery problems. It frequently happens that the refractive power of the implanted intraocular lens (IOL) is insufficient for restoring good vision. Such problems may, for example, be caused by changes in eye geometry as consequence of the surgery as well as irregular wound healing and positioning errors that result in the ophthalmic device not having the optimal optical properties. As a result the patient will still require corrective vision aids, e.g. glasses, to be able to see correctly. In some cases the resulting refractive power of the implanted ophthalmic device is so far removed from the required refractive power that further surgery will be required. Particularly for aged persons this is not desirable because the body's capability for healing is reduced with increasing age. Furthermore, there is the risk of attracting endophthalmitis, an inflammation of the eye, which can even lead to a complete loss of vision or worse, loss of the eye.

There is therefore a need in the health sector for optically active ophthalmic devices, and particularly for artificial intraocular lenses, that would allow for non-invasive adjustment of refractive power after implantation of the lens, thereby preferably further reducing the need for post-surgery vision aids.

Some developments in this sense have already been made, as for example evidenced by WO 2007033831, WO 2009074520, US 20100324165, WO 2017032442, WO 2017032443, WO 2017032444, WO 2018149850, WO 2018149852, WO 2018149853, WO 2018149855, WO 2018149856, WO 2018149857, WO2019121642. M. Schraub et al, European Polymer Journal 51 (2014) 21-27 describe the photochemistry of 3-phenyl-coumarin containing polymethacrylates.

When a chromophore undergoes cycloaddition there is a change in both density and polarizability. Refractive index is a function of density and polarizability according to the Lorentz-Lorenz equation (Equation 1), where $M$ is the molar mass, $\rho$ is the density, $N$ is the number density of molecules, and $\alpha$ is the polarizability. The conversion of a carbon-carbon double bond to carbon-carbon single bond results in a reduction of volume of 22 $cm^3$/mole (Patel, M.P. et al., Biomaterials, 1987, 8, 53-56).

Equation 1. $$\frac{n^2-1}{n^2+2}\frac{M}{\rho} = \frac{4}{3}\pi N\alpha$$

This alone would lead to an increase in refractive index due to cycloaddition. However, by taking advantage of the nonlinear decrease in refractivity caused by breaking of conjugated systems, large negative refractive index changes can be seen for certain chromophores, in far excess of the positive refractive index change of volume reduction.

However, large conjugated chromophores also show large optical dispersion. Optical dispersion is characterized by Abbe number, $V_D$, defined by Equation 2.

Equation 2. $$V_D = \frac{n_D-1}{n_F-n_C}$$

High optical dispersion is characterized by low Abbe number and is detrimental in optical applications where more than one wavelength of light passes through the material such as ophthalmic devices e.g. lenses. The higher the Abbe number

is, the lower the chromatic aberration.

S. Helmstetter et al, J Polym Res, 2016, 23:249, describes qinolinone-based cross-linked homo- as well as copolymers showing refractive indices at 589 nm ranging from 1.60 up to 1.68 and Abbe numbers of 19 to 25. It is further reported that the higher the refractive index of a lens material is, the thinner the intraocular lens becomes but in most cases this is accompanied by an increase in glass transition temperature and a decrease of the Abbe number. It is reported that the optimal balance between high refractive index, low glass transition temperature, and high Abbe number is the chemical challenge in polymer synthesis for IOL manufacture and that state-of-the-art foldable but not adjustable hydrophobic IOLs have refractive indices up to 1.55, glass transition temperatures of about 14 to 15°C and Abbe numbers up to 37.

C. E. Wheelock, J. Am. Chem. Soc., 1959, 81, 6, 1348-1352 describes the fluorescence of some coumarins.

K. Muthuramu and V. Ramamurthy, Ind. J. Chem. B, 1984, 23B, 6, 502-508 describe photodimerization of substituted coumarins in micelles.

J. Träger et al, Macromolecular Bioscience, 2008, 8, 2, 177-183 describe polymers for *in vivo* tuning of refractive properties in intraocular lenses.

M. Schraub and N. Hampp, Proc. Of SPIE, 2011, 7885 (Ophthalmic Technologies XXI), 78851Z-1-78851Z-11 describe smart polymers containing substituted coumarin side groups enabling photo-induced tuning of focal length of intraocular lenses.

K. Tietz et al, Macromol. Chem. Phys., 2014, 215(16), 1563-1572 describe photocrosslinkable materials produced via self-assembly of poly(methyl methacrylate)-*block*-poly(*n*-butyl acrylate) block copolymers, in which the poly(methyl methacrylate) block is decorated with coumarin moieties.

US5587444 describes polymers or oligomers of coumarin derivatives or quinolinone derivatives which can be photochemically dimerized and their use as a liquid crystal orientation layer.

WO9610049 describes polymers or oligomers of coumarin derivatives or quinolinone derivatives which can be photochemically dimerized and their use as a liquid crystal orientation layer.

JPH1174077 describes a multilayered electroluminescent element using copolymers.

JP2009099253 describes optical recording materials.

WO2009074520 and WO2009074521 describe acrylic ophthalmologic compositions and their use.

WO2010049044 describes a liquid-crystal medium comprising coumarin derivatives.

WO2012097858 describes polymerizable compounds and the use thereof in liquid-crystal displays.

US2013033975 describes copolymers comprising 4-methyl substituted coumarin moieties as part of a medium for reversible recording.

US20140356788 describes fluorinated photoresist with integrated sensitzer.

Photosensitive reactive polymers are disclosed e.g. in US2016018737, US2017081532 or US20170306121.

However, there is still a need to provide alternative or improved ophthalmic devices e.g. contact lenses or lenses to be implanted by state of the art surgical methods, especially cataract surgical methods, and there is still a need to provide special compounds for the manufacture of ophthalmic devices e.g. of intraocular lenses to be implanted by state of the art surgical methods, particularly by state of the art micro-incision cataract surgical methods. Preferably, high refractive index-change materials with high Abbe numbers are needed to fulfill the above mentioned needs.

Consequently, it is an objective of the present application to provide for alternative or improved ophthalmic devices and suitable compounds for the manufacture of such ophthalmic devices.
It is also an objective of the present application to provide for compounds, the optical properties of which may be changed, preferably by non-invasive techniques.

It is a further objective of the present application to provide alternative compounds or compounds having advantages over currently known compounds, preferably in combination with being suitable for ophthalmic devices.

Advantages for polymers or copolymers comprising polymerized monomers of formula (I) according to the invention are shown in the experimental section. The polymers or copolymers as well as the ophthalmic device comprising said material according to the invention preferably show a significant polarizability change or refractive index change after irradiation. In addition, the said polymers or copolymers ensure a much higher Abbe number compared to prior art materials.

Advantages for polymers or copolymers comprising polymerized monomers of formula (I) according to the invention are further good flexibilities and low glass transition temperatures.

Based on this advantage of the polymers or copolymers of the invention, the ophthalmic device comprising said materials has an intrinsically lower chromatic aberration.

## Summary of the Invention

The present inventors have now found that the above objects may be attained either individually or in any combination by ophthalmic devices and the compounds of the present application.

The invention relates to an ophthalmic device or a precursor article for an ophthalmic device comprising at least one polymerized compound of formula (I),

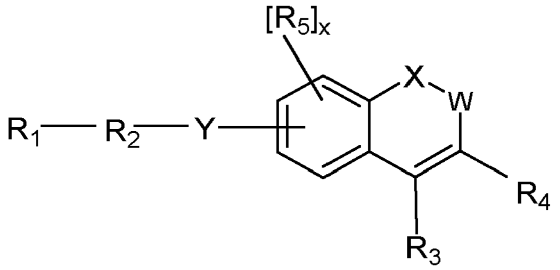

(I)

,

wherein

X-W is O-C(=O), O-C(=S), O-$SO_2$, $SO_2$-O, $SO_2$-C(=S), S-C(=O), S-C(=S), $NR_B$-C(=O) or $NR_B$-C(=S);

$R_B$ is at each occurrence independently H, a linear or branched alkyl group having 1 to 6 C atoms or a linear or branched partially or fully fluorinated alkyl group having 1 to 10 C atoms;

$R_3$ is a linear or branched alkyl group having 1 to 8 C atoms or a cycloalkyl group having 3 to 7 C atoms;

$R_4$ is F, Cl, CN, a linear or branched alkyl group having 1 to 8 C atoms or a partially or fully fluorinated or partially or fully chlorinated alkyl group having 1 to 8 C atoms, a linear or branched partially or fully fluorinated alkoxy group having 1 to 8 C atoms, a linear or branched optionally partially or fully fluorinated alkoxyalkyl group having 2 to 8 C atoms or a cycloalkyl group having 3 to 6 C atoms which may be partially or fully fluorinated;

$R_5$ is at each occurrence independently a linear or branched alkyl group having 1 to 8 C atoms or a linear or branched alkoxy group having 1 to 8 C atoms;

x is 0 or 1;

Y is O, S, SO, $SO_2$, Se, $NR_0$ or a bond;

$R_0$ is at each occurrence independently selected from the group consisting of a linear or branched alkyl group having 1 to 4 C atoms;

$R_1$ is a trialkoxysilyl group or a dialkoxyalkylsilyl group where the alkyl and/or alkoxy groups are each independently linear or branched having 1 to 6 C atoms, or a silyl group of formula (1), (2) or (3) or a polymerizable group of formula (4),

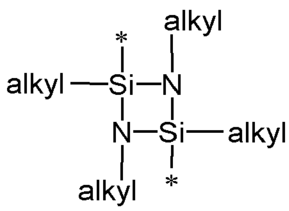

(1)

,

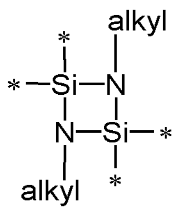

(2)

,

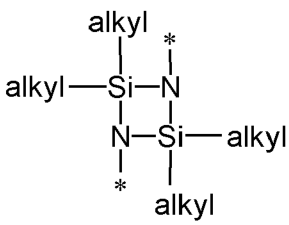

(3)

,

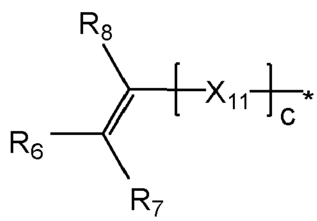

(4)

,

where alkyl means at each occurrence independently of each other a linear or branched alkyl group having 1 to 6 C atoms and the asterisk "*" denotes at each occurrence independently of each other a linkage to the linker -$R_2$-Y-;

and wherein

$X_{11}$ is selected from the group consisting of O, S, O-$SO_2$, $SO_2$-O, C(=O), OC(=O), C(=O)O, S(C=O) and (C=O)S,

$R_6$, $R_7$, $R_8$ are at each occurrence independently of each other selected from the group consisting of H, F, a linear or branched, non-fluorinated, partially or completely fluorinated alkyl group having 1 to 20 C atoms and

aryl with 6 to 14 C atoms and
c is 0 or 1;

-$R_2$- is -$(C(R)_2)_o$-, or -$(C(R)_2)_p$-$X_8$-$(C(R)_2)_q$-$(X_9)_s$-$(C(R)_2)_r$-$(X_{10})_t$-$(C(R)_2)_u$-; R is at each occurrence independently selected from the group consisting of H, F, a linear or branched alkyl group having 1 to 4 C atoms or a linear or branched partially or fully fluorinated alkyl group having 1 to 4 C atoms;
o is selected from the group consisting of 5 to 20,
$X_8$, $X_9$, $X_{10}$ are at each occurrence independently O, S, $SO_2$, or $NR_B$,
s, t is 0 or 1,
p, q are at each occurrence independently selected from the group consisting of 1 to 10,
r, u are at each occurrence independently selected from the group consisting of 0 to 10, wherein the overall number of atoms for -$(C(R)_2)_p$-$X_8$-$(C(R)_2)_q$-$(X_9)_s$-$(C(R)_2)_r$-$(X_{10})_t$-$(C(R)_2)_u$- is up to 20 atoms.

The invention relates further to a process of forming an ophthalmic device or a precursor article for an ophthalmic device as described before or preferably described below, said process comprising the steps of

- providing a composition comprising at least one compound of formula (I) as described before or preferably described below and/or an oligomer or polymer derived from a compound of formula (I) as described below or preferably described below and having at least one reactive group left for polymerization and optionally further monomers different from compounds of formula (I) and/or crosslinking agents and/or UV absorbers and/or radical initiators;
- subsequently forming the ophthalmic device or precursor article of said composition.

The invention relates further to a process of changing the optical properties of an ophthalmic device or a precursor article for an ophthalmic device as described before or preferably described below said process comprising the steps of

- providing an ophthalmic device or a precursor article with the process as described before or preferably described below, and
- subsequently exposing said ophthalmic device or precursor article to irradiation having a wavelength of at least 200 nm and at most 1500 nm.

The invention relates further to an ophthalmic device or precursor article for an ophthalmic device obtainable by said process.

The invention relates further to compounds of formula (I),

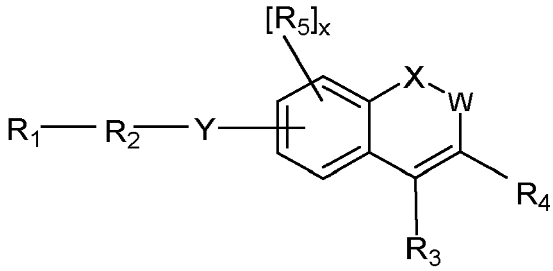

(I)

,

wherein

X-W is O-C(=O), O-C(=S), O-$SO_2$, $SO_2$-O, $SO_2$-C(=S), S-C(=O), S-C(=S), $NR_B$-C(=O) or $NR_B$-C(=S);

$R_B$ is at each occurrence independently H, a linear or branched alkyl group having 1 to 6 C atoms or a linear or branched partially or fully fluorinated alkyl group having 1 to 10 C atoms;

$R_3$ is a linear or branched alkyl group having 1 to 8 C atoms or a cycloalkyl group having 3 to 7 C atoms;

$R_4$ is F, Cl, a linear or branched alkyl group having 1 to 8 C atoms or a partially or fully fluorinated or partially or fully chlorinated alkyl group having 1 to 8 C atoms, a linear or branched partially or fully fluorinated alkoxy group having 1 to 8 C atoms, a linear or branched optionally partially or fully fluorinated alkoxyalkyl group having 2 to 8 C atoms or or a cycloalkyl group having 3 to 7 C atoms which may be partially or fully fluorinated;

$R_5$ is at each occurrence independently a linear or branched alkyl group having 1 to 8 C atoms or a linear or branched alkoxy group having 1 to 8 C atoms;

x is 0 or 1;

Y is O, S, SO, $SO_2$, Se, $NR_0$ or a bond;

$R_0$ is at each occurrence independently selected from the group consisting of a linear or branched alkyl group having 1 to 4 C atoms;

$R_1$ is a trialkoxysilyl group or a dialkoxyalkylsilyl group where the alkyl and/or alkoxy groups are each independently linear or branched having 1 to 6 C atoms, or a silyl group of formula (1), (2) or (3) or a polymerizable group of formula (4),

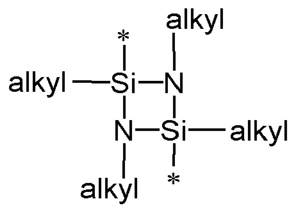 (1) ,

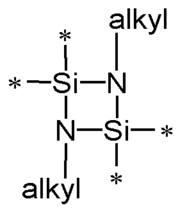 (2) ,

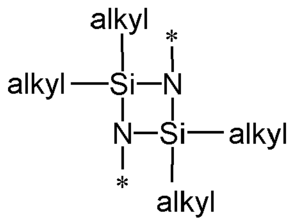 (3) ,

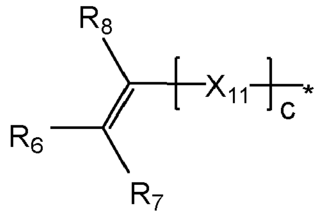 (4) ,

where alkyl means at each occurrence independently of each other a linear or branched alkyl group having 1 to 6 C atoms and the asterisk "*" denotes at each occurrence independently of each other a linkage to the linker $-R_2$-Y-;
and wherein
$X_{11}$ is selected from the group consisting of O, S, O-$SO_2$, $SO_2$-O, C(=O), OC(=O), C(=O)O, S(C=O) and (C=O)S,
$R_6$, $R_7$, $R_8$ are at each occurrence independently of each other selected from the group consisting of H, F, a linear or branched, non-fluorinated, partially or completely fluorinated alkyl group having 1 to 20 C atoms and aryl with 6 to 14 C atoms and
c is 0 or 1;

$-R_2-$ is $-(C(R)_2)_o-$;
R is at each occurrence independently selected from the group consisting of H, F, a linear or branched alkyl group having 1 to 4 C atoms or a linear or branched partially or fully fluorinated alkyl group having 1 to 4 C atoms; and
o is selected from the group consisting of 5 to 20.

The invention relates further to oligomers, polymers or copolymers comprising at least one polymerized compound of formula (I) as described before or preferably described below, where $R_4$ is F, Cl, a linear or branched alkyl group having 1 to 8 C atoms or a partially or fully fluorinated or partially or fully chlorinated alkyl group having 1 to 8 C atoms, a linear or branched partially or fully fluorinated alkoxy group having 1 to 8 C atoms, a linear or branched optionally partially or fully fluorinated alkoxyalkyl group having 2 to 8 C atoms or a cycloalkyl group having 3 to 7 C atoms which may be partially or fully fluorinated and $-R_2-$ is $-(C(R)_2)_o-$, where R and o have a meaning as defined before.

The invention relates further to compositions comprising at least one compound of formula (I) as described before or preferably described below, where $R_4$ is F, Cl, a linear or branched alkyl group having 1 to 8 C atoms or a partially or fully fluorinated or partially or fully chlorinated alkyl group having 1 to 8 C atoms, a linear or branched partially or fully fluorinated alkoxy group having 1 to 8 C atoms, a linear or branched optionally partially or fully fluorinated alkoxyalkyl group having 2 to 8 C atoms or a cycloalkyl group having 3 to 7 C atoms which may be partially or fully fluorinated and $-R_2-$ is $-(C(R)_2)_o-$, where R and o have a meaning as defined before and/or an oligomer or polymer derived from said compounds of formula (I) and having at least one reactive group left for polymerization.

Detailed Description of the Invention

Compounds of formula (I) as described before or preferably described below can be preferably used as monomers for the preparation of a precursor article such as a blank which may be transformed to an ophthalmic device such as an eye-implant or specifically an intraocular lens or can be preferably used for the preparation of the ophthalmic

device as such as described before or preferably described below.

The compounds of formula (I) and all preferred embodiments of compounds of formula (I) including any monomeric units according to the present invention include all stereoisomers or racemic mixtures.

The compounds of formula (I) provide several advantages over prior art materials for the preparation of ophthalmic devices or precursor articles for an ophthalmic device according to the invention.

One way chosen in prior art to increase the polarizability was by extending the aromatic system of the photoactive unit. However, the Abbe number usually tends to lower with an increase of the aromatic system as described before. The inventors found that making use of the substitution in 3-position or 4-position by the substituent Y-$R_2$-$R_1$ as described still enables a high polarizability of the double bond and maintains high Abbe numbers.

Moreover, the addition of the substituent $R_4$ in 3-position and $R_3$ in 4-position of the central chromophore in the compounds of formula (I) or oligomers, polymers and copolymers comprising polymerized compounds of formula (I) has a significant impact on the optical properties over prior art compounds as described before. With the above described substitution pattern of compounds of formula (I) the melting point of said compounds and $T_g$ values of the respective polymers/copolymers can be lowered compared to prior art materials e. g. compared to those materials as described in M. Schraub and N. Hampp, Proc. Of SPIE, 2011, 7885 (Ophthalmic Technologies XXI), 78851Z-1-78851Z-11. Also solubility of compounds of formula (I) with common co-monomers is increased compared to compounds which bear a halogen atom on the main aromatic ring within the coumarine structure motif as described in M. Schraub and N. Hampp, Proc. Of SPIE, 2011, 7885 (Ophthalmic Technologies XXI), 78851Z-1-78851Z-11.

Another general aspect in the evaluation of polymers/copolymers in intraocular lenses during their material testing is the behavior upon irradiation of light in so called photostability tests. Compounds of formula (I) with the above described substitution pattern are found to become polymers/copolymers exhibiting an increased photostability over prior art materials for the preparation of ophthalmic devices or precursor articles for an ophthalmic device.

Polymers/copolymers that are foldable at room temperature generally exhibit glass transition temperatures ($T_g$) lower than room temperature (ca. 21°C). They are easily deformable at this temperature without causing physical damage to the polymer, for example by inducing creep, stress or fissures. For polymers in intraocular lenses, $T_g$'s of less than or equal to 15°C are preferred.

Polymers/copolymers used in ophthalmic device manufacturing, preferably in intraocular lens manufacturing, have preferably relatively high refractive indices, which enable the fabrication of thinner ophthalmic devices such as intraocular lenses. Preferably, the polymer used in an ophthalmic device, preferably an intraocular lens, will have a refractive index greater than about 1.5 and presently most preferably greater than about 1.55.

Polymers/copolymers used in ophthalmic device manufacturing, preferably in intraocular lens manufacturing, have preferably relatively high Abbe numbers. Preferably, the polymer/copolymer used in an ophthalmic device, preferably an intraocular lens, will have an Abbe number greater than 35 and presently most preferably greater than or equal to 37.

In case an asterisk ("*") is used within the description of the present invention, it denotes a linkage to an adjacent unit or group or, in case of a polymer, to an adjacent repeating unit or any other group whenever it is not specifically defined.

A linear or branched alkyl group having 1 to 10 C atoms denotes an alkyl group having 1, 2, 3, 4, 5, 6, 7, 8, 9 or 10 C atoms, for example methyl, ethyl, *iso*-propyl, *n*-propyl, *iso*-butyl, *n*-butyl, *tert*-butyl, *n*-pentyl, 1-, 2- or 3-methylbutyl, 1,1-, 1,2- or 2,2-dimethylpropyl, 1-ethylpropyl, *n*-hexyl, *n*-heptyl, *n*-octyl, ethylhexyl, *n*-nonyl or *n*-decyl. A linear or branched alkyl group having 1 to 20 C atoms include all examples for a linear or branched alkyl group having 1 to 10 C atoms including any alkyl group having 11, 12, 13, 14, 15, 16, 17, 18, 19 and 20 C atoms such as *n*-undecyl, *n*-dodecyl, *n*-tridecyl, *n*-tetradecyl, *n*-pentadecyl, *n*-hexadecyl, *n*-heptadecyl, *n*-octadecyl, *n*-nonadecyl and *n*-eicosyl.

The term partially fluorinated alkyl group denotes that at least one H atom of the alkyl group is replaced by F. A preferred partially flourinated alkyl group is $CH_2CF_3$.

The term completely fluorinated alkyl group denotes that all H atoms of the alkyl group are replaced by F. A preferred completely fluorinated alkyl group is trifluoromethyl or pentafluoroethyl.

The term partially chlorinated alkyl group denotes that at least one H atom of the alkyl group is replaced by Cl. A preferred partially chlorinated alkyl group is $CH_2CCl_3$.

The term completely chlorinated alkyl group denotes that all H atoms of the alkyl group are replaced by Cl. A preferred completely fluorinated alkyl group is trichloromethyl or pentachloroethyl.

The term chlorinated or fluorinated corresponds additionally to other groups such as a chlorinated or fluorinated cycloalkyl group or fluorinated alkoxy group or partially or fully fluorinated alkoxyalkyl group.

A cycloalkyl group having 3 to 7 C atoms includes cyclopropyl, cyclobutyl, cyclopentyl, cyclohexyl and cycloheptyl. Preferably, the cycloalkyl group is cyclopentyl or cyclohexyl.

A linear or branched alkoxy group having 1 to 8 C atoms denotes an O-alkyl group having 1, 2, 3, 4, 5, 6, 7 or 8 C atoms, for example methoxy, ethoxy, *iso*-propoxy, *n*-propoxy, *iso*-butoxy, n-butoxy, *tert*-butoxy, *n*-pentyloxy, 1-, 2- or 3-methylbutyloxy, 1,1-, 1,2- or 2,2-dimethylpropoxy, 1-ethylpropoxy, *n*-hexyloxy, *n*-heptyloxy, *n*-octyloxy and ethylhexyloxy.

A preferred partially fluorinated alkoxy group is $OCH_2F_3$.
A preferred fully fluorinated alkoxy group is trifluoromethoxy.

A linear or branched optionally partially or fully fluorinated alkoxyalkyl group having 2 to 8 C atoms denotes an alkyl group which is substituted by an alkoxy group where at least one H atom is substituted by F in case it is a partially fluorinated alkoxyalkyl group. The alkoxyalkyl group as described before has 2 to 8 C atoms in total, for example methoxymethyl, ethoxymethyl, propoxymethyl, butoxymethyl, pentoxymethyl, methoxyethan-2-yl, methoxypropan-3-yl, methoxybutan-4-yl, methoxypentan-5-yl, methoxyhexan-6-yl, ethoxyethane-2-yl, propoxyethan-2-yl, butoxyethan-2-yl, pentoxyethan-2-yl, ethoxypropan-3-yl, ethoxybutan-4-yl, ethoxypentan-5-yl, propoxypropan-3-yl, propoxybutan-4-yl, propoxypentan-5-yl, butoxypropan-3-yl, pentoxypropan-3-yl, butoxybutan-4-yl, trifluoromethoxymethyl, trifluoromethoxyethan-2-yl, trifluoromethoxypropan-3-yl, trifluoromethoxybutan-4-yl, trifluoromethoxypentan-5-yl, trifluoromethoxyhexan-6-yl, trifluoromethoxydifluoromethyl, pentafluoroethoxydifluoromethyl. A preferred optionally fluorinated alkoxyalkyl group is methoxymethyl, methoxyethan-2-yl, trifluoromethoxymethyl and trifluoromethoxyethan-2-yl.

Preferred alkyl radicals have 1, 2, 3, 4, 5, 6, 7, 8, 9 or 10 C atoms.

An aryl group in the context of this invention contains 6 to 14 ring atoms. An aryl group is understood here to mean either a simple aromatic cycle, i.e. phenyl, or a fused (annelated) aryl, for example naphthyl, anthracenyl or phenanthrenyl.

An aryl group is preferably derived from benzene, naphthalene, anthracene, phenanthrene, biphenyl, biphenylene, fluorene.

A polymerizable group is a group which can be subject to or can undergo polymerization thus forming an oligomer or a polymer.

Polymerization is the process of taking individual monomers and chaining them together to make longer units. These longer units are called polymers. The compounds of formula (I) as described before and preferably described below are suitable monomers for the preparation of an ophthalmic device or a precursor article for an ophthalmic device.

Within the gist of the invention, the polymerizable group $R_1$ once oligomerized or polymerized thus forms or is part of the backbone of the oligomer, polymer or copolymer comprising polymerized compounds of formula (I). Suitable polymerizable groups are defined to be
a trialkoxysilyl group or a dialkoxyalkylsilyl group where the alkyl and/or alkoxy groups are each independently linear or branched having 1 to 6 C atoms, or a silyl group of formula (1), (2) or (3) or a polymerizable group of formula (4),

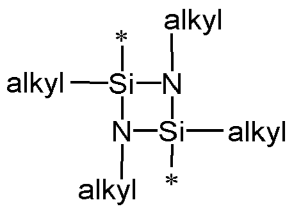 (1) , 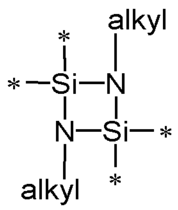 (2) ,

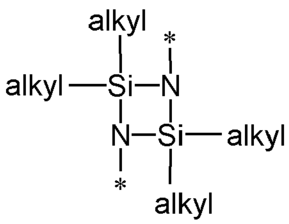 (3) , 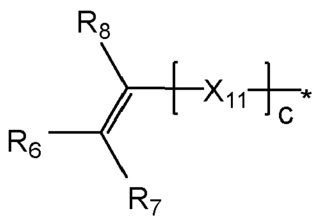 (4) ,

where alkyl means at each occurrence independently of each other a linear or branched alkyl group having 1 to 6 C atoms and the asterisk "*" denotes at each occurrence independently of each other a linkage to the linker $-R_2-Y-$; and wherein
$X_{11}$ is selected from the group consisting of O, S, $O-SO_2$, $SO_2-O$, C(=O), OC(=O), C(=O)O, S(C=O) and (C=O)S,
$R_6$, $R_7$, $R_8$ are at each occurrence independently of each other selected from the group consisting of H, F, a linear or branched, non-fluorinated, partially or completely fluorinated alkyl group having 1 to 20 C atoms and aryl with 6 to 14 C atoms and
c is 0 or 1.

Particularly preferred polymerizable groups are described below.

Aryl with 6 to 14 C atoms is an aryl group preferably selected from the group consisting of phenyl, naphthyl or anthryl, particularly preferably phenyl.

The linker Y-$R_2$-$R_1$ in compounds of formula (I) may be in any position of the photoactive ring system.

In one preferred embodiment, the compounds of formula (I) acting as monomers for the preparation of the ophthalmic device or precursor article of the ophthalmic device as described before or for the preparation of an oligomer, polymer or copolymer according to the invention or as compound according to the invention contain the linker Y-$R_2$-$R_1$ in 7-position of the photoactive ring system which can be described according to formula (Ia),

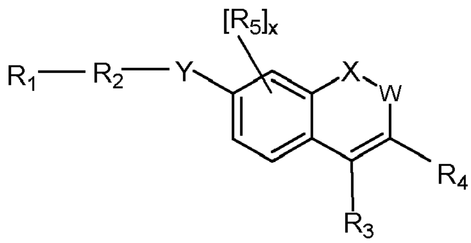

(Ia)

,

wherein X-W, Y, $R_1$, -$R_2$-, $R_3$, $R_4$, x and $R_5$ have a meaning as described before or preferably described below.

The invention is therefore additionally directed to an ophthalmic device or a precursor article for an ophthalmic device comprising at least one polymerized compound of formula (Ia),

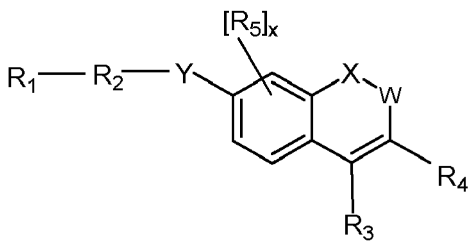

(Ia)

,

wherein X-W, Y, $R_1$, -$R_2$-, $R_3$, $R_4$, x and $R_5$ have a meaning as described before or preferably described before or below. This embodiment is particularly preferred.

In another preferred embodiment, the compounds of formula (I) acting as monomers for the preparation of the ophthalmic device or precursor article of the ophthalmic device as described before or for the preparation of an oligomer, polymer or copolymer according to the invention or as compound according to the invention contain the linker Y-$R_2$-$R_1$ in 5-position of the photoactive ring system which can be described according to formula (Ib),

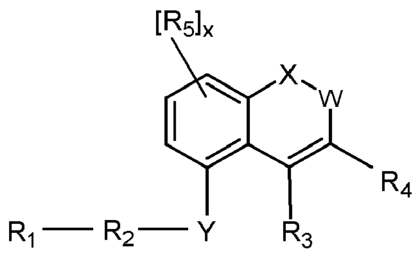

(Ib)

,

wherein X-W, Y, $R_1$, -$R_2$-, $R_3$, $R_4$, x and $R_5$ have a meaning as described before or preferably described below.

The invention is therefore additionally directed to an ophthalmic device or a precursor article for an ophthalmic device comprising at least one polymerized compound of formula (Ib),

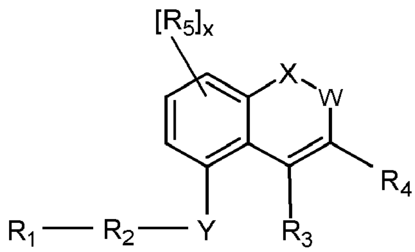

(Ib)

,

wherein X-W, Y, $R_1$, -$R_2$-, $R_3$, $R_4$, x and $R_5$ have a meaning as described before or preferably described before or below.

In another preferred embodiment, the compounds of formula (I) acting as monomers for the preparation of the ophthalmic device or precursor article of the ophthalmic device as described before or for the preparation of an oligomer, polymer or copolymer according to the invention or as compound according to the invention contain the linker Y-$R_2$-$R_1$ in 8-position of the photoactive ring system which can be described according to formula (Ic),

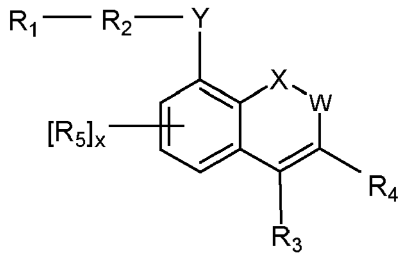

(Ic)

,

wherein X-W, Y, $R_1$, -$R_2$-, $R_3$, $R_4$, x and $R_5$ have a meaning as described before or preferably described below.

The invention is therefore additionally directed to an ophthalmic device or a precursor article for an ophthalmic device comprising at least one polymerized compound of formula (Ic),

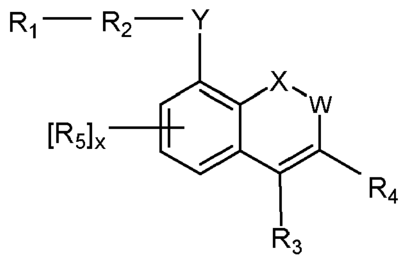

(Ic)

,

wherein X-W, Y, $R_1$, -$R_2$-, $R_3$, $R_4$, x and $R_5$ have a meaning as described before or preferably described before or below.

The invention is therefore additionally directed to compounds of formulae (Ia), (Ib) or (Ic),

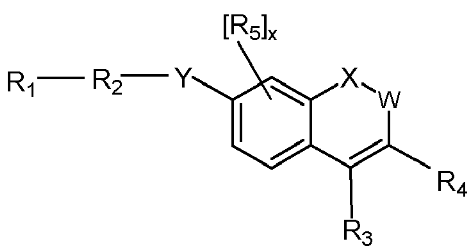

(Ia)

,

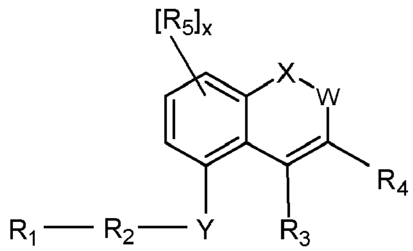

(Ib)

,

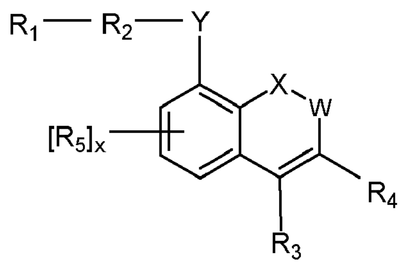

(Ic)

,

wherein X-W, Y, $R_1$, $-R_2-$, $R_3$, x and $R_5$ have a meaning as described before or preferably described before or below and $R_4$ is F, Cl, a linear or branched alkyl group having 1 to 8 C atoms or a partially or fully fluorinated or partially or fully chlorinated alkyl group having 1 to 8 C atoms, a linear or branched partially or fully fluorinated alkoxy group having 1 to 8 C atoms, a linear or branched optionally partially or fully fluorinated alkoxyalkyl group having 2 to 8 C atoms or a cycloalkyl group having 3 to 7 C atoms which may be partially or fully fluorinated and $-R_2-$ is $-(C(R)_2)_o-$, where R and o have a meaning as described before.

In compounds of formulae (I), (Ia), (Ib) or (Ic) acting as monomers for the preparation of the ophthalmic device or precursor article of the ophthalmic device as described before or for the preparation of an oligomer, polymer or copolymer according to the invention or as compound according to the invention, the symbol X-W is O-C(=O), O-C(=S), O-$SO_2$, $SO_2$-O, $SO_2$-C(=S), S-C(=O), S-C(=S), $NR_B$-C(=O) or $NR_B$-C(=S) where $R_B$ has a meaning as described before or preferably described below. Preferably, X-W is O-C(=O), O-$SO_2$, $SO_2$-O, $SO_2$-C(=S), S-C(=O) or $NR_B$-C(=O) where $R_B$ has a meaning as described before or preferably described below. Particularly preferably, X-W is O-C(=O), O-$SO_2$, $SO_2$-O, S-C(=O) or $NR_B$-C(=O) where $R_B$ has a meaning as described before or preferably described below. Very particularly preferably, X-W is O-C(=O), S-C(=O) or $NR_B$-C(=O) where $R_B$ has a meaning as described before or preferably described below.

Within $NR_B$-C(=O), $R_B$ is preferably a linear or branched alkyl group having 1 to 6 C atoms or a linear or branched partially or fully fluorinated alkyl group having 1 to 10 C atoms. Within $NR_B$-C(=O), $R_B$ is particularly preferably a linear or branched alkyl group having 1 to 6 C atoms, preferably methyl, ethyl, *n*-propyl or *n*-butyl.

The invention is therefore additionally directed to an ophthalmic device or a precursor article for an ophthalmic device comprising at least one polymerized compound of formulae (I), (Ia), (Ib) or (Ic) wherein X-W is O-C(=O), O-$SO_2$, $SO_2$-O, $SO_2$-C(=S), S-C(=O) or $NR_B$-C(=O) and where $R_B$ has a meaning as described before.

The invention is therefore additionally directed to compounds of formulae (I), (Ia), (Ib) or (Ic) wherein X-W is O-C(=O), O-$SO_2$, $SO_2$-O, $SO_2$-C(=S), S-C(=O) or $NR_B$-C(=O) and where $R_B$ has a meaning as described before and $R_4$ is F, Cl, a linear or branched alkyl group having 1 to 8 C atoms or a partially or fully fluorinated or partially or fully chlorinated alkyl group having 1 to 8 C atoms, a linear or branched partially or fully fluorinated alkoxy group having 1 to 8 C atoms or a fluorinated, a linear or branched optionally partially or fully fluorinated alkoxyalkyl group having 2 to 8 C atoms or a cycloalkyl group having 3 to 6 C atoms which may be partially or fully fluorinated and $-R_2-$ is $-(C(R)_2)_o-$, where R and o have a meaning as described before.

As described before within the ophthalmic device or precursor article, compounds of formulae (I), (Ia), (Ib), (Ic) and any oligomers, polymers or copolymers derived therefrom according to the invention, the substituent $R_3$ is a linear or branched alkyl group having 1 to 8 C atoms or a cycloalkyl group having 3 to 7 C atoms, preferably a linear or branched alkyl group having 1 to 8 C atoms, particularly preferably methyl, ethyl, *n*-propyl, *i*-propyl, *n*-butyl or 2-methyl-propan-3-yl. $R_3$ is very particularly preferably methyl.

As described before within the inventive compounds of formulae (I), (Ia), (Ib) or (Ic), and any oligomers, polymers or copolymers derived therefrom according to the invention, the substituent $R_3$ is a linear or branched alkyl group having 1 to 8 C atoms or a cycloalkyl group having 3 to 7 C atoms, preferably a linear or branched alkyl group having 1 to 8 C atoms, particularly preferably methyl, ethyl, *n*-propyl, *i*-propyl, *n*-butyl or 2-methyl-propan-3-yl, 2-methyl-butan-4-yl, *n*-pentyl, cyclopropyl, cyclobutyl, cyclopentyl, cyclohexyl, cycloheptyl, very particularly preferably methyl.

As described before within the ophthalmic device, precursor article comprising polymerized compounds of formulae (I), (Ia), (Ib), (Ic), or comprising preferred compounds of formulae (I), (Ia), (Ib), (Ic) and any oligomers, polymers or copolymers derived therefrom according to the invention, the substituent $R_4$ is F, Cl, CN, a linear or branched alkyl group having 1 to 8 C atoms or a partially or fully fluorinated or partially or fully chlorinated alkyl group having 1 to 8 C atoms, a linear or branched partially or fully fluorinated alkoxy group having 1 to 8 C atoms, a linear or branched optionally partially or fully fluorinated alkoxyalkyl group having 2 to 8 C atoms or a cycloalkyl group having 3 to 7 C atoms which may be partially or fully fluorinated, preferably F, Cl, CN, a partially or fully fluorinated alkyl group or alkoxy group having 1 to 8 C atoms, a linear or branched partially fluorinated alkoxyalkyl group having 2 to 8 C atoms or a cycloalkyl group having 3 to 7 C atoms which may be partially or fully fluorinated, particularly preferably F, Cl, CN, methyl, trifluoromethyl, pentafluoroethyl, trifluoromethoxy, trifluoromethoxymethyl, very particularly preferably F.

Preferred fluorinated cycloalkyl groups are

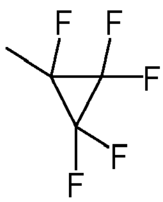 , 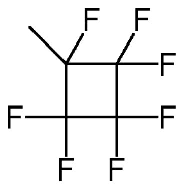 , 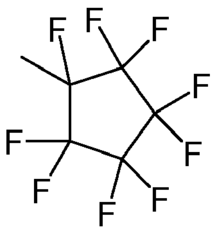 or 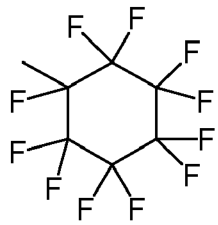 .

As described before within the inventive compounds of formulae (I), (Ia), (Ib) or (Ic), and any oligomers, polymers or copolymers derived therefrom according to the invention, the substituent $R_4$ is F, Cl, a linear or branched alkyl group having 1 to 8 C atoms or a partially or fully fluorinated or partially or fully chlorinated alkyl group having 1 to 8 C atoms, a linear or branched partially or fully fluorinated alkoxy group having 1 to 8 C atoms or a fluorinated or chlorinated cycloalkyl group having 3 to 6 C atoms, preferably F, fully fluorinated alkyl group or alkoxy group having 1 to 8 C atoms, or a cycloalkyl group having 3 to 7 C atoms which may be partially or fully fluorinated, particularly preferably F, trifluoromethyl, trifluoromethoxy, very particularly preferably F or trifluoromethyl.

As described before within the ophthalmic device, precursor article, compounds of formulae (I), (Ia), (Ib) (Ic), preferred compounds of formulae (I), (Ia), (Ib) (Ic) and any oligomers, polymers or copolymers derived therefrom according to the invention, the substituent $R_5$, if present, is a linear or branched alkyl group having 1 to 8 C atoms or a linear or branched alkoxy group having 1 to 8 C atoms, preferably a linear or branched alkyl group having 1 to 4 C atoms or a linear or branched alkoxy group having 1 to 4 C atoms, particularly preferably methyl, methoxy, ethyl or ethoxy.

As described before within the ophthalmic device, precursor article, compounds of formulae (I), (Ia), (Ib), (Ic) preferred compounds of formulae (I), (Ia), (Ib), (Ic) and any oligomers, polymers or copolymers derived therefrom according to the invention, the index x is 0 or 1, preferably 0.

As described before within the inventive compounds of formulae (I), (Ia), (Ib) or (Ic), and any oligomers, polymers or copolymers derived therefrom according to the invention, the substituent $R_5$, if present, is a linear or branched alkyl group having 1 to 8 C atoms or a linear or branched alkoxy group having 1 to 8 C atoms, preferably a linear or branched alkyl group having 1 to 4 C atoms or a linear or branched alkoxy group having 1 to 4 C atoms, particularly preferably methyl, methoxy, ethyl or ethoxy.

As described before within the ophthalmic device, precursor article, compounds of formulae (I), (Ia), (Ib) or (Ic), preferred compounds of formulae (I), (Ia), (Ib) or (Ic) and any oligomers, polymers or copolymers derived therefrom according to the invention, Y is O, S, SO, $SO_2$, Se, $NR_0$ or a bond and $R_0$ is a linear or branched alkyl group having 1 to 4 C atoms.

As described before within the ophthalmic device, precursor article, compounds of formulae (I), (Ia), (Ib) or (Ic), preferred compounds of formulae (I), (Ia), (Ib) or (Ic) and any oligomers, polymers or copolymers derived therefrom according to the invention, Y is preferably O, S, SO, $NR_0$ or a bond and $R_0$ is a linear or branched alkyl group having 1 to 4 C atoms.

As described before within the ophthalmic device, precursor article, compounds of formulae (I), (Ia), (Ib) or (Ic), preferred compounds of formulae (I), (Ia), (Ib) or (Ic) and any oligomers, polymers or copolymers derived therefrom according to the invention, Y is preferably O, S or $NR_0$ and $R_0$ is a linear or branched alkyl group having 1 to 4 C atoms.

$R_0$ is preferably methyl or ethyl.

As described before within the ophthalmic device, precursor article, compounds of formulae (I), (Ia), (Ib) or (Ic), preferred compounds of formulae (I), (Ia), (Ib) or (Ic) and any oligomers, polymers or copolymers derived therefrom according to the invention, Y is preferably O or $NR_0$ and $R_0$ is a linear or branched alkyl group having 1 to 4 C atoms.

$R_0$ is preferably methyl or ethyl.

According to the invention, compounds of formulae (I), (Ia), (Ib) or (Ic) with substituents as described before or preferably described before have a polymerizable group as described before or preferably described before or below and have one linking element Y-$R_2$ where Y has a meaning as described before.

According to the invention, the linking element $-R_2-$ is $-(C(R)_2)_o-$, or $-(C(R)_2)_p-X_8-(C(R)_2)_q-(X_9)_s-(C(R)_2)_r-(X_{10})_t-(C(R)_2)_u-$, R is at each occurrence independently selected from the group consisting of H, F, a linear or branched alkyl group having 1 to 4 C atoms or a linear or branched partially or fully fluorinated alkyl group having 1 to 4 C atoms and o is 5 to 20, $X_8$, $X_9$ and $X_{10}$ are at each occurrence O, S, $SO_2$, or $NR_B$, s and t are at each occurrence independently 0 or 1, p and q are at each occurrence independently selected from the group consisting of 1 to 10, r and u are at each occurrence independently selected from the group consisting of 0 to 10, wherein the overall number of atoms for$-(C(R)_2)_p-X_8-(C(R)_2)_q-(X_9)_s-(C(R)_2)_r-(X_{10})_t-(C(R)_2)_u-$, is up to 20 C atoms. $R_B$ in $NR_B$ within $-R_2-$ is at each occurrence independently selected from the group consisting of a linear or branched alkyl group having 1 to 4 C atoms and a linear or branched partially or fully fluorinated alkyl group having 1 to 4 C atoms. $R_B$

within $-R_2-$ is independently at each occurrence and preferably methyl, ethyl or trifluoromethyl. $R_B$ within $-R_2-$ is independently at each occurrence particularly preferably methyl.
R within $-R_2-$ is at each occurrence independently preferably H, a linear or branched alkyl group having 1 to 4 C atoms and a linear or branched partially or fully fluorinated alkyl group having 1 to 4 C atoms.
R within $-R_2-$ is at each occurrence independently particularly preferably H, ethyl, *n*-propyl or trifluoromethyl.
R within $-R_2-$ is at each occurrence independently particularly preferably H.

In another preferred embodiment of the invention, o is preferably selected from the group consisting of 5, 6, 7, 8, 9, 10, 11, 12 and 13 within the compounds of formulae (I), (Ia), (Ib) and (Ic) acting as monomers for the preparation of the ophthalmic device or precursor article of the ophthalmic device as described before or for the preparation of an oligomer, polymer or copolymer according to the invention or within the compounds according to the invention. Preferably, o is selected from the group consisting of 6, 7, 8, 9, 10, 11 and 12. Particularly preferably, o is selected from the group consisting of 7, 8, 9 and 12, where 6, 9 or 12 are especially preferred. With regards to the compounds according to the invention, the same definition of $-R_2-$ and preferably o apply.

In another preferred embodiment of the invention, s, t, $X_8$, $X_9$, $X_{10}$, p, q, r and u within the compounds of formulae (I), (Ia), (Ib) and (Ic) acting as monomers for the preparation of the ophthalmic device or precursor article of the ophthalmic device as described before have the following preferred meaning:

Preferably, s is 1. Preferably, s is 0.
Preferably t is 0 or 1.
Preferably, s and t are 0.

Preferably, $X_8$, Xg and $X_{10}$ are O, S or $SO_2$. Particularly preferably, $X_8$, Xg and $X_{10}$ are O. Particularly preferably, $X_8$, Xg and $X_{10}$ are S. Particularly preferably, $X_8$, $X_9$ and $X_{10}$ are $SO_2$. Particularly preferably, $X_8$, and $X_{10}$ are O and $X_9$ is S. Particularly preferably, $X_8$, and $X_{10}$ are S and Xg is O.

Preferably, p and q are each independently 1, 3, 3, 4, 5 or 6, particularly preferably 2 or 3, very particularly preferably 2.
Preferably, r and u are each independently 0, 1, 2 or 3, particularly preferably 0 or 2, very particularly preferably 0.

According to the invention, suitable examples for $-R_2-$ are $-(CH_2)_5-$, $-(CH_2)_6-$,$-(CH_2)_{7-}$, $-(CH_2)_8-$, $-(CH_2)_9-$, $-(CH_2)_{10}-$, $-(CH_2)_{11}-$, $-(CH_2)_{12}-$, $-(CH_2)_{13}-$, $-(CH_2)_{14}-$,$-(CH_2)_{15}-$, $-(CH_2)_{16}-$, $-(CH_2)_{17}-$, $-(CH_2)_{18}-$, $-(CH_2)_{19}-$, $-(CH_2)_{20}-$, $-(CHCH_3)_5-$, $-(CHCH_3)_6-$, $-(CHCH_3)_{7-}$, $-(CHCH_3)_8-$, $-(CHCH_3)_9-$, $-(CHCH_3)_{10}-$, $-(CHCH_3)_{11}-$, $-(CHCH_3)_{12}-$,$-(CHCH_3)_{13}-$, $-(CHCH_3)_{14}-$, $-(CHCH_3)_{15}-$, $-(CHCH_3)_{16}-$, $-(CHCH_3)_{17}-$, $-(CHCH_3)_{18}-$,$-(CHCH_3)_{19}-$, $-(CHCH_3)_{20}-$, $-(C(CH_3)_2)_5-$, $-(C(CH_3)_2)_6-$, $-(C(CH_3)_2)_{7-}$, $-(C(CH_3)_2)_8-$,$-(C(CH_3)_2)g-$, $-(C(CH_3)_2)_{10}-$, $-(C(CH_3)_2)_{11}-$, $-(C(CH_3)_2)_{12}-$, $-(C(CH_3)_2)_{13}-$, $-(C(CH_3)_2)_{14}-$,$-(C(CH_3)_2)_{15}-$, $-(C(CH_3)_2)_{16}-$, $-(C(CH_3)_2)_{17}-$, $-(C(CH_3)_2)_{18}-$, $-(C(CH_3)_2)_{19}-$, $-(C(CH_3)_2)_2o-$, $-(CHC_2H_5)_5-$, $-(CHC_2H_5)_6-$, $-(CHC_2H_5)_7-$, $-(CHC_2H_5)_8-$, $-(CHC_2H_5)_9-$, $-(CHC_2H_5)_{10}-$,$-(CHC_2H_5)_{11}-$, $-(CHC_2H_5)_{12}-$, $-(CHC_2H_5)_{13}-$, $-(CHC_2H_5)_{14}-$, $-(CHC_2H_5)_{15}-$, $-(CHC_2H_5)_{16}-$, $-(CHC_2H_5)_{17}-$, $-(CHC_2H_5)_{18}-$, $-(CHC_2H_5)_{19}-$, $-(CHC_2H_5)_{20}-$, $-(CH_2)-(CHCH_3)-(CH_2)_3-$, $-(CH_2)-(CHCH_3)-(CH_2)_4-$, $-(CH_2)_2-(CHCH_3)-(CH_2)_2-$, $-(CH_2)_3-(CHCH_3)-(CH_2)-$,$-(CH_2)_{11}-(CHCH_3)-(CH_2)-$, $-(CH(C_3H_7)-(CH_2)_4-$, $-(CH(C_3H_7)-(CH_2)_5-$, $-(CH(C_3H_7)-(CH_2)_6-$, $-(CH(C_3H_7)-(CH_2)_{7-}$, $-(CH(C_2H_5)-(CH_2)_3-$, $-(CH(C_2H_5)-(CH_2)_4-$, $-(CH(C_2H_5)-(CH_2)_5-$, $-(CH(C_2H_5)-(CH_2)_6-$, $-(CH(C_2H_5)-(CH_2)_7-$, $-(CH_2)_2-O-(CH_2)_2-$, $-(CH_2)_3-O-(CH_2)_3-$, $-(CH_2)_2-O-(CH_2)_2-O-(CH_2)_2-$, $-(CH_2)_3-O-(CH_2)_3-O-(CH_2)_3-$, $-(CH_2)_2-O-(CH_2)_2-O-(CH_2)_6-$, $-(CH_Z)_s-O-(CH_Z)_2-O-(CH_Z)_2-$, $-(CH_Z)_2-O-(CH_Z)_2-O-(CH_Z)_s-$, $-(CH_2)_8-O-(CH_2)_2-O-(CH_2)_2-$, $-(CH_2)_2-S-(CH_2)_2-$, $-(CH_2)_3-S-(CH_2)_3-$, $-(CH_2)_2-S-(CH_2)_2-S-(CH_2)_2-$, $-(CH_2)_3-S-(CH_2)_3-S-(CH_2)_3-$, $-(CH_2)_2-S-(CH_2)_2-S-(CH_2)_6-$, $-(CH_2)_6-S-(CH_2)_2-S-(CH_2)_2-$, $-(CH_2)_2-S-(CH_2)_2-S-(CH_2)_8-$, $-(CH_2)_8-S-(CH_2)_2-S-(CH_2)_2-$, $-(CH_2)_2-SO_2-(CH_2)_2-$, $-(CH_2)_4-SO_2-(CH_2)_4-$, $-(CH_2)_3-SO_2-(CH_2)_3-$, $-(CH_2)_2-SO_2-(CH_2)_2-SO_2-(CH_2)_2-$, $-(CH_2)_3-S0_2-(CH_2)_3-S0_2-(CH_2)_3-$, $-(CH_2)_2-SO_2-(CH_2)_2-SO_2-(CH_2)_6-$, $-(CH_2)_6-SO_2-(CH_2)_2-SO_2-(CH_2)_2-$,$-(CH_2)_2-SO_2-(CH_2)_2-SO_2-(CH_2)_8-$,$-(CH_2)_8-SO_2-(CH_2)_2-SO_2-(CH_2)_2-$, $-(CH_2)-S-(CH_2)_2-O-(CH_2)-$, $-(CH_2)_2-S-(CH_2)_2-O-(CH_2)_2-$, $-(CH_2)_2-O-(CH_2)_2-S-(CH_2)_2-$, $-(CH_Z)-O-(CH_Z)_2-SO_Z-(CH_Z)_2-$, $-(CH_2)-O-(CH_2)_2-SO_2-(CH_2)_3-$, $-(CH_2)_2-O-(CH_2)_2-SO_2-(CH_2)_2-$, $-(CH_2)-SO_2-(CH_2)_2-O-(CH_2)-$, $-(CH_2)-SO_2-(CH_2)_2-S-(CH_2)-$,$-(CH_2)-O-(CH_2)_2-S-(CH_2)_2-O-(CH_2)-$, $-(CH_2)-S-(CH_2)_2-O-(CH_2)_2-S-(CH_2)-$, $-(CH_2)-SO_2-(CH_2)_2-O-(CH_2)_2-SO_2-(CH_2)-$, $-(CH_2)-S-(CH_2)_2-S-(CH_2)_2-S-(CH_2)-$, $-(CH_2)-SO_2-(CH_2)_2-SO_2-(CH_2)_2-SO_2-(CH_2)-$, $-(CH_2)-O-(CH_2)_2-SO_2-(CH_2)_2-O-(CH_2)-$, $-(CH_2)_2-S-(CH_2)_2-0-(CH_2)_2-S-(CH_2)_2-$, $-(CH_2)_2-0-(CH_2)_2-S-(CH_2)_2-0-(CH_2)_2-$, $-(CH_2)_2-0-(CH_2)_2-O-(CH_2)_2-O-(CH_2)_2-$, $-(CH_2)_2-(NCH_3)-(CH_2)_2-$, $-(CH_2)_3-(NCH_3)-(CH_2)_3-$, $-(CH_2)_{2-}(NCH_3)-(CH_2)_2-(NCH_3)-(CH_2)_2-$, $-(CH_2)_3-(NCH_3)-(CH_2)_3-(NCH_3)-(CH_2)_3-$, $-(CH_2)_2-(NCH_3)-(CH_2)_2-(NCH_3)-(CH_2)_6-$, $-(CH_2)_6-(NCH_3)-(CH_2)_2-(NCH_3)-(CH_2)_2-$, $-(CH_2)_2-(NCH_3)-(CH_2)_2-(NCH_3)-(CH_2)_8-$ and$-(CH_2)_8-(NCH_3)-(CH_2)_2-(NCH_3)-(CH_2)_2-$; $-(CH_2)-(CF_2)-(CH_2)_3-$, $-(CH_2)-(CF_2)-(CH_2)_4-$, $-(CH_2)-(CF_2)-(CH_2)_5-$, $-(CH_2)-(CF_2)-(CH_2)_6-$, $-(CH_2)-(CF_2)-(CH_2)_{7-}$, $-(CH_2)-(CF_2)-(CH_2)_8-$, $-(CH_2)-(CF_2)-(CH_2)_9-$, $-(CH_2)-(CF_2)-(CH_2)_{10}-$, $-(CH_2)_3-(CF_2)-(CH_2)-$, $-(CH_2)_4-(CF_2)-(CH_2)-$, $-(CH_2)_5-(CF_2)-(CH_2)-$,$-(CH_2)_6-(CF_2)-(CH_2)-$, $-(CH_2)_7-(CF_2)-(CH_2)-$, $-(CH_2)_8-(CF_2)-(CH_2)-$, $-(CH_2)_9-(CF_2)-(CH_2)-$, $-(CH_2)_w-(CF_2)-(CH_2)-$, $-(CH_2)_2-(CF_2)-(CH_2)_2-$, $-(CH_2)_3-(CF_2)-(CH_2)_3-$,

$-(CH_2)_4-(CF_2)-(CH_2)_4-$, $-(CH_2)_5-(CF_2)-(CH_2)_5-$, $-(CH_2)_2-(CF_2)-(CH_2)_3-$, $-(CH_2)_2-(CF_2)-(CH_2)_4-$,$-(CH_2)_2-(CF_2)-(CH_2)_5-$, $-(CH_2)_2-(CF_2)-(CH_2)_6-$, $-(CH_2)_2-(CF_2)-(CH_2)_7-$, $-(CH_2)_2-(CF_2)-(CH_2)_8-$, $-(CH_2)_2-(CF_2)-(CH_2)_9-$, $-(CH_2)_3-(CF_2)-(CH_2)-$, $-(CH_2)_3-(CF_2)-(CH_2)_2-$, $-(CH_2)_3-(CF_2)-(CH_2)_4-$, $-(CH_2)_3-(CF_2)-(CH_2)_5-$, $-(CH_2)_3-(CF_2)-(CH_2)_6-$, $-(CH_2)_3-(CF_2)-(CH_2)_7-$ ,$-(CH_2)_3-(CF_2)-(CH_2)_8-$, $-(CH_2)_4-(CF_2)-(CH_2)-$, $-(CH_2)_4-(CF_2)-(CH_2)_2-$, $-(CH_2)_4-(CF_2)-(CH_2)_3-$, $-(CH_2)_4-(CF_2)-(CH_2)_5-$, $-(CH_2)_4-(CF_2)-(CH_2)_6-$, $-(CH_2)_4-(CF_2)-(CH_2)_7-$,$-(CH_2)_5-(CF_2)-(CH_2)-$, $-(CH_2)_5-(CF_2)-(CH_2)_2-$, $-(CH_2)_5-(CF_2)-(CH_2)_3-$, $-(CH_2)_5-(CF_2)-(CH_2)_4-$, $-(CH_2)_5-(CF_2)-(CH_2)_6-$, $-(CH_2)_6-(CF_2)-(CH_2)-$, $-(CH_2)_6-(CF_2)-(CH_2)_2-$, $-(CH_2)_6-(CF_2)-(CH_2)_3-$, $-(CH_2)_6-(CF_2)-(CH_2)_4-$, $-(CH_2)_6-(CF_2)-(CH_2)_5-$,

$-(CH_2)-(CFH)-(CH_2)_3-$, $-(CH_2)-(CFH)-(CH_2)_4-$, $-(CH_2)-(CFH)-(CH_2)_5-$, $-(CH_2)-(CFH)-(CH_2)_6-$, $-(CH_2)-(CFH)-(CH_2)_7-$, $-(CH_2)-(CFH)-(CH_2)_8-$, $-(CH_2)-(CFH)-(CH_2)_9-$, $-(CH_2)-(CFH)-(CH_2)_{10}-$, $-(CH_2)_3-(CFH)-(CH_2)-$, $-(CH_2)_4-(CFH)-(CH_2)-$, $-(CH_2)_5-(CFH)-(CH_2)-$, $-(CH_2)_6-(CFH)-(CH_2)-$, $-(CH_2)_7-(CFH)-(CH_2)-$, $-(CH_2)_8-(CFH)-(CH_2)-$, $-(CH_2)_9-(CFH)-(CH_2)-$, $-(CH_2)_{10}-(CFH)-(CH_2)-$, $-(CH_2)_2-(CFH)-(CH_2)_2-$, $-(CH_2)_3-(CFH)-(CH_2)_3-$,$-(CH_2)_4-(CFH)-(CH_2)_4-$, $-(CH_2)_5-(CFH)-(CH_2)_5-$, $-(CH_2)_2-(CFH)-(CH_2)_3-$, $-(CH_2)_2-(CFH)-(CH_2)_4-$, $-(CH_2)_2-(CFH)-(CH_2)_5-$, $-(CH_2)_2-(CFH)-(CH_2)_6-$, $-(CH_2)_2-(CFH)-(CH_2)_7-$ , $-(CH_2)_2-(CFH)-(CH_2)_8-$, $-(CH_2)_2-(CFH)-(CH_2)_9-$, $-(CH_2)_3-(CFH)-(CH_2)-$,$-(CH_2)_3-(CFH)-(CH_2)_2-$, $-(CH_2)_3-(CFH)-(CH_2)_4-$, $-(CH_2)_3-(CFH)-(CH_2)_5-$, $-(CH_2)_3-(CFH)-(CH_2)_6-$, $-(CH_2)_3-(CFH)-(CH_2)_7-$, $-(CH_2)_3-(CFH)-(CH_2)_8-$, $-(CH_2)_4-(CFH)-(CH_2)-$, $-(CH_2)_4-(CFH)-(CH_2)_2-$, $-(CH_2)_4-(CFH)-(CH_2)_3-$, $-(CH_2)_4-(CFH)-(CH_2)_5-$, $-(CH_2)_4-(CFH)-(CH_2)_6-$, $-(CH_2)_4-(CFH)-(CH_2)_7-$ , $-(CH_2)_5-(CFH)-(CH_2)-$, $-(CH_2)_5-(CFH)-(CH_2)_2-$, $-(CH_2)_5-(CFH)-(CH_2)_3-$, $-(CH_2)_5-(CFH)-(CH_2)_4-$, $-(CH_2)_5-(CFH)-(CH_2)_6-$, $-(CH_2)_6-(CFH)-(CH_2)-$, $-(CH_2)_6-(CFH)-(CH_2)_2-$, $-(CH_2)_6-(CFH)-(CH_2)_3-$, $-(CH_2)_6-(CFH)-(CH_2)_4-$, $-(CH_2)_6-(CFH)-(CH_2)_5-$,

$-(CH_2)-(CF_2)_2-(CH_2)_2-$, $-(CH_2)-(CF_2)_2-(CH_2)_3-$, $-(CH_2)-(CF_2)_2-(CH_2)_4-$, $-(CH_2)-(CF_2)_2-(CH_2)_5-$, $-(CH_2)-(CF_2)_2-(CH_2)_6-$, $-(CH_2)-(CF_2)_2-(CH_2)_7-$, $-(CH_2)-(CF_2)_2-(CH_2)_8-$, $-(CH_2)-(CF_2)_2-(CH_2)_9-$, $-(CH_2)_2-(CF_2)_2-(CH_2)-$, $-(CH_2)_3-(CF_2)_2-(CH_2)-$, $-(CH_2)_4-(CF_2)_2-(CH_2)-$, $-(CH_2)_5-(CF_2)_2-(CH_2)-$, $-(CH_2)_6-(CF_2)_2-(CH_2)-$, $-(CH_2)_7-(CF_2)_2-(CH_2)-$, $-(CH_2)_8-(CF_2)_2-(CH_2)-$, $-(CH_2)_9-(CF_2)_2-(CH_2)-$, $-(CH_2)_2-(CF_2)_2-(CH_2)_2-$, $-(CH_2)_3-(CF_2)_2-(CH_2)_3-$,$-(CH_2)_4-(CF_2)_2-(CH_2)_4-$, $-(CH_2)_5-(CF_2)_2-(CH_2)_5-$, $-(CH_2)_2-(CF_2)_2-(CH_2)-$,$-(CH_2)_2-(CF_2)_2-(CH_2)_3-$, $-(CH_2)_2-(CF_2)_2-(CH_2)_4-$, $-(CH_2)_2-(CF_2)_2-(CH_2)_5-$, $-(CH_2)_2-(CF_2)_2-(CH_2)_6-$,$-(CH_2)_2-(CF_2)_2-(CH_2)_7-$, $-(CH_2)_2-(CF_2)_2-(CH_2)_8-$, $-(CH_2)_3-(CF_2)_2-(CH_2)-$, $-(CH_2)_3-(CF_2)_2-(CH_2)_2-$, $-(CH_2)_3-(CF_2)_2-(CH_2)_4-$, $-(CH_2)_3-(CF_2)_2-(CH_2)_5-$, $-(CH_2)_3-(CF_2)_2-(CH_2)_6-$,$-(CH_2)_3-(CF_2)_2-(CH_2)_7-$, $-(CH_2)_4-(CF_2)_2-(CH_2)-$, $-(CH_2)_4-(CF_2)_2-(CH_2)_2-$, $-(CH_2)_4-(CF_2)_2-(CH_2)_3-$, $-(CH_2)_4-(CF_2)_2-(CH_2)_5-$, $-(CH_2)_4-(CF_2)_2-(CH_2)_6-$, $-(CH_2)_5-(CF_2)_2-(CH_2)-(CH_2)_5-(CF_2)_2-(CH_2)_2-$, $-(CH_2)_5-(CF_2)_2-(CH_2)_3-$, $-(CH_2)_5-(CF_2)_2-(CH_2)_4-$, $-(CH_2)_6-(CF_2)_2-(CH_2)-$, $-(CH_2)_6-(CF_2)_2-(CH_2)_2-$, $-(CH_2)_6-(CF_2)_2-(CH_2)_3-$, $-(CH_2)_6-(CF_2)_2-(CH_2)_4-$,

$-(CH_2)-(CFH)_2-(CH_2)_2-$, $-(CH_2)-(CFH)_2-(CH_2)_3-$, $-(CH_2)-(CFH)_2-(CH_2)_4-$, $-(CH_2)-(CFH)_2-(CH_2)_5-$, $-(CH_2)-(CFH)_2-(CH_2)_6-$, $-(CH_2)-(CFH)_2-(CH_2)_7-$, $-(CH_2)-(CFH)_2-(CH_2)_8-$, $-(CH_2)-(CFH)_2-(CH_2)_9-$, $-(CH_2)_2-(CFH)_2-(CH_2)-$, $-(CH_2)_3-(CFH)_2-(CH_2)-$,$-(CH_2)_4-(CFH)_2-(CH_2)-$, $-(CH_2)_5-(CFH)_2-(CH_2)-$, $-(CH_2)_6-(CFH)_2-(CH_2)-$, $-(CH_2)_7-(CFH)_2-(CH_2)-$, $-(CH_2)_8-(CFH)_2-(CH_2)-$, $-(CH_2)_9-(CFH)_2-(CH_2)-$, $-(CH_2)_2-(CFH)_2-(CH_2)_2-$, $-(CH_2)_3-(CFH)_2-(CH_2)_3-$, $-(CH_2)_4-(CFH)_2-(CH_2)_4-$, $-(CH_2)_5-(CFH)_2-(CH_2)_5-$,$-(CH_2)_2-(CFH)_2-(CH_2)-$,$-(CH_2)_2-(CFH)_2-(CH_2)_3-$, $-(CH_2)_2-(CFH)_2-(CH_2)_4-$, $-(CH_2)_2-(CFH)_2-(CH_2)_5-$, $-(CH_2)_2-(CFH)_2-(CH_2)_6-$, $-(CH_2)_2-(CFH)_2-(CH_2)_7-$, $-(CH_2)_2-(CFH)_2-(CH_2)_8-$, $-(CH_2)_3-(CFH)_2-(CH_2)-$, $-(CH_2)_3-(CFH)_2-(CH_2)_2-$, $-(CH_2)_3-(CFH)_2-(CH_2)_4-$,$-(CH_2)_3-(CFH)_2-(CH_2)_5-$, $-(CH_2)_3-(CFH)_2-(CH_2)_6-$, $-(CH_2)_3-(CFH)_2-(CH_2)_7-$, $-(CH_2)_4-(CFH)_2-(CH_2)-$, $-(CH_2)_4-(CFH)_2-(CH_2)_2-$, $-(CH_2)_4-(CFH)_2-(CH_2)_3-$, $-(CH_2)_4-(CFH)_2-(CH_2)_5-$, $-(CH_2)_4-(CFH)_2-(CH_2)_6-$, $-(CH_2)_5-(CFH)_2-(CH_2)-$, $-(CH_2)_5-(CFH)_2-(CH_2)_2-$,$-(CH_2)_5-(CFH)_2-(CH_2)_3-$, $-(CH_2)_5-(CFH)_2-(CH_2)_4-$, $-(CH_2)_6-(CFH)_2-(CH_2)-$, $-(CH_2)_6-(CFH)_2-(CH_2)_2-$, $-(CH_2)_6-(CFH)_2-(CH_2)_3-$, $-(CH_2)_6-(CFH)_2-(CH_2)_4-$, $-(CH_2)-(CF_2)_3-(CH_2)-$, $-(CH_2)-(CF_2)_3-(CH_2)_2-$, $-(CH_2)-(CF_2)_3-(CH_2)_3-$, $-(CH_2)-(CF_2)_3-(CH_2)_4-$, $-(CH_2)-(CF_2)_3-(CH_2)_5-$, $-(CH_2)-(CF_2)_3-(CH_2)_6-$, $-(CH_2)-(CF_2)_3-(CH_2)_7-$, $-(CH_2)-(CF_2)_3-(CH_2)_8-$, $-(CH_2)_2-(CF_2)_3-(CH_2)-$, $-(CH_2)_3-(CF_2)_3-(CH_2)-$, $-(CH_2)_4-(CF_2)_3-(CH_2)-$,$-(CH_2)_5-(CF_2)_3-(CH_2)-$, $-(CH_2)_6-(CF_2)_3-(CH_2)-$, $-(CH_2)_7-(CF_2)_3-(CH_2)-$, $-(CH_2)_8-(CF_2)_3-(CH_2)-$, $-(CH_2)_2-(CF_2)_3-(CH_2)_2-$, $-(CH_2)_3-(CF_2)_3-(CH_2)_3-$, $-(CH_2)_4-(CF_2)_3-(CH_2)_4-$,$-(CH_2)_2-(CF_2)_3-(CH_2)-$,$-(CH_2)_2-(CF_2)_3-(CH_2)_3-$, $-(CH_2)_2-(CF_2)_3-(CH_2)_4-$, $-(CH_2)_2-(CF_2)_3-(CH_2)_5-$, $-(CH_2)_2-(CF_2)_3-(CH_2)_6-$,$-(CH_2)_2-(CF_2)_3-(CH_2)_7-$,$-(CH_2)_3-(CF_2)_3-(CH_2)-$,$-(CH_2)_3-(CF_2)_3-(CH_2)_2-$,$-(CH_2)_3-(CF_2)_3-(CH_2)_4-$, $-(CH_2)_3-(CF_2)_3-(CH_2)_5-$, $-(CH_2)_3-(CF_2)_3-(CH_2)_6-$, $-(CH_2)_4-(CF_2)_3-(CH_2)-$, $-(CH_2)_4-(CF_2)_3-(CH_2)_2-$, $-(CH_2)_4-(CF_2)_3-(CH_2)_3-$,$-(CH_2)_4-(CF_2)_3-(CH_2)_5-$, $-(CH_2)_5-(CF_2)_3-(CH_2)-$, $-(CH_2)_5-(CF_2)_3-(CH_2)_2-$, $-(CH_2)_5-(CF_2)_3-(CH_2)_3-$, $-(CH_2)_5-(CF_2)_3-(CH_2)_4-$, $(CH_2)_6-(CF_2)_3-(CH_2)-$, $-(CH_2)_6-(CF_2)_3-(CH_2)_2-$, $-(CH_2)_6-(CF_2)_3-(CH_2)_3-$, $-(CH_2)-(CF_2)_4-$, $-(CH_2)-(CF_2)_4-(CH_2)-$, $-(CH_2)-(CF_2)_4-(CH_2)_2-$, $-(CH_2)-(CF_2)_4-(CH_2)_3-$,$-(CH_2)-(CF_2)_4-(CH_2)_4-$, $-(CH_2)-(CF_2)_4-(CH_2)_5-$, $-(CH_2)-(CF_2)_4-(CH_2)_6-$, $-(CH_2)-(CF_2)_4-(CH_2)_7-$, $-(CH_2)-(CF_2)_4-(CH_2)_8-$, $-(CH_2)-(CF_2)_4-(CH_2)_9-$, $-(CH_2)-(CF_2)_4-(CH_2)_{10}-$,$-(CH_2)_2-(CF_2)_4-(CH_2)-$, $-(CH_2)_3-(CF_2)_4-(CH_2)-$, $-(CH_2)_4-(CF_2)_4-(CH_2)-$, $-(CH_2)_5-(CF_2)_4-(CH_2)-$, $-(CH_2)_6-(CF_2)_4-(CH_2)-$, $-(CH_2)_7-(CF_2)_4-(CH_2)-$, $-(CH_2)_2-(CF_2)_4-(CH_2)_2-$,$-(CH_2)_3-(CF_2)_4-(CH_2)_3-$, $-(CH_2)_4-(CF_2)_4-(CH_2)_4-$, $-(CH_2)_5-(CF_2)_4-(CH_2)_5-$, $-(CH_2)_2-(CF_2)_4-(CH_2)_3-$, $-(CH_2)_2-(CF_2)_4-(CH_2)_4-$, $-(CH_2)_2-(CF_2)_4-(CH_2)_5-$, $-(CH_2)_2-(CF_2)_4-(CH_2)_6-$, $-(CH_2)_3-(CF_2)_4-(CH_2)_2-$, $-(CH_2)_3-(CF_2)_4-(CH_2)_4-$, $-(CH_2)_4-(CF_2)_4-(CH_2)_2-$,$-(CH_2)_4-(CF_2)_4-(CH_2)_3-$, $-(CH_2)_5-(CF_2)_4-(CH_2)_2-$, $-(CH_2)_5-(CF_2)_4-(CH_2)_3-$, $-(CH_2)_6-(CF_2)_4-(CH_2)_2-$,

$-(CF_2)_5-(CH_2)-$, $-(CH_2)-(CF_2)_5-$, $-(CH_2)-(CF_2)_5-(CH_2)-$, $-(CH_2)-(CF_2)_5-(CH_2)_2-$, $-(CH_2)-(CF_2)_5-(CH_2)_3-$, $-(CH_2)-(CF_2)_5-(CH_2)_4-$, $-(CH_2)-(CF_2)_5-(CH_2)_5-$, $-(CH_2)-(CF_2)_5-(CH_2)_6-$, $-(CH_2)_2-(CF_2)_5-(CH_2)-$, $-(CH_2)_3-(CF_2)_5-(CH_2)-$,

$-(CH_2)_4-(CF_2)_5-(CH_2)-$, $-(CH_2)_5-(CF_2)_5-(CH_2)-$, $-(CH_2)_6-(CF_2)_5-(CH_2)-$, $-(CH_2)_2-(CF_2)_5-(CH_2)_2-$, $-(CH_2)_3-(CF_2)_5-(CH_2)_3-$,$-(CH_2)_4-(CF_2)_5-(CH_2)_4-$, $-(CH_2)_2-(CF_2)_5-(CH_2)_3-$, $-(CH_2)_2-(CF_2)_5-(CH_2)_4-$, $-(CH_2)_2-(CF_2)_5-(CH_2)_5-$, $-(CH_2)_2-(CF_2)_5-(CH_2)_6-$, $-(CH_2)_3-(CF_2)_5-(CH_2)_2-$, $-(CH_2)_3-(CF_2)_5-(CH_2)_4-$, $-(CH_2)_4-(CF_2)_5-(CH_2)_2-$, $-(CH_2)_4-(CF_2)_5-(CH_2)_3-$, $-(CH_2)_5-(CF_2)_5-(CH_2)_2-$, $-(CH_2)-(CHCF_3)-(CH_2)_3-$, $-(CHC_2H_5)-(CH_2)-(CHCF_3)-(CH_2)_3-$, $-(CH_2)-(CHCF_3)-(CH_2)_4-$, $-(CH_2)-(CHCF_3)-(CH_2)_5-$, $-(CH_2)-(CHCF_3)-(CH_2)_6-$, $-(CH_2)-(CHCF_3)-(CH_2)_7-$, $-(CH_2)-(CHCF_3)-(CH_2)_8-$, $-(CH_2)-(CHCF_3)-(CH_2)_9-$, $-(CH_2)-(CHCF_3)-(CH_2)_{10}-$,$-(CH_2)_3-(CHCF_3)-(CH_2)-$, $-(CH_2)_4-(CHCF_3)-(CH_2)-$, $-(CH_2)_5-(CHCF_3)-(CH_2)-$, $-(CH_2)_6-(CHCF_3)-(CH_2)-$, $-(CH_2)_7-(CHCF_3)-(CH_2)-$, $-(CH_2)_8-(CHCF_3)-(CH_2)-$, $-(CH_2)_9-(CHCF_3)-(CH_2)-$, $-(CH_2)_{10}-(CHCF_3)-(CH_2)-$, $-(CH_2)_2-(CHCF_3)-(CH_2)_2-$, $-(CH_2)_3-(CHCF_3)-(CH_2)_3-$, $-(CH_2)_4-(CHCF_3)-(CH_2)_4-$, $-(CH_2)_5-(CHCF_3)-(CH_2)_5-$, $-(CH_2)_2-(CHCF_3)-(CH_2)_3-$, $-(CH_2)_2-(CHCF_3)-(CH_2)_4-$, $-(CH_2)_2-(CHCF_3)-(CH_2)_5-$, $-(CH_2)_2-(CHCF_3)-(CH_2)_6-$, $-(CH_2)_2-(CHCF_3)-(CH_2)_{7-}$., $-(CH_2)_2-(CHCF_3)-(CH_2)_8-$, $-(CH_2)_2-(CHCF_3)-(CH_2)_9-$, $-(CH_2)_3-(CHCF_3)-(CH_2)_2-$, $-(CH_2)_3-(CHCF_3)-(CH_2)_4-$, $-(CH_2)_3-(CHCF_3)-(CH_2)_5-$, $-(CH_2)_3-(CHCF_3)-(CH_2)_6-$, $-(CH_2)_3-(CHCF_3)-(CH_2)_{7-}$., $-(CH_2)_3-(CHCF_3)-(CH_2)_8-$, $-(CH_2)_4-(CHCF_3)-(CH_2)_2-$, $-(CH_2)_4-(CHCF_3)-(CH_2)_3-$, $-(CH_2)_4-(CHCF_3)-(CH_2)_5-$, $-(CH_2)_4-(CHCF_3)-(CH_2)_6-$, $-(CH_2)_4-(CHCF_3)-(CH_2)_{7-}$., $-(CH_2)_5-(CHCF_3)-(CH_2)_2-$, $-(CH_2)_5-(CHCF_3)-(CH_2)_3-$, $-(CH_2)_5-(CHCF_3)-(CH_2)_4-$, $-(CH_2)_5-(CHCF_3)-(CH_2)_6-$, $-(CH_2)_6-(CHCF_3)-(CH_2)_2-$, $-(CH_2)_6-(CHCF_3)-(CH_2)_3-$, $-(CH_2)_6-(CHCF_3)-(CH_2)_4-$, $-(CH_2)_6-(CHCF_3)-(CH_2)_5-$,
$-(CH_2)-(CHCF_3)_2-(CH_2)_3-$, $-(CH_2)-(CHCF_3)_2-(CH_2)_4-$, $-(CH_2)-(CHCF_3)_2-(CH_2)_5-$,$-(CH_2)-(CHCF_3)_2-(CH_2)_6-$, $-(CH_2)-(CHCF_3)_2-(CH_2)_{7-}$., $-(CH_2)-(CHCF_3)_2-(CH_2)_8-$,$-(CH_2)-(CHCF_3)_2-(CH_2)_9-$, $-(CH_2)_2-(CHCF_3)_2-(CH_2)-$, $-(CH_2)_3-(CHCF_3)_2-(CH_2)-$,$-(CH_2)_4-(CHCF_3)_2-(CH_2)-$, $-(CH_2)_5-(CHCF_3)_2-(CH_2)-$, $-(CH_2)_6-(CHCF_3)_2-(CH_2)-$,$-(CH_2)_7-(CHCF_3)_2-(CH_2)-$, $-(CH_2)_8-(CHCF_3)_2-(CH_2)-$, $-(CH_2)_9-(CHCF_3)_2-(CH_2)-$,$-(CH_2)_2-(CHCF_3)_2-(CH_2)_2-$, $-(CH_2)_3-(CHCF_3)_2-(CH_2)_3-$, $-(CH_2)_4-(CHCF_3)_2-(CH_2)_4-$, $-(CH_2)_5-(CHCF_3)_2-(CH_2)_5-$, $-(CH_2)_2-(CHCF_3)_2-(CH_2)_3-$, $-(CH_2)_2-(CHCF_3)_2-(CH_2)_4-$,$-(CH_2)_2-(CHCF_3)_2-(CH_2)_5-$, $-(CH_2)_2-(CHCF_3)_2-(CH_2)_6-$, $-(CH_2)_2-(CHCF_3)_2-(CH_2)_7-$,$-(CH_2)_2-(CHCF_3)_2-(CH_2)_8-$, $-(CH_2)_3-(CHCF_3)_2-(CH_2)_2-$, $-(CH_2)_3-(CHCF_3)_2-(CH_2)_4-$,$-(CH_2)_3-(CHCF_3)_2-(CH_2)_5-$, $-(CH_2)_3-(CHCF_3)_2-(CH_2)_6-$, $-(CH_2)_3-(CHCF_3)_2-(CH_2)_7-$,$-(CH_2)_4-(CHCF_3)_2-(CH_2)_2-$, $-(CH_2)_4-(CHCF_3)_2-(CH_2)_3-$, $-(CH_2)_4-(CHCF_3)_2-(CH_2)_5-$,$-(CH_2)_4-(CHCF_3)_2-(CH_2)_6-$, $-(CH_2)_5-(CHCF_3)_2-(CH_2)_2-$, $-(CH_2)_5-(CHCF_3)_2-(CH_2)_3-$,$-(CH_2)_5-(CHCF_3)_2-(CH_2)_4-$, $-(CH_2)_6-(CHCF_3)_2-(CH_2)_2-$, $-(CH_2)_6-(CHCF_3)_2-(CH_2)_3-$,$-(CH_2)_6-(CHCF_3)_2-(CH_2)_4-$,
$-(CH_2)-(CHCF_3)_3-(CH_2)-$, $-(CH_2)-(CHCF_3)_3-(CH_2)_2-$, $-(CH_2)-(CHCF_3)_3-(CH_2)_3-$,$-(CH_2)-(CHCF_3)_3-(CH_2)_4-$, $-(CH_2)-(CHCF_3)_3-(CH_2)_5-$, $-(CH_2)-(CHCF_3)_3-(CH_2)_6-$,$-(CH_2)-(CHCF_3)_3-(CH_2)_{7-}$., $-(CH_2)-(CHCF_3)_3-(CH_2)_8-$, $-(CH_2)_2-(CHCF_3)_3-(CH_2)-$,$-(CH_2)_3-(CHCF_3)_3-(CH_2)-$, $-(CH_2)_4-(CHCF_3)_3-(CH_2)-$, $-(CH_2)_5-(CHCF_3)_3-(CH_2)-$,$-(CH_2)_6-(CHCF_3)_3-(CH_2)-$, $-(CH_2)_{7-}(CHCF_3)_3-(CH_2)-$, $-(CH_2)_8-(CHCF_3)_3-(CH_2)-$,$-(CH_2)_2-(CHCF_3)_3-(CH_2)_2-$, $-(CH_2)_3-(CHCF_3)_3-(CH_2)_3-$, $-(CH_2)_4-(CHCF_3)_3-(CH_2)_4-$,$-(CH_2)_2-(CHCF_3)_3-(CH_2)_3-$, $-(CH_2)_2-(CHCF_3)_3-(CH_2)_4-$, $-(CH_2)_2-(CHCF_3)_3-(CH_2)_5-$,$-(CH_2)_2-(CHCF_3)_3-(CH_2)_6-$, $-(CH_2)_2-(CHCF_3)_3-(CH_2)_{7-}$., $-(CH_2)_3-(CHCF_3)_3-(CH_2)_2-$,$-(CH_2)_3-(CHCF_3)_3-(CH_2)_4-$, $-(CH_2)_3-(CHCF_3)_3-(CH_2)_5-$, $-(CH_2)_3-(CHCF_3)_3-(CH_2)_6-$,$-(CH_2)_4-(CHCF_3)_3-(CH_2)_2-$, $-(CH_2)_4-(CHCF_3)_3-(CH_2)_3-$, $-(CH_2)_4-(CHCF_3)_3-(CH_2)_5-$,$-(CH_2)_5-(CHCF_3)_3-(CH_2)_2-$, $-(CH_2)_5-(CHCF_3)_3-(CH_2)_3-$, $-(CH_2)_5-(CHCF_3)_3-(CH_2)_4-$,$-(CH_2)_6-(CHCF_3)_3-(CH_2)_2-$, $-(CH_2)_6-(CHCF_3)_3-(CH_2)_3-$,
$-(CHCF_3)_4-(CH_2)-$, $-(CH_2)-(CHCF_3)_4-$, $-(CH_2)-(CHCF_3)_4-(CH_2)-$, $-(CH_2)-(CHCF_3)_4-(CH_2)_2-$, $-(CH_2)-(CHCF_3)_4-(CH_2)_3-$, $-(CH_2)-(CHCF_3)_4-(CH_2)_4-$, $-(CH_2)-(CHCF_3)_4-(CH_2)_5-$, $-(CH_2)-(CHCF_3)_4-(CH_2)_6-$, $-(CH_2)-(CHCF_3)_4-(CH_2)_{7-}$., $-(CH_2)-(CHCF_3)_4-(CH_2)_8-$, $-(CH_2)-(CHCF_3)_4-(CH_2)_9-$, $-(CH_2)-(CHCF_3)_4-(CH_2)_{10}-$, $-(CH_2)_2-(CHCF_3)_4-(CH_2)-$, $-(CH_2)_3-(CHCF_3)_4-(CH_2)-$, $-(CH_2)_4-(CHCF_3)_4-(CH_2)-$, $-(CH_2)_5-(CHCF_3)_4-(CH_2)-$, $-(CH_2)_6-(CHCF_3)_4-(CH_2)-$, $-(CH_2)_7-(CHCF_3)_4-(CH_2)-$, $-(CH_2)_2-(CHCF_3)_4-(CH_2)_2-$, $-(CH_2)_3-(CHCF_3)_4-(CH_2)_3-$, $-(CH_2)_4-(CHCF_3)_4-(CH_2)_4-$, $-(CH_2)_5-(CHCF_3)_4-(CH_2)_5-$, $-(CH_2)_2-(CHCF_3)_4-(CH_2)_3-$, $-(CH_2)_2-(CHCF_3)_4-(CH_2)_4-$, $-(CH_2)_2-(CHCF_3)_4-(CH_2)_5-$, $-(CH_2)_2-(CHCF_3)_4-(CH_2)_6-$, $-(CH_2)_3-(CHCF_3)_4-(CH_2)_2-$, $-(CH_2)_3-(CHCF_3)_4-(CH_2)_4-$, $-(CH_2)_4-(CHCF_3)_4-(CH_2)_2-$, $-(CH_2)_4-(CHCF_3)_4-(CH_2)_3-$, $-(CH_2)_5-(CHCF_3)_4-(CH_2)_2-$, $-(CH_2)_5-(CHCF_3)_4-(CH_2)_3-$, $-(CH_2)_6-(CHCF_3)_4-(CH_2)_2-$,
$-(CHCF_3)_5-(CH_2)-$, $-(CH_2)-(CHCF_3)_5-$, $-(CH_2)-(CHCF_3)_5-(CH_2)-$, $-(CH_2)-(CHCF_3)_5-(CH_2)_2-$, $-(CH_2)-(CHCF_3)_5-(CH_2)_3-$, $-(CH_2)-(CHCF_3)_5-(CH_2)_4-$, $-(CH_2)-(CHCF_3)_5-(CH_2)_5-$, $-(CH_2)-(CHCF_3)_5-(CH_2)_6-$, $-(CH_2)_2-(CHCF_3)_5-(CH_2)-$, $-(CH_2)_3-(CHCF_3)_5-(CH_2)-$, $-(CH_2)_4-(CHCF_3)_5-(CH_2)-$, $-(CH_2)_5-(CHCF_3)_5-(CH_2)-$, $-(CH_2)_6$,$-(CHCF_3)_5-(CH_2)-$, $-(CH_2)_2-(CHCF_3)_5-(CH_2)_2-$, $-(CH_2)_3-(CHCF_3)_5-(CH_2)_3-$, $-(CH_2)_4-(CHCF_3)_5-(CH_2)_4-$, $-(CH_2)_2-(CHCF_3)_5-(CH_2)_3-$, $-(CH_2)_2-(CHCF_3)_5-(CH_2)_4-$, $-(CH_2)_2-(CHCF_3)_5-(CH_2)_5-$, $-(CH_2)_2-(CHCF_3)_5-(CH_2)_6-$, $-(CH_2)_3-(CHCF_3)_5-(CH_2)_2-$, $-(CH_2)_3-(CHCF_3)_5-(CH_2)_4-$, $-(CH_2)_4-(CHCF_3)_5-(CH_2)_2-$, $-(CH_2)_4-(CHCF_3)_5-(CH_2)_3-$, $-(CH_2)_5-(CHCF_3)_5-(CH_2)_2-$,
$-(CH_2)-[C(CH_3)CF_3]-(CH_2)_3-$, $-(CH_2)-[C(CH_3)CF_3]-(CH_2)_4-$, $-(CH_2)-[C(CH_3)CF_3]-(CH_2)_5-$, $-(CH_2)-[C(CH_3)CF_3]-(CH_2)_6-$, $-(CH_2)-[C(CH_3)CF_3]-(CH_2)_{7-}$., $-(CH_2)-[C(CH_3)CF_3]-(CH_2)_8-$, $-(CH_2)-[C(CH_3)CF_3]-(CH_2)_9-$, $-(CH_2)-[C(CH_3)CF_3]-(CH_2)_{10}-$,$-(CH_2)_3-[C(CH_3)CF_3]-(CH_2)-$, $-(CH_2)_4-[C(CH_3)CF_3]-(CH_2)-$, $-(CH_2)_5-[C(CH_3)CF_3]-(CH_2)-$, $-(CH_2)_6-[C(CH_3)CF_3]-(CH_2)-$, $-(CH_2)_7-[C(CH_3)CF_3]-(CH_2)-$, $-(CH_2)_8-[C(CH_3)CF_3]-(CH_2)-$, $-(CH_2)_9-[C(CH_3)CF_3]-(CH_2)-$, $-(CH_2)_{10}-[C(CH_3)CF_3]-(CH_2)-$,$-(CH_2)_2-[C(CH_3)CF_3]-(CH_2)_2-$, $-(CH_2)_3-[C(CH_3)CF_3]-(CH_2)_3-$, $-(CH_2)_4-[C(CH_3)CF_3]-(CH_2)_4-$, $-(CH_2)_5-[C(CH_3)CF_3]-(CH_2)_5-$, $-(CH_2)_2-[C(CH_3)CF_3]-(CH_2)_3-$,

$-(CH_2)_2-[C(CH_3)CF_3]-(CH_2)_4-$, $-(CH_2)_2-[C(CH_3)CF_3]-(CH_2)_5-$, $-(CH_2)_2-[C(CH_3)CF_3]-(CH_2)_6-$, $-(CH_2)_2-[C(CH_3)CF_3]-(CH_2)_7-$, $-(CH_2)_2-[C(CH_3)CF_3]-(CH_2)_8-$, $-(CH_2)_2-[C(CH_3)CF_3]-(CH_2)_9-$, $-(CH_2)_3-[C(CH_3)CF_3]-(CH_2)_2-$, $-(CH_2)_3-[C(CH_3)CF_3]-(CH_2)_4-$, $-(CH_2)_3-[C(CH_3)CF_3]-(CH_2)_5-$, $-(CH_2)_3-[C(CH_3)CF_3]-(CH_2)_6-$, $-(CH_2)_3-[C(CH_3)CF_3]-(CH_2)_7-$, $-(CH_2)_3-[C(CH_3)CF_3]-(CH_2)_8-$, $-(CH_2)_4-[C(CH_3)CF_3]-(CH_2)_2-$, $-(CH_2)_4-[C(CH_3)CF_3]-(CH_2)_3-$, $-(CH_2)_4-[C(CH_3)CF_3]-(CH_2)_5-$, $-(CH_2)_4-[C(CH_3)CF_3]-(CH_2)_6-$, $-(CH_2)_4-[C(CH_3)CF_3]-(CH_2)_7-$, $-(CH_2)_5-[C(CH_3)CF_3]-(CH_2)_2-$, $-(CH_2)_5-[C(CH_3)CF_3]-(CH_2)_3-$, $-(CH_2)_5-[C(CH_3)CF_3]-(CH_2)_4-$, $-(CH_2)_5-[C(CH_3)CF_3]-(CH_2)_6-$, $-(CH_2)_6-[C(CH_3)CF_3]-(CH_2)_2-$, $-(CH_2)_6-[C(CH_3)CF_3]-(CH_2)_3-$, $-(CH_2)_6-[C(CH_3)CF_3]-(CH_2)_4-$, $-(CH_2)_6-[C(CH_3)CF_3]-(CH_2)_5-$, $-(CH_2)-[C(CH_3)CF_3]_2-(CH_2)_2-$, $-(CH_2)-[C(CH_3)CF_3]_2-(CH_2)_3-$, $-(CH_2)-[C(CH_3)CF_3]_2-(CH_2)_4-$, $-(CH_2)-[C(CH_3)CF_3]_2-(CH_2)_5-$, $-(CH_2)-[C(CH_3)CF_3]_2-(CH_2)_6-$, $-(CH_2)-[C(CH_3)CF_3]_2-(CH_2)_7-$, $-(CH_2)-[C(CH_3)CF_3]_2-(CH_2)_8-$, $-(CH_2)-[C(CH_3)CF_3]_2-(CH_2)_9-$, $-(CH_2)_2-[C(CH_3)CF_3]_2-(CH_2)-$, $-(CH_2)_3-[C(CH_3)CF_3]_2-(CH_2)-$, $-(CH_2)_4-[C(CH_3)CF_3]_2-(CH_2)-$, $-(CH_2)_5-[C(CH_3)CF_3]_2-(CH_2)-$, $-(CH_2)_6-[C(CH_3)CF_3]_2-(CH_2)-$, $-(CH_2)_7-[C(CH_3)CF_3]_2-(CH_2)-$, $-(CH_2)_8-[C(CH_3)CF_3]_2-(CH_2)-$, $-(CH_2)_9-[C(CH_3)CF_3]_2-(CH_2)-$, $-(CH_2)_2-[C(CH_3)CF_3]_2-(CH_2)_2-$, $-(CH_2)_3-[C(CH_3)CF_3]_2-(CH_2)_3-$, $-(CH_2)_4-[C(CH_3)CF_3]_2-(CH_2)_4-$, $-(CH_2)_5-[C(CH_3)CF_3]_2-(CH_2)_5-$, $-(CH_2)_2-[C(CH_3)CF_3]_2-(CH_2)_3-$, $-(CH_2)_2-[C(CH_3)CF_3]_2-(CH_2)_4-$, $-(CH_2)_2-[C(CH_3)CF_3]_2-(CH_2)_5-$, $-(CH_2)_2-[C(CH_3)CF_3]_2-(CH_2)_6-$, $-(CH_2)_2-[C(CH_3)CF_3]_2-(CH_2)_7-$, $-(CH_2)_2-[C(CH_3)CF_3]_2-(CH_2)_8-$, $-(CH_2)_3-[C(CH_3)CF_3]_2-(CH_2)_2-$, $-(CH_2)_3-[C(CH_3)CF_3]_2-(CH_2)_4-$, $-(CH_2)_3-[C(CH_3)CF_3]_2-(CH_2)_5-$, $-(CH_2)_3-[C(CH_3)CF_3]_2-(CH_2)_6-$, $-(CH_2)_3-[C(CH_3)CF_3]_2-(CH_2)_7-$, $-(CH_2)_4-[C(CH_3)CF_3]_2-(CH_2)_2-$, $-(CH_2)_4-[C(CH_3)CF_3]_2-(CH_2)_3-$, $-(CH_2)_4-[C(CH_3)CF_3]_2-(CH_2)_5-$, $-(CH_2)_4-[C(CH_3)CF_3]_2-(CH_2)_6-$, $-(CH_2)_5-[C(CH_3)CF_3]_2-(CH_2)_2-$, $-(CH_2)_5-[C(CH_3)CF_3]_2-(CH_2)_3-$, $-(CH_2)_5-[C(CH_3)CF_3]_2-(CH_2)_4-$, $-(CH_2)_6-[C(CH_3)CF_3]_2-(CH_2)_2-$, $-(CH_2)_6-[C(CH_3)CF_3]_2-(CH_2)_3-$, $-(CH_2)_6-[C(CH_3)CF_3]_2-(CH_2)_4-$, $-(CH_2)-[C(CH_3)CF_3]_3-(CH_2)-$, $-(CH_2)-[C(CH_3)CF_3]_3-(CH_2)_2-$, $-(CH_2)-[C(CH_3)CF_3]_3-(CH_2)_3-$, $-(CH_2)-[C(CH_3)CF_3]_3-(CH_2)_4-$, $-(CH_2)-[C(CH_3)CF_3]_3-(CH_2)_5-$, $-(CH_2)-[C(CH_3)CF_3]_3-(CH_2)_6-$, $-(CH_2)-[C(CH_3)CF_3]_3-(CH_2)_7-$, $-(CH_2)-[C(CH_3)CF_3]_3-(CH_2)_8-$, $-(CH_2)_2-[C(CH_3)CF_3]_3-(CH_2)-$, $-(CH_2)_3-[C(CH_3)CF_3]_3-(CH_2)-$, $-(CH_2)_4-[C(CH_3)CF_3]_3-(CH_2)-$, $-(CH_2)_5-[C(CH_3)CF_3]_3-(CH_2)-$, $-(CH_2)_6-[C(CH_3)CF_3]_3-(CH_2)-$, $-(CH_2)_7-[C(CH_3)CF_3]_3-(CH_2)-$, $-(CH_2)_8-[C(CH_3)CF_3]_3-(CH_2)-$, $-(CH_2)_2-[C(CH_3)CF_3]_3-(CH_2)_2-$, $-(CH_2)_3-[C(CH_3)CF_3]_3-(CH_2)_3-$, $-(CH_2)_4-[C(CH_3)CF_3]_3-(CH_2)_4-$, $-(CH_2)_2-[C(CH_3)CF_3]_3-(CH_2)_3-$, $-(CH_2)_2-[C(CH_3)CF_3]_3-(CH_2)_4-$, $-(CH_2)_2-[C(CH_3)CF_3]_3-(CH_2)_5-$, $-(CH_2)_2-[C(CH_3)CF_3]_3-(CH_2)_6-$, $-(CH_2)_2-[C(CH_3)CF_3]_3-(CH_2)_7-$, $-(CH_2)_3-[C(CH_3)CF_3]_3-(CH_2)_2-$, $-(CH_2)_3-[C(CH_3)CF_3]_3-(CH_2)_4-$, $-(CH_2)_3-[C(CH_3)CF_3]_3-(CH_2)_5-$, $-(CH_2)_3-[C(CH_3)CF_3]_3-(CH_2)_6-$, $-(CH_2)_4-[C(CH_3)CF_3]_3-(CH_2)_2-$, $-(CH_2)_4-[C(CH_3)CF_3]_3-(CH_2)_3-$, $-(CH_2)_4-[C(CH_3)CF_3]_3-(CH_2)_5-$, $-(CH_2)_5-[C(CH_3)CF_3]_3-(CH_2)_2-$, $-(CH_2)_5-[C(CH_3)CF_3]_3-(CH_2)_3-$, $-(CH_2)_5-[C(CH_3)CF_3]_3-(CH_2)_4-$, $-(CH_2)_6-[C(CH_3)CF_3]_3-(CH_2)_2-$, $-(CH_2)_6-[C(CH_3)CF_3]_3-(CH_2)_3-$, $-[C(CH_3)CF_3]_4-(CH_2)-$, $-(CH_2)-[C(CH_3)CF_3]_4-$, $-(CH_2)-[C(CH_3)CF_3]_4-(CH_2)-$, $-(CH_2)-[C(CH_3)CF_3]_4-(CH_2)_2-$, $-(CH_2)-[C(CH_3)CF_3]_4-(CH_2)_3-$, $-(CH_2)-[C(CH_3)CF_3]_4-(CH_2)_4-$, $-(CH_2)-[C(CH_3)CF_3]_4-(CH_2)_5-$, $-(CH_2)-[C(CH_3)CF_3]_4-(CH_2)_6-$, $-(CH_2)-[C(CH_3)CF_3]_4-(CH_2)_7-$., $-(CH_2)-[C(CH_3)CF_3]_4-(CH_2)_8-$, $-(CH_2)-[C(CH_3)CF_3]_4-(CH_2)_9-$, $-(CH_2)-[C(CH_3)CF_3]_4-(CH_2)_{10}-$, $-(CH_2)_2-[C(CH_3)CF_3]_4-(CH_2)-$, $-(CH_2)_3-[C(CH_3)CF_3]_4-(CH_2)-$, $-(CH_2)_4-[C(CH_3)CF_3]_4-(CH_2)-$, $-(CH_2)_5-[C(CH_3)CF_3]_4-(CH_2)-$, $-(CH_2)_6-[C(CH_3)CF_3]_4-(CH_2)-$, $-(CH_2)_7-[C(CH_3)CF_3]_4-(CH_2)-$, $-(CH_2)_2-[C(CH_3)CF_3]_4-(CH_2)_2-$, $-(CH_2)_3-[C(CH_3)CF_3]_4-(CH_2)_3-$, $-(CH_2)_4-[C(CH_3)CF_3]_4-(CH_2)_4-$, $-(CH_2)_5-[C(CH_3)CF_3]_4-(CH_2)_5-$, $-(CH_2)_2-[C(CH_3)CF_3]_4-(CH_2)_3-$, $-(CH_2)_2-[C(CH_3)CF_3]_4-(CH_2)_4-$, $-(CH_2)_2-[C(CH_3)CF_3]_4-(CH_2)_5-$, $-(CH_2)_2-[C(CH_3)CF_3]_4-(CH_2)_6-$, $-(CH_2)_3-[C(CH_3)CF_3]_4-(CH_2)_2-$, $-(CH_2)_3-[C(CH_3)CF_3]_4-(CH_2)_4-$, $-(CH_2)_4-[C(CH_3)CF_3]_4-(CH_2)_2-$, $-(CH_2)_4-[C(CH_3)CF_3]_4-(CH_2)_3-$, $-(CH_2)_5-[C(CH_3)CF_3]_4-(CH_2)_2-$, $-(CH_2)_5-[C(CH_3)CF_3]_4-(CH_2)_3-$, $-(CH_2)_6-[C(CH_3)CF_3]_4-(CH_2)_2-$, $-[C(CH_3)CF_3]_5-(CH_2)-$, $-(CH_2)-[C(CH_3)CF_3]_5-$, $-(CH_2)-[C(CH_3)CF_3]_5-(CH_2)-$, $-(CH_2)-[C(CH_3)CF_3]_5-(CH_2)_2-$, $-(CH_2)-[C(CH_3)CF_3]_5-(CH_2)_3-$, $-(CH_2)-[C(CH_3)CF_3]_5-(CH_2)_4-$, $-(CH_2)-[C(CH_3)CF_3]_5-(CH_2)_5-$, $-(CH_2)-[C(CH_3)CF_3]_5-(CH_2)_6-$, $-(CH_2)_2-[C(CH_3)CF_3]_5-(CH_2)-$, $-(CH_2)_3-[C(CH_3)CF_3]_5-(CH_2)-$, $-(CH_2)_4-[C(CH_3)CF_3]_5-(CH_2)-$, $-(CH_2)_5-[C(CH_3)CF_3]_5-(CH_2)-$, $-(CH_2)_6-[C(CH_3)CF_3]_5-(CH_2)-$, $-(CH_2)_2-[C(CH_3)CF_3]_5-(CH_2)_2-$, $-(CH_2)_3-[C(CH_3)CF_3]_5-(CH_2)_3-$, $-(CH_2)_4-[C(CH_3)CF_3]_5-(CH_2)_4-$, $-(CH_2)_2-[C(CH_3)CF_3]_5-(CH_2)_3-$, $-(CH_2)_2-[C(CH_3)CF_3]_5-(CH_2)_4-$, $-(CH_2)_2-[C(CH_3)CF_3]_5-(CH_2)_5-$, $-(CH_2)_2-[C(CH_3)CF_3]_5-(CH_2)_6-$, $-(CH_2)_3-[C(CH_3)CF_3]_5-(CH_2)_2-$, $-(CH_2)_3-[C(CH_3)CF_3]_5-(CH_2)_4-$, $-(CH_2)_4-[C(CH_3)CF_3]_5-(CH_2)_2-$, $-(CH_2)_4-[C(CH_3)CF_3]_5-(CH_2)_3-$, $-(CH_2)_5-[C(CH_3)CF_3]_5-(CH_2)_2-$, $-(CH_2)-[CH(CH_2CF_3)]-(CH_2)_3-$, $-(CH_2)-[CH(CH_2CF_3)]-(CH_2)_4-$, $-(CH_2)-[CH(CH_2CF_3)]-(CH_2)_5-$, $-(CH_2)-[CH(CH_2CF_3)]-(CH_2)_6-$, $-(CH_2)-[CH(CH_2CF_3)]-(CH_2)_7-$, $-(CH_2)-[CH(CH_2CF_3)]-(CH_2)_8-$, $-(CH_2)-[CH(CH_2CF_3)]-(CH_2)_9-$, $-(CH_2)-[CH(CH_2CF_3)]-(CH_2)_{10}-$, $-(CH_2)_3-[CH(CH_2CF_3)]-(CH_2)-$, $-(CH_2)_4-[CH(CH_2CF_3)]-(CH_2)-$, $-(CH_2)_5-[CH(CH_2CF_3)]-(CH_2)-$, $-(CH_2)_6-[CH(CH_2CF_3)]-(CH_2)-$,

$-(CH_2)_7-[CH(CH_2CF_3)]-(CH_2)-$, $-(CH_2)_8-[CH(CH_2CF_3)]-(CH_2)-$, $-(CH_2)_9-[CH(CH_2CF_3)]-(CH_2)-$, $-(CH_2)_{10}-[CH(CH_2CF_3)]-(CH_2)-$, $-(CH_2)_2-[CH(CH_2CF_3)]-(CH_2)_2-$, $-(CH_2)_3-[CH(CH_2CF_3)]-(CH_2)_3-$, $-(CH_2)_4-[CH(CH_2CF_3)]-(CH_2)_4-$, $-(CH_2)_5-[CH(CH_2CF_3)]-(CH_2)_5-$, $-(CH_2)_2-[CH(CH_2CF_3)]-(CH_2)_3-$, $-(CH_2)_2-[CH(CH_2CF_3)]-(CH_2)_4-$, $-(CH_2)_2-[CH(CH_2CF_3)]-(CH_2)_5-$, $-(CH_2)_2-[CH(CH_2CF_3)]-(CH_2)_6-$, $-(CH_2)_2-[CH(CH_2CF_3)]-(CH_2)_7-$, $-(CH_2)_2-[CH(CH_2CF_3)]-(CH_2)_8-$, $-(CH_2)_2-[CH(CH_2CF_3)]-(CH_2)_9-$, $-(CH_2)_3-[CH(CH_2CF_3)]-(CH_2)_2-$, $-(CH_2)_3-[CH(CH_2CF_3)]-(CH_2)_4-$, $-(CH_2)_3-[CH(CH_2CF_3)]-(CH_2)_5-$, $-(CH_2)_3-[CH(CH_2CF_3)]-(CH_2)_6-$, $-(CH_2)_3-[CH(CH_2CF_3)]-(CH_2)_7-$, $-(CH_2)_3-[CH(CH_2CF_3)]-(CH_2)_8-$, $-(CH_2)_4-[CH(CH_2CF_3)]-(CH_2)_2-$, $-(CH_2)_4-[CH(CH_2CF_3)]-(CH_2)_3-$, $-(CH_2)_4-[CH(CH_2CF_3)]-(CH_2)_5-$, $-(CH_2)_4-[CH(CH_2CF_3)]-(CH_2)_6-$, $-(CH_2)_4-[CH(CH_2CF_3)]-(CH_2)_{7-.}$, $-(CH_2)_5-[CH(CH_2CF_3)]-(CH_2)_2-$, $-(CH_2)_5-[CH(CH_2CF_3)]-(CH_2)_3-$, $-(CH_2)_5-[CH(CH_2CF_3)]-(CH_2)_4-$, $-(CH_2)_5-[CH(CH_2CF_3)]-(CH_2)_6-$, $-(CH_2)_6-[CH(CH_2CF_3)]-(CH_2)_2-$, $-(CH_2)_6-[CH(CH_2CF_3)]-(CH_2)_3-$, $-(CH_2)_6-[CH(CH_2CF_3)]-(CH_2)_4-$, $-(CH_2)_6-[CH(CH_2CF_3)]-(CH_2)_5-$,

$-(CH_2)-[CH(CH_2CF_3)]_2-(CH_2)_2-$, $-(CH_2)-[CH(CH_2CF_3)]_2-(CH_2)_3-$, $-(CH_2)-[CH(CH_2CF_3)]_2-(CH_2)_4-$, $-(CH_2)-[CH(CH_2CF_3)]_2-(CH_2)_5-$, $-(CH_2)-[CH(CH_2CF_3)]_2-(CH_2)_6-$, $-(CH_2)-[CH(CH_2CF_3)]_2-(CH_2)_7-$, $-(CH_2)-[CH(CH_2CF_3)]_2-(CH_2)_8-$, $-(CH_2)-[CH(CH_2CF_3)]_2-(CH_2)_9-$, $-(CH_2)_2-[CH(CH_2CF_3)]_2-(CH_2)-$, $-(CH_2)_3-[CH(CH_2CF_3)]_2-(CH_2)-$, $-(CH_2)_4-[CH(CH_2CF_3)]_2-(CH_2)-$, $-(CH_2)_5-[CH(CH_2CF_3)]_2-(CH_2)-$, $-(CH_2)_6-[CH(CH_2CF_3)]_2-(CH_2)-$, $-(CH_2)_7-[CH(CH_2CF_3)]_2-(CH_2)-$, $-(CH_2)_8-[CH(CH_2CF_3)]_2-(CH_2)-$, $-(CH_2)_9-[CH(CH_2CF_3)]_2-(CH_2)-$, $-(CH_2)_2-[CH(CH_2CF_3)]_2-(CH_2)_2-$, $-(CH_2)_3-[CH(CH_2CF_3)]_2-(CH_2)_3-$, $-(CH_2)_4-[CH(CH_2CF_3)]_2-(CH_2)_4-$, $-(CH_2)_5-[CH(CH_2CF_3)]_2-(CH_2)_5-$, $-(CH_2)_2-[CH(CH_2CF_3)]_2-(CH_2)_3-$, $-(CH_2)_2-[CH(CH_2CF_3)]_2-(CH_2)_4-$, $-(CH_2)_2-[CH(CH_2CF_3)]_2-(CH_2)_5-$, $-(CH_2)_2-[CH(CH_2CF_3)]_2-(CH_2)_6-$, $-(CH_2)_2-[CH(CH_2CF_3)]_2-(CH_2)_{7-.}$, $-(CH_2)_2-[CH(CH_2CF_3)]_2-(CH_2)_8-$, $-(CH_2)_3-[CH(CH_2CF_3)]_2-(CH_2)_2-$, $-(CH_2)_3-[CH(CH_2CF_3)]_2-(CH_2)_4-$, $-(CH_2)_3-[CH(CH_2CF_3)]_2-(CH_2)_5-$, $-(CH_2)_3-[CH(CH_2CF_3)]_2-(CH_2)_6-$, $-(CH_2)_3-[CH(CH_2CF_3)]_2-(CH_2)_7-$, $-(CH_2)_4-[CH(CH_2CF_3)]_2-(CH_2)_2-$, $-(CH_2)_4-[CH(CH_2CF_3)]_2-(CH_2)_3-$, $-(CH_2)_4-[CH(CH_2CF_3)]_2-(CH_2)_5-$, $-(CH_2)_4-[CH(CH_2CF_3)]_2-(CH_2)_6-$, $-(CH_2)_5-[CH(CH_2CF_3)]_2-(CH_2)_2-$, $-(CH_2)_5-[CH(CH_2CF_3)]_2-(CH_2)_3-$, $-(CH_2)_5-[CH(CH_2CF_3)]_2-(CH_2)_4-$, $-(CH_2)_6-[CH(CH_2CF_3)]_2-(CH_2)_2-$, $-(CH_2)_6-[CH(CH_2CF_3)]_2-(CH_2)_3-$, $-(CH_2)_6-[CH(CH_2CF_3)]_2-(CH_2)_4-$,

$-(CH_2)-[CH(CH_2CF_3)]_3-(CH_2)-$, $-(CH_2)-[CH(CH_2CF_3)]_3-(CH_2)_2-$, $-(CH_2)-[CH(CH_2CF_3)]_3-(CH_2)_3-$, $-(CH_2)-[CH(CH_2CF_3)]_3-(CH_2)_4-$, $-(CH_2)-[CH(CH_2CF_3)]_3-(CH_2)_5-$, $-(CH_2)-[CH(CH_2CF_3)]_3-(CH_2)_6-$, $-(CH_2)-[CH(CH_2CF_3)]_3-(CH_2)_7-$, $-(CH_2)-[CH(CH_2CF_3)]_3-(CH_2)_8-$, $-(CH_2)_2-[CH(CH_2CF_3)]_3-(CH_2)-$, $-(CH_2)_3-[CH(CH_2CF_3)]_3-(CH_2)-$, $-(CH_2)_4-[CH(CH_2CF_3)]_3-(CH_2)-$, $-(CH_2)_5-[CH(CH_2CF_3)]_3-(CH_2)-$, $-(CH_2)_6-[CH(CH_2CF_3)]_3-(CH_2)-$, $-(CH_2)_7-[CH(CH_2CF_3)]_3-(CH_2)-$, $-(CH_2)_8-[CH(CH_2CF_3)]_3-(CH_2)-$, $-(CH_2)_2-[CH(CH_2CF_3)]_3-(CH_2)_2-$, $-(CH_2)_3-[CH(CH_2CF_3)]_3-(CH_2)_3-$, $-(CH_2)_4-[CH(CH_2CF_3)]_3-(CH_2)_4-$, $-(CH_2)_2-[CH(CH_2CF_3)]_3-(CH_2)_3-$, $-(CH_2)_2-[CH(CH_2CF_3)]_3-(CH_2)_4-$, $-(CH_2)_2-[CH(CH_2CF_3)]_3-(CH_2)_5-$, $-(CH_2)_2-[CH(CH_2CF_3)]_3-(CH_2)_6-$, $-(CH_2)_2-[CH(CH_2CF_3)]_3-(CH_2)_7-$, $-(CH_2)_3-[CH(CH_2CF_3)]_3-(CH_2)_2-$, $-(CH_2)_3-[CH(CH_2CF_3)]_3-(CH_2)_4-$, $-(CH_2)_3-[CH(CH_2CF_3)]_3-(CH_2)_5-$, $-(CH_2)_3-[CH(CH_2CF_3)]_3-(CH_2)_6-$, $-(CH_2)_4-[CH(CH_2CF_3)]_3-(CH_2)_2-$, $-(CH_2)_4-[CH(CH_2CF_3)]_3-(CH_2)_3-$, $-(CH_2)_4-[CH(CH_2CF_3)]_3-(CH_2)_5-$, $-(CH_2)_5-[CH(CH_2CF_3)]_3-(CH_2)_2-$, $-(CH_2)_5-[CH(CH_2CF_3)]_3-(CH_2)_3-$, $-(CH_2)_5-[CH(CH_2CF_3)]_3-(CH_2)_4-$, $-(CH_2)_6-[CH(CH_2CF_3)]_3-(CH_2)_2-$, $-(CH_2)_6-[CH(CH_2CF_3)]_3-(CH_2)_3-$,

$-[CH(CH_2CF_3)]_4-(CH_2)-$, $-(CH_2)-[CH(CH_2CF_3)]_4-$, $-(CH_2)-[CH(CH_2CF_3)]_4-(CH_2)-$, - $(CH_2)-[CH(CH_2CF_3)]_4-(CH_2)_2-$, $-(CH_2)-[CH(CH_2CF_3)]_4-(CH_2)_3-$, $-(CH_2)-[CH(CH_2CF_3)]_4-(CH_2)_4-$, $-(CH_2)-[CH(CH_2CF_3)]_4-(CH_2)_5-$, $-(CH_2)-[CH(CH_2CF_3)]_4-(CH_2)_6-$, $-(CH_2)-[CH(CH_2CF_3)]_4-(CH_2)_7-$, $-(CH_2)-[CH(CH_2CF_3)]_4-(CH_2)_8-$, $-(CH_2)-[CH(CH_2CF_3)]_4-(CH_2)_9-$, $-(CH_2)-[CH(CH_2CF_3)]_4-(CH_2)_{10}-$, $-(CH_2)_2-[CH(CH_2CF_3)]_4-(CH_2)-$, $-(CH_2)_3-[CH(CH_2CF_3)]_4-(CH_2)-$, $-(CH_2)_4-[CH(CH_2CF_3)]_4-(CH_2)-$, $-(CH_2)_5-[CH(CH_2CF_3)]_4-(CH_2)-$, $-(CH_2)_6-[CH(CH_2CF_3)]_4-(CH_2)-$, $-(CH_2)_7-[CH(CH_2CF_3)]_4-(CH_2)-$, $-(CH_2)_2-[CH(CH_2CF_3)]_4-(CH_2)_2-$, $-(CH_2)_3-[CH(CH_2CF_3)]_4-(CH_2)_3-$, $-(CH_2)_4-[CH(CH_2CF_3)]_4-(CH_2)_4-$, $-(CH_2)_5-[CH(CH_2CF_3)]_4-(CH_2)_5-$, $-(CH_2)_2-[CH(CH_2CF_3)]_4-(CH_2)_3-$, $-(CH_2)_2-[CH(CH_2CF_3)]_4-(CH_2)_4-$, $-(CH_2)_2-[CH(CH_2CF_3)]_4-(CH_2)_5-$, $-(CH_2)_2-[CH(CH_2CF_3)]_4-(CH_2)_6-$, $-(CH_2)_3-[CH(CH_2CF_3)]_4-(CH_2)_2-$, $-(CH_2)_3-[CH(CH_2CF_3)]_4-(CH_2)_4-$, $-(CH_2)_4-[CH(CH_2CF_3)]_4-(CH_2)_2-$, $-(CH_2)_4-[CH(CH_2CF_3)]_4-(CH_2)_3-$, $-(CH_2)_5-[CH(CH_2CF_3)]_4-(CH_2)_2-$, $-(CH_2)_5-[CH(CH_2CF_3)]_4-(CH_2)_3-$, $-(CH_2)_6-[CH(CH_2CF_3)]_4-(CH_2)_2-$,

$-[CH(CH_2CF_3)]_5-(CH_2)-$, $-(CH_2)-[CH(CH_2CF_3)_5-$, $-(CH_2)-[CH(CH_2CF_3)]_5-(CH_2)-$, - $(CH_2)-[CH(CH_2CF_3)]_5-(CH_2)_2-$, $-(CH_2)-[CH(CH_2CF_3)]_5-(CH_2)_3-$, $-(CH_2)-[CH(CH_2CF_3)]_5-(CH_2)_4-$, $-(CH_2)-[CH(CH_2CF_3)]_5-(CH_2)_5-$, $-(CH_2)-[CH(CH_2CF_3)]_5-(CH_2)_6-$, $-(CH_2)_2-[CH(CH_2CF_3)]_5-(CH_2)-$, $-(CH_2)_3-[CH(CH_2CF_3)]_5-(CH_2)-$, $-(CH_2)_4-[CH(CH_2CF_3)]_5-(CH_2)-$, $-(CH_2)_5-[CH(CH_2CF_3)]_5-(CH_2)-$, $-(CH_2)_6-[CH(CH_2CF_3)]_5-(CH_2)-$, $-(CH_2)_2-[CH(CH_2CF_3)]_5-(CH_2)_2-$, $-(CH_2)_3-[CH(CH_2CF_3)]_5-(CH_2)_3-$, $-(CH_2)_4-[CH(CH_2CF_3)]_5-(CH_2)_4-$, $-(CH_2)_2-[CH(CH_2CF_3)]_5-(CH_2)_3-$, $-(CH_2)_2-[CH(CH_2CF_3)]_5-(CH_2)_4-$, $-(CH_2)_2-[CH(CH_2CF_3)]_5-(CH_2)_5-$, $-(CH_2)_2-[CH(CH_2CF_3)]_5-(CH_2)_6-$, $-(CH_2)_3-[CH(CH_2CF_3)]_5-(CH_2)_2-$, $-(CH_2)_3-[CH(CH_2CF_3)]_5-(CH_2)_4-$, $-(CH_2)_4-[CH(CH_2CF_3)]_5-(CH_2)_2-$, $-(CH_2)_4-[CH(CH_2CF_3)]_5-(CH_2)_3-$, $-(CH_2)_5-[CH(CH_2CF_3)]_5-(CH_2)_2-$,

$-(CH_2)-[C(CH_3)(CH_2CF_3)]-(CH_2)_3-$, $-(CH_2)-[C(CH_3)(CH_2CF_3)]-(CH_2)_4-$, $-(CH_2)-[C(CH_3)(CH_2CF_3)]-(CH_2)_5-$,

$-(CH_2)-[C(CH_3)(CH_2CF_3)]-(CH_2)_6-$, $-(CH_2)-[C(CH_3)(CH_2CF_3)]-(CH_2)_7-$, $-(CH_2)-[C(CH_3)(CH_2CF_3)]-(CH_2)_8-$,
$-(CH_2)-[C(CH_3)(CH_2CF_3)]-(CH_2)_9-$, $-(CH_2)-[C(CH_3)(CH_2CF_3)]-(CH_2)_{10}-$, $-(CH_2)_3-[C(CH_3)(CH_2CF_3)]-(CH_2)-$,
$-(CH_2)_4-[C(CH_3)(CH_2CF_3)]-(CH_2)-$, $-(CH_2)_5-[C(CH_3)(CH_2CF_3)]-(CH_2)-$, $-(CH_2)_6-[C(CH_3)(CH_2CF_3)]-(CH_2)-$,
$-(CH_2)_7-[C(CH_3)(CH_2CF_3)]-(CH_2)-$, $-(CH_2)_8-[C(CH_3)(CH_2CF_3)]-(CH_2)-$, $-(CH_2)_9-[C(CH_3)(CH_2CF_3)]-(CH_2)-$,
$-(CH_2)_{10}-[C(CH_3)(CH_2CF_3)]-(CH_2)-$, $-(CH_2)_2-[C(CH_3)(CH_2CF_3)]-(CH_2)_2-$, $-(CH_2)_3-[C(CH_3)(CH_2CF_3)]-(CH_2)_3-$,
$-(CH_2)_4-[C(CH_3)(CH_2CF_3)]-(CH_2)_4-$, $-(CH_2)_5-[C(CH_3)(CH_2CF_3)]-(CH_2)_5-$, $-(CH_2)_2-[C(CH_3)(CH_2CF_3)]-(CH_2)_3-$,
$-(CH_2)_2-[C(CH_3)(CH_2CF_3)]-(CH_2)_4-$, $-(CH_2)_2-[C(CH_3)(CH_2CF_3)]-(CH_2)_5-$, $-(CH_2)_2-[C(CH_3)(CH_2CF_3)]-(CH_2)_6-$,
$-(CH_2)_2-[C(CH_3)(CH_2CF_3)]-(CH_2)_7-$, $-(CH_2)_2-[C(CH_3)(CH_2CF_3)]-(CH_2)_8-$, $-(CH_2)_2-[C(CH_3)(CH_2CF_3)]-(CH_2)_9-$,
$-(CH_2)_3-[C(CH_3)(CH_2CF_3)]-(CH_2)_2-$, $-(CH_2)_3-[C(CH_3)(CH_2CF_3)]-(CH_2)_4-$, $-(CH_2)_3-[C(CH_3)(CH_2CF_3)]-(CH_2)_5-$,
$-(CH_2)_3-[C(CH_3)(CH_2CF_3)]-(CH_2)_6-$, $-(CH_2)_3-[C(CH_3)(CH_2CF_3)]-(CH_2)_7-$, $-(CH_2)_3-[C(CH_3)(CH_2CF_3)]-(CH_2)_8-$,
$-(CH_2)_4-[C(CH_3)(CH_2CF_3)]-(CH_2)_2-$, $-(CH_2)_4-[C(CH_3)(CH_2CF_3)]-(CH_2)_3-$, $-(CH_2)_4-[C(CH_3)(CH_2CF_3)]-(CH_2)_5-$,
$-(CH_2)_4-[C(CH_3)(CH_2CF_3)]-(CH_2)_6-$, $-(CH_2)_4-[C(CH_3)(CH_2CF_3)]-(CH_2)_7-$, $-(CH_2)_5-[C(CH_3)(CH_2CF_3)]-(CH_2)_2-$,
$-(CH_2)_5-[C(CH_3)(CH_2CF_3)]-(CH_2)_3-$, $-(CH_2)_5-[C(CH_3)(CH_2CF_3)]-(CH_2)_4-$, $-(CH_2)_5-[C(CH_3)(CH_2CF_3)]-(CH_2)_6-$,
$-(CH_2)_6-[C(CH_3)(CH_2CF_3)]-(CH_2)_2-$, $-(CH_2)_6-[C(CH_3)(CH_2CF_3)]-(CH_2)_3-$, $-(CH_2)_6-[C(CH_3)(CH_2CF_3)]-(CH_2)_4-$,
$-(CH_2)_6-[C(CH_3)(CH_2CF_3)]-(CH_2)_5-$, $-(CH_2)-[C(CH_3)(CH_2CF_3)]_2-(CH_2)_2-$, $-(CH_2)-[C(CH_3)(CH_2CF_3)]_2-(CH_2)_3-$,
$-(CH_2)-[C(CH_3)(CH_2CF_3)]_2-(CH_2)_4-$, $-(CH_2)-[C(CH_3)(CH_2CF_3)]_2-(CH_2)_5-$, $-(CH_2)-[C(CH_3)(CH_2CF_3)]_2-(CH_2)_6-$,
$-(CH_2)-[C(CH_3)(CH_2CF_3)]_2-(CH_2)_7-$, $-(CH_2)-[C(CH_3)(CH_2CF_3)]_2-(CH_2)_8-$, $-(CH_2)-[C(CH_3)(CH_2CF_3)]_2-(CH_2)_9-$,
$-(CH_2)_2-[C(CH_3)(CH_2CF_3)]_2-(CH_2)-$, $-(CH_2)_3-[C(CH_3)(CH_2CF_3)]_2-(CH_2)-$, $-(CH_2)_4-[C(CH_3)(CH_2CF_3)]_2-(CH_2)-$,
$-(CH_2)_5-[C(CH_3)(CH_2CF_3)]_2-(CH_2)-$, $-(CH_2)_6-[C(CH_3)(CH_2CF_3)]_2-(CH_2)-$, $-(CH_2)_7-[C(CH_3)(CH_2CF_3)]_2-(CH_2)-$,
$-(CH_2)_8-[C(CH_3)(CH_2CF_3)]_2-(CH_2)-$, $-(CH_2)_9-[C(CH_3)(CH_2CF_3)]_2-(CH_2)-$, $-(CH_2)_2-[C(CH_3)(CH_2CF_3)]_2-(CH_2)_2-$,
$-(CH_2)_3-[C(CH_3)(CH_2CF_3)]_2-(CH_2)_3-$, $-(CH_2)_4-[C(CH_3)(CH_2CF_3)]_2-(CH_2)_4-$, $-(CH_2)_5-[C(CH_3)(CH_2CF_3)]_2-(CH_2)_5-$,
$-(CH_2)_2-[C(CH_3)(CH_2CF_3)]_2-(CH_2)_3-$, $-(CH_2)_2-[C(CH_3)(CH_2CF_3)]_2-(CH_2)_4-$, $-(CH_2)_2-[C(CH_3)(CH_2CF_3)]_2-(CH_2)_5-$,
$-(CH_2)_2-[C(CH_3)(CH_2CF_3)]_2-(CH_2)_6-$, $-(CH_2)_2-[C(CH_3)(CH_2CF_3)]_2-(CH_2)_7-$, $-(CH_2)_2-[C(CH_3)(CH_2CF_3)]_2-(CH_2)_8-$,
$-(CH_2)_3-[C(CH_3)(CH_2CF_3)]_2-(CH_2)_2-$, $-(CH_2)_3-[C(CH_3)(CH_2CF_3)]_2-(CH_2)_4-$, $-(CH_2)_3-[C(CH_3)(CH_2CF_3)]_2-(CH_2)_5-$,
$-(CH_2)_3-[C(CH_3)(CH_2CF_3)]_2-(CH_2)_6-$, $-(CH_2)_3-[C(CH_3)(CH_2CF_3)]_2-(CH_2)_7-$, $-(CH_2)_4-[C(CH_3)(CH_2CF_3)]_2-(CH_2)_2-$,
$-(CH_2)_4-[C(CH_3)(CH_2CF_3)]_2-(CH_2)_3-$, $-(CH_2)_4-[C(CH_3)(CH_2CF_3)]_2-(CH_2)_5-$, $-(CH_2)_4-[C(CH_3)(CH_2CF_3)]_2-(CH_2)_6-$,
$-(CH_2)_5-[C(CH_3)(CH_2CF_3)]_2-(CH_2)_2-$, $-(CH_2)_5-[C(CH_3)(CH_2CF_3)]_2-(CH_2)_3-$, $-(CH_2)_5-[C(CH_3)(CH_2CF_3)]_2-(CH_2)_4-$,
$-(CH_2)_6-[C(CH_3)(CH_2CF_3)]_2-(CH_2)_2-$, $-(CH_2)_6-[C(CH_3)(CH_2CF_3)]_2-(CH_2)_3-$, $-(CH_2)_6-[C(CH_3)(CH_2CF_3)]_2-(CH_2)_4-$,
$-(CH_2)-[C(CH_3)(CH_2CF_3)]_3-(CH_2)-$, $-(CH_2)-[C(CH_3)(CH_2CF_3)]_3-(CH_2)_2-$, $-(CH_2)-[C(CH_3)(CH_2CF_3)]_3-(CH_2)_3-$,
$-(CH_2)-[C(CH_3)(CH_2CF_3)]_3-(CH_2)_4-$, $-(CH_2)-[C(CH_3)(CH_2CF_3)]_3-(CH_2)_5-$, $-(CH_2)-[C(CH_3)(CH_2CF_3)]_3-(CH_2)_6-$,
$-(CH_2)-[C(CH_3)(CH_2CF_3)]_3-(CH_2)_7-$, $-(CH_2)-[C(CH_3)(CH_2CF_3)]_3-(CH_2)_8-$, $-(CH_2)_2-[C(CH_3)(CH_2CF_3)]_3-(CH_2)-$,
$-(CH_2)_3-[C(CH_3)(CH_2CF_3)]_3-(CH_2)-$, $-(CH_2)_4-[C(CH_3)(CH_2CF_3)]_3-(CH_2)-$, $-(CH_2)_5-[C(CH_3)(CH_2CF_3)]_3-(CH_2)-$,
$-(CH_2)_6-[C(CH_3)(CH_2CF_3)]_3-(CH_2)-$, $-(CH_2)_7-[C(CH_3)(CH_2CF_3)]_3-(CH_2)-$, $-(CH_2)_8-[C(CH_3)(CH_2CF_3)]_3-(CH_2)-$,
$-(CH_2)_2-[C(CH_3)(CH_2CF_3)]_3-(CH_2)_2-$, $-(CH_2)_3-[C(CH_3)(CH_2CF_3)]_3-(CH_2)_3-$, $-(CH_2)_4-[C(CH_3)(CH_2CF_3)]_3-(CH_2)_4-$,
$-(CH_2)_2-[C(CH_3)(CH_2CF_3)]_3-(CH_2)_3-$, $-(CH_2)_2-[C(CH_3)(CH_2CF_3)]_3-(CH_2)_4-$, $-(CH_2)_2-[C(CH_3)(CH_2CF_3)]_3-(CH_2)_5-$,
$-(CH_2)_2-[C(CH_3)(CH_2CF_3)]_3-(CH_2)_6-$, $-(CH_2)_2-[C(CH_3)(CH_2CF_3)]_3-(CH_2)_7-$, $-(CH_2)_3-[C(CH_3)(CH_2CF_3)]_3-(CH_2)_2-$,
$-(CH_2)_3-[C(CH_3)(CH_2CF_3)]_3-(CH_2)_4-$, $-(CH_2)_3-[C(CH_3)(CH_2CF_3)]_3-(CH_2)_5-$, $-(CH_2)_3-[C(CH_3)(CH_2CF_3)]_3-(CH_2)_6-$,
$-(CH_2)_4-[C(CH_3)(CH_2CF_3)]_3-(CH_2)_2-$, $-(CH_2)_4-[C(CH_3)(CH_2CF_3)]_3-(CH_2)_3-$, $-(CH_2)_4-[C(CH_3)(CH_2CF_3)]_3-(CH_2)_5-$,
$-(CH_2)_5-[C(CH_3)(CH_2CF_3)]_3-(CH_2)_2-$, $-(CH_2)_5-[C(CH_3)(CH_2CF_3)]_3-(CH_2)_3-$, $-(CH_2)_5-[C(CH_3)(CH_2CF_3)]_3-(CH_2)_4-$,
$-(CH_2)_6-[C(CH_3)(CH_2CF_3)]_3-(CH_2)_2-$, $-(CH_2)_6-[C(CH_3)(CH_2CF_3)]_3-(CH_2)_3-$,
$-[C(CH_3)(CH_2CF_3)]_4-(CH_2)-$, $-(CH_2)-[C(CH_3)(CH_2CF_3)]_4-$, $-(CH_2)-[C(CH_3)(CH_2CF_3)]_4-(CH_2)-$,
$-(CH_2)-[C(CH_3)(CH_2CF_3)]_4-(CH_2)_2-$, $-(CH_2)-[C(CH_3)(CH_2CF_3)]_4-(CH_2)_3-$, $-(CH_2)-[C(CH_3)(CH_2CF_3)]_4-(CH_2)_4-$,
$-(CH_2)-[C(CH_3)(CH_2CF_3)]_4-(CH_2)_5-$, $-(CH_2)-[C(CH_3)(CH_2CF_3)]_4-(CH_2)_6-$, $-(CH_2)-[C(CH_3)(CH_2CF_3)]_4-(CH_2)_7-$,
$-(CH_2)-[C(CH_3)(CH_2CF_3)]_4-(CH_2)_8-$, $-(CH_2)-[C(CH_3)(CH_2CF_3)]_4-(CH_2)_9-$, $-(CH_2)-[C(CH_3)(CH_2CF_3)]_4-(CH_2)_{10}-$,
$-(CH_2)_2-[C(CH_3)(CH_2CF_3)]_4-(CH_2)-$, $-(CH_2)_3-[C(CH_3)(CH_2CF_3)]_4-(CH_2)-$, $-(CH_2)_4-[C(CH_3)(CH_2CF_3)]_4-(CH_2)-$,
$-(CH_2)_5-[C(CH_3)(CH_2CF_3)]_4-(CH_2)-$, $-(CH_2)_6-[C(CH_3)(CH_2CF_3)]_4-(CH_2)-$, $-(CH_2)_7-[C(CH_3)(CH_2CF_3)]_4-(CH_2)-$,
$-(CH_2)_2-[C(CH_3)(CH_2CF_3)]_4-(CH_2)_2-$, $-(CH_2)_3-[C(CH_3)(CH_2CF_3)]_4-(CH_2)_3-$, $-(CH_2)_4-[C(CH_3)(CH_2CF_3)]_4-(CH_2)_4-$,
$-(CH_2)_5-[C(CH_3)(CH_2CF_3)]_4-(CH_2)_5-$, $-(CH_2)_2-[C(CH_3)(CH_2CF_3)]_4-(CH_2)_3-$, $-(CH_2)_2-[C(CH_3)(CH_2CF_3)]_4-(CH_2)_4-$,
$-(CH_2)_2-[C(CH_3)(CH_2CF_3)]_4-(CH_2)_5-$, $-(CH_2)_2-[C(CH_3)(CH_2CF_3)]_4-(CH_2)_6-$, $-(CH_2)_3-[C(CH_3)(CH_2CF_3)]_4-(CH_2)_2-$,
$-(CH_2)_3-[C(CH_3)(CH_2CF_3)]_4-(CH_2)_4-$, $-(CH_2)_4-[C(CH_3)(CH_2CF_3)]_4-(CH_2)_2-$, $-(CH_2)_4-[C(CH_3)(CH_2CF_3)]_4-(CH_2)_3-$,
$-(CH_2)_5-[C(CH_3)(CH_2CF_3)]_4-(CH_2)_2-$, $-(CH_2)_5-[C(CH_3)(CH_2CF_3)]_4-(CH_2)_3-$, $-(CH_2)_6-[C(CH_3)(CH_2CF_3)]_4-(CH_2)_2-$,
$-[C(CH_3)(CH_2CF_3)]_5-(CH_2)-$, $-(CH_2)-[C(CH_3)(CH_2CF_3)]_5-$, $-(CH_2)-[C(CH_3)(CH_2CF_3)]_5-(CH_2)-$,
$-(CH_2)-[C(CH_3)(CH_2CF_3)]_5-(CH_2)_2-$, $-(CH_2)-[C(CH_3)(CH_2CF_3)]_5-(CH_2)_3-$, $-(CH_2)-[C(CH_3)(CH_2CF_3)]_5-(CH_2)_4-$,
$-(CH_2)-[C(CH_3)(CH_2CF_3)]_5-(CH_2)_5-$, $-(CH_2)-[C(CH_3)(CH_2CF_3)]_5-(CH_2)_6-$, $-(CH_2)_2-[C(CH_3)(CH_2CF_3)]_5-(CH_2)-$,
$-(CH_2)_3-[C(CH_3)(CH_2CF_3)]_5-(CH_2)-$, $-(CH_2)_4-[C(CH_3)(CH_2CF_3)]_5-(CH_2)-$, $-(CH_2)_5-[C(CH_3)(CH_2CF_3)]_5-(CH_2)-$,
$-(CH_2)_6-[C(CH_3)(CH_2CF_3)]_5-(CH_2)-$, $-(CH_2)_2-[C(CH_3)(CH_2CF_3)]_5-(CH_2)_2-$, $-(CH_2)_3-[C(CH_3)(CH_2CF_3)]_5-(CH_2)_3-$,
$-(CH_2)_4-[C(CH_3)(CH_2CF_3)]_5-(CH_2)_4-$, $-(CH_2)_2-[C(CH_3)(CH_2CF_3)]_5-(CH_2)_3-$, $-(CH_2)_2-[C(CH_3)(CH_2CF_3)]_5-(CH_2)_4-$,
$-(CH_2)_2-[C(CH_3)(CH_2CF_3)]_5-(CH_2)_5-$, $-(CH_2)_2-[C(CH_3)(CH_2CF_3)]_5-(CH_2)_6-$, $-(CH_2)_3-[C(CH_3)(CH_2CF_3)]_5-(CH_2)_2-$,

$-(CH_2)_3-[C(CH_3)(CH_2CF_3)]_5-(CH_2)_4-$, $-(CH_2)_4-[C(CH_3)(CH_2CF_3)]_5-(CH_2)_2-$, $-(CH_2)_4-[C(CH_3)(CH_2CF_3)]_5-(CH_2)_3-$,
$-(CH_2)_5-[C(CH_3)(CH_2CF_3)]_5-(CH_2)_2-$, $-(CH_2)_2-(CF_2)-O-(CF_2)-(CH_2)_2-$, $-(CH_2)_2-(CF_2)-O-(CH_2)-O-(CF_2)-(CH_2)_2-$,
$-(CH_2)_2-(CF_2)-O-(CH_2)_2-O-(CF_2)-(CH_2)_2$,
$-(CH_2)-(CF_2)-O-(CF_2)-O-(CF_2)-(CH_2)-$, $-(CH_2)_2-(CF_2)-O-(CF_2)-O-(CF_2)-(CH_2)_2-$,
$-(CH_2)_3-(CF_2)-O-(CF_2)-O-(CF_2)-(CH_2)_3-$, $-(CH_2)_4-(CF_2)-O-(CF_2)-O-(CF_2)-(CH_2)_4-$,
$-(CH_2)-(CF_2)-O-(CF_2)-O-(CF_2)-(CH_2)_2-$, $-(CH_2)-(CF_2)-O-(CF_2)-O-(CF_2)-(CH_2)_3--(CH_2)-(CF_2)-O-(CF_2)-O-(CF_2)-(CH_2)_4-$,
$-(CH_2)-(CF_2)-O-(CF_2)-O-(CF_2)-(CH_2)_5-$, $-(CH_2)-(CF_2)-O-(CF_2)-O-(CF_2)-(CH_2)_6-$,
$-(CH_2)-(CF_2)-O-(CF_2)-O-(CF_2)-(CH_2)_7-$, $-(CH_2)_2-(CF_2)-O-(CF_2)-O-(CF_2)-(CH_2)-$,
$-(CH_2)_3-(CF_2)-O-(CF_2)-O-(CF_2)-(CH_2)-$, $-(CH_2)_4-(CF_2)-O-(CF_2)-O-(CF_2)-(CH_2)-$,
$-(CH_2)_5-(CF_2)-O-(CF_2)-O-(CF_2)-(CH_2)-$, $-(CH_2)_6-(CF_2)-O-(CF_2)-O-(CF_2)-(CH_2)-$,
$-(CH_2)_7-(CF_2)-O-(CF_2)-O-(CF_2)-(CH_2)-$, $-(CH_2)_2-(CF_2)-O-(CF_2)-O-(CF_2)-(CH_2)_3-$,
$-(CH_2)_2-(CF_2)-O-(CF_2)-O-(CF_2)-(CH_2)_4-$, $-(CH_2)_2-(CF_2)-O-(CF_2)-O-(CF_2)-(CH_2)_5-$,
$-(CH_2)_2-(CF_2)-O-(CF_2)-O-(CF_2)-(CH_2)_6-$, $-(CH_2)_3-(CF_2)-O-(CF_2)-O-(CF_2)-(CH_2)_2-$,
$-(CH_2)_4-(CF_2)-O-(CF_2)-O-(CF_2)-(CH_2)_2-$, $-(CH_2)_5-(CF_2)-O-(CF_2)-O-(CF_2)-(CH_2)_2-$,
$-(CH_2)_6-(CF_2)-O-(CF_2)-O-(CF_2)-(CH_2)_2-$, $-(CH_2)_3-(CF_2)-O-(CF_2)-O-(CF_2)-(CH_2)_4-$,
$-(CH_2)_4-(CF_2)-O-(CF_2)-O-(CF_2)-(CH_2)_3-$, $-(CH_2)_3-(CF_2)-O-(CF_2)-O-(CF_2)-(CH_2)_5-$,
$-(CH_2)_5-(CF_2)-O-(CF_2)-O-(CF_2)-(CH_2)_3-$, $-(CH_2)-(CF_2)-O-(CF_2)_2-O-(CF_2)-(CH_2)-$,
$-(CH_2)_2-(CF_2)-O-(CF_2)_2-O-(CF_2)-(CH_2)_2-$, $-(CH_2)_3-(CF_2)-O-(CF_2)_2-O-(CF_2)-(CH_2)_3-$,
$-(CH_2)_4-(CF_2)-O-(CF_2)_2-O-(CF_2)-(CH_2)_4-$, $-(CH_2)-(CF_2)-O-(CF_2)_2-O-(CF_2)-(CH_2)_2-$,
$-(CH_2)-(CF_2)-O-(CF_2)_2-O-(CF_2)-(CH_2)_3-$, $-(CH_2)-(CF_2)-O-(CF_2)_2-O-(CF_2)-(CH_2)_4-$,
$-(CH_2)-(CF_2)-O-(CF_2)_2-O-(CF_2)-(CH_2)_5-$, $-(CH_2)-(CF_2)-O-(CF_2)_2-O-(CF_2)-(CH_2)_6-$,
$-(CH_2)-(CF_2)-O-(CF_2)_2-O-(CF_2)-(CH_2)_7-$,$-(CH_2)_2-(CF_2)-O-(CF_2)_2-O-(CF_2)-(CH_2)-$,
$-(CH_2)_3-(CF_2)-O-(CF_2)_2-O-(CF_2)-(CH_2)--(CH_2)_4-(CF_2)-O-(CF_2)_2-O-(CF_2)-(CH_2)-$,
$-(CH_2)_5-(CF_2)-O-(CF_2)_2-O-(CF_2)-(CH_2)--(CH_2)_6-(CF_2)-O-(CF_2)_2-O-(CF_2)-(CH_2)-$,
$-(CH_2)_7-(CF_2)-O-(CF_2)_2-O-(CF_2)-(CH_2)-$, $-(CH_2)_2-(CF_2)-O-(CF_2)_2-O-(CF_2)-(CH_2)_3-$,
$-(CH_2)_2-(CF_2)-O-(CF_2)_2-O-(CF_2)-(CH_2)_4-$,$-(CH_2)_2-(CF_2)-O-(CF_2)_2-O-(CF_2)-(CH_2)_6-$,$-(CH_2)_2-(CF_2)-O-(CF_2)_2-O-(CF_2)-(CH_2)_6-$, $-(CH_2)_3-(CF_2)-O-(CF_2)_2-O-(CF_2)-(CH_2)_2-$, $-(CH_2)_4-(CF_2)-O-(CF_2)_2-O-(CF_2)-(CH_2)_2-$,
$-(CH_2)_5-(CF_2)-O-(CF_2)_2-O-(CF_2)-(CH_2)_2-$, $-(CH_2)_6-(CF_2)-O-(CF_2)_2-O-(CF_2)-(CH_2)_2-$,
$-(CH_2)_3-(CF_2)-O-(CF_2)_2-O-(CF_2)-(CH_2)_4-$, $-(CH_2)_4-(CF_2)-O-(CF_2)_2-O-(CF_2)-(CH_2)_3-$,
$-(CH_2)_3-(CF_2)-O-(CF_2)_2-O-(CF_2)-(CH_2)_6-$, $-(CH_2)_6-(CF_2)-O-(CF_2)_2-O-(CF_2)-(CH_2)_3-$,
$-(CH_2)-(CF_2)_2-O-(CF_2)_2-O-(CF_2)_2-(CH_2)-$, $-(CH_2)_2-(CF_2)_2-O-(CF_2)_2-O-(CF_2)_2-(CH_2)_2-$,
$-(CH_2)_3-(CF_2)_2-O-(CF_2)_2-O-(CF_2)_2-(CH_2)_3-$, $-(CH_2)_4-(CF_2)_2-O-(CF_2)_2-O-(CF_2)_2-(CH_2)_4-$,
$-(CH_2)-(CF_2)_2-O-(CF_2)_2-O-(CF_2)_2-(CH_2)_2-$, $-(CH_2)-(CF_2)_2-O-(CF_2)_2-O-(CF_2)_2-(CH_2)_3-$,
$-(CH_2)-(CF_2)_2-O-(CF_2)_2-O-(CF_2)_2-(CH_2)_3-$, $-(CH_2)-(CF_2)_2-O-(CF_2)_2-O-(CF_2)_2-(CH_2)_4-$,
$-(CH_2)-(CF_2)_2-O-(CF_2)_2-O-(CF_2)_2-(CH_2)_5-$, $-(CH_2)-(CF_2)_2-O-(CF_2)_2-O-(CF_2)_2-(CH_2)_6-$,
$-(CH_2)-(CF_2)_2-O-(CF_2)_2-O-(CF_2)_2-(CH_2)_7-$, $-(CH_2)_2-(CF_2)_2-O-(CF_2)_2-O-(CF_2)_2-(CH_2)-$,
$-(CH_2)_3-(CF_2)_2-O-(CF_2)_2-O-(CF_2)_2-(CH_2)-$, $-(CH_2)_4-(CF_2)_2-O-(CF_2)_2-O-(CF_2)_2-(CH_2)-$,
$-(CH_2)_5-(CF_2)_2-O-(CF_2)_2-O-(CF_2)_2-(CH_2)-$, $-(CH_2)_6-(CF_2)_2-O-(CF_2)_2-O-(CF_2)_2-(CH_2)-$,
$-(CH_2)_7-(CF_2)_2-O-(CF_2)_2-O-(CF_2)_2-(CH_2)-$, $-(CH_2)_2-(CF_2)_2-O-(CF_2)_2-O-(CF_2)_2-(CH_2)_3-$,
$-(CH_2)_2-(CF_2)_2-O-(CF_2)_2-O-(CF_2)_2-(CH_2)_4-$, $-(CH_2)_2-(CF_2)_2-O-(CF_2)_2-O-(CF_2)_2-(CH_2)_5-$,
$-(CH_2)_2-(CF_2)_2-O-(CF_2)_2-O-(CF_2)_2-(CH_2)_6-$, $-(CH_2)_3-(CF_2)_2-O-(CF_2)_2-O-(CF_2)_2-(CH_2)_2-$,
$-(CH_2)_4-(CF_2)_2-O-(CF_2)_2-O-(CF_2)_2-(CH_2)_2-$, $-(CH_2)_5-(CF_2)_2-O-(CF_2)_2-O-(CF_2)_2-(CH_2)_2-$,
$-(CH_2)_6-(CF_2)_2-O-(CF_2)_2-O-(CF_2)_2-(CH_2)_2-$, $-(CH_2)_3-(CF_2)_2-O-(CF_2)_2-O-(CF_2)_2-(CH_2)_4-$,
$-(CH_2)_4-(CF_2)_2-O-(CF_2)_2-O-(CF_2)_2-(CH_2)_3-$, $-(CH_2)_3-(CF_2)_2-O-(CF_2)_2-O-(CF_2)_2-(CH_2)_5-$,
$-(CH_2)_5-(CF_2)_2-O-(CF_2)_2-O-(CF_2)_2-(CH_2)_3-$, $-(CH_2)-(CF_2)-O-(CF_2)-O-(CF_2)-O-(CF_2)-(CH_2)-$,
$-(CH_2)_2-(CF_2)-O-(CF_2)-O-(CF_2)-O-(CF_2)-(CH_2)_2-$,$-(CH_2)_3-(CF_2)-O-(CF_2)-O-(CF_2)-O-(CF_2)-(CH_2)_3-$,
$-(CH_2)_4-(CF_2)-O-(CF_2)-O-(CF_2)-O-(CF_2)-(CH_2)_4-$, $-(CH_2)-(CF_2)-O-(CF_2)-O-(CF_2)-O-(CF_2)-(CH_2)_2-$,
$-(CH_2)-(CF_2)-O-(CF_2)-O-(CF_2)-O-(CF_2)-(CH_2)_3-$, $-(CH_2)-(CF_2)-O-(CF_2)-O-(CF_2)-O-(CF_2)-(CH_2)_4-$,
$-(CH_2)-(CF_2)-O-(CF_2)-O-(CF_2)-O-(CF_2)-(CH_2)_5-$, $-(CH_2)-(CF_2)-O-(CF_2)-O-(CF_2)-O-(CF_2)-(CH_2)_6-$,
$-(CH_2)-(CF_2)-O-(CF_2)-O-(CF_2)-O-(CF_2)-(CH_2)_7-$, $-(CH_2)_2-(CF_2)-O-(CF_2)-O-(CF_2)-O-(CF_2)-(CH_2)-$,
$-(CH_2)_3-(CF_2)-O-(CF_2)-O-(CF_2)-O-(CF_2)-(CH_2)-$, $-(CH_2)_4-(CF_2)-O-(CF_2)-O-(CF_2)-O-(CF_2)-(CH_2)-$,
$-(CH_2)_6-(CF_2)-O-(CF_2)-O-(CF_2)-O-(CF_2)-(CH_2)-$, $-(CH_2)_6-(CF_2)-O-(CF_2)-O-(CF_2)-O-(CF_2)-(CH_2)-$,
$-(CH_2)_7-(CF_2)-O-(CF_2)-O-(CF_2)-O-(CF_2)-(CH_2)-$, $-(CH_2)_2-(CF_2)-O-(CF_2)-O-(CF_2)-O-(CF_2)-(CH_2)_3-$,
$-(CH_2)_2-(CF_2)-O-(CF_2)-O-(CF_2)-O-(CF_2)-(CH_2)_4-$, $-(CH_2)_2-(CF_2)-O-(CF_2)-O-(CF_2)-O-(CF_2)-(CH_2)_5-$,
$-(CH_2)_2-(CF_2)-O-(CF_2)-O-(CF_2)-O-(CF_2)-(CH_2)_6-$, $-(CH_2)_3-(CF_2)-O-(CF_2)-O-(CF_2)-O-(CF_2)-(CH_2)_2-$,
$-(CH_2)_4-(CF_2)-O-(CF_2)-O-(CF_2)-O-(CF_2)-(CH_2)_2-$, $-(CH_2)_5-(CF_2)-O-(CF_2)-O-(CF_2)-O-(CF_2)-(CH_2)_2-$,
$-(CH_2)_6-(CF_2)-O-(CF_2)-O-(CF_2)-O-(CF_2)-(CH_2)_2-$,$-(CH_2)_3-(CF_2)-O-(CF_2)-O-(CF_2)-O-(CF_2)-(CH_2)_4-$,
$-(CH_2)_4-(CF_2)-O-(CF_2)-O-(CF_2)-O-(CF_2)-(CH_2)_3-$, $-(CH_2)_3-(CF_2)-O-(CF_2)-O-(CF_2)-O-(CF_2)-(CH_2)_5-$,

$-(CH_2)_5-(CF_2)-O-(CF_2)-O-(CF_2)-O-(CF_2)-(CH_2)_3-$, $-(CH_2)-(CF_2)_2-O-(CF_2)_2-O-(CF_2)_2-O-(CF_2)_2-(CH_2)-$, $-(CH_2)_2-(CF_2)_2-O-(CF_2)_2-O-(CF_2)_2-O-(CF_2)_2-(CH_2)_2-$, $-(CH_2)_3-(CF_2)_2-O-(CF_2)_2-O-(CF_2)_2-O-(CF_2)_2-(CH_2)_3-$, $-(CH_2)_4-(CF_2)_2-O-(CF_2)_2-O-(CF_2)_2-O-(CF_2)_2-(CH_2)_4-$, $-(CH_2)-(CF_2)_2-O-(CF_2)_2-O-(CF_2)_2-O-(CF_2)_2-(CH_2)_2-$, $-(CH_2)-(CF_2)_2-O-(CF_2)_2-O-(CF_2)_2-O-(CF_2)_2-(CH_2)_3-$, $-(CH_2)-(CF_2)_2-O-(CF_2)_2-O-(CF_2)_2-O-(CF_2)_2-(CH_2)_4-$, $-(CH_2)-(CF_2)_2-O-(CF_2)_2-O-(CF_2)_2-O-(CF_2)_2-(CH_2)_5-$, $-(CH_2)-(CF_2)_2-O-(CF_2)_2-O-(CF_2)_2-O-(CF_2)_2-(CH_2)_6-$, $-(CH_2)-(CF_2)_2-O-(CF_2)_2-O-(CF_2)_2-O-(CF_2)_2-(CH_2)_7-$, $-(CH_2)_2-(CF_2)_2-O-(CF_2)_2-O-(CF_2)_2-O-(CF_2)_2-(CH_2)-$, $-(CH_2)_3-(CF_2)_2-O-(CF_2)_2-O-(CF_2)_2-O-(CF_2)_2-(CH_2)-$, $-(CH_2)_4-(CF_2)_2-O-(CF_2)_2-O-(CF_2)_2-O-(CF_2)_2-(CH_2)-$, $-(CH_2)_5-(CF_2)_2-O-(CF_2)_2-O-(CF_2)_2-O-(CF_2)_2-(CH_2)-$, $-(CH_2)_6-(CF_2)_2-O-(CF_2)_2-O-(CF_2)_2-O-(CF_2)_2-(CH_2)-$, $-(CH_2)_7-(CF_2)_2-O-(CF_2)_2-O-(CF_2)_2-O-(CF_2)_2-(CH_2)-$, $-(CH_2)_2-(CF_2)_2-O-(CF_2)_2-O-(CF_2)_2-O-(CF_2)_2-(CH_2)_3-$, $-(CH_2)_2-(CF_2)_2-O-(CF_2)_2-O-(CF_2)_2-O-(CF_2)_2-(CH_2)_4-$, $-(CH_2)_2-(CF_2)_2-O-(CF_2)_2-O-(CF_2)_2-O-(CF_2)_2-(CH_2)_5-$, $-(CH_2)_2-(CF_2)_2-O-(CF_2)_2-O-(CF_2)_2-O-(CF_2)_2-(CH_2)_6-$, $-(CH_2)_3-(CF_2)_2-O-(CF_2)_2-O-(CF_2)_2-O-(CF_2)_2-(CH_2)_2-$, $-(CH_2)_4-(CF_2)_2-O-(CF_2)_2-O-(CF_2)_2-O-(CF_2)_2-(CH_2)_2-$, $-(CH_2)_5-(CF_2)_2-O-(CF_2)_2-O-(CF_2)_2-O-(CF_2)_2-(CH_2)_2-$, $-(CH_2)_6-(CF_2)_2-O-(CF_2)_2-O-(CF_2)_2-O-(CF_2)_2-(CH_2)_2-$, $-(CH_2)_3-(CF_2)_2-O-(CF_2)_2-O-(CF_2)_2-O-(CF_2)_2-(CH_2)_4-$, $-(CH_2)_4-(CF_2)_2-O-(CF_2)_2-O-(CF_2)_2-O-(CF_2)_2-(CH_2)_3-$, $-(CH_2)_3-(CF_2)_2-O-(CF_2)_2-O-(CF_2)_2-O-(CF_2)_2-(CH_2)_5-$, $-(CH_2)5-(CF_2)_2-O-(CF_2)_2-O-(CF_2)_2-O-(CF_2)_2-(CH_2)_3-$ .

Preferred examples for $-R_2-$ are $-(CH_2)_5-$, $-(CH_2)_6-$, $-(CH_2)_7-$, $-(CH_2)_8-$, $-(CH_2)_9-$,$-(CH_2)_{10}-$, $-(CH_2)_{11}-$, $-(CH_2)_{12}-$, $-(CH_2)_{13}-$, $-(CH(C_2H_5)-(CH_2)_3-$, $-(CH(C_2H_5)-(CH_2)_4-$,$-(CH(C_2H_5)-(CH_2)_7-$, $-(CH_2)_2-S-(CH_2)_2-$, $-(CH_2)_2-SO_2-(CH_2)_2-$, $-(CH_2)_4-SO_2-(CH_2)_4-$,$-(CH_2)_2-O-(CH_2)_2-$, $-(CH_2)_3-O-(CH_2)_3-$, $-(CH_2)_3-S-(CH_2)_3-$, $-(CH_2)_2-S-(CH_2)_2-S-(CH_2)_2-$, $-(CH_2)_2-O-(CH_2)_2-O-(CH_2)_2-$,$-(CH_2)_2-S-(CH_2)_2-O-(CH_2)_2-$,$-(CH_2)_2-O-(CH_2)_2-S-(CH_2)_2-$, $-(CH_2)_2-O-(CH_2)_2-SO_2-(CH_2)_2-$, $-(CH_2)_2-SO_2-(CH_2)_2-O-(CH_2)_2-$, $-(CH_2)_2-SO_2-(CH_2)_2-S-(CH_2)_2-$, $-(CH_2)_2-O-(CH_2)_2-O-(CH_2)_2-O-(CH_2)_2-$, $-(CH_2)_2-O-(CH_2)_2-S-(CH_2)_2-O-(CH_2)_2-$, $-(CH_2)_2-S-(CH_2)_2-O-(CH_2)_2-S-(CH_2)_2-$, $-(CH_2)_2-SO_2-(CH_2)_2-O-(CH_2)_2-SO_2-(CH_2)_2-$, $-(CH_2)_2-S-(CH_2)_2-S-(CH_2)_2-S-(CH_2)_2-$, $-(CH_2)_2-SO_2-(CH_2)_2-S-(CH_2)_2-SO_2-(CH_2)_2-$, $-(CH_2)_2-SO_2-(CH_2)_2-SO_2-(CH_2)_2-SO_2-(CH_2)_2-$, $-(CH_2)_2-O-(CH_2)_2-SO_2-(CH_2)_2-O-(CH_2)_2-$, $-(CH_2)_3-(CF_2)-(CH_2)_3-$, $-(CH_2)_2-(CF_2)-(CH_2)_2-$, $-(CH_2)-(CF_2)_3-(CH_2)-$, $-(CH_2)_2-(CF_2)_4-(CH_2)_2-$, $-(CH_2)_3-(CHCF_3)-(CH_2)_3-$,$-(CH_2)_2-(CF_2)-O-(CF_2)-O-(CF_2)-(CH_2)_2-$ and $-(CH_2)-(CF_2)-O-(CF_2)-O-(CF_2)-O-(CF_2)-(CH_2)-$ according to the invention.

Particularly preferred examples for $-R_2-$ are $-(CH_2)_5-$, $-(CH_2)_6-$, $-(CH_2)_7-$, $-(CH_2)_8-$,$-(CH_2)_9-$, $-(CH_2)_{10}-$, $-(CH_2)_{11}-$, $-(CH_2)_{12}-$, $-(CH(C_2H_5)-(CH_2)_3-$, $-(CH(C_2H_5)-(CH_2)_4-$,$-(CH_2)_2-O-(CH_2)_2-$, $-(CH_2)_3-O-(CH_2)_3-$, $-(CH_2)_2-O-(CH_2)_2-O-(CH_2)_2-$, $-(CH_2)_2-S-(CH_2)_2-$, $-(CH_2)_3-S-(CH_2)_3-$, $-(CH_2)_2-S-(CH_2)_2-S-(CH_2)_2-$, $-(CH_2)_2-SO_2-(CH_2)_2-$, $-(CH_2)_2-S-(CH_2)_2-O-(CH_2)_2-$, $-(CH_2)_2-O-(CH_2)_2-S-(CH_2)_2-$, $-(CH_2)_2-O-(CH_2)_2-SO_2-(CH_2)_2-$,$-(CH_2)-S-(CH_2)_2-O-(CH_2)_2-S-(CH_2)-$, $-(CH_2)_2-O-(CH_2)_2-S-(CH_2)_2-O-(CH_2)_2-$, $-(CH_2)_2-O-(CH_2)_2-O-(CH_2)_2-O-(CH_2)_2-$, $-(CH_2)_2-(CF_2)-(CH_2)_2-$, $-(CH_2)_3-(CF_2)-(CH_2)_3-$, $-(CH_2)_3-(CHCF_3)-(CH_2)_3-$ according to the invention.

Compounds of formulae (I), (Ia), (Ib) and (Ic) with substituents as described before or preferably described before having a polymerizable group as described before or preferably described before or below are preferred in case the substituent $-R_2-$within the at least one linking element $Y-R_2-$ corresponds to $-(C(R)_2)_o-$, wherein R and o has a meaning as described or preferably described before.

Accordingly, monomers of formulae (I), (Ia), (Ib) and (Ic) for the preparation of an ophthalmic device or precursor article for an ophthalmic device as described before with substituents as described before or preferably described before having a polymerizable group as described before or preferably described before or below are preferred in case the substituent $-R_2-$ within the at least one linking element $Y-R_2-$ corresponds to $-(C(R)_2)_o-$, wherein R and o has a meaning as described or preferably described before. Such ophthalmic devices and precursor articles prepared by using these monomers are especially preferred.

Particularly preferred examples for $-R_2-$ are $-(CH_2)_5-$, $-(CH_2)_6-$, $-(CH_2)_7-$, $-(CH_2)_8-$,$-(CH_2)_9-$, $-(CH_2)_{10}-$, $-(CH_2)_{11}-$, $-(CH_2)_{12}-$, $-(CH_2)_{13}-$, $-(CH(C_2H_5)-(CH_2)_4-$and $-(CH(C_2H_5)-(CH_2)_7-$ according to the invention. Very particularly preferably,$-R_2-$ is $-(CH_2)_7-$, $-(CH_2)_8-$ or $-(CH_2)_9-$ according to the invention.

Therefore, the invention is furthermore directed to an ophthalmic device or precursor article for an ophthalmic device comprising polymerized compounds of formulae (I), (Ia), (Ib) or (Ic) as described before or preferably described before wherein $-R_2-$ is at each occurrence independently $-(C(R)_2)_o-$, wherein R and o have a meaning as described or preferably described before.

The invention therefore relates to compounds of formulae (I), (Ia), (Ib) or (Ic) as described before or preferably described before wherein $-R_2-$ is at each occurrence independently $-(C(R)_2)_o-$, wherein R and o have a meaning as preferably described before.

The substituent $Y-R_2-$ within formulae (I), (Ia), (Ib) and (Ic) is selected from the group consisting of $O-R_2-$, $S-R_2-$, $SO-R_2-$, $SO_2-R_2-$, $Se-R_2-$, $NR_0-R_2-$ and $-R_2-$ where Y is a bond and wherein $-R_2-$ has a meaning as described before or preferably or particularly preferably described before.

The substituent $Y-R_2-$ is preferably selected from the group consisting of $O-R_2-$, $S-R_2-$, $SO-R_2-$, $NR_0-R_2-$ and $-R_2-$ where Y is a bond and wherein $-R_2-$ has a meaning as described before or preferably or particularly preferably described before.

The substituent Y-$R_2$- is preferably selected from the group consisting of O-$R_2$-, S-$R_2$- and $NR_0$-$R_2$- and -$R_2$- where Y is a bond and wherein -$R_2$- has a meaning as described before or preferably or particularly preferably described before.

The substituent Y-$R_2$- is preferably selected from the group consisting of O-$R_2$- and $NR_0$-$R_2$- and wherein -$R_2$- has a meaning as described before or preferably or particularly preferably described before.

The substituent Y-$R_2$- is preferably selected from the group consisting of S-$R_2$- and -$R_2$- has a meaning as described before or preferably or particularly preferably described before.

The substituent Y-$R_2$-$R_1$ within formulae (I), (Ia), (Ib) and (Ic) is selected from the group consisting of O-$R_2$-$R_1$, S-$R_2$-$R_1$, SO-$R_2$-$R_1$, SO-$R_2$-$R_1$, Se-$R_2$-$R_1$, $NR_0$-$R_2$-$R_1$ and -$R_2$-$R_1$, or very preferably selected from the group consisting of O-$R_2$-$R_1$, S-$R_2$-$R_1$ and $NR_0$-$R_2$-$R_1$, wherein -$R_2$- has a meaning as described before or preferably or particularly preferably described before and wherein $R_1$ is preferably trimethoxysilyl, triethoxysilyl, dimethoxymethylsilyl, diethoxymethylsilyl or a polymerizable group according to formula (4),

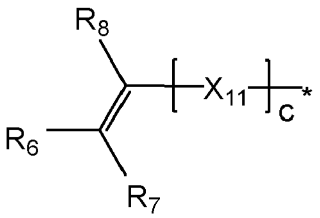

(4)

,

wherein

| | |
|---|---|
| $X_{11}$ | is selected from the group consisting of O, S, O-$SO_2$, $SO_2$-O, C(=O), OC(=O), C(=O)O, S(C=O) and (C=O)S, |
| $R_6$, $R_7$, $R_8$ | are at each occurrence independently of each other selected from the group consisting of H, F, a linear or branched, non-fluorinated, partially or completely fluorinated alkyl group having 1 to 20 C atoms and aryl with 6 to 14 C atoms and |
| c | is 0 or 1. |

In another preferred embodiment of the invention, c, $X_{11}$, $R_6$, $R_7$ and $R_8$ within the compounds of formulae (I), (Ia), (Ib) and (Ic) acting as monomers for the preparation of the ophthalmic device or precursor article of the ophthalmic device as described before or for the preparation of an oligomer, polymer or copolymer according to the invention or within the compounds according to the invention have the following preferred meaning:

Preferably, $R_6$ and $R_7$ are H. Preferably, c is 1.

Preferably, $R_8$ is H, methyl, ethyl or phenyl. Particularly preferably, $R_8$ is H or methyl.

Preferably, $X_{11}$ is C(=O), OC(=O), C(=O)O or SC(=O). Particularly preferably, $X_{11}$ is C(=O)O.

Preferred alkenyl groups of formula (4) as polymerizable groups $R_1$ according to the invention are therefore represented by any one selected from the group consisting of formulae (4-1), (4-2), (4-3), (4-4), (4-5), (4-6), (4-7), (4-8), (4-9), (4-10), (4-11) and (4-12):

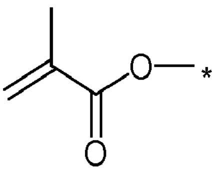

(4-1) ,

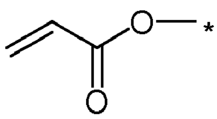

(4-2) ,

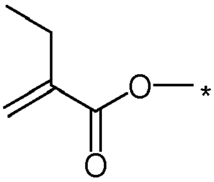

(4-3) ,

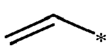

(4-4) ,

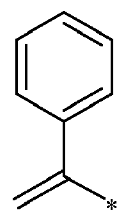

(4-5) ,

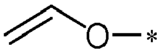 (4-6), 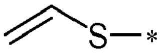 (4-7), 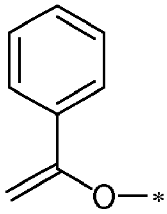 (4-8)

,

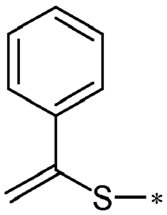 (4-9), 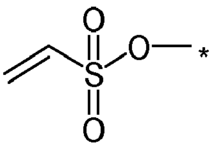 (4-10),

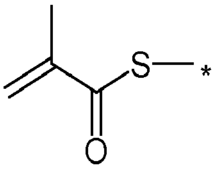 (4-11), 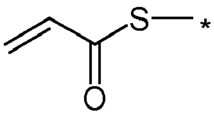 (4-12).

Particularly preferred alkenyl groups of formula (4) as polymerizable groups $R_1$ according to the invention are represented by any one selected from the group consisting of formulae (4-1), (4-2), (4-3), (4-5), (4-6), (4-8), (4-9), (4-11) and (4-12) as described before.

Particularly preferred alkenyl groups of formula (4) as polymerizable groups $R_1$ according to the invention are represented by any one selected from the group consisting of formulae (4-1), (4-2), (4-3), (4-5), (4-6), (4-11) and (4-12) as described before.

The alkenyl group represented by formula (4-1) is called methacrylate. The alkenyl group represented by formula (4-2) is called acrylate.

The preferred groups $R_1$ are preferably combined with preferred groups of the linking element $-R_2-$ and/or the linking element $Y-R_2-$. Combinations are excluded where two O atoms or one O atom and one S atom are directly bonded to each other as known for a skilled artisan in the field of organic chemistry.

Particularly preferably, the compounds of formulae (I), (Ia), (Ib) and (Ic) comprise a polymerizable group $R_1$ which is represented by formulae (4-1), (4-2), (4-5), (4-6), (4-8), (4-9), (4-11) and (4-12).

Particularly preferably, the compounds of formulae (I), (Ia), (Ib) and (Ic) comprise a polymerizable group $R_1$ which is represented by formulae (4-1), (4-2), (4-5), (4-6), (4-11) and (4-12).

Very particularly preferably, the compounds of formulae (I), (Ia), (Ib) and (Ic) comprise a polymerizable group $R_1$ which is a methacryl or an acryl group represented by formula (4-1) and (4-2).

The invention therefore relates further to an ophthalmic device or a precursor article for an ophthalmic device comprising polymerized compounds of formulae (I), (Ia), (Ib) and/or (Ic) as described before or preferably described before wherein $R_1$ is at each occurrence independently an acryl or methacryl group.

The invention therefore relates further to compounds of formulae (I), (Ia), (Ib) and/or (Ic) as described before or preferably described before wherein $R_1$ is at each occurrence independently an acryl or methacryl group.

Examples for compounds/monomers of formulae (I), (Ia), (Ib) and/or (Ic) are the following compounds (A-001) to (A-106) as shown in table 1.

Table 1:

| | |
|---|---|
| 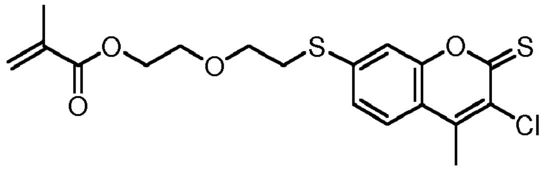 | A-001 |
| 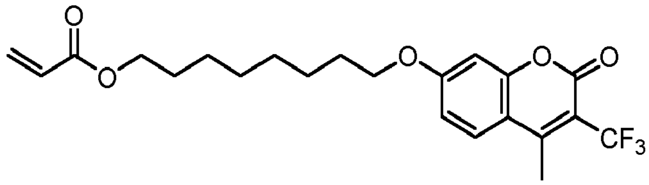 | A-002 |
| 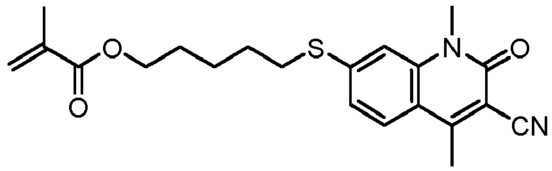 | A-003 |
| 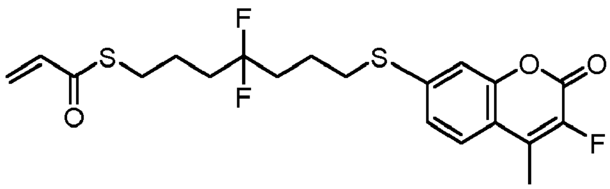 | A-004 |
| 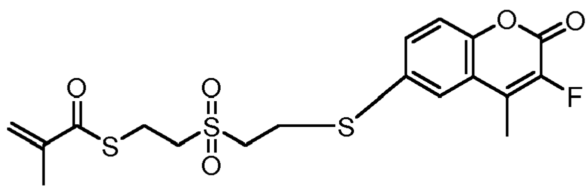 | A-005 |
| 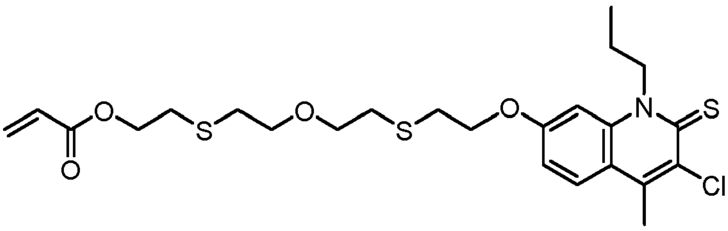 | A-006 |
| 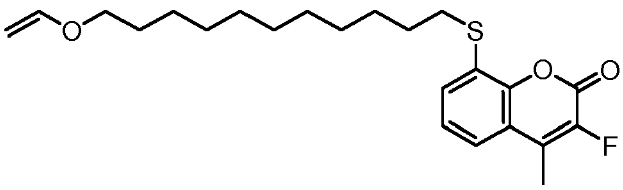 | A-007 |
| 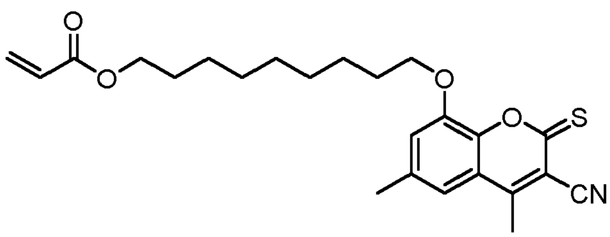 | A-008 |

(continued)

| | |
|---|---|
| 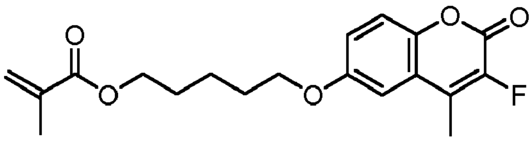 | A-009 |
| 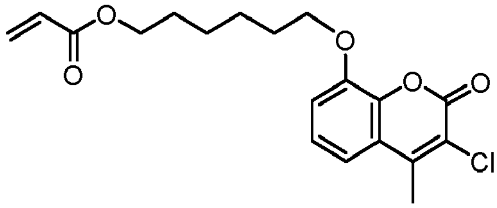 | A-010 |
| 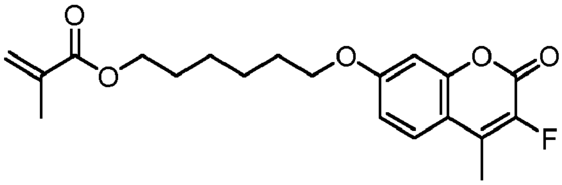 | A-011 |
| 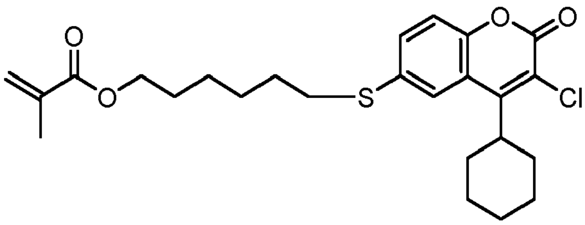 | A-012 |
| 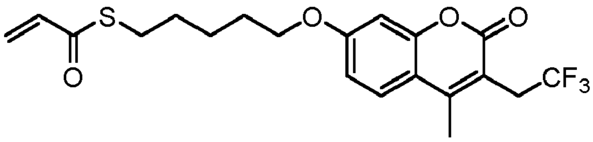 | A-013 |
| 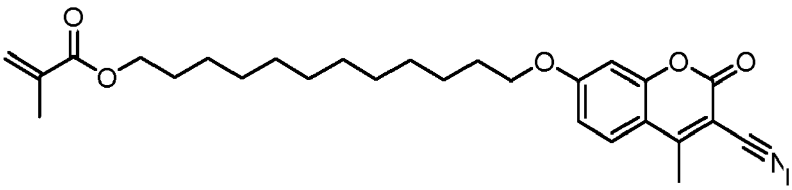 | A-014 |
| 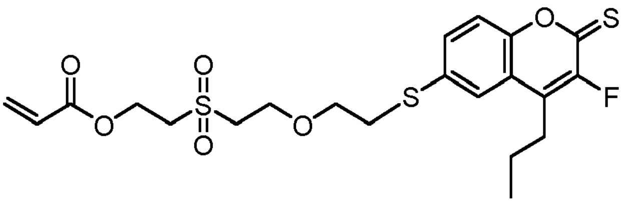 | A-015 |
| 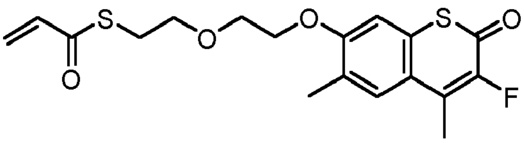 | A-016 |

(continued)

| | |
|---|---|
| 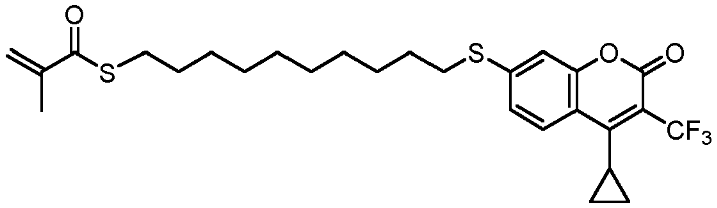 | A-017 |
| 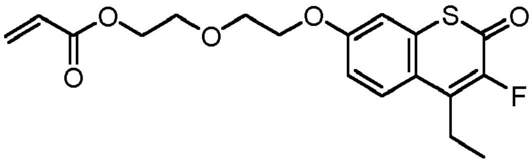 | A-018 |
| 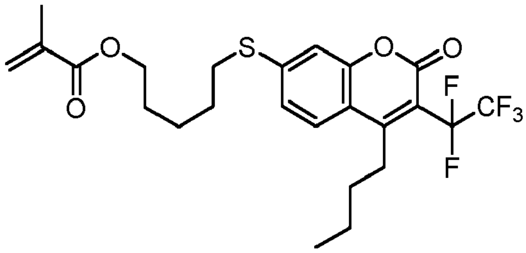 | A-019 |
| 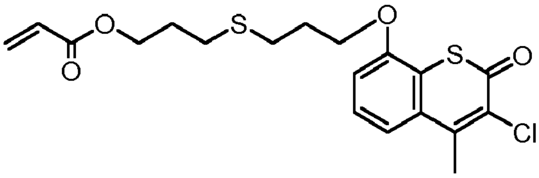 | A-020 |
| 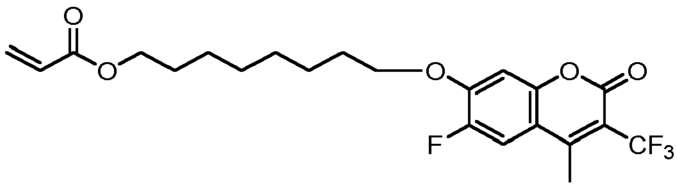 | A-021 |
| 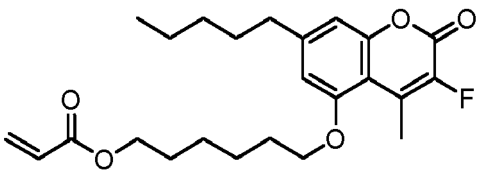 | A-022 |
| 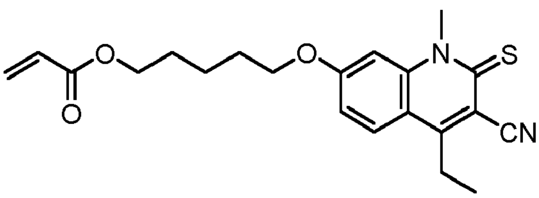 | A-023 |

(continued)

| | |
|---|---|
| 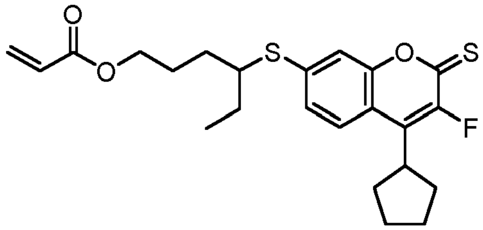 | A-024 |
| 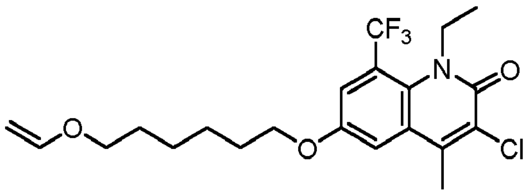 | A-025 |
| 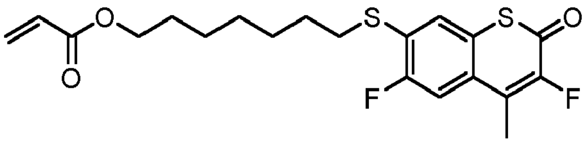 | A-026 |
| 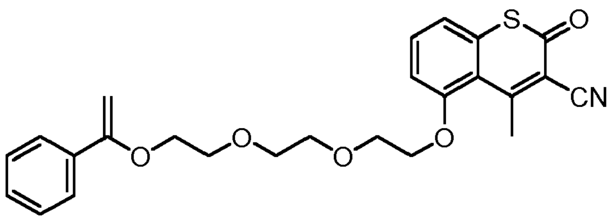 | A-027 |
| 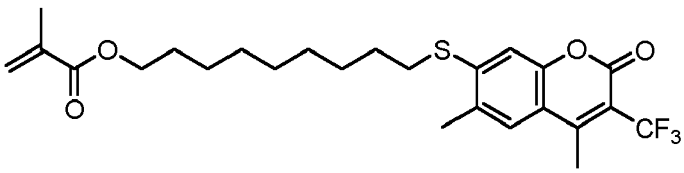 | A-028 |
| 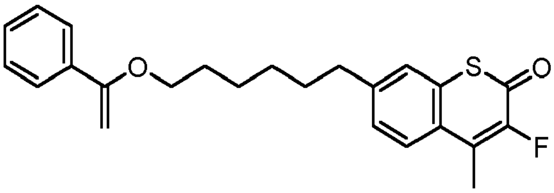 | A-029 |
| 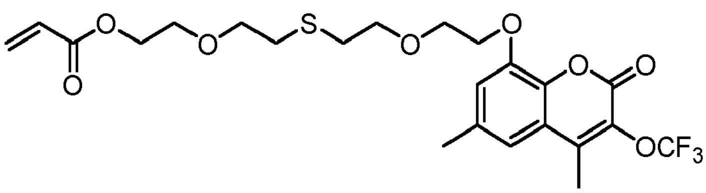 | A-030 |
| 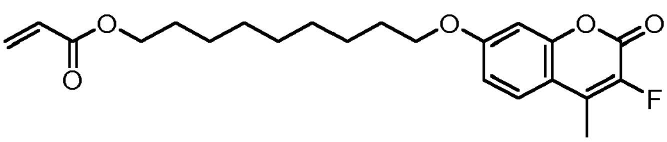 | A-031 |

(continued)

| | |
|---|---|
| 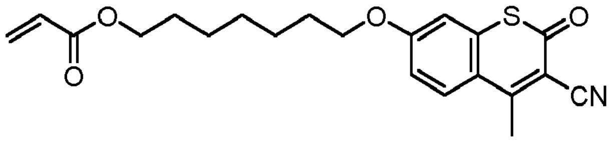 | A-032 |
| 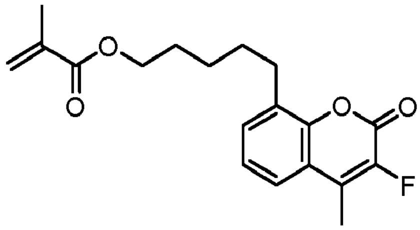 | A-033 |
| 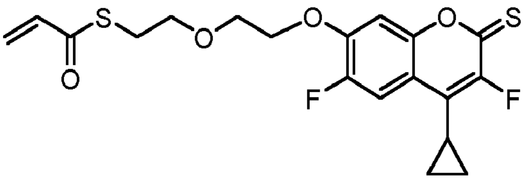 | A-034 |
| 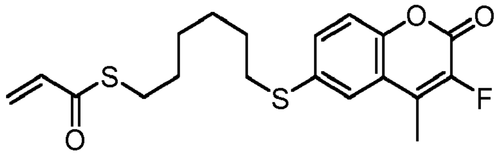 | A-035 |
| 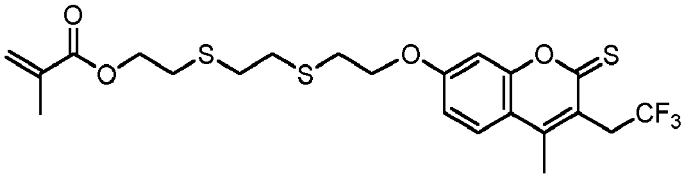 | A-036 |
| 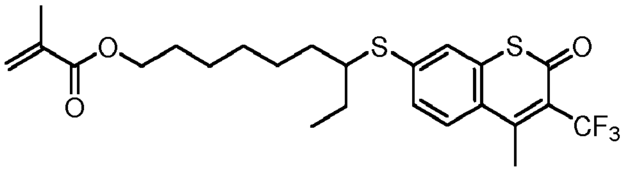 | A-037 |
| 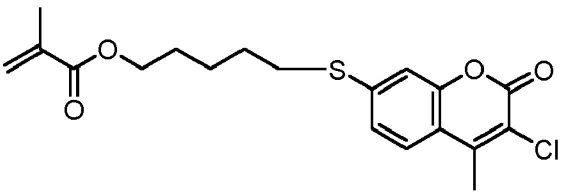 | A-038 |
| 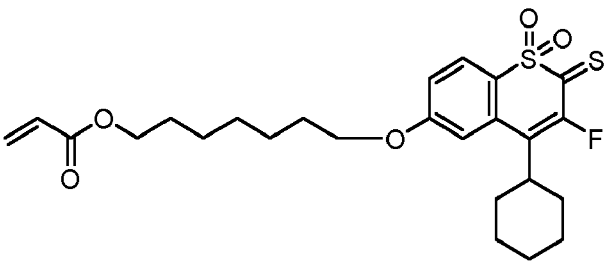 | A-039 |

(continued)

| | |
|---|---|
| 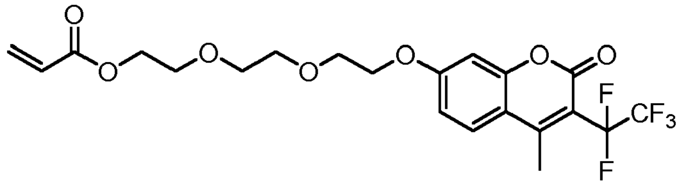 | A-040 |
| 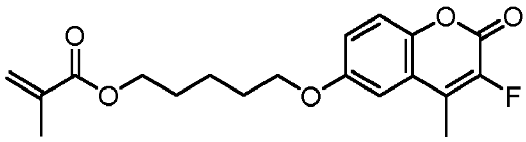 | A-041 |
| 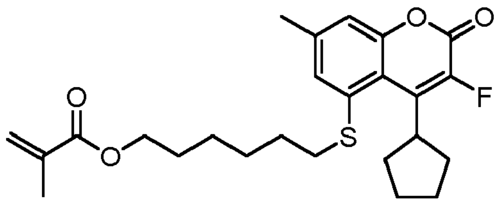 | A-042 |
| 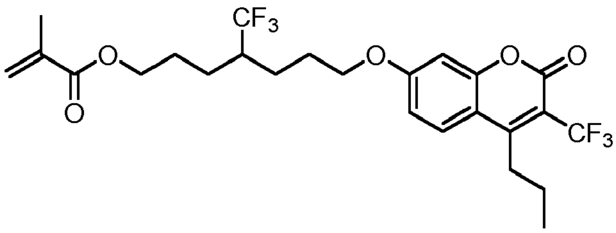 | A-043 |
| 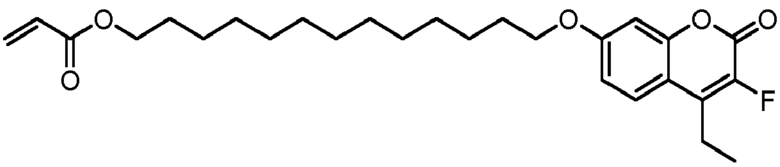 | A-044 |
| 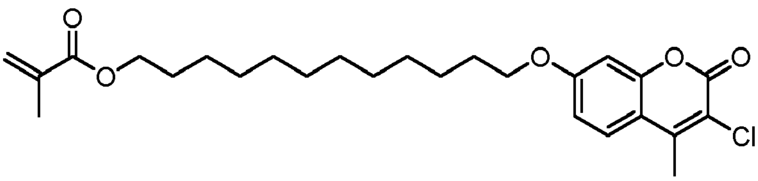 | A-045 |
| 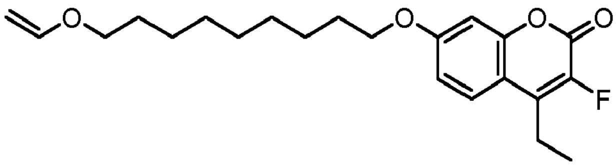 | A-046 |
| 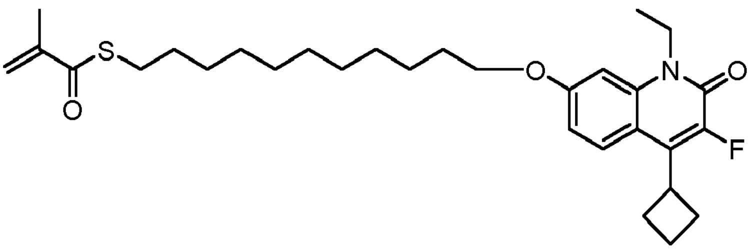 | A-047 |

(continued)

| | |
|---|---|
| 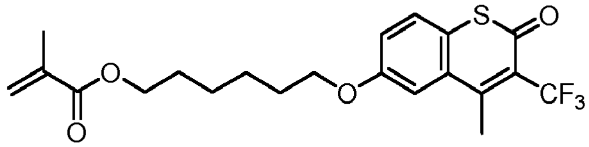 | A-048 |
| 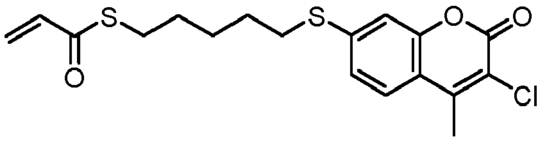 | A-049 |
| 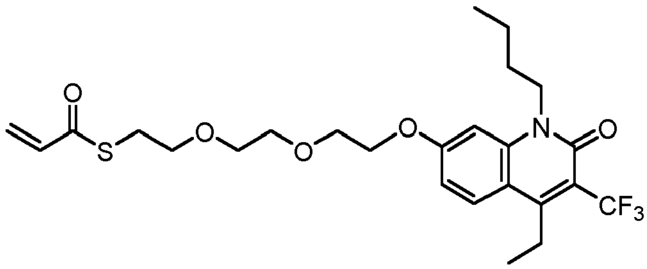 | A-050 |
| 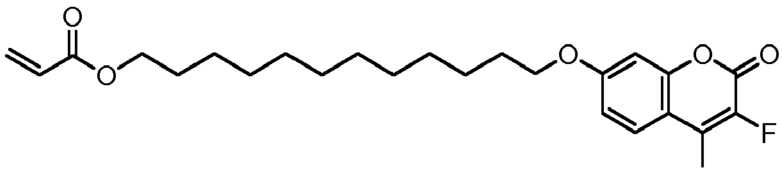 | A-051 |
| 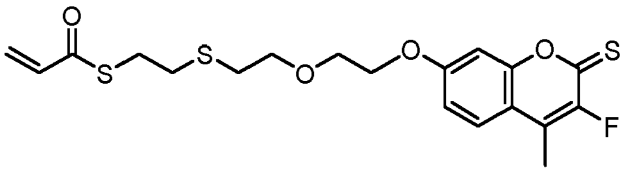 | A-052 |
| 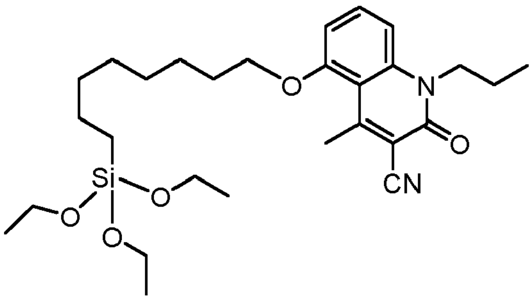 | A-053 |
| 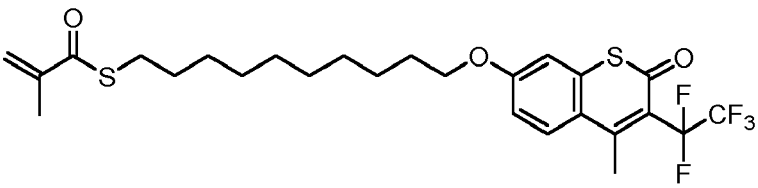 | A-054 |
| 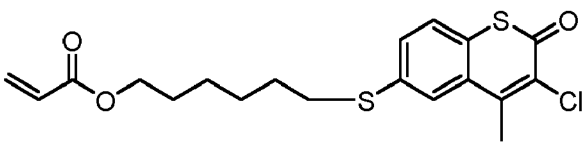 | A-055 |

(continued)

| | |
|---|---|
| 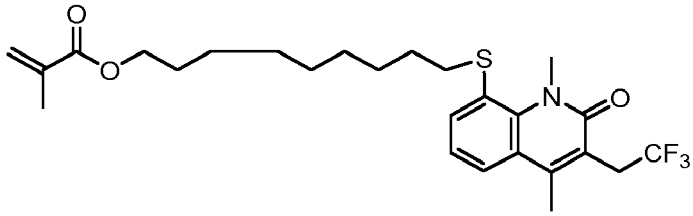 | A-056 |
| 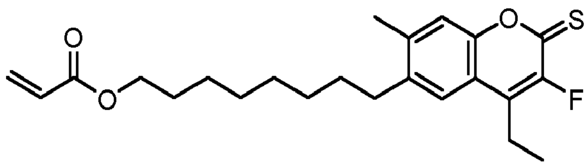 | A-057 |
| 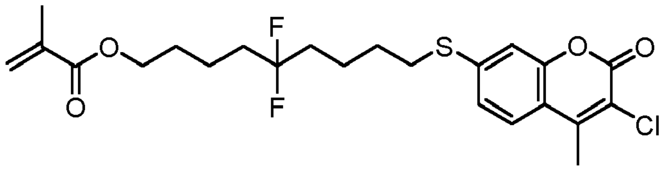 | A-058 |
| 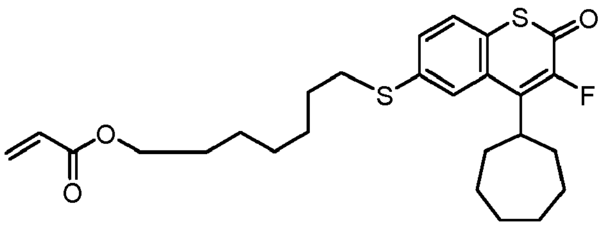 | A-059 |
| 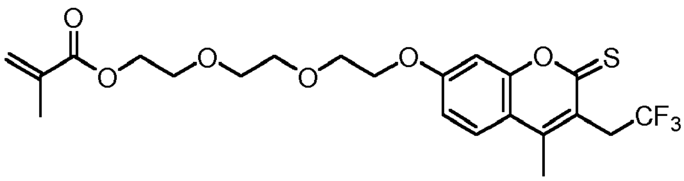 | A-060 |
| 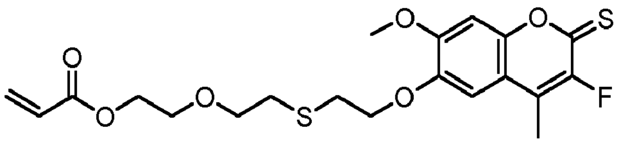 | A-061 |
| 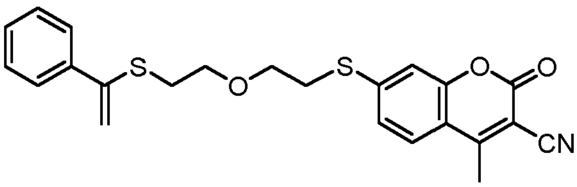 | A-062 |
| 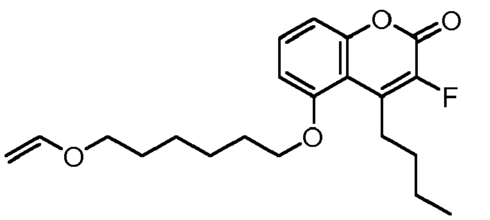 | A-063 |

(continued)

| | |
|---|---|
| 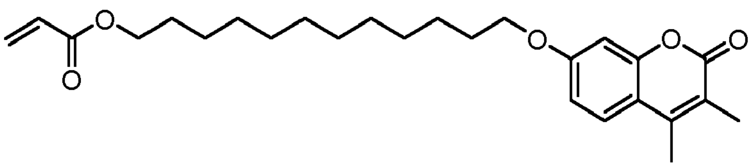 | A-064 |
| 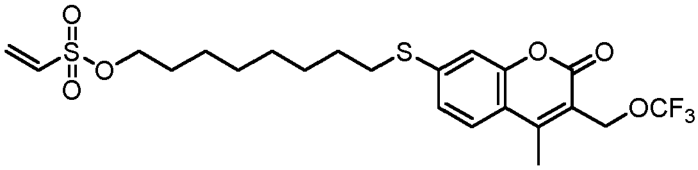 | A-065 |
| 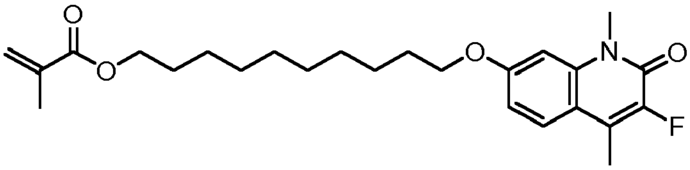 | A-066 |
| 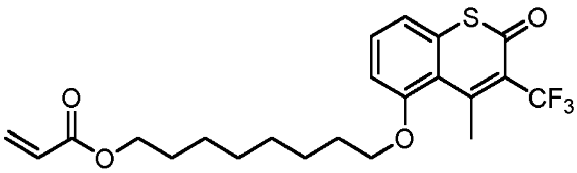 | A-067 |
| 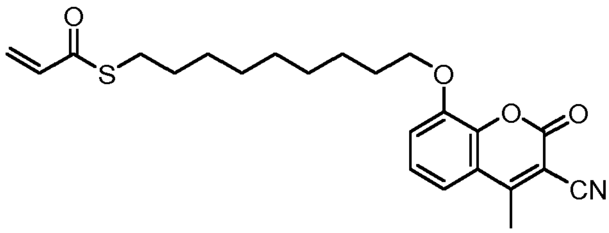 | A-068 |
| 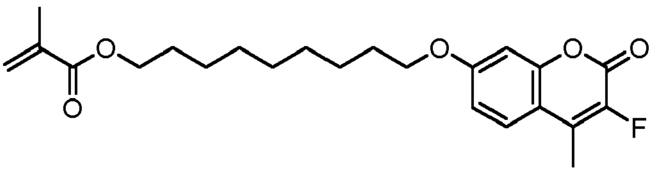 | A-069 |
| 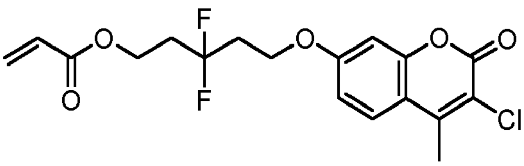 | A-070 |
| 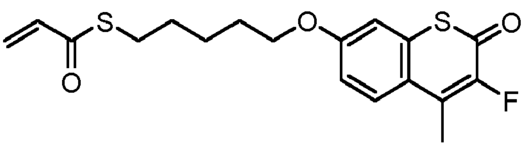 | A-071 |

(continued)

| | |
|---|---|
| 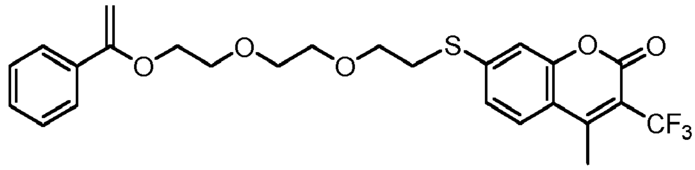 | A-072 |
| 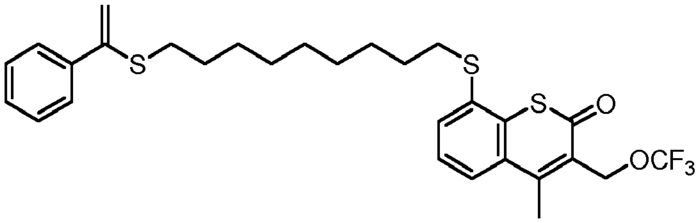 | A-073 |
| 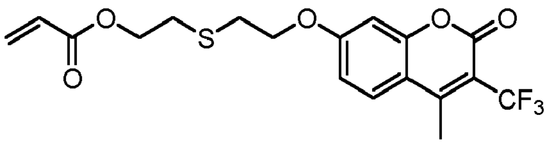 | A-074 |
| 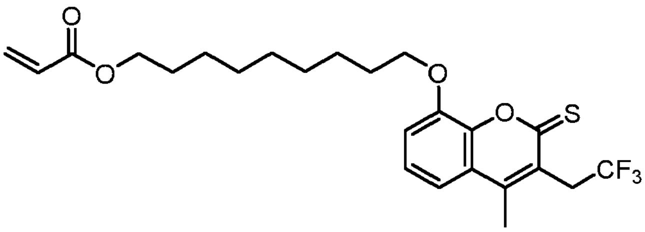 | A-075 |
| 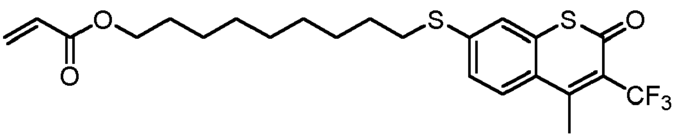 | A-076 |
| 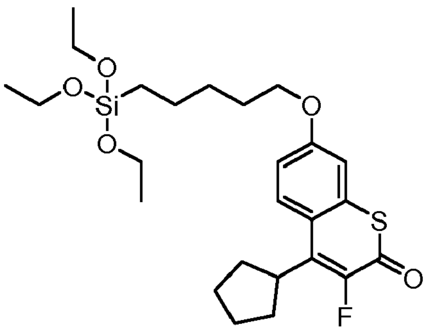 | A-077 |
| 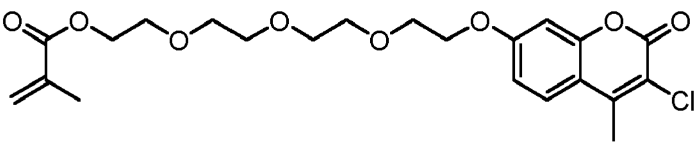 | A-078 |

(continued)

| | |
|---|---|
| 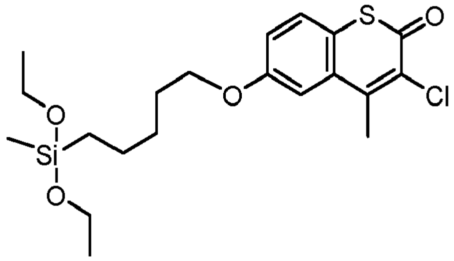 | A-079 |
| 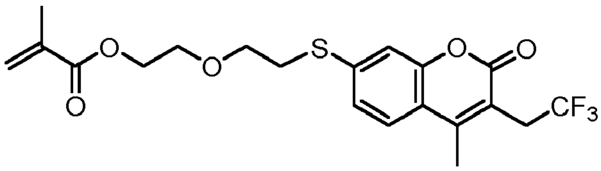 | A-080 |
| 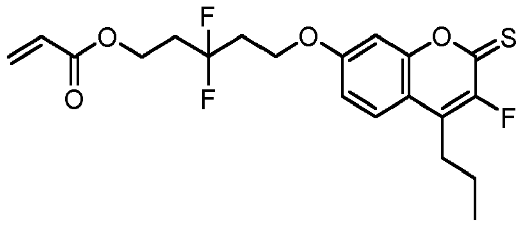 | A-081 |
| 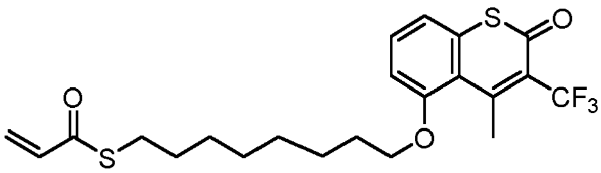 | A-082 |
| 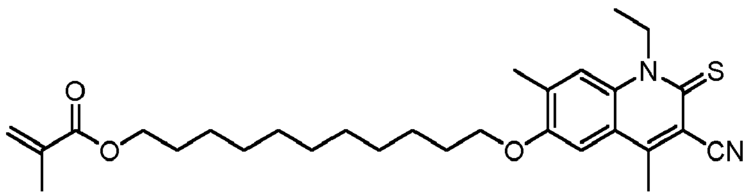 | A-083 |
| 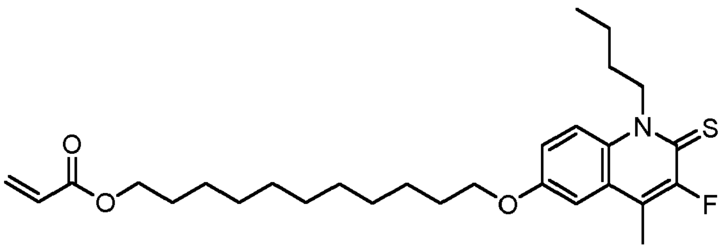 | A-084 |
| 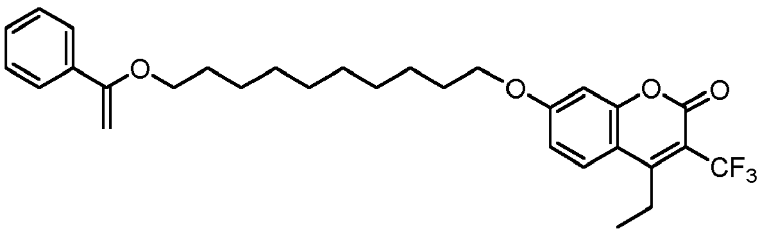 | A-085 |

(continued)

| | |
|---|---|
| 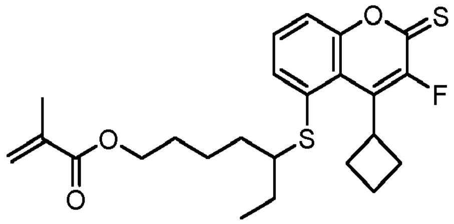 | A-086 |
| 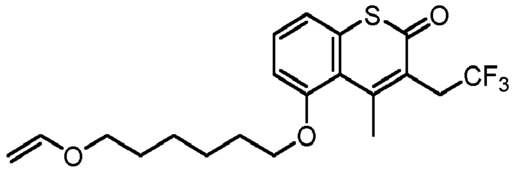 | A-087 |
| 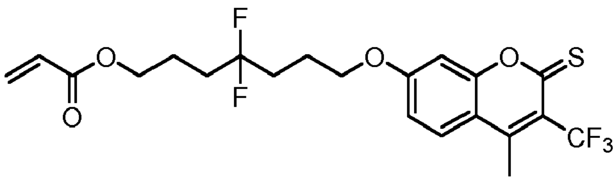 | A-088 |
| 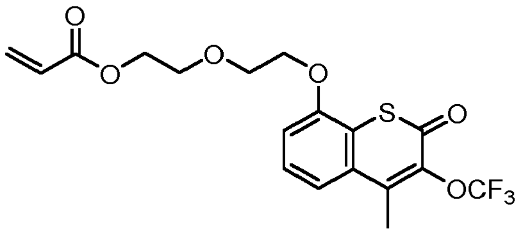 | A-089 |
| 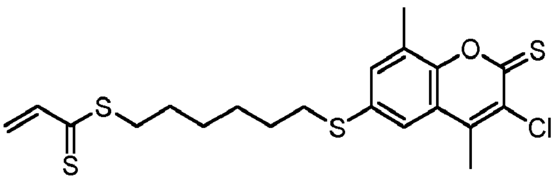 | A-090 |
| 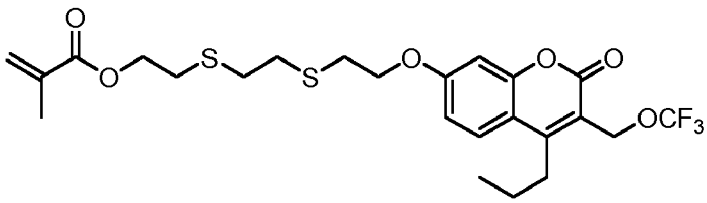 | A-091 |
| 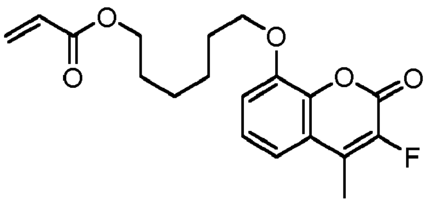 | A-092 |
| 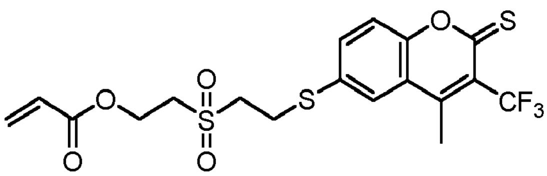 | A-093 |

(continued)

| | |
|---|---|
| 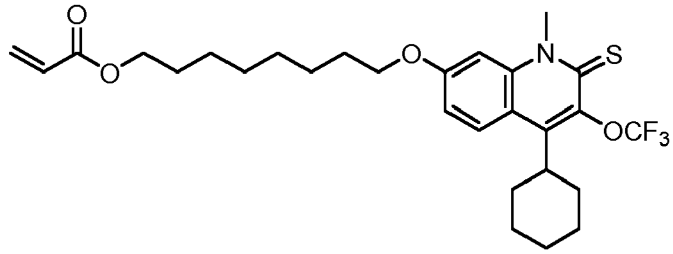 | A-094 |
| 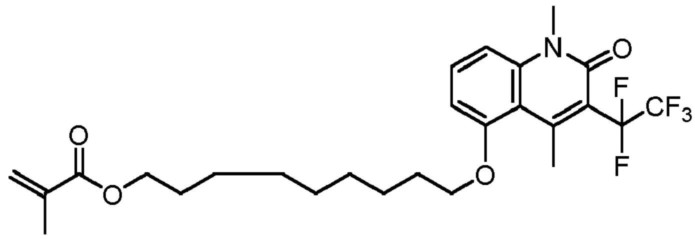 | A-095 |
| 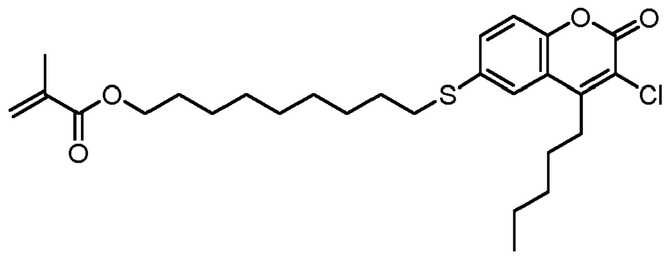 | A-096 |
| 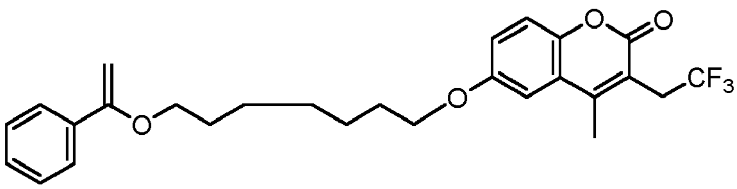 | A-097 |
| 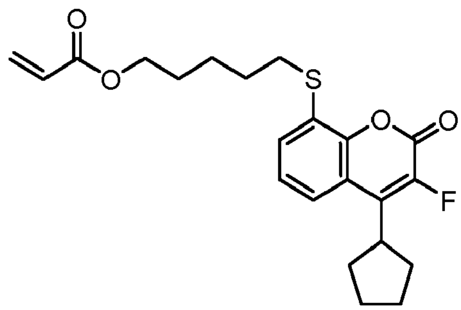 | A-098 |
| 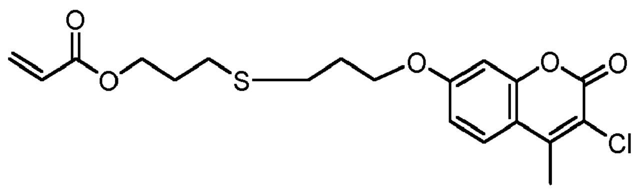 | A-099 |

(continued)

| | |
|---|---|
| 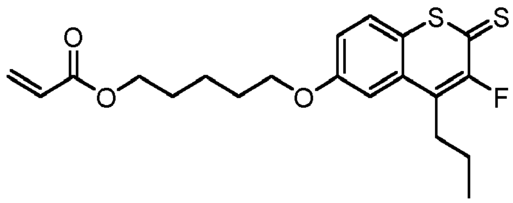 | A-0100 |
| 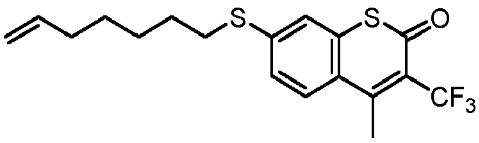 | A-101 |
| 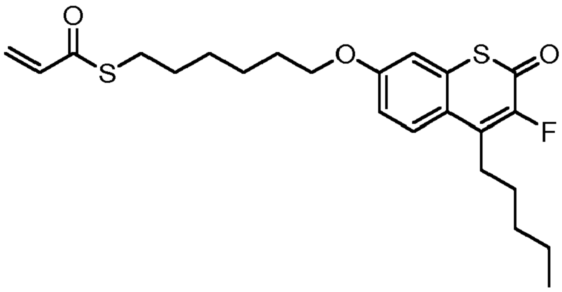 | A-102 |
| 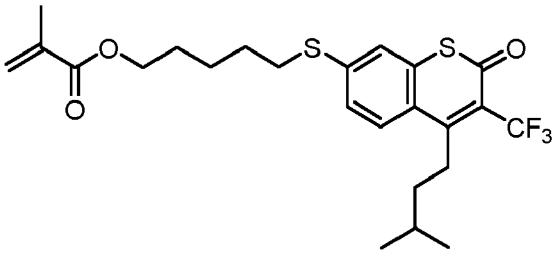 | A-103 |
| 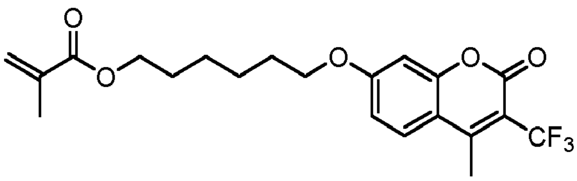 | A-104 |
| 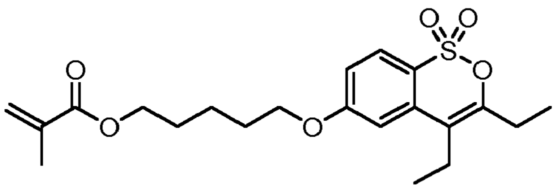 | A-105 |
| 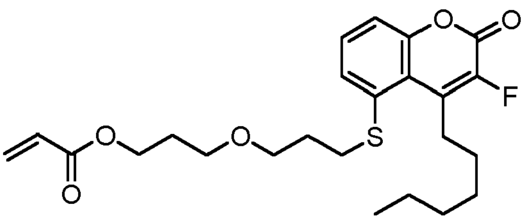 | A-106 |

Parts of the compounds of the present application may be synthesized by methods well known to the skilled person. Preferably, all syntheses are carried out under an inert atmosphere using dried solvents.

An exemplary reaction sequence is shown in Scheme 1 for the compounds of formula (I) where X-W is O-C(=O), Y is O and all further symbols and indices have a meaning as described before and the polymerizable group $R_1$ is as shown in scheme 1.

Scheme 1:

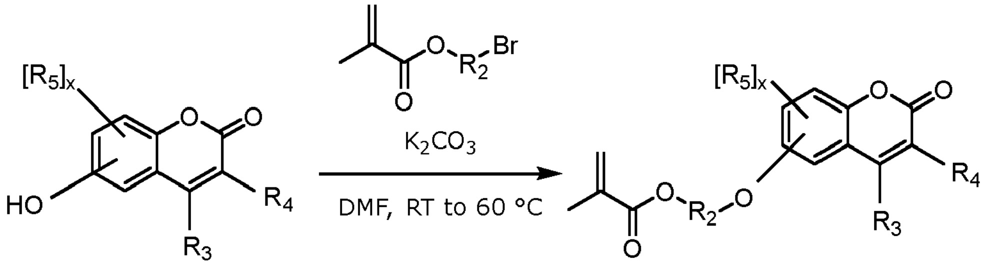


The first type of reaction is a Williamson ether synthesis.
An exemplary reaction sequence is shown in Scheme 2 for the compounds of formula (I) where X-W is O-C(=O), Y is S and all further symbols and indices have a meaning as described before and the polymerizable group $R_1$ is as shown in scheme 2. DMAP means 4-N,N-dimethylaminopyridine.

Scheme 2:

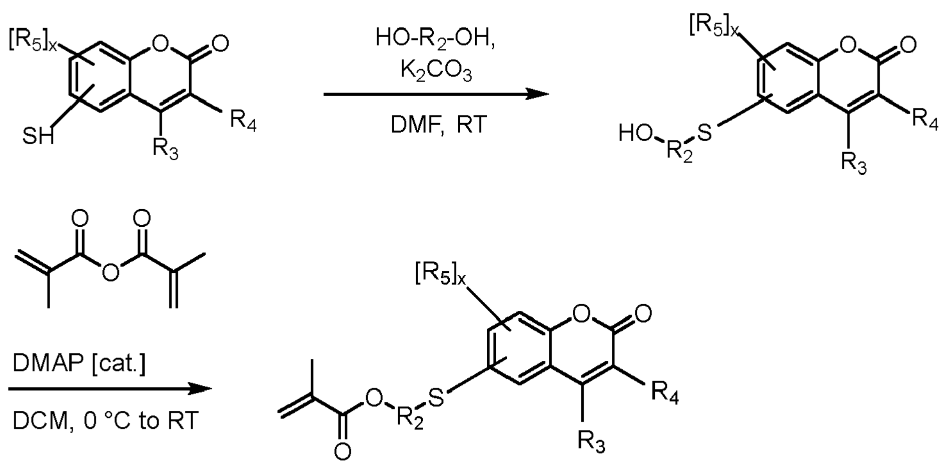


The first type of reaction is a Williamson thioether synthesis. The second type of reaction is an esterification reaction.
An exemplary reaction sequence is shown in Scheme 3 for the compounds of formula (I) where X-W is $NR_B$-C(=O), $R_B$ is methyl, Y is S and all further symbols and indices have a meaning as described before and the polymerizable group $R_1$ is as shown in scheme 3.

Scheme 3:

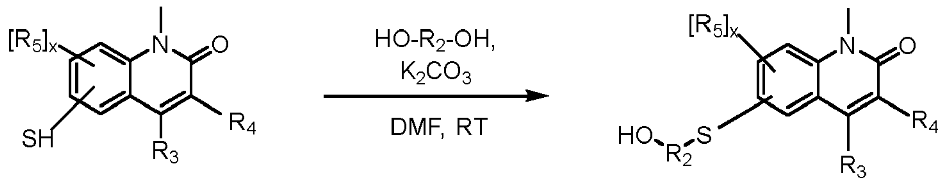

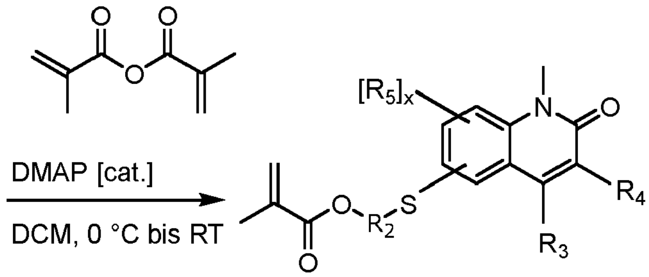


The first type of reaction is a Williamson thioether synthesis. The second type of reaction is an esterification reaction.

An exemplary reaction sequence is shown in Scheme 4 for the compounds of formula (I) where X-W is O-C(=O), Y is $NR_0$, $R_0$ is methyl and all further symbols and indices have a meaning as described before and the polymerizable group $R_1$ is as shown in scheme 4.

Scheme 4:

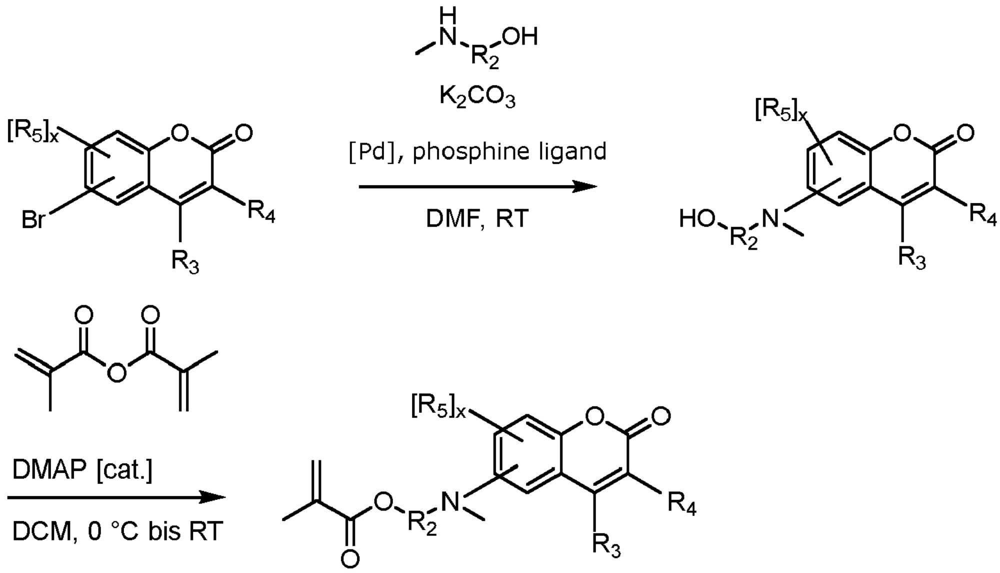


The first type of reaction is a Buchwald-Hartwig amination reaction. The second type of reaction is an esterification reaction.

An exemplary reaction sequence is shown in Scheme 5 for the compounds of formula (I) where X-W is $-SO_2$-O, Y is O and all further symbols and indices have a meaning as described before and the polymerizable group $R_1$ is as shown in scheme 5.

Scheme 5:

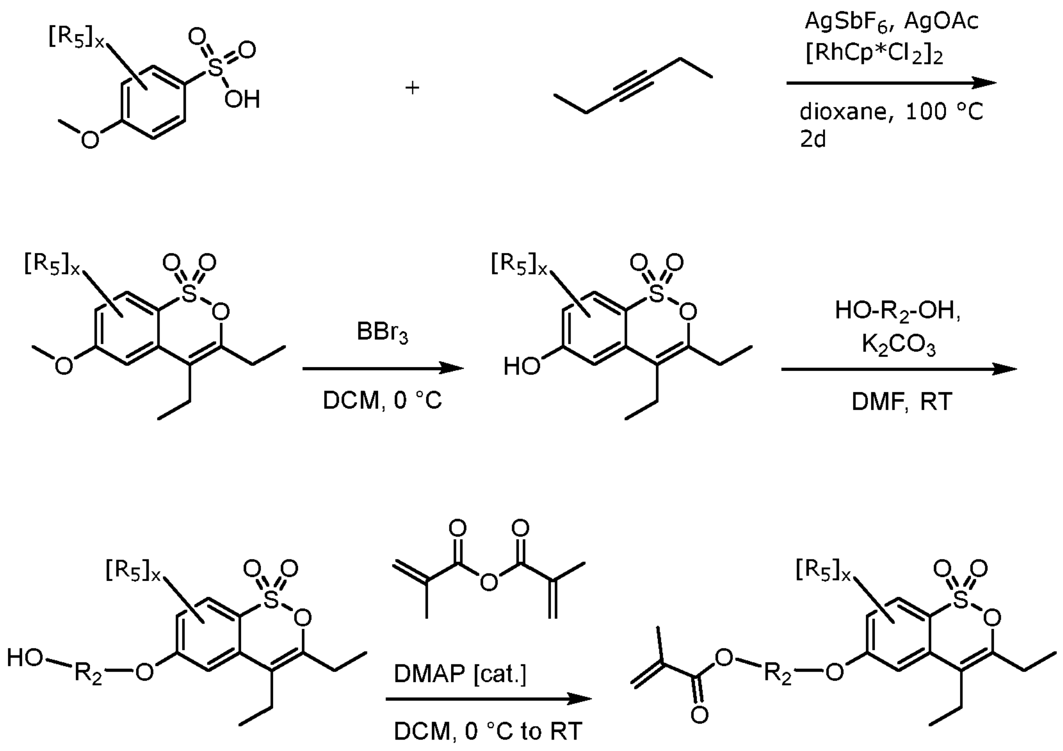


The first type of reaction is a rhodium-catalyzed hydroalkynylation reaction.
The second type of reaction is a methoxy ether deprotection reaction.
The third type of reaction is Williamson ether synthesis.
The fourth type of reaction is an esterification reaction.

As described before, the compounds/monomers of formulae (I), (Ia), (Ib) and (Ic) as described before or preferably described before contain a polymerizable group and are predestinated as monomers for an oligomerization or a polymerization. The resulting oligomers, polymers or copolymers are advantageous materials being at least part of the ophthalmic device or precursor article for an ophthalmic device or building the material for the ophthalmic device or precursor article for the ophthalmic device according to the invention.

The term "polymer" generally means a molecule of high relative molecular mass, the structure of which essentially comprises the multiple repetition of units derived, actually or conceptually, from molecules of low relative molecular mass (PAC, 1996, 68, 2291). The term "polymer" includes homopolymers and copolymers if not mentioned otherwise within the description. The term "oligomer" generally means a molecule of intermediate relative molecular mass, the structure of which essentially comprises a small plurality of units derived, actually or conceptually, from molecules of lower relative molecular mass (PAC, 1996, 68, 2291). In a preferred sense according to the present invention a polymer means a compound having ≥ 30 repeating units, and an oligomer means a compound with > 1 and < 30 repeating units.

Above and below, in formulae showing a polymer, an oligomer, a compound of formulae (I), (Ia), (Ib) or (Ic) or a monomeric unit or a polymer formed from a compound of formulae (I), (Ia), (Ib) or (Ic), an asterisk ("*") denotes a linkage to the adjacent repeating unit in the polymer chain or oligomer chain or to a terminal end group.

Suitable terminal end groups are known to the skilled artisan and depend on the polymerization method used.

The terms "repeating unit" and "monomeric unit" mean the constitutional repeating unit (CRU), which is the smallest constitutional unit the repetition of which constitutes a regular macromolecule, a regular oligomer molecule, a regular block or a regular chain (PAC, 1996, 68, 2291).

Unless stated otherwise, the molecular weight is given as the number average molecular weight $M_n$ or weight average molecular weight Mw, which is determined by gel permeation chromatography (GPC) against polystyrene standards in eluent solvents such as tetrahydrofuran, trichloromethane (TCM, chloroform), chlorobenzene or 1,2,4-trichloro-benzene. Unless stated otherwise, tetrahydrofuran is used as solvent. The degree of polymerization (n) means the number average degree of polymerization given as $n = M_n/M_U$, wherein Mu is the molecular weight of the single repeating unit as described in J. M. G. Cowie, Polymers: Chemistry & Physics of Modern Materials, Blackie, Glasgow, 1991.

In the polymers or copolymers according to the present invention as described below or within the ophthalmic

device or precursor material for an ophthalmic device according to the invention, the total number of repeating units n is preferably ≥ 30, very preferably ≥ 100, most preferably ≥ 200, and preferably up to 5000, very preferably up to 3000, most preferably up to 2000, including any combination of the aforementioned lower and upper limits of n.

The invention is therefore further directed to an oligomer, polymer or copolymer comprising at least one polymerized compound of formula (I)

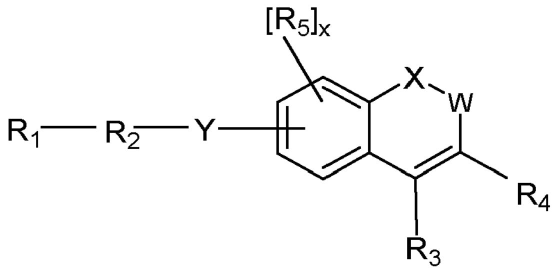

(I)

,

wherein

X-W is O-C(=O), O-C(=S), O-$SO_2$, $SO_2$-O, $SO_2$-C(=S), S-C(=O), S-C(=S), $NR_B$-C(=O) or $NR_B$-C(=S);

$R_B$ is at each occurrence independently H, a linear or branched alkyl group having 1 to 6 C atoms or a linear or branched partially or fully fluorinated alkyl group having 1 to 10 C atoms;

$R_3$ is a linear or branched alkyl group having 1 to 8 C atoms or a cycloalkyl group having 3 to 7 C atoms;

$R_4$ is F, Cl, a linear or branched alkyl group having 1 to 8 C atoms or a partially or fully fluorinated or partially or fully chlorinated alkyl group having 1 to 8 C atoms, a linear or branched partially or fully fluorinated alkoxy group having 1 to 8 C atoms, a linear or branched optionally partially or fully fluorinated alkoxyalkyl group having 2 to 8 C atoms or a cycloalkyl group having 3 to 6 C atoms which may be partially or fully fluorinated;

$R_5$ is at each occurrence independently a linear or branched alkyl group having 1 to 8 C atoms or a linear or branched alkoxy group having 1 to 8 C atoms;

x is 0 or 1;

Y is O, S, SO, $SO_2$, Se, $NR_0$ or a bond;

$R_0$ is at each occurrence independently selected from the group consisting of a linear or branched alkyl group having 1 to 4 C atoms;

$R_1$ is a trialkoxysilyl group or a dialkoxyalkylsilyl group where the alkyl and/or alkoxy groups are each independently linear or branched having 1 to 6 C atoms, or a silyl group of formula (1), (2) or (3) or a polymerizable group of formula (4),

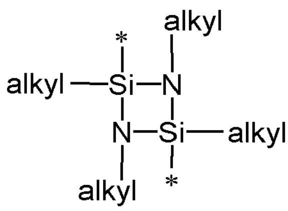

(1)

,

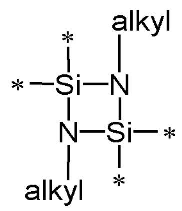

(2)

,

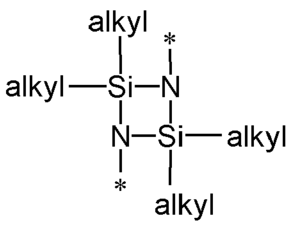

(3)

,

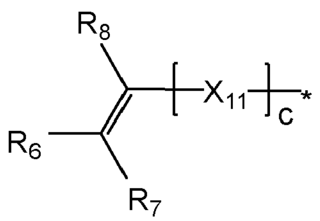

(4)

,

where alkyl means at each occurrence independently of each other a linear or branched alkyl group having 1 to 6 C atoms and the asterisk "*" denotes at each occurrence independently of each other a linkage to the linker -$R_2$-Y-;

and wherein

$X_{11}$ is selected from the group consisting of O, S, O-$SO_2$, $SO_2$-O, C(=O), OC(=O), C(=O)O, S(C=O) and (C=O)S, $R_6$, $R_7$, $R_8$ are at each occurrence independently of each other selected from the group consisting of H, F, a linear or branched, non-fluorinated, partially or completely fluorinated alkyl group having 1 to 20 C atoms and aryl with 6 to 14 C atoms and

c is 0 or 1;

-$R_2$- is -$(C(R)_2)_o$-;

R is at each occurrence independently selected from the group consisting of H, F, a linear or branched alkyl group having 1 to 4 C atoms or a linear or branched partially or fully fluorinated alkyl group having 1 to 4 C atoms; and

o is selected from the group consisting of 5 to 20 or comprising polymerized compounds of formulae (I), (Ia), (Ib) or (Ic) with substituents as described before or preferably described before.

The polymers within the ophthalmic device or precursor material for an ophthalmic device according to the invention and/or the polymers of the present invention include homopolymers, statistical copolymers, random copolymers, alternating copolymers and block copolymers, and combinations of the aforementioned. The polymers within the ophthalmic device or precursor material for an ophthalmic device according to the invention and/or the polymers of the present invention are preferably random copolymers.

Throughout the description and claims of this specification, the words "comprise" and "contain" and variations of the words, for example "comprising" and "comprises", mean "including but not limited to", and are not intended to (and do not) exclude other components.

Preferably, the polymers within the ophthalmic device or precursor material for an ophthalmic device according to the invention and/or the oligomers, polymers or copolymers according to the invention comprise a constitutional unit $M^0$ based on formulae (I), (Ia), (Ib) or (Ic),

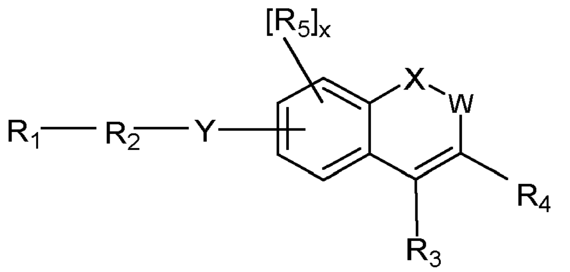

(I)

,

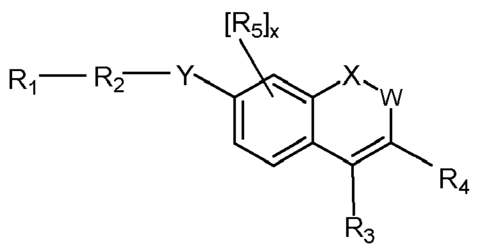

(Ia)

,

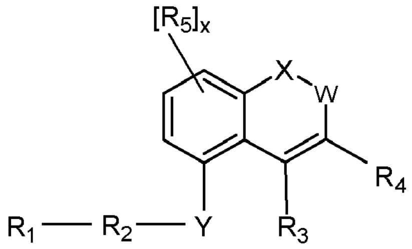

(Ib)

,

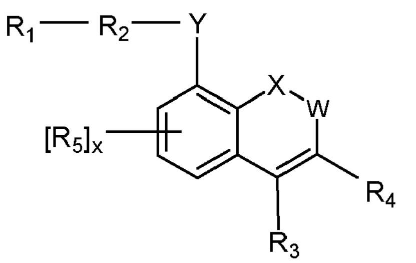

(Ic)

,

where the polymerizable group $R_1$ on each occurrence is polymerized and forms the regioregular, alternated, regiorandom, statistical, block or random oligomer or polymer backbone or is part of the copolymer backbone and where all the symbols and indices used within the formulae (I), (Ia), (Ib) and (Ic) have a meaning as described before or preferably

described before.

Preferably the polymerizable group $R_1$ forms the regioregular, alternated, regiorandom, statistical, block or random homopolymer or copolymer backbone or is part of the polymer backbone where $R_1$ has a meaning as described or preferably described before.

The invention is furthermore directed to an ophthalmic device or a precursor article for an ophthalmic device as described before or preferably described below comprising an oligomer, polymer or copolymer comprising a constitutional unit $M^0$ based on formulae (I), (Ia), (Ib) or (Ic) as described before or preferably described before where $R_1$ on each occurrence is polymerized and forms the regioregular, alternated, regiorandom, statistical, block or random oligomer or polymer backbone or is part of the copolymer backbone.

Preferably, such polymerized groups $R_1$ are of formulae (1-p), (2-p), (3-p) or (4-p)

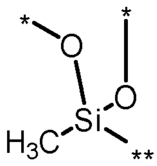
(1-p) ,

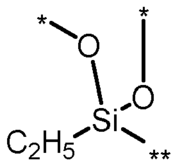
(2-p) ,

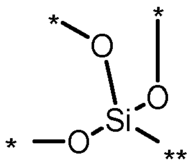
(3-p) ,

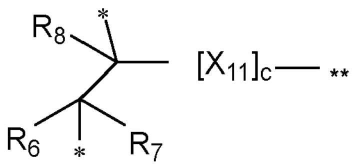
(4-p) ,

where the asterisk "*" within formulae (1-p) to (4-p) denotes a linkage to the adjacent repeating unit in the polymer chain or oligomer chain or to a terminal end group, the asterisk "**" within formulae (1-p) to (4-p) denotes the linkage to the remainder of formula (I) as described before or preferably described before and $R_6$, $R_7$, $R_8$, $X_{11}$ and c have a meaning as described before or preferably described before.

The invention is furthermore directed to an ophthalmic device or a precursor article for an ophthalmic device as described before or preferably described below where said polymerized group $R_1$ is of formulae (1-p), (2-p), (3-p) or (4-p) as described before.

The invention is furthermore directed to an oligomer, polymer or copolymer with susymbols and indices as described before or preferably described below where said polymerized group $R_1$ is of formulae (1-p), (2-p), (3-p) or (4-p) as described before.

Particularly preferably, such polymers within the ophthalmic device or precursor material for an ophthalmic device according to the invention and/or such oligomers, polymers or copolymers according to the invention comprise a constitutional unit $M^0$ of formulae ($M^0$-I), ($M^0$-Ia), ($M^0$-Ib) or ($M^0$-Ic),

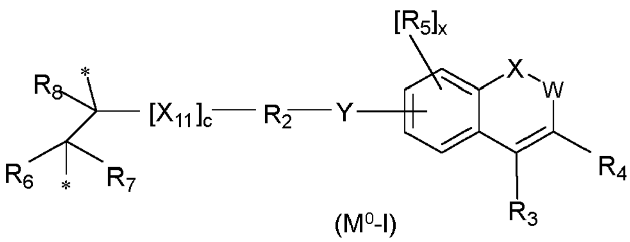
($M^0$-I) ,

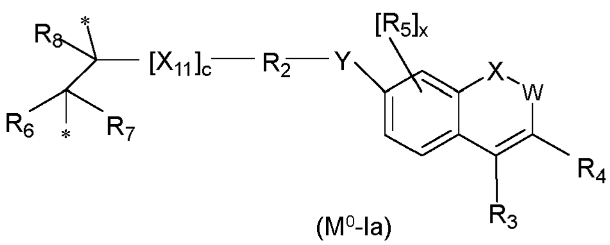
($M^0$-Ia) ,

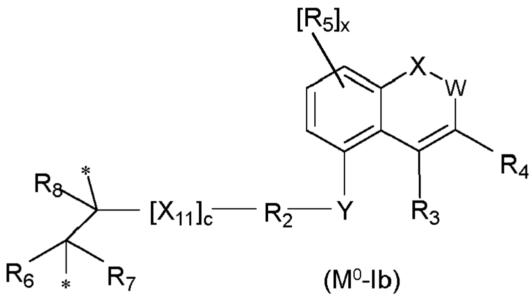

(M$^0$-Ib) ,

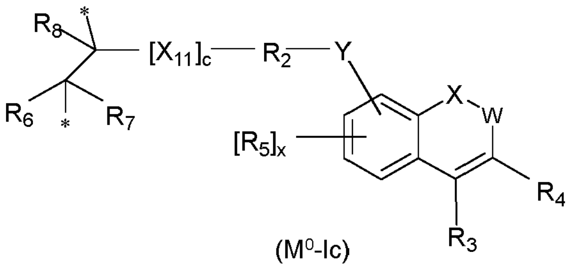

(M$^0$-Ic) ,

wherein -$R_2$-, X-W, Y, $R_3$, $R_4$, $R_5$, x, $R_6$, $R_7$, $R_8$, $X_{11}$ and c have a meaning as described before or preferably described before and where the asterisk "*" denotes at each occurrence a linkage to the adjacent repeating unit in the polymer chain or oligomer chain or a linkage to a terminal end group.

Combinations are excluded where two O atoms or an O atom and a S atom are directly linked to each other as known for a skilled artisan in the field of organic chemistry.

The invention is furthermore directed to an ophthalmic device or a precursor article for an ophthalmic device as described before or preferably described below wherein the constitutional unit $M^0$ is of formulae (M$^0$-I), (M$^0$-Ia), (M$^0$-Ib) or (M$^0$-Ic) as described before and where the asterisk "*" denotes at each occurrence a linkage to the adjacent repeating unit in the polymer chain or oligomer chain or a linkage to a terminal end group.

Preferably, such polymers within the ophthalmic device or precursor material for an ophthalmic device according to the invention and/or oligomers, polymers or copolymers according to the invention comprise a constitutional unit (M$^0$-I), (M$^0$-Ia), (M$^0$-Ib) or (M$^0$-Ic) as described before, wherein

-$R_2$- is selected from $-(CH_2)_5-$, $-(CH_2)_6-$, $-(CH_2)_7-$, $-(CH_2)_8-$, $-(CH_2)_9-$, $-(CH_2)_{10}-$, $-(CH_2)_{11}-$, $-(CH_2)_{12}-$, $-(CH_2)_{13}-$, $-(CH(C_2H_5)-(CH_2)_3-$, $-(CH(C_2H_5)-(CH_2)_4-$, $-(CH(C_2H_5)-(CH_2)_7-$, $-(CH_2)_2-S-(CH_2)_2-$, $-(CH_2)_2-SO_2-(CH_2)_2-$, $-(CH_2)_4SO_2-(CH_2)_4$, $-(CH_2)_2-O-(CH_2)_2-$, $-(CH_2)_3-O-(CH_2)_3-$, $-(CH_2)_3-S-(CH_2)_3-$, $-(CH_2)_2-S-(CH_2)_2-S-(CH_2)_2-$, $-(CH_2)_2-O-(CH_2)_2-O-(CH_2)_2-$, $-(CH_2)_2-S-(CH_2)_2-O-(CH_2)_2-$, $-(CH_2)_2-O-(CH_2)_2-S-(CH_2)_2-$, $-(CH_2)_2-O-(CH_2)_2-SO_2-(CH_2)_2-$, $-(CH_2)_2-SO_2-(CH_2)_2-O-(CH_2)_2-$, $-(CH_2)_2-SO_2-(CH_2)_2-S-(CH_2)_2-$, $-(CH_2)_2-O-(CH_2)_2-O-(CH_2)_2-O-(CH_2)_2-$, $-(CH_2)_2-O-(CH_2)_2-S-(CH_2)_2-O-(CH_2)_2-$, $-(CH_2)_2-S-(CH_2)_2-O-(CH_2)_2-S-(CH_2)_2-$, $-(CH_2)_2-SO_2-(CH_2)_2-O-(CH_2)_2-SO_2-(CH_2)_2-$, $-(CH_2)_2-S-(CH_2)_2-S-(CH_2)_2-S-(CH_2)_2-$, $-(CH_2)_2-SO_2-(CH_2)_2-S-(CH_2)_2-SO_2-(CH_2)_2-$, $-(CH_2)_2-SO_2-(CH_2)_2-SO_2-(CH_2)_2-SO_2-(CH_2)_2-$, $-(CH_2)_2-O-(CH_2)_2-SO_2-(CH_2)_2-O-(CH_2)_2-$, $-(CH_2)_3-(CF_2)-(CH_2)_3-$, $-(CH_2)_2-(CF_2)-(CH_2)_2-$, $-(CH_2)-(CF_2)_3-(CH_2)-$, $-(CH_2)_2-(CF_2)_4-(CH_2)_2-$, $-(CH_2)_3-(CHCF_3)-(CH_2)_3-$, $-(CH_2)_2-(CF_2)-O-(CF_2)-O-(CF_2)-(CH_2)_2-$ and $-(CH_2)-(CF_2)-O-(CF_2)-O-(CF_2)-O-(CF_2)-(CH_2)-$ according to the invention or has a meaning as preferably described before.

The copolymer within the ophthalmic device or precursor material for an ophthalmic device according to the invention or the copolymer according to the invention may be an oligomer or polymer comprising one or more polymerized compounds of formulae (I), (Ia), (Ib) or (Ic) as described before or preferably described before or one or more constitutional units $M^0$ of formulae (M$^0$-I), (M$^0$-Ia), (M$^0$-Ib) and/or (M$^0$-Ic) as described before or preferably described before or one or more constitutional units (M$^0$-001) to (M$^0$-106) as described below, which may be the same or different from one another, and one or more constitutional units $M^2$, which may be the same or different from one another. Said one or more constitutional units $M^2$ are chemically different from the units $M^0$. Preferably, said one or more constitutional units $M^2$ are derived by polymerization of one or more monomers selected from the group consisting of styrene, ethoxyethyl methacrylate (EOEMA), methyl methacrylate (MMA), methyl acrylate, *n*-alkyl acrylates (the *n*-alkyl group comprising 2-20 C-atoms), *n*-alkyl methacrylates (the *n*-alkyl group comprising 2-20 C-atoms), *i*-alkyl acrylates (the *i*-alkyl group comprising 3-20 C-atoms), *i*-alkyl methacrylates (the *i*-alkyl group comprising 3-20 C-atoms), ethoxyethoxy ethylacrylate (EEEA), *n*-hydroxalkyl acrylate (the *n*-alkyl group comprising 2 to 10 C-atoms), *n*-hydroxalkyl methacrylate (the *n*-alkyl group comprising 2 to 10 C-atoms), tetrahydrofuryl methacrylate (THFMA), glycidylmethacrylate (GMA), 16-hydroxyhexadecyl acrylate, 16-hydroxyhexadecyl methacrylate, 18-hydroxyoctadecyl acrylate, 18-hydroxyoctadecyl methacrylate, 2-phenoxyethyl acrylate (EGPEA), heptafluorobutyl acrylate, heptafluorobutyl methacrylate, hexafluorobutyl acrylate, hexafluorobutyl methacrylate, hexafluoroisopropyl acrylate, hexafluoroisopropyle methacrylate, octafluoropentyl acrylate, octafluoropentyl methacrylate, petanfluoropropyl acrylate, pentafluoropropyl methacrylate, tetrafluoropropyl methacrylate, trifluoroethyl acrylate, trifluoroethyl methacrylate, trimethylcyclohexyl acrylate, trimethylcyclohexyl methacrylate, 2-(4-bromophenyl)ethyl methacrylate, 4-phenylbutyl methacrylate, 4-methylphenyl methacrylate, phenyl methacrylate, 4-methylphenyl acrylate, benzyl acrylate, benzyl methacrylate, 2-benzyloxyethyl methacrylate, 3-benzyloxypropyl methacrylate, phenylethyl acrylate, phenylethyl methacrylate, 3-phenylpropyl methacrylate, 2-phenoxyethyl acrylate, 2-benzyloxyethyl acrylate, 3-phenylpropyl acrylate, 4-methylbenzyl acrylate, phenyl acrylate, 2-(phenylthio)ethyl methacrylate, 4-phenylbutyl acrylate, 5-phenylpentyl acrylate, 3-benzyloxypropyl acrylate, 2-(phenylthio)propyl acrylate, 2-(phenyl-thio)ethyl acrylate, 4-phenylbutyl acrylate, 4-methylbenzyl methacrylate, 2-(2-methylphenyl)ethyl acrylate, 2-(2-methylphenyl)ethyl methacrylate, 2-(3-methylphenyl)ethyl acrylate, 2-(3-methylphenyl)ethyl methacrylate, 2-(4-methylphenyl)ethyl acrylate, 2-(4-methylphenyl)ethyl methacrylate, 2-(4-propylphenyl)ethyl acrylate, 2-(4-propylphenyl)ethyl methacrylate, 2-(4-(1-methylethyl)phenyl)ethyl acrylate, 2-(4-(1-methylethyl)phenyl)ethyl methacrylate, 2-(4-methoxyphenyl)ethyl methacrylate, 2-(4-methoxyphenyl)ethyl acrylate, 2-(4-cyclohexylphenyl)ethyl acrylate, 2-(4-cyclohexylphenyl)ethyl methacrylate, 2-(4-chlorophenyl)ethyl methacrylate, 2-(4-chlorophenyl)ethyl acrylate, 2-(3-chlorophenyl)ethyl methacrylate, 2-(3-chlorophenyl)ethyl acrylate, 2-(2-chlorophenyl)ethyl methacrylate, 2-(2-chlorophenyl)ethyl acrylate, 2-(4-bromophenyl)ethyl acrylate, 2-(4-phenylphenyl)ethyl methacrylate, 2-(4-phenylphenyl)ethyl acrylate, 2-(3-phenylphenyl)ethyl methacrylate, 2-(3-phenylphenyl)ethyl acrylate, 2-(4-benzylphenyl)ethyl methacrylate, 2-(4-benzylphenyl)ethyl acrylate, 2-(phenylthio)ethyl acrylate, 3-benzyloxypropyl acrylate, 2-[2-(benzyloxy)ethoxy]ethyl acrylate, 2-[2-(benzyloxy)ethoxy]ethyl methacrylate, pentafluorophenyl methacrylate, heptadecafluorodecyl methacrylate, dodecafluoroheptyl methacrylate, heptadecafluorodecyl acrylate, pentafluorophenyl acrylate.

The invention therefore relates further to an ophthalmic device or a precursor article for the ophthalmic device as described or preferably described before comprising beside of the at least one polymerized compound of formulae (I), (Ia), (Ib) or (Ic) or the constitutional unit $M^0$ of formulae ($M^0$-I), ($M^0$-Ia), ($M^0$-Ib) and/or ($M^0$-Ic) as described before or preferably described before or one or more constitutional units ($M^0$-001) to ($M^0$-106) as described one or more constitutional units $M^2$ derived by polymerization of one or more co-monomers selected from the group consisting of styrene, ethoxyethyl methacrylate (EOEMA), methyl methacrylate (MMA), methyl acrylate, *n*-alkyl acrylates (the *n*-alkyl group comprising 2-20 C-atoms), *n*-alkyl methacrylates (the *n*-alkyl group comprising 2-20 C-atoms), *i*-alkyl acrylates (the *i*-alkyl group comprising 3-20 C-atoms), *i*-alkyl methacrylates (the *i*-alkyl group comprising 3-20 C-atoms), ethoxyethoxy ethylacrylate (EEEA), *n*-hydroxalkyl acrylate (the *n*-alkyl group comprising 2 to 10 C-atoms), *n*-hydroxalkyl methacrylate (the *n*-alkyl group comprising 2 to 10 C-atoms), tetrahydrofuryl methacrylate (THFMA), glycidyl methacrylate (GMA), 16-hydroxyhexadecyl acrylate, 16-hydroxyhexadecyl methacrylate, 18-hydroxyoctadecyl acrylate, 18-hydroxyoctadecyl methacrylate, 2-phenoxyethyl acrylate (EGPEA), heptafluorobutyl acrylate, heptafluorobutyl methacrylate, hexafluorobutyl acrylate, hexafluorobutyl methacrylate, hexafluoroisopropyl acrylate, hexafluoroisopropyle methacrylate, octafluoropentyl acrylate, octafluoropentyl methacrylate, petanfluoropropyl acrylate, pentafluoropropyl methacrylate, tetrafluoropropyl methacrylate, trifluoroethyl acrylate, trifluoroethyl methacrylate, trimethylcyclohexyl acrylate, trimethylcyclohexyl methacrylate, 2-(4-bromophenyl)ethyl methacrylate, 4-phenylbutyl methacrylate, 4-methylphenyl methacrylate, phenyl methacrylate, 4-methylphenyl acrylate, benzyl acrylate, benzyl methacrylate, 2-benzyloxyethyl methacrylate, 3-benzyloxypropyl methacrylate, phenylethyl acrylate, phenylethyl methacrylate, 3-phenylpropyl methacrylate, 2-phenoxyethyl acrylate, 2-benzyloxyethyl acrylate, 3-phenylpropyl acrylate, 4-methylbenzyl acrylate, phenyl acrylate, 2-(phenylthio)ethyl methacrylate, 4-phenylbutyl acrylate, 5-phenylpentyl acrylate, 3-benzyloxypropyl acrylate, 2-(phenylthio)propyl acrylate, 2-(phenylthio)ethyl acrylate, 4-phenylbutyl acrylate, 4-methylbenzyl methacrylate, 2-(2-methylphenyl)ethyl acrylate, 2-(2-methylphenyl)ethyl methacrylate, 2-(3-methylphenyl)ethyl acrylate, 2-(3-methylphenyl)ethyl methacrylate, 2-(4-methylphenyl)ethyl acrylate, 2-(4-methylphenyl)ethyl methacrylate, 2-(4-propylphenyl)ethyl acrylate, 2-(4-propylphenyl)ethyl methacrylate, 2-(4-(1-methylethyl)phenyl)ethyl acrylate, 2-(4-(1-methylethyl)phenyl)ethyl methacrylate, 2-(4-methoxyphenyl)ethyl methacrylate, 2-(4-methoxyphenyl)ethyl acrylate, 2-(4-cyclohexylphenyl)ethyl acrylate, 2-(4-cyclohexylphenyl)ethyl methacrylate, 2-(4-chlorophenyl)ethyl methacrylate, 2-(4-chlorophenyl)ethyl acrylate, 2-(3-chlorophenyl)ethyl methacrylate, 2-(3-chlorophenyl)ethyl acrylate, 2-(2-chlorophenyl)ethyl methacrylate, 2-(2-chlorophenyl)ethyl acrylate, 2-(4-bromophenyl)ethyl acrylate, 2-(4-phenylphenyl)ethyl methacrylate, 2-(4-phenylphenyl)ethyl acrylate, 2-(3-phenylphenyl)ethyl methacrylate, 2-(3-phenylphenyl)ethyl acrylate, 2-(4-benzylphenyl)ethyl methacrylate, 2-(4-benzylphenyl)ethyl acrylate, 2-(phenylthio)ethyl acrylate, 3-benzyloxypropyl acrylate, 2-[2-(benzyloxy)ethoxy]ethyl acrylate, 2-[2-(benzyloxy)ethoxy]ethyl methacrylate, pentafluorophenyl methacrylate, heptadecafluorodecyl methacrylate, dodecafluoroheptyl methacrylate, heptadecafluorodecyl acrylate, pentafluorophenyl acrylate.

The term constitutional units $M^2$ derived by polymerization of one or more co-monomers may comprise compounds having the function of a cross-linker, blue absorber or UV absorber as further described below.

By using *n*-alkyl acrylates as co-monomer beside the compounds of formulae (I), (Ia), (Ib) or (Ic), the *n*-alkyl group has preferably 2 to 10 C-atoms, particularly preferably 2 to 5 C-atoms and very particularly preferably 4 C-atoms.

By using *n*-alkyl methacrylates as co-monomer beside the compounds of formulae (I), (Ia), (Ib) or (Ic), the *n*-alkyl group has preferably 2 to 10 C-atoms, particularly preferably 2 to 5 C-atoms and very particularly preferably 4 C-atoms.
By using *i*-alkyl acrylates as co-monomer beside the compounds of formulae (I), (Ia), (Ib) or (Ic), the *i*-alkyl group has preferably 3 to 10 C-atoms, particularly preferably 5 to 10 C-atoms and very particularly preferably 10 C-atoms.
By using *i*-alkyl methacrylates as co-monomer beside the compounds of formulae (I), (Ia), (Ib) or (Ic), the *i*-alkyl group has preferably 3 to 10 C-atoms, particularly preferably 5 to 10 C-atoms and very particularly preferably 10 C-atoms.
By using n-hydroxyalkyl acrylates as further monomer beside the compounds of formulae (I), (Ia), (Ib) or (Ic), the *n*-hydroxyalkyl group has preferably 2 to 8 C-atoms, particularly preferably 2 to 5 C-atoms, more preferably 2 to 4 C-atoms, and very particularly preferably 2 or 4 C-atoms.
By using *n*-hydroxyalkyl methacrylates as further monomer beside the compounds of formulae (I), (Ia), (Ib) or (Ic), the *n*-hydroxyalkyl group has preferably 2 to 8 C-atoms, particularly preferably 2 to 5 C-atoms, more preferably 2 to 4 C-atoms, and very particularly preferably 2 or 4 C-atoms.

Particularly preferably, the at least one further co-monomer beside of crosslinkers and/or UV absorbers as described below is selected from methyl methacrylate, *n*-alkyl acrylates (the *n*-alkyl group comprising 2-20 C-atoms), fluorinated *n*-alkyl acrylates (the *n*-alkyl group comprising 2-20 C-atoms), *n*-alkyl methacrylates (the *n*-alkyl group comprising 2-20 C-atoms), fluorinated *n*-alkyl methacrylates (the *n*-alkyl group comprising 2-20 C-atoms), *i*-alkyl acrylates (the *i*-alkyl group comprising 3-20 C-atoms), fluorinated *i*-alkyl acrylates (the *i*-alkyl group comprising 3-20 C-atoms), *i*-alkyl methacrylates (the *i*-alkyl group comprising 3-20 C-atoms), fluorinated *i*-alkyl methacrylates, *n*-hydroxyalkyl acrylates (the *n*-alkyl group comprising 2-10 C-atoms), *n*-hydroxyalkyl methacrylates (the *n*-alkyl group comprising 2-10 C-atoms) or a mixture thereof.

Particularly preferably, the at least one further co-monomer beside of crosslinkers and/or UV absorbers as described below is selected from methyl methacrylate, 2-hydroxyethyl methacrylate, 2-hydroxyethyl acrylate, 3-hydroxypropyl methacrylate, 3-hydroxypropyl acrylate, 4-hydroxybutyl methacrylate, 4-hydroxybutyl acrylate, 5-hydroxypentyl methacrylate, 5-hydroxypentyl acrylate, 8-methylnonyl methacrylate, *n*-butyl-acrylate, *n*-butyl methacrylate, ethyl methacrylate, 2-ethyl hexylmethacrylate, 2,2,3,3,4,4,5,5-octafluoropentyl acrylate or a mixture thereof.

Both hydrophobic, soft acrylate polymers and siloxane polymers, which are known for use for the production of intraocular lenses, exhibit glistening. The tendency towards glistening is more pronounced in the first-mentioned acrylate polymers. Glistening is the emerging of water filled voids in a hydrophobic material when placed in an aqueous environment like the human eye. Thus, water and the material of the ophthalmic device usually have different refractive indices glistening can decrease the quality of the patient's vision. A. Miyata and S. Yaguchi, J Cataract Refract Surg 2004; 30:1768-1772 describes the phenomenon glistening and introduced a scale for it.

This undesirable phenomenon can be avoided by equipping the material for the ophthalmic device or precursor material for the ophthalmic device with hydrophilic properties. One option is the addition of an adequate amount of a hydrophilic co-monomer. A hydrophilic component or co-monomer is a monomer whose uncrosslinked homopolymer is water-soluble or swellable in water. Examples are described before and below.

For the division of intraocular lenses into hydrophilic and hydrophobic lenses, Bozukova et al indicate their water content as the division criterion. If this water content is less than 10 - 20% by weight, it is a hydrophobic intraocular lens; if this water content is greater than 10 - 20% by weight, it is a hydrophilic intraocular lens (literature: Bozukova D, Pagnoulle C, Jêrome R, Jêrome C, (2010) Polymers in modern ophthalmic implants -Historical background and recent advances, Material Science and Engineering R 69:63-83). The water absorption of a material is the more appropriate decision criterion with respect to hydrophilicity and hydrophobicity for the polymers prepared in the context of this invention with respect to their area of application. For division of monomers into hydrophilic and hydrophobic monomers, it is useful in the context of this invention and the application aims thereof to use a division described by Bozukova et al: if a monomer gives a homopolymer which is either completely water-soluble or has a water absorption of greater than 10% by weight, this exhibits a hydrophilic property and is classified as hydrophilic monomer for the purposes of this invention. The hydrophilic property is caused by functional groups in the monomer which are capable of binding water molecules. The property can be confirmed in the swollen state by the presence of non-freezable water. The non-freezable water here is based on the fact that functional groups in the polymer bind water per se and this water is therefore not capable of forming ice crystals, irrespective of the temperature. Unaffected by this, a polymer of this type may also contain freezable water which forms ice crystals, but this is not utilised for the division into hydrophilic and hydrophobic monomers. Examples of functional groups which are capable of binding non-freezable water are, inter alia, hydroxyl groups, amino groups, ammonium groups, carboxyl groups, sulfone groups, sulfate groups, ether bridges or amide groups. The presence of functional groups of this type in a monomer is thus an indicator of a hydrophilic monomer.
Therefore, if a monomer satisfies one or more of the said criteria water solubility of the resultant homopolymer, water absorption of the resultant homopolymer greater than 10% by weight and/or presence of non-freezable water in the resultant homopolymer, this is a "hydrophilic monomer" for the purposes of the invention. If a monomer does not satisfy any of the said criteria, this is referred to as "non-hydrophilic monomer" for the purposes of the invention.

By using a hydrophilic co-monomer, thus building a constitutional unit $M^2$, as described before beside of the

at least one compound of formulae (I), (Ia) or (Ib) as described before or preferably described before, the resulting copolymer to be used in the ophthalmic device according to the invention or to be used in the precursor material for an ophthalmic device according to the invention exhibits little glistening or no glistening and is sufficiently hydrophobic at the same time so that calcification cannot occur. This is a further advantage for the ophthalmic device according to the invention.

The invention therefore relates further to an ophthalmic device or a precursor article for the ophthalmic device as described or preferably described before comprising beside of the at least one polymerized compound of formulae (I), (Ia), (Ib) or (Ic) or the constitutional unit $M^0$ of formulae ($M^0$-I), ($M^0$-Ia), ($M^0$-Ib) and/or ($M^0$-Ic) as described before or preferably described before or one or more constitutional units ($M^0$-001) to ($M^0$-106) as described below at least one further polymerized hydrophilic monomer as described before, preferably selected from the group consisting of *n*-hydroxalkyl acrylate (the *n*-alkyl group comprising 2 to 10 C-atoms), *n*-hydroxalkyl methacrylate (the *n*-alkyl group comprising 2 to 10 C-atoms), glycidyl methacrylate (GMA), *N*-vinylpyrrolidone and 2-phenoxyethyl acrylate (EGPEA).

The invention therefore relates further to an ophthalmic device or a precursor article for the ophthalmic device as described or preferably described before comprising beside of the at least one polymerized compound of formulae (I), (Ia), (Ib) or (Ic) or the constitutional unit $M^0$ of formulae ($M^0$-I), ($M^0$-Ia), ($M^0$-Ib) and/or ($M^0$-Ic) as described before or preferably described before or one or more constitutional units ($M^0$-001) to ($M^0$-106) as described below at least one further polymerized hydrophilic monomer as described before, preferably selected from the group consisting of *n*-hydroxalkyl acrylate (the *n*-alkyl group comprising 2 to 10 C-atoms), *n*-hydroxalkyl methacrylate (the *n*-alkyl group comprising 2 to 10 C-atoms), glycidyl methacrylate (GMA), *N*-vinylpyrrolidone and 2-phenoxyethyl acrylate (EGPEA).

Particularly preferably, such copolymer is comprised in the ophthalmic device or precursor article for an ophthalmic device according to the invention.

Alternatively the oligomer or polymer, preferably the polymer, according to the invention is a homopolymer, i.e. an oligomer or polymer, preferably a polymer, comprising one or more constitutional units $M^0$ of formula ($M^0$-I), ($M^0$-Ia), ($M^0$-Ib) or ($M^0$-Ic) as described before or preferably described before or ($M^0$-001) to ($M^0$-106) as described below and wherein all constitutional units $M^0$ are the same.

Exemplary homopolymeric compounds based on compounds of formulae (I), (Ia), (Ib) and/or (Ic) are the following compounds (P-001) to (P-106) as shown in table 2.

Table 2:

| | |
|---|---|
| 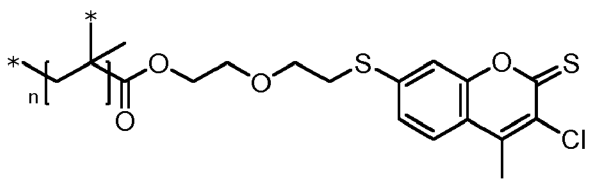 | P-001 |
| 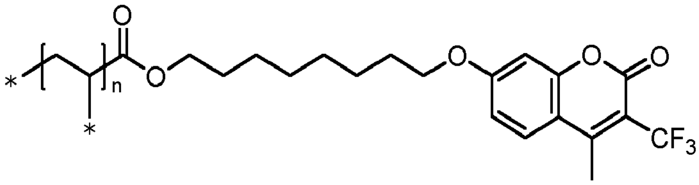 | P-002 |
| 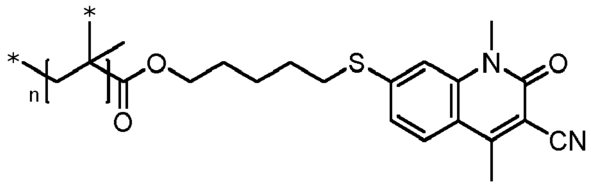 | P-003 |
| 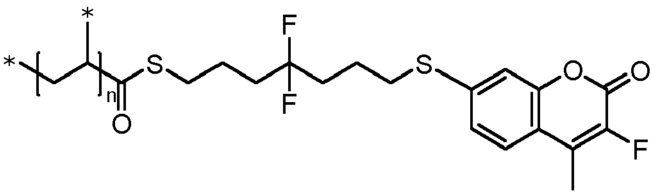 | P-004 |

(continued)

| | |
|---|---|
| 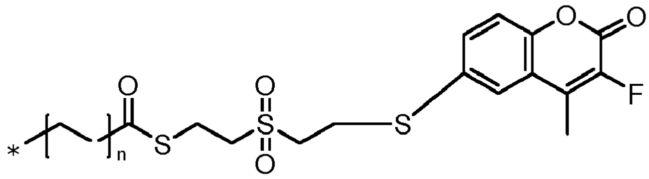 | P-005 |
| 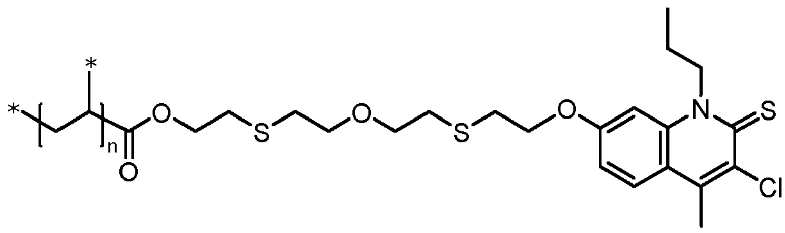 | P-006 |
| 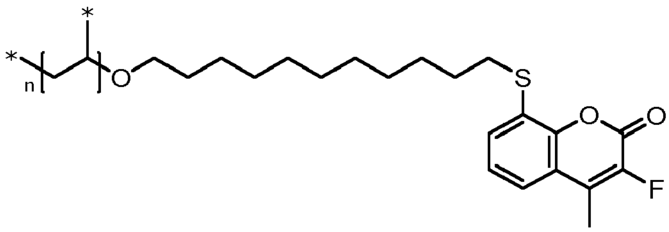 | P-007 |
| 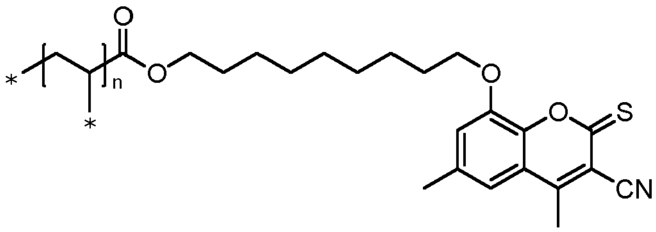 | P-008 |
| 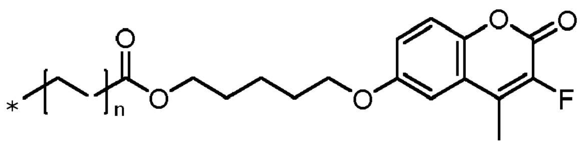 | P-009 |
| 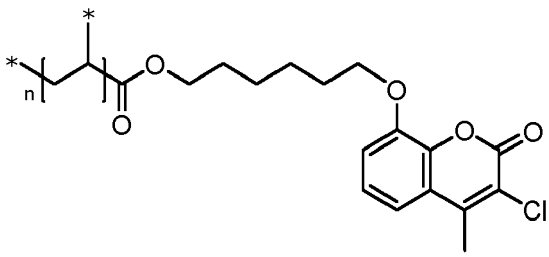 | P-010 |
| 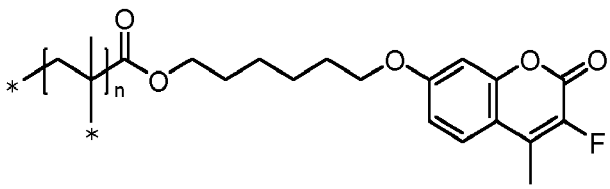 | P-011 |

(continued)

| | |
|---|---|
| 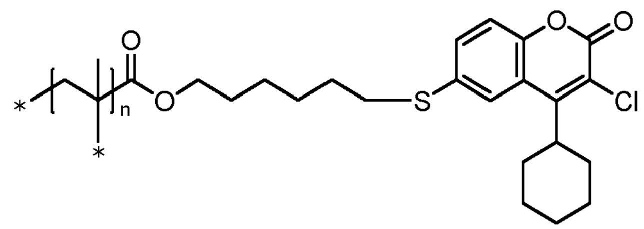 | P-012 |
| 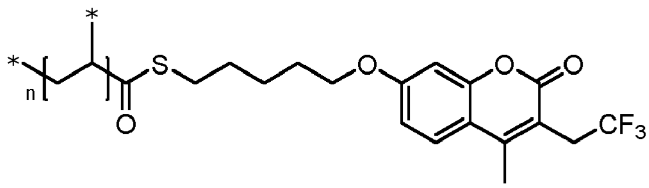 | P-013 |
| 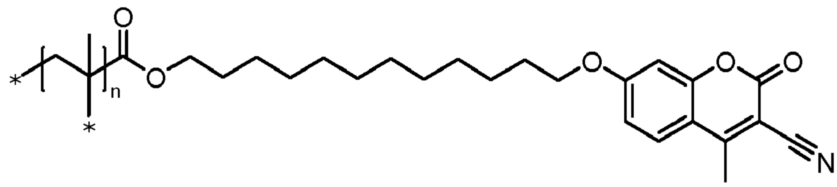 | P-014 |
| 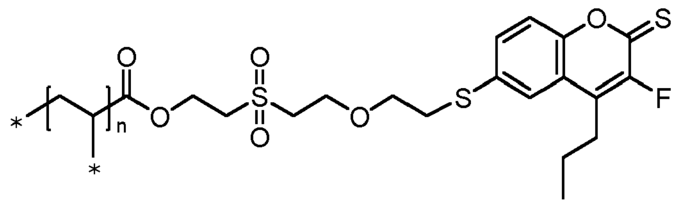 | P-015 |
| 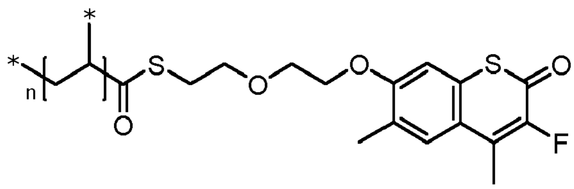 | P-016 |
| 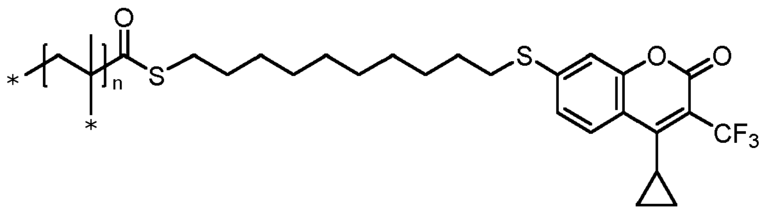 | P-017 |
| 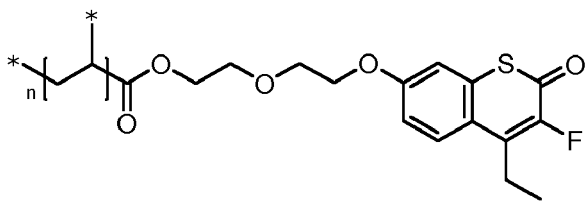 | P-018 |

(continued)

| | |
|---|---|
| 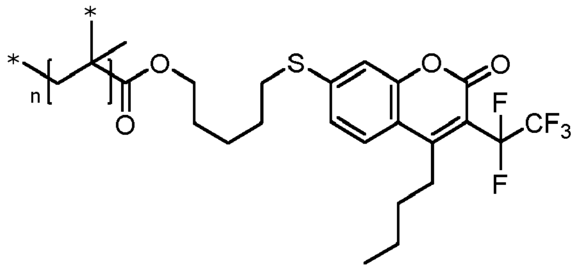 | P-019 |
| 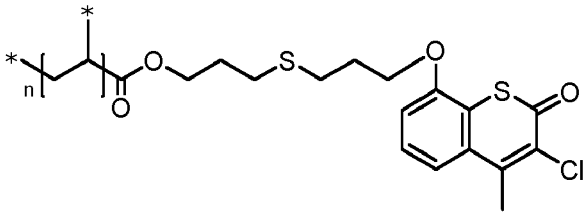 | P-020 |
| 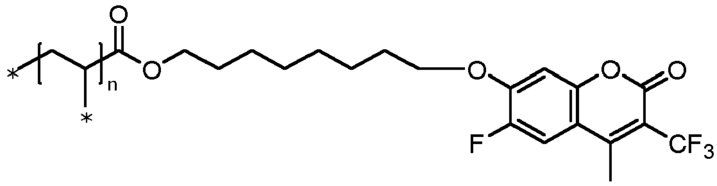 | P-021 |
| 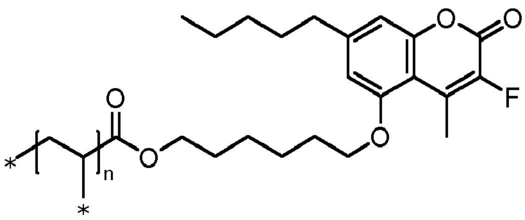 | P-022 |
| 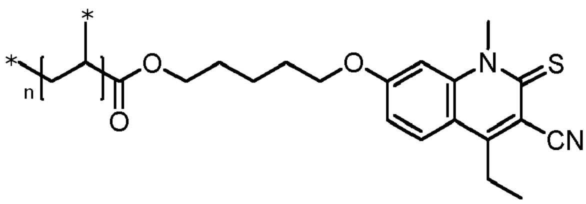 | P-023 |
| 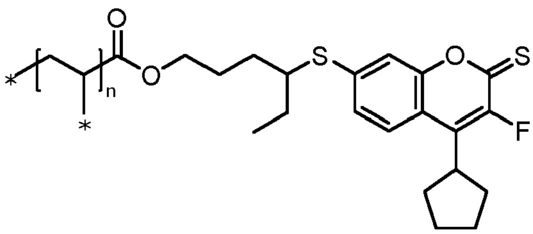 | P-024 |
| 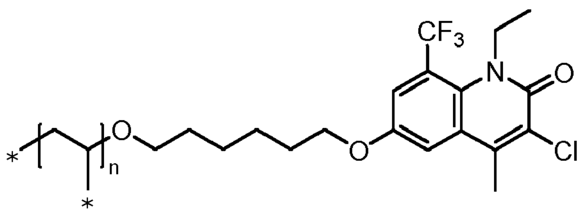 | P-025 |

(continued)

| | |
|---|---|
| 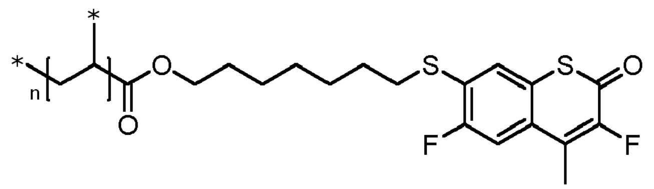 | P-026 |
| 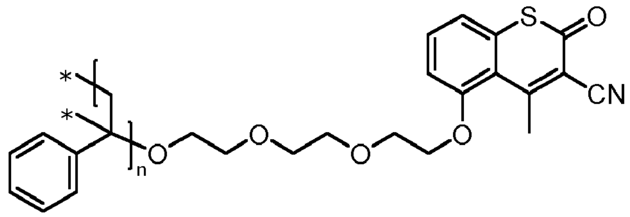 | P-027 |
| 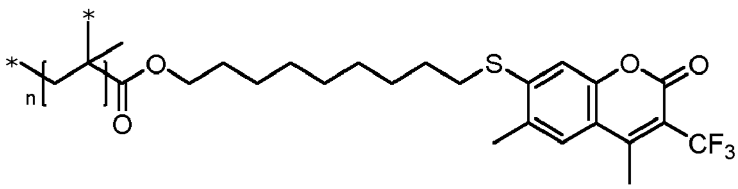 | P-028 |
| 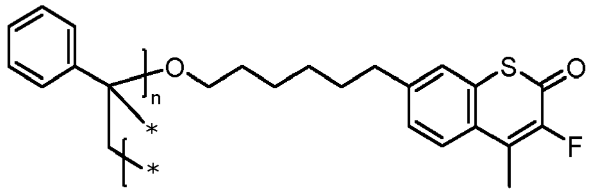 | P-029 |
| 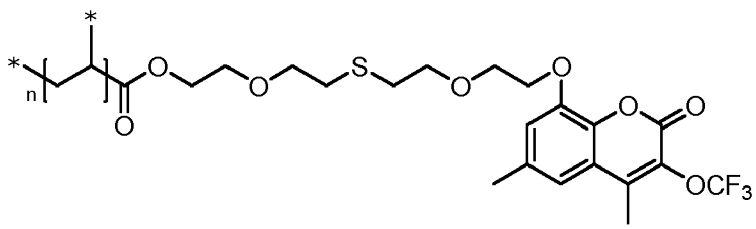 | P-030 |
| 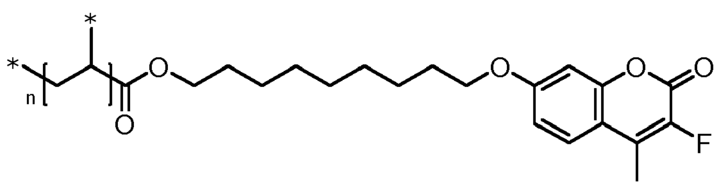 | P-031 |
| 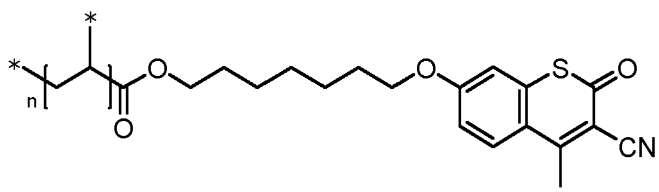 | P-032 |

(continued)

| | |
|---|---|
| 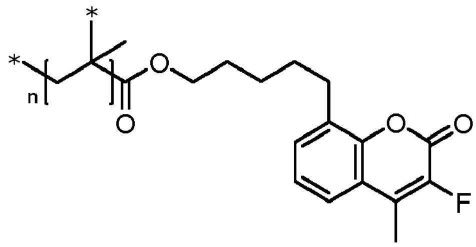 | P-033 |
| 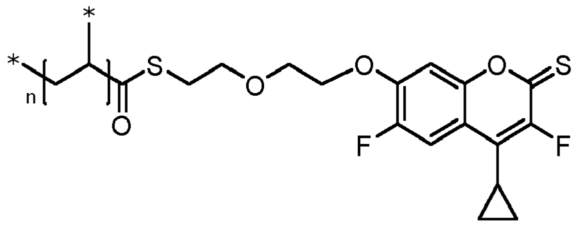 | P-034 |
| 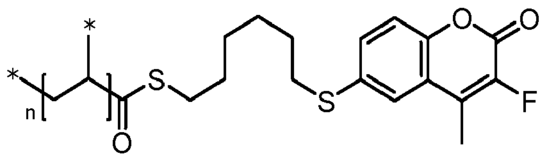 | P-035 |
| 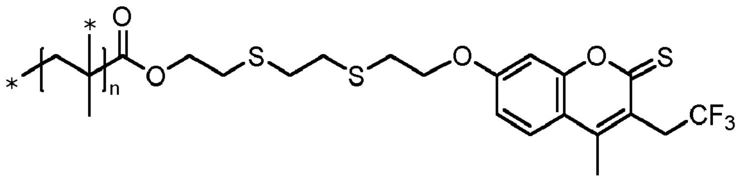 | P-036 |
| 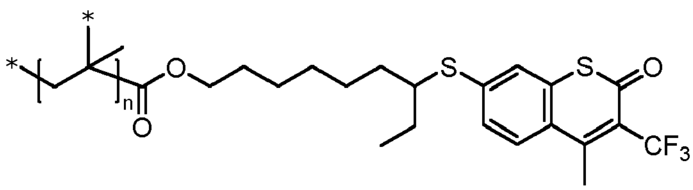 | P-037 |
| 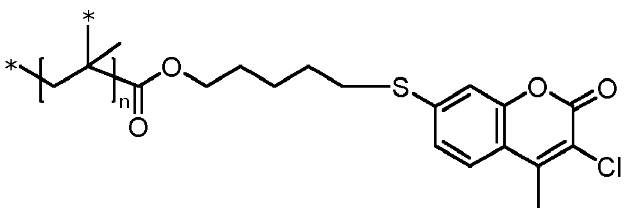 | P-038 |
| 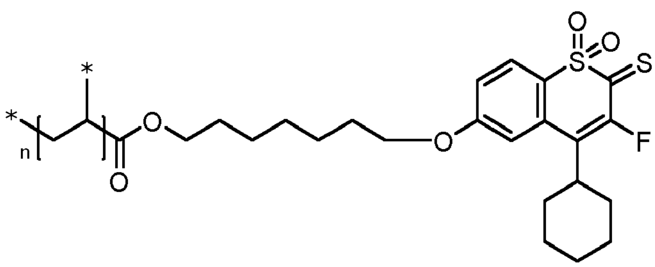 | P-039 |

(continued)

| | |
|---|---|
| 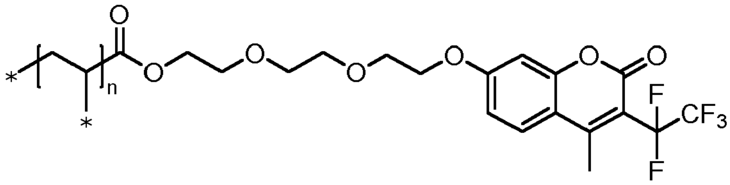 | P-040 |
| 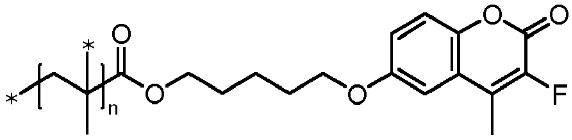 | P-041 |
| 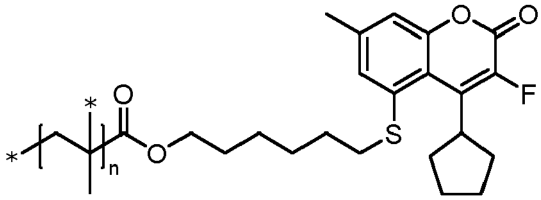 | P-042 |
| 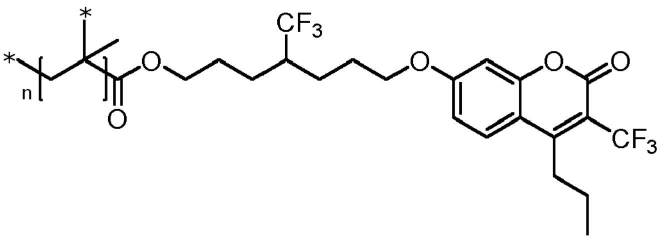 | P-043 |
| 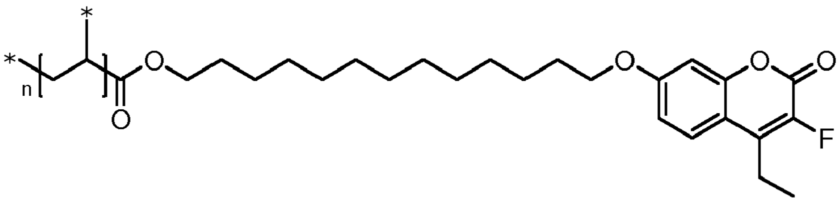 | P-044 |
| 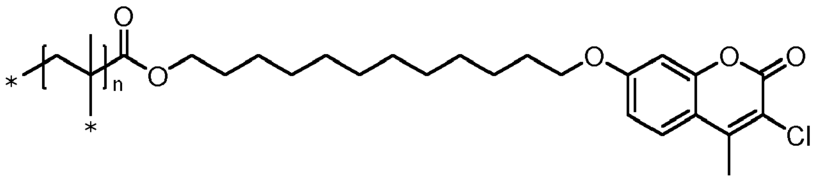 | P-045 |
| 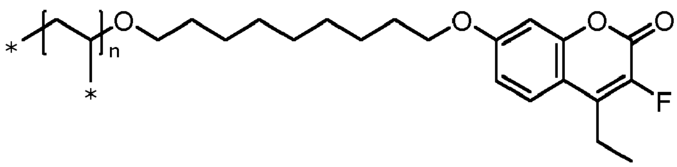 | P-046 |

(continued)

| | |
|---|---|
| 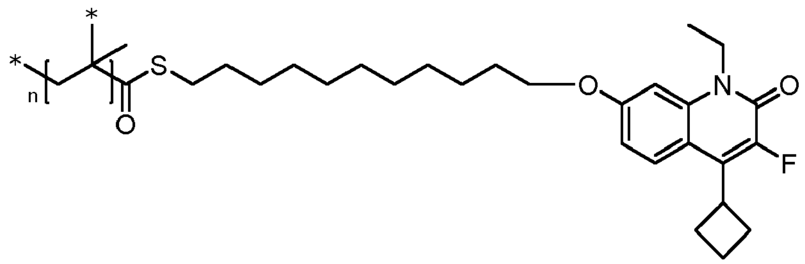 | P-047 |
| 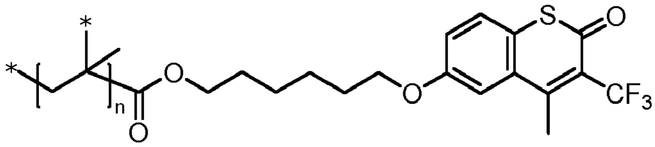 | P-048 |
| 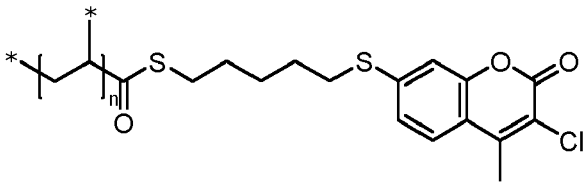 | P-049 |
| 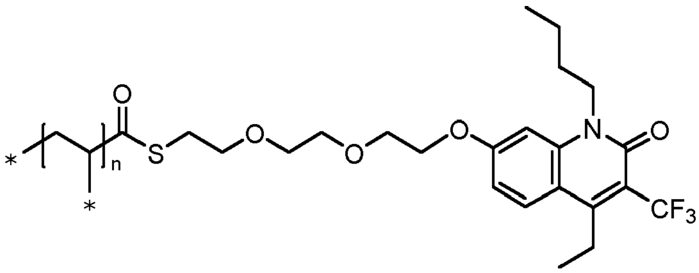 | P-050 |
| 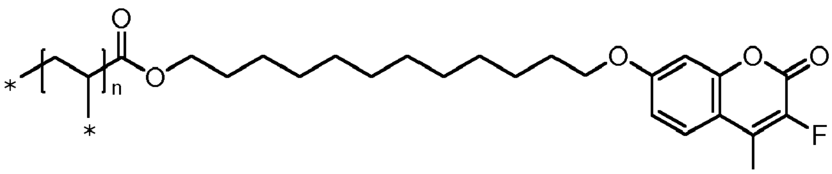 | P-051 |
| 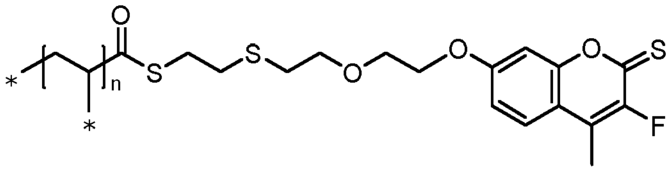 | P-052 |

(continued)

| | |
|---|---|
| 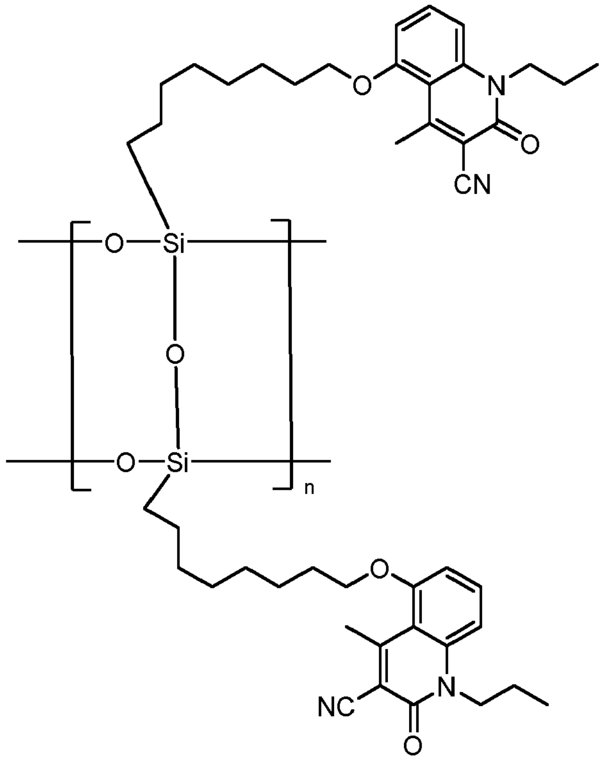 | P-053 |
| 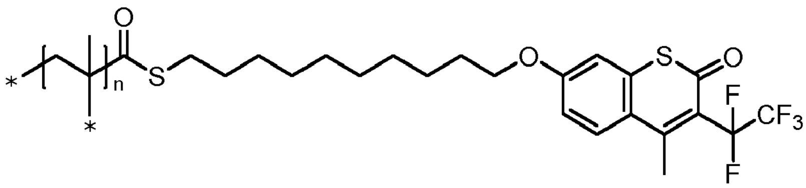 | P-054 |
| 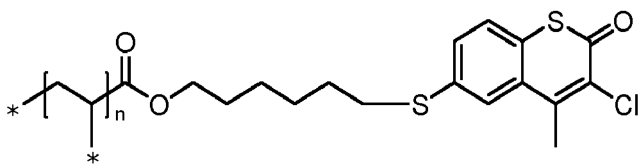 | P-055 |
| 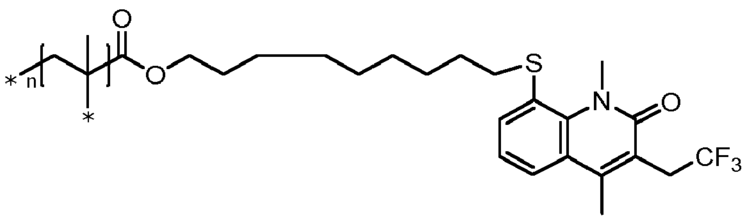 | P-056 |
| 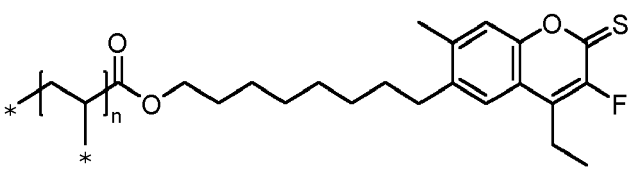 | P-057 |

(continued)

| | |
|---|---|
| 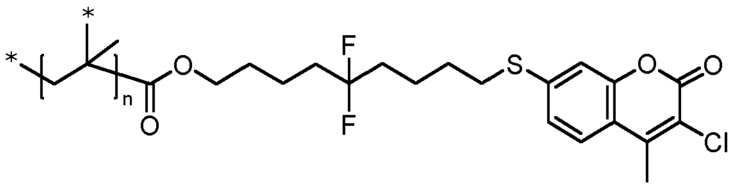 | P-058 |
| 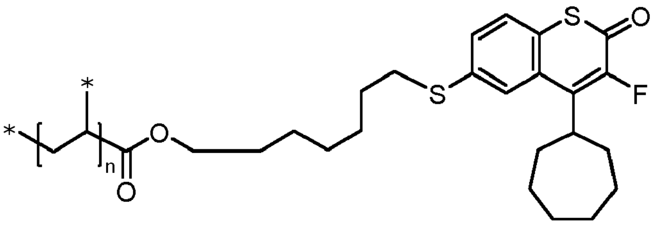 | P-059 |
| 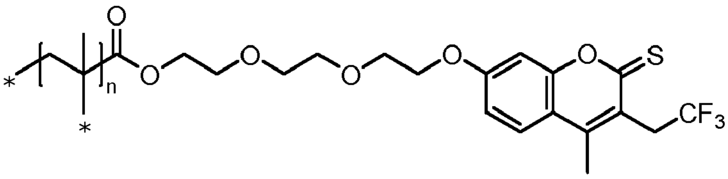 | P-060 |
| 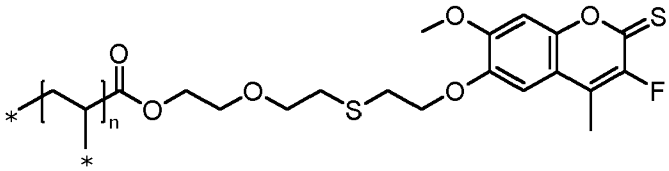 | P-061 |
| 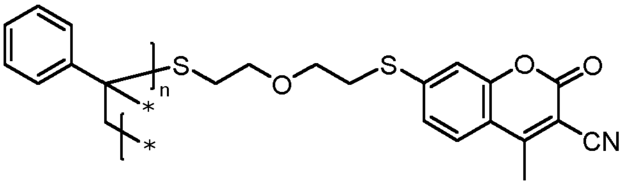 | P-062 |
| 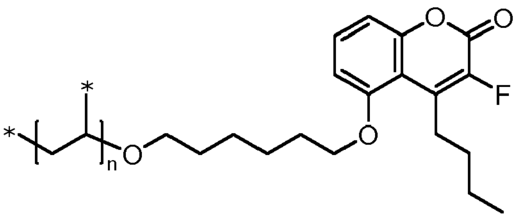 | P-063 |
| 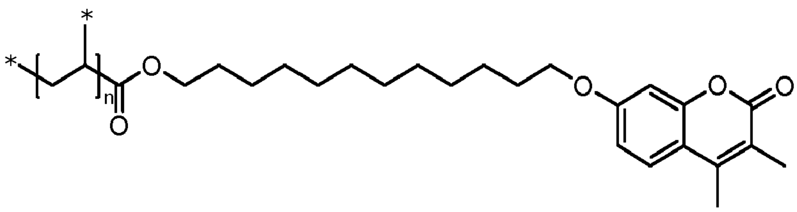 | P-064 |
| 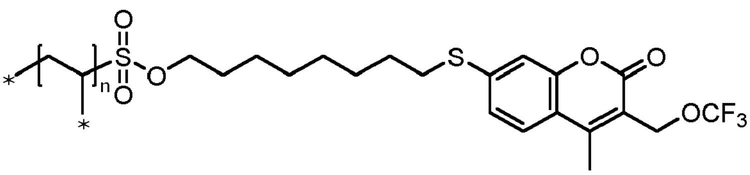 | P-065 |

(continued)

| | |
|---|---|
| 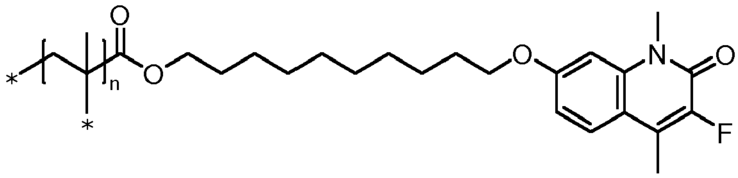 | P-066 |
| 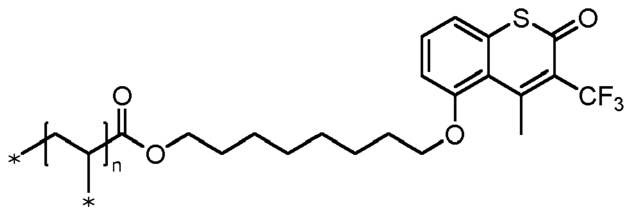 | P-067 |
| 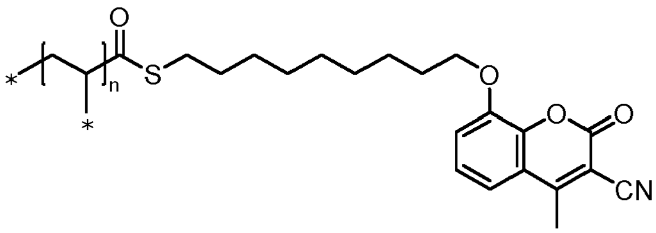 | P-068 |
| 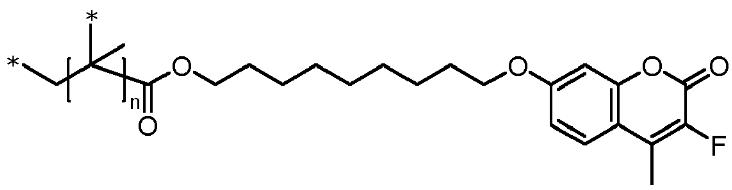 | P-069 |
| 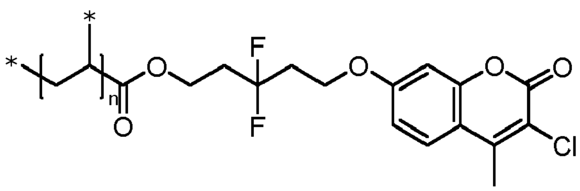 | P-070 |
| 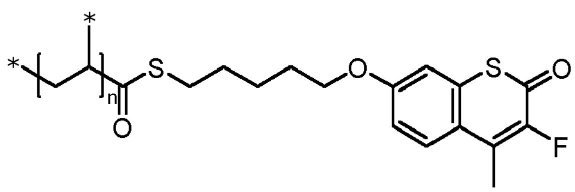 | P-071 |
| 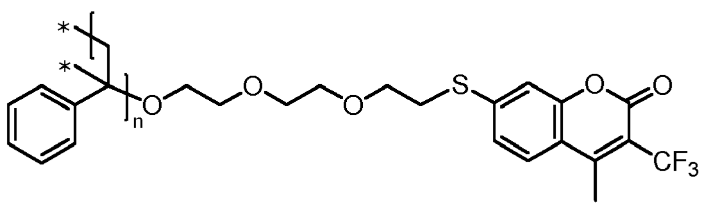 | P-072 |

(continued)

| | |
|---|---|
| 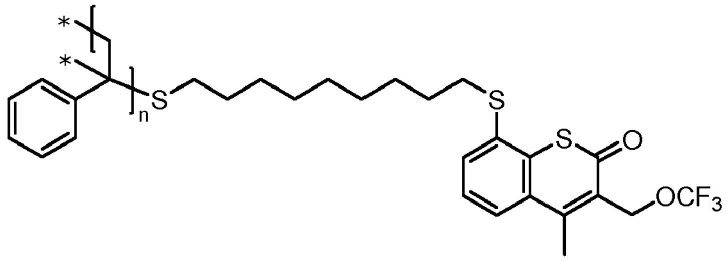 | P-073 |
| 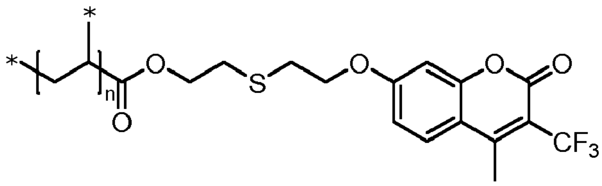 | P-074 |
| 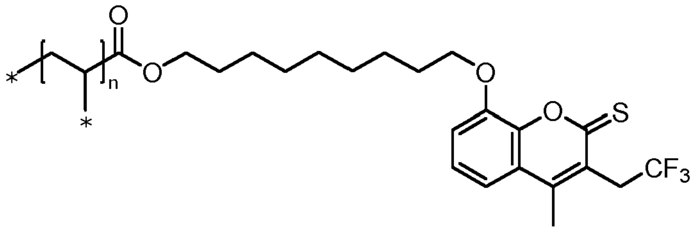 | P-075 |
| 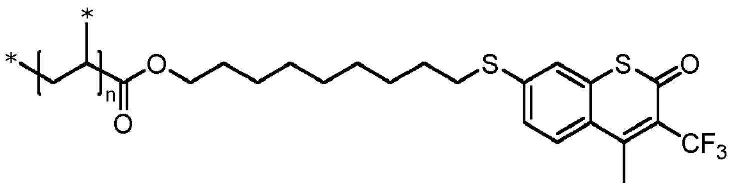 | P-076 |
| 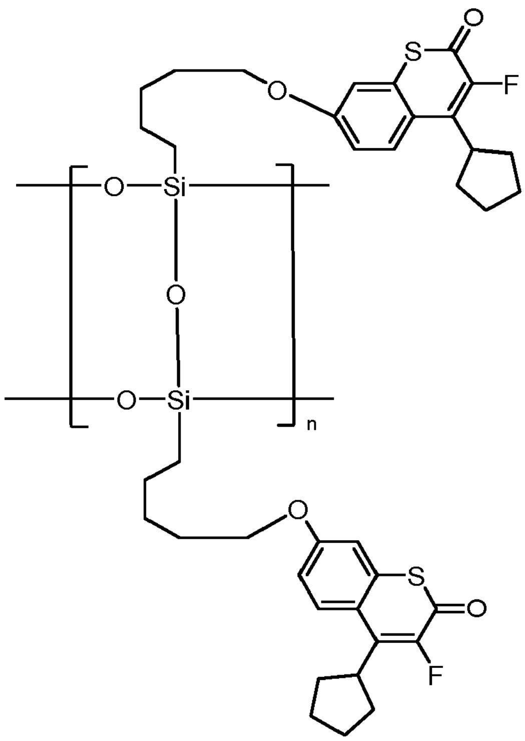 | P-077 |

(continued)

| | |
|---|---|
| 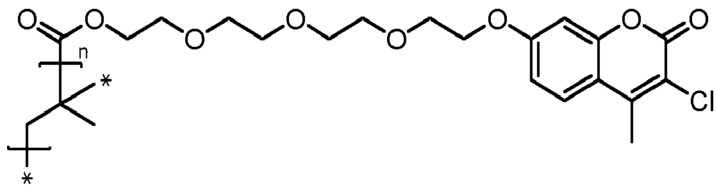 | P-078 |
| 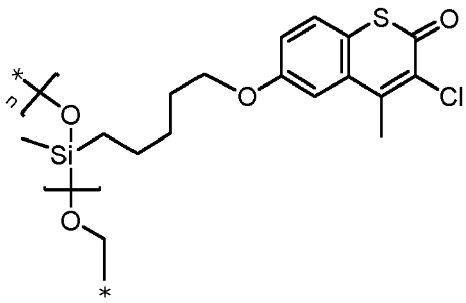 | P-079 |
| 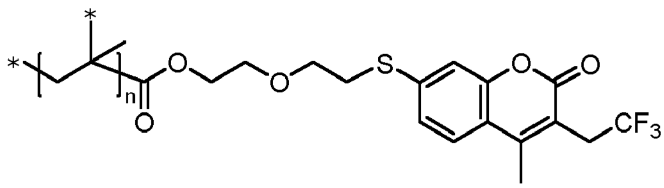 | P-080 |
| 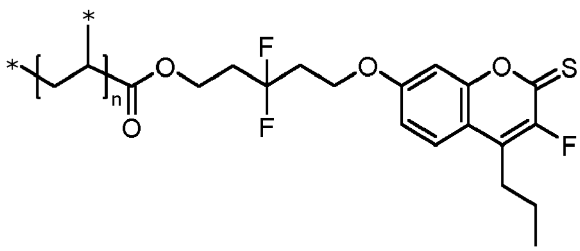 | P-081 |
| 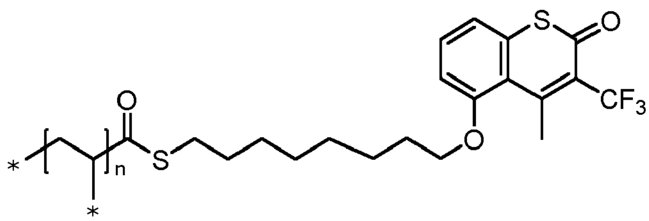 | P-082 |
| 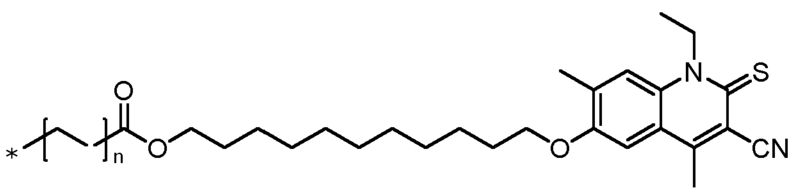 | P-083 |
| 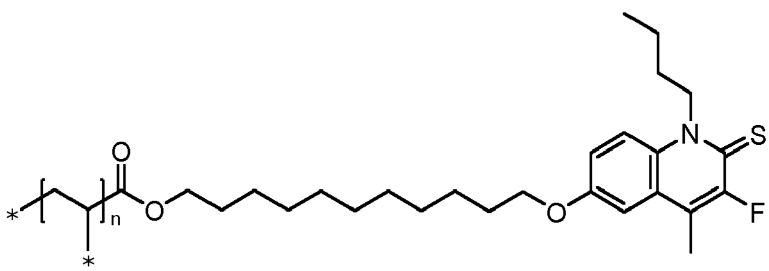 | P-084 |

(continued)

| | |
|---|---|
| 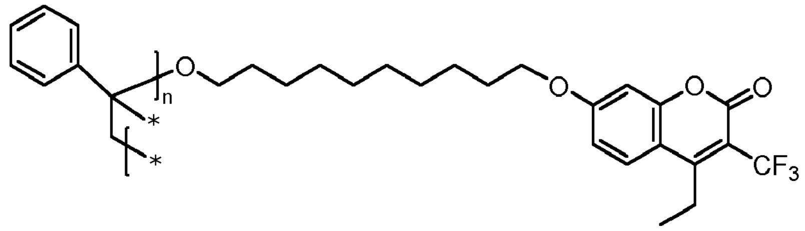 | P-085 |
| 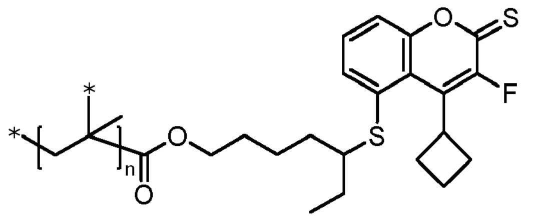 | P-086 |
| 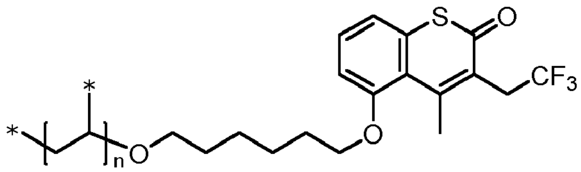 | P-087 |
| 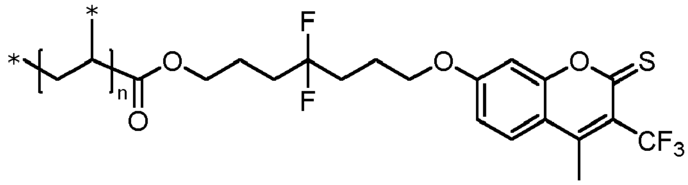 | P-088 |
| 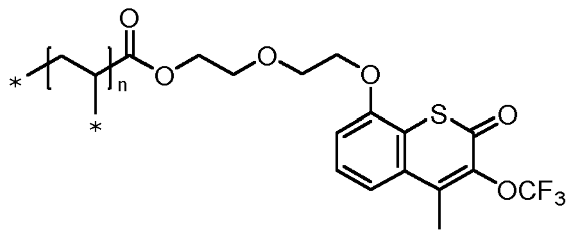 | P-089 |
| 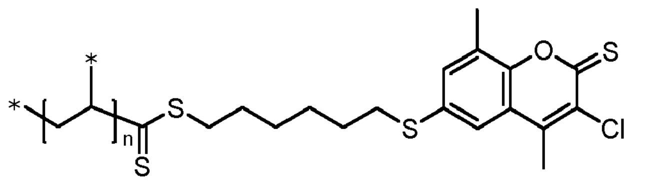 | P-090 |
| 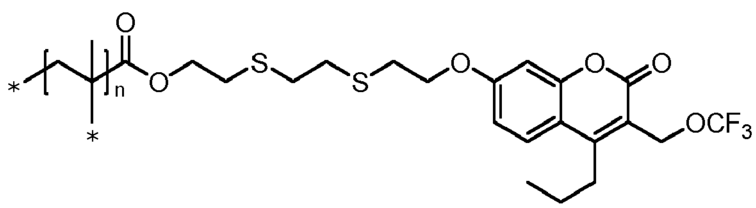 | P-091 |

(continued)

| | |
|---|---|
| 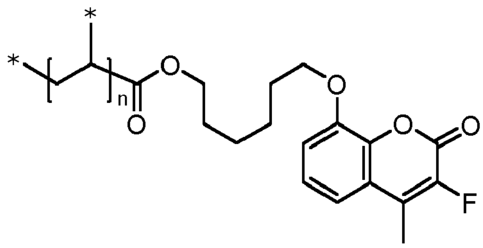 | P-092 |
| 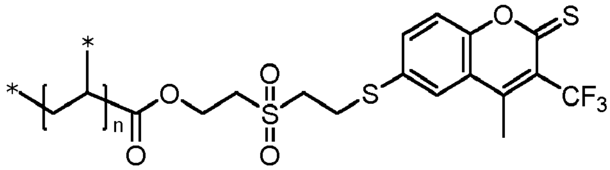 | P-093 |
| 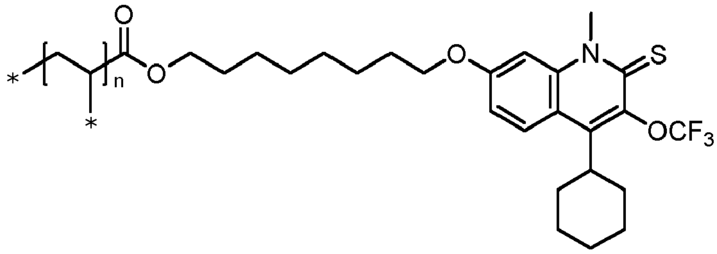 | P-094 |
| 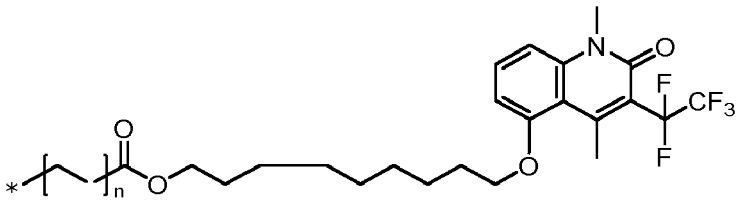 | P-095 |
| 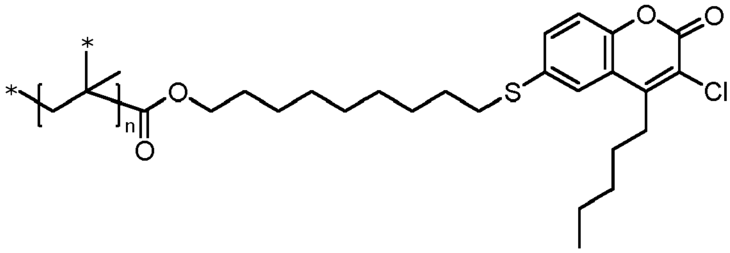 | P-096 |
| 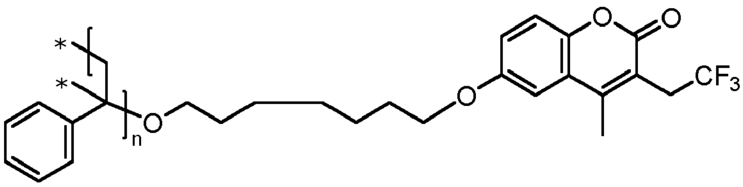 | P-097 |

(continued)

| | |
|---|---|
| 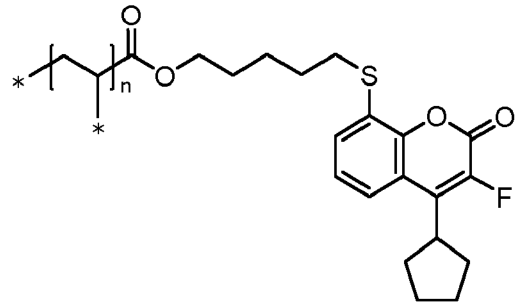 | P-098 |
| 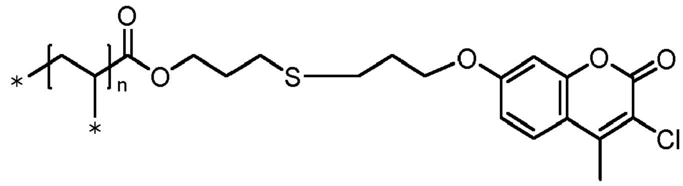 | P-099 |
| 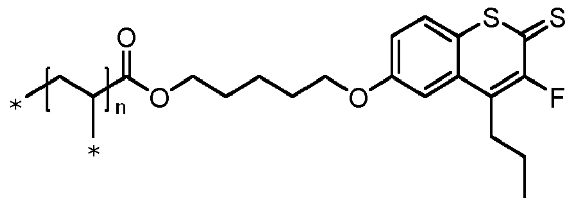 | P-100 |
| 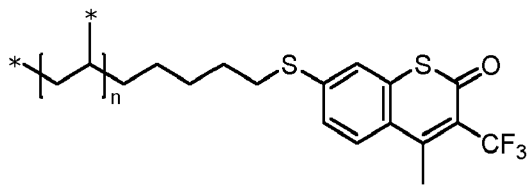 | P-101 |
| 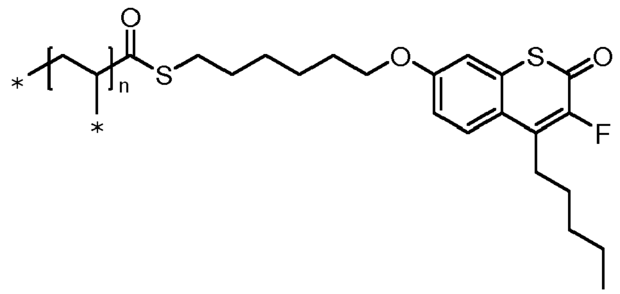 | P-102 |
| 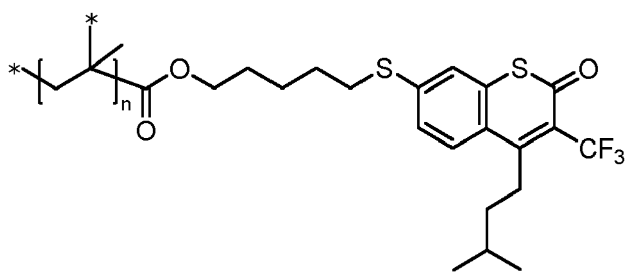 | P-103 |

(continued)

| | |
|---|---|
| 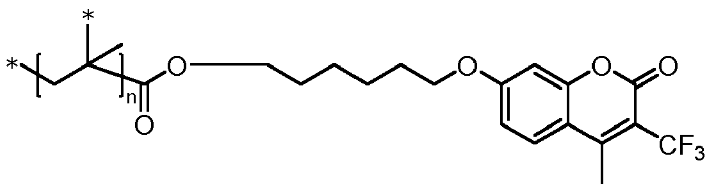 | P-104 |
| 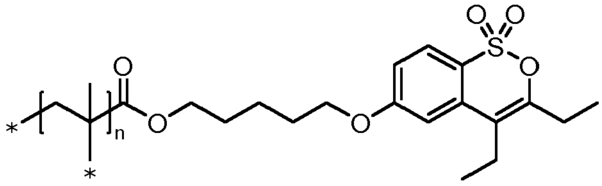 | P-105 |
| 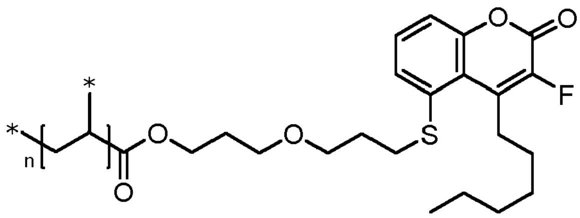 | P-106 |

The letter n gives the degree of polymerization as explained before.

Exemplary constitutional units $M^0$ based on compounds of formulae (I), (Ia), (Ib) and/or (Ic) or constitutional units $M^0$ of formulae ($M^0$-I), ($M^0$-Ia), ($M^0$-Ib) or ($M^0$-Ic) are the following compounds ($M^0$-001) to ($M^0$-106) as shown in table 2-1.

Table 2-1:

| | |
|---|---|
| 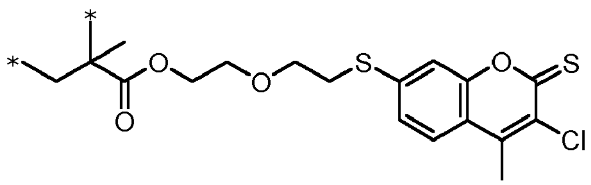 | $M^0$-001 |
| 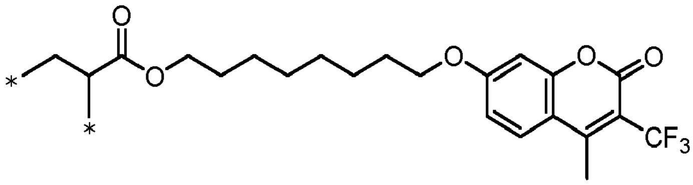 | $M^0$-002 |
| 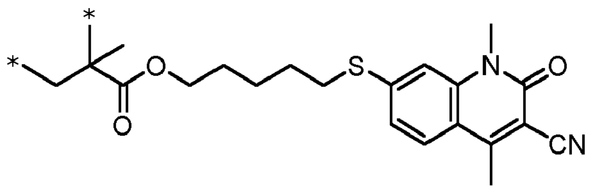 | $M^0$-003 |
| 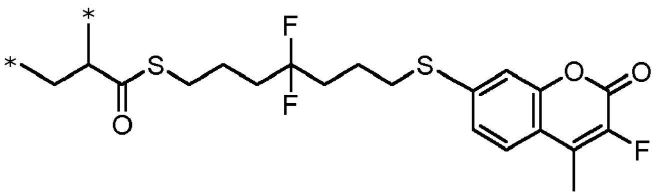 | $M^0$-004 |

(continued)

| | |
|---|---|
| 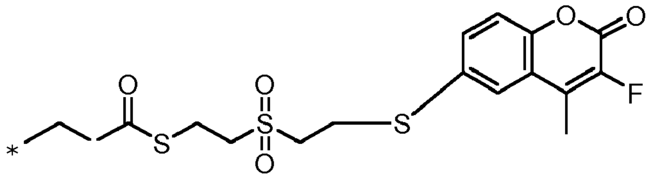 | M0-005 |
| 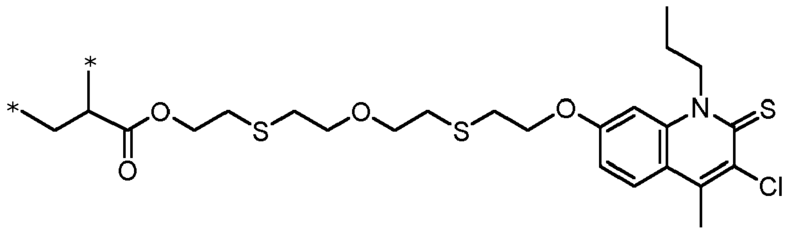 | M0-006 |
| 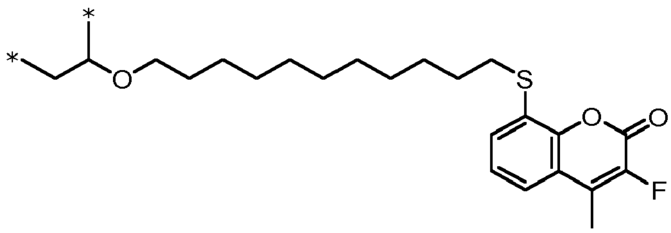 | M0-007 |
| 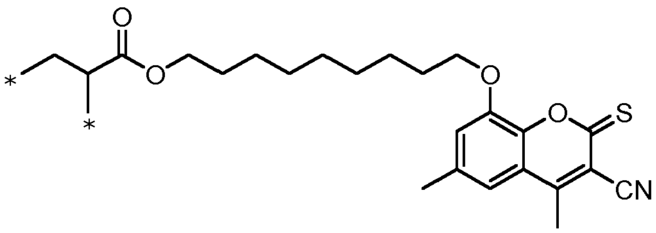 | M0-008 |
| 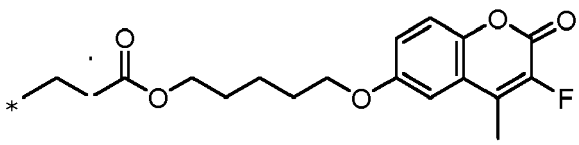 | M0-009 |
| 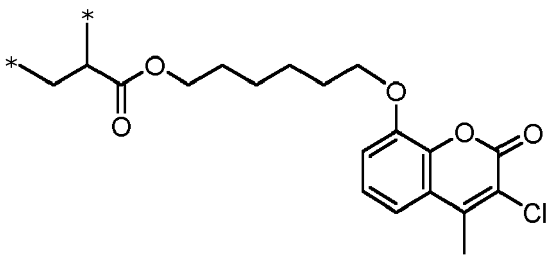 | M0-010 |
| 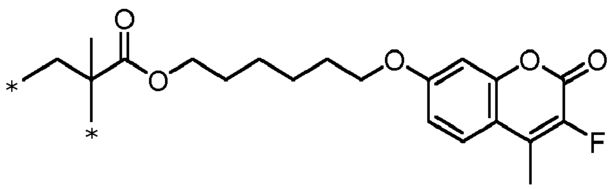 | M0-011 |

(continued)

| | |
|---|---|
| 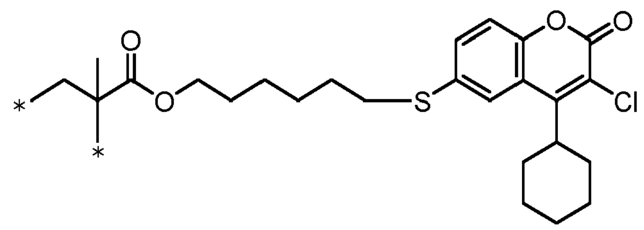 | $M^0$-012 |
| 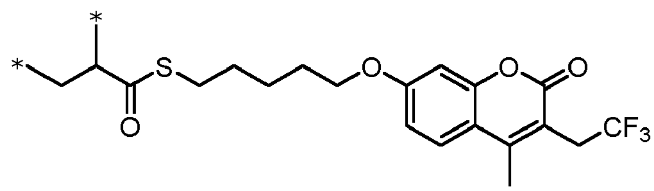 | $M^0$-013 |
| 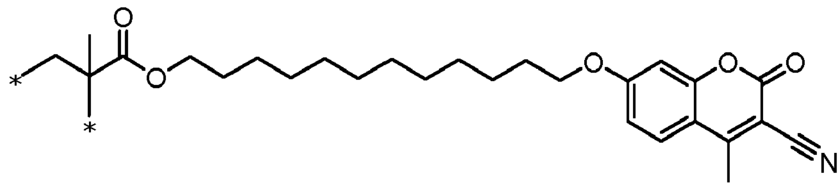 | $M^0$-014 |
| 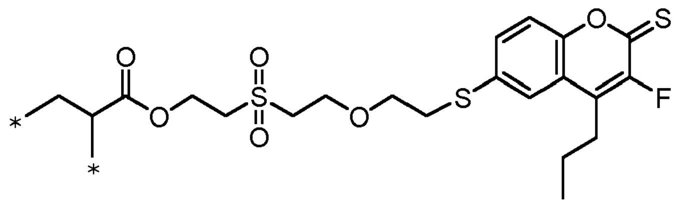 | $M^0$-015 |
| 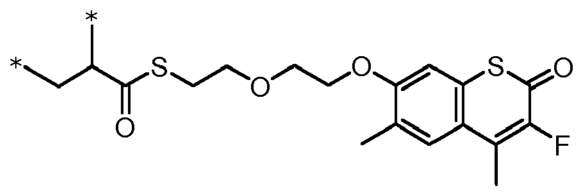 | $M^0$-016 |
| 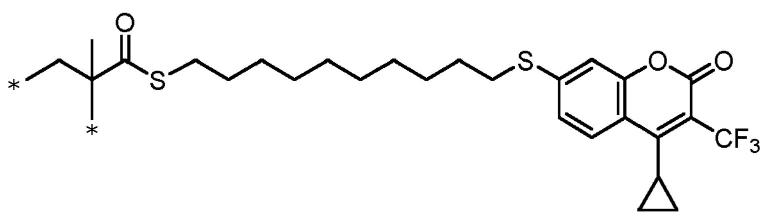 | $M^0$-017 |
| 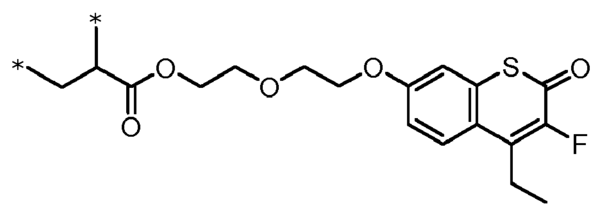 | $M^0$-018 |

(continued)

| | |
|---|---|
| 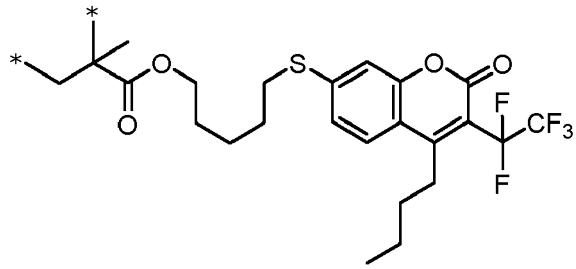 | $M^0$-019 |
| 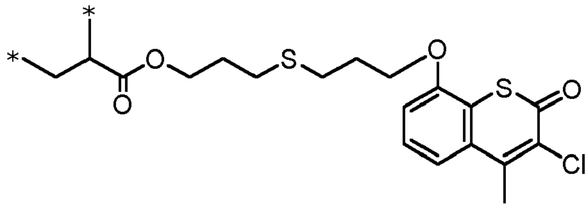 | $M^0$-020 |
| 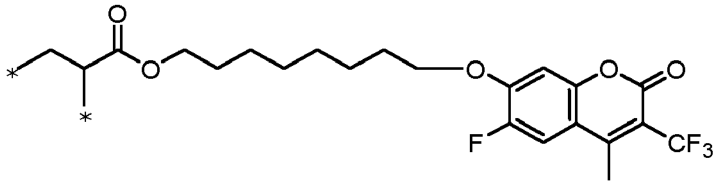 | $M^0$-021 |
| 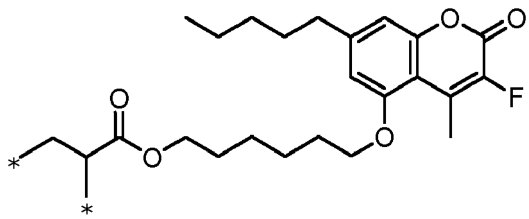 | $M^0$-022 |
| 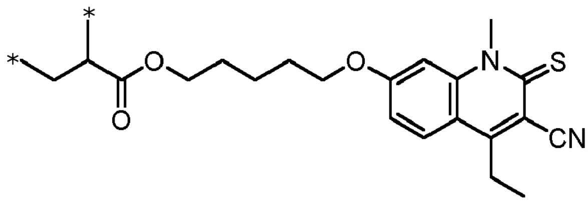 | $M^0$-023 |
| 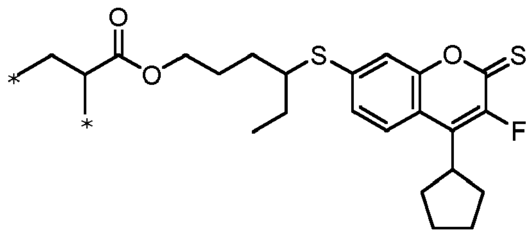 | $M^0$-024 |
| 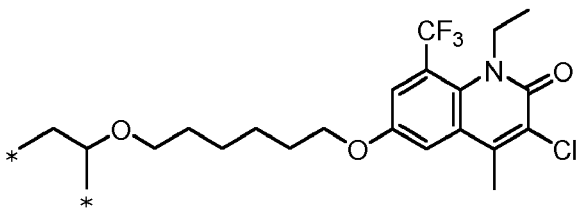 | $M^0$-025 |

(continued)

| | |
|---|---|
| 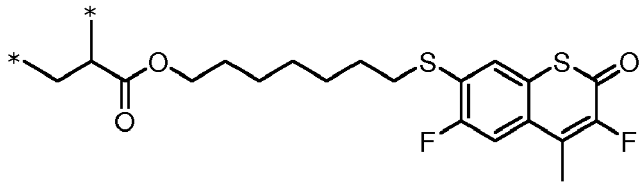 | M0-026 |
| 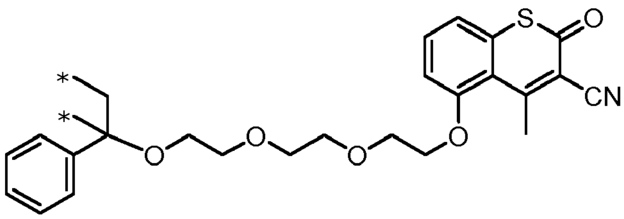 | M0-027 |
| 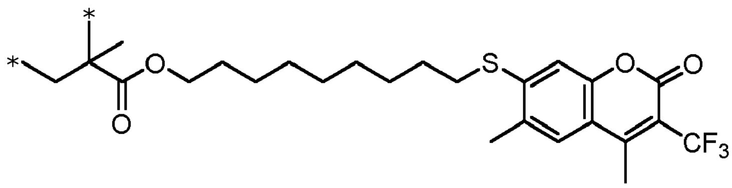 | M0-028 |
| 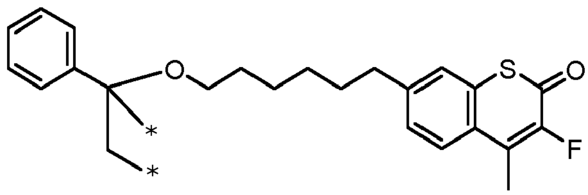 | M0-029 |
| 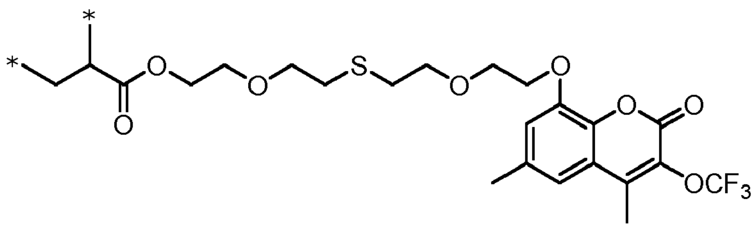 | M0-030 |
| 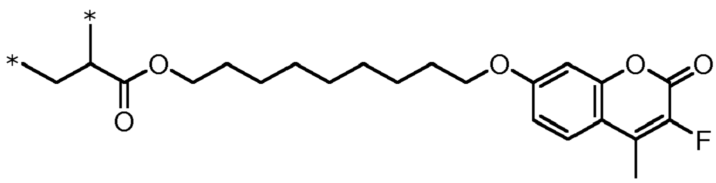 | M0-031 |
| 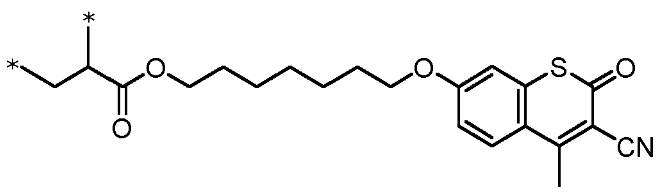 | M0-032 |

(continued)

| | |
|---|---|
| 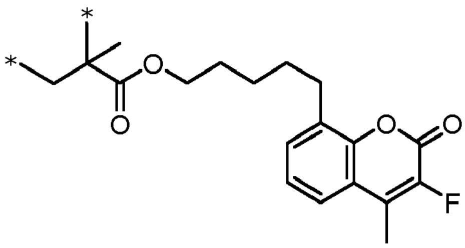 | $M^0$-033 |
| 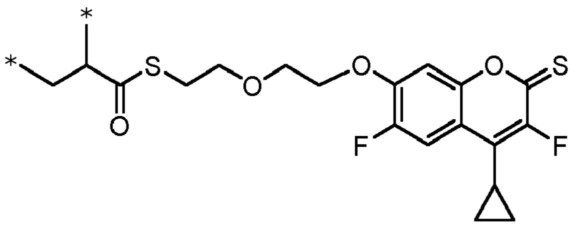 | $M^0$-034 |
| 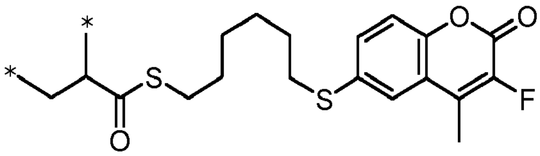 | $M^0$-035 |
| 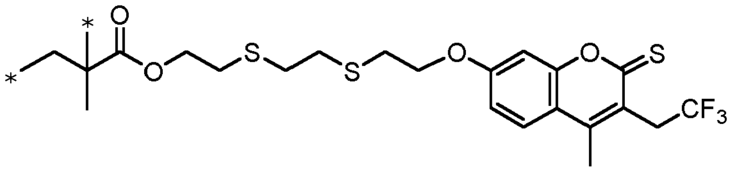 | $M^0$-036 |
| 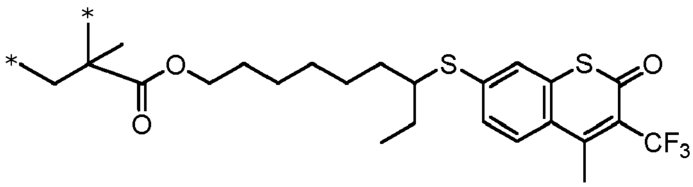 | $M^0$-037 |
| 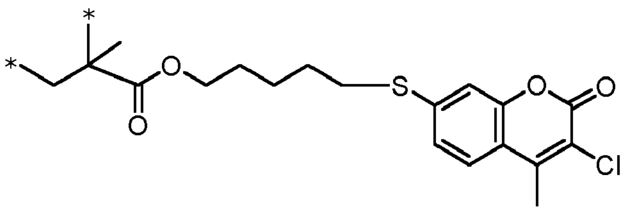 | $M^0$-038 |
| 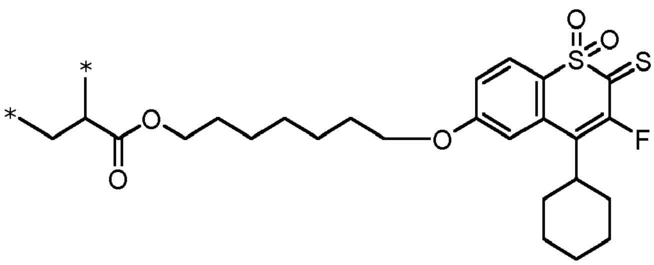 | $M^0$-039 |

(continued)

| | |
|---|---|
| 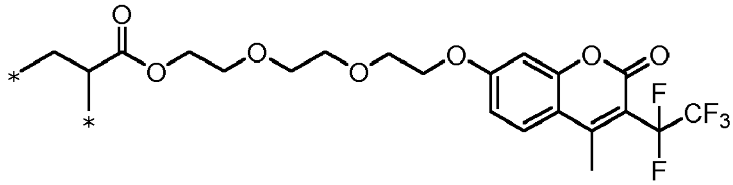 | $M^0$-040 |
| 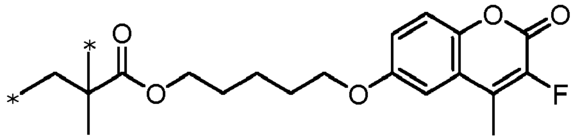 | $M^0$-041 |
| 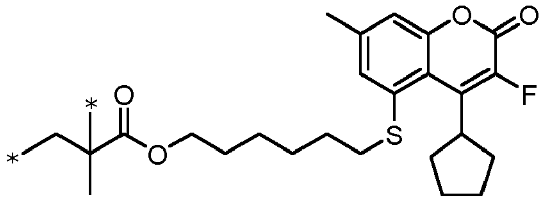 | $M^0$-042 |
| 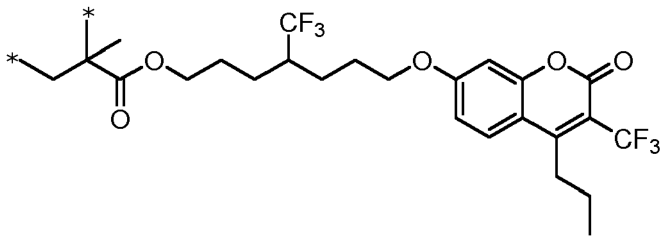 | $M^0$-043 |
| 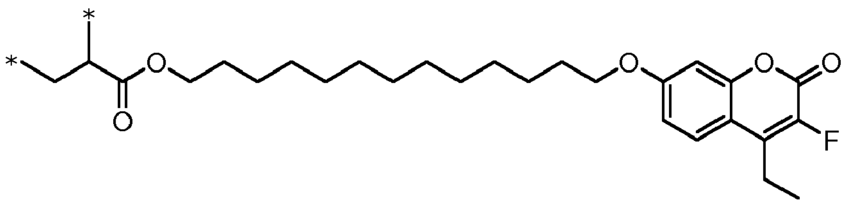 | $M^0$-044 |
| 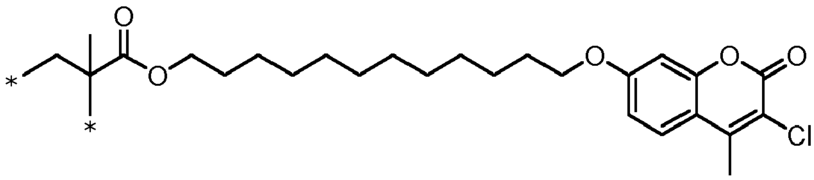 | $M^0$-045 |
| 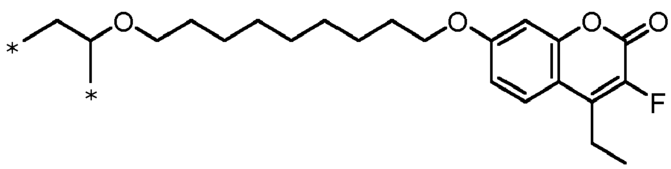 | $M^0$-046 |

(continued)

| | |
|---|---|
| 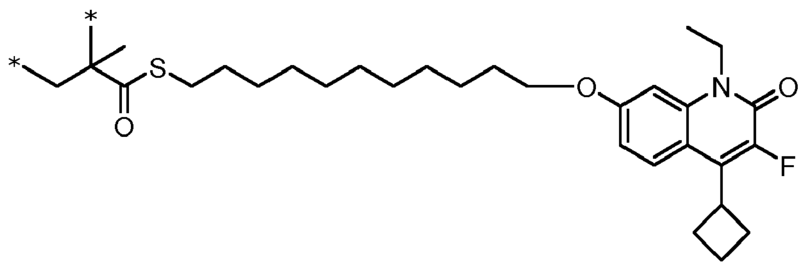 | $M^0$-047 |
| 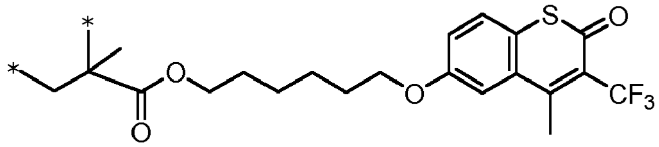 | $M^0$-048 |
| 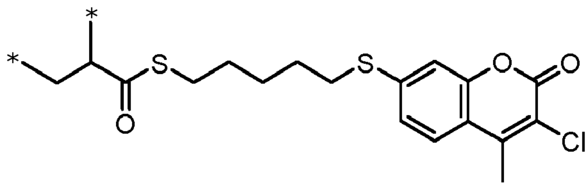 | $M^0$-049 |
| 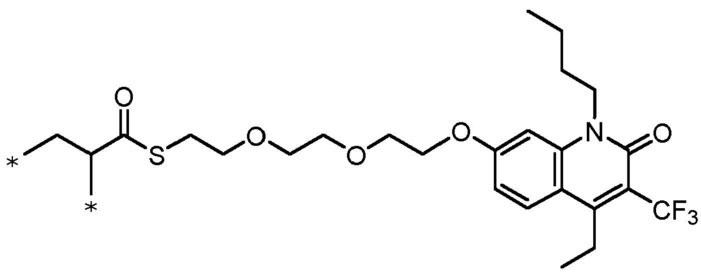 | $M^0$-050 |
| 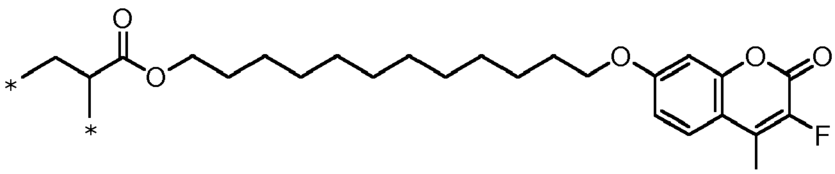 | $M^0$-051 |
| 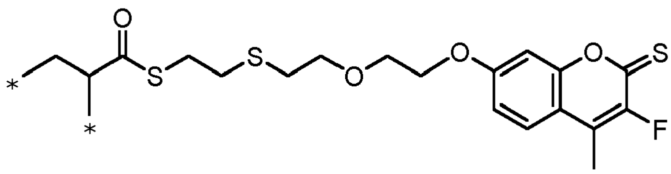 | $M^0$-052 |

(continued)

| | |
|---|---|
| 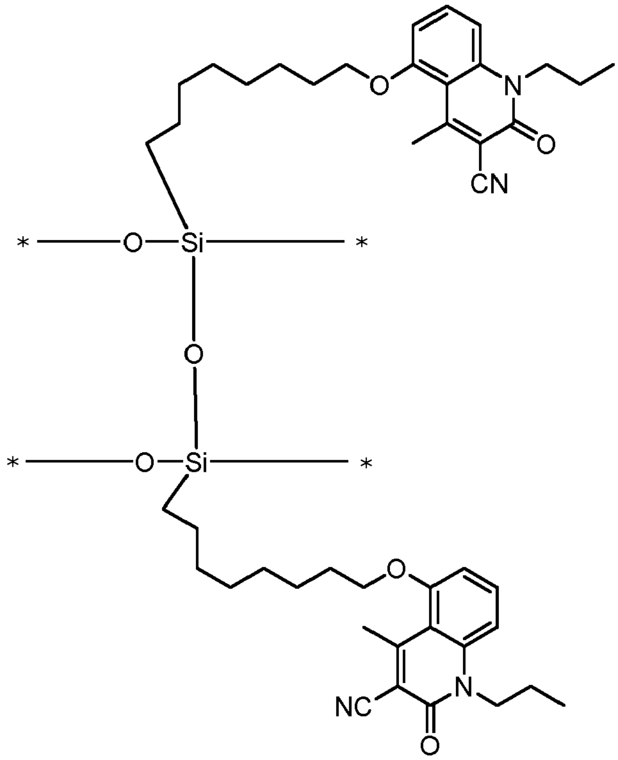 | $M^0$-053 |
| 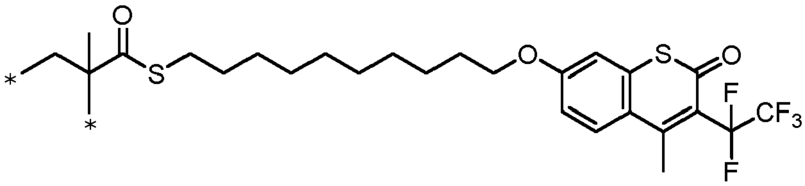 | $M^0$-054 |
| 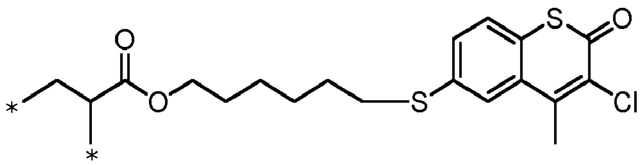 | $M^0$-055 |
| 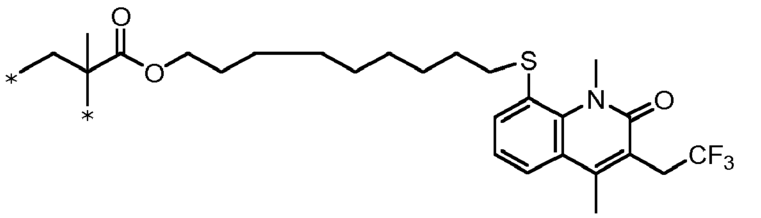 | $M^0$-056 |
| 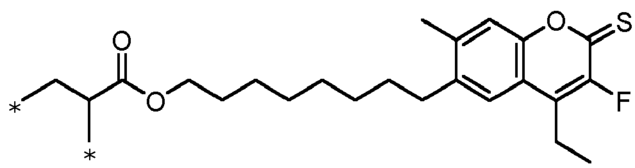 | $M^0$-057 |

(continued)

| | |
|---|---|
| 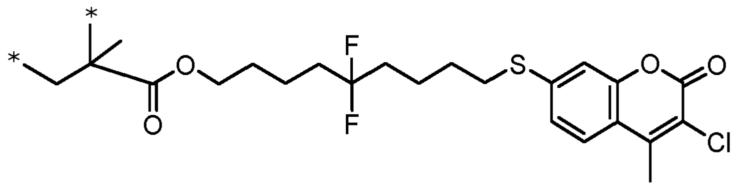 | $M^0$-058 |
| 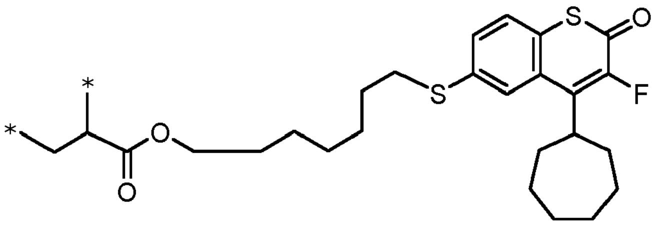 | $M^0$-059 |
| 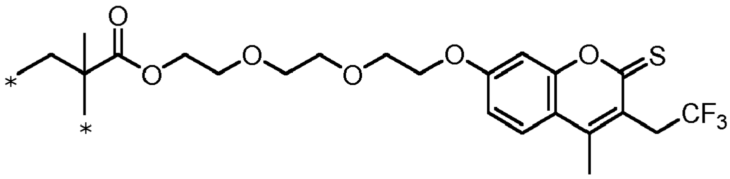 | $M^0$-060 |
| 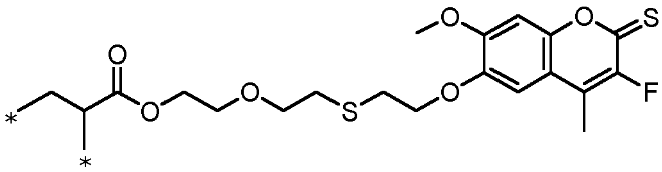 | $M^0$-061 |
| 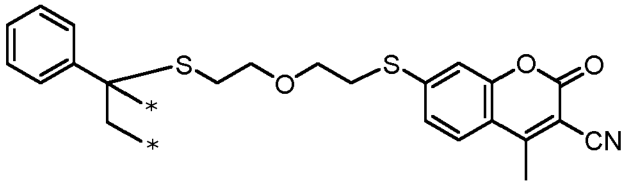 | $M^0$-062 |
| 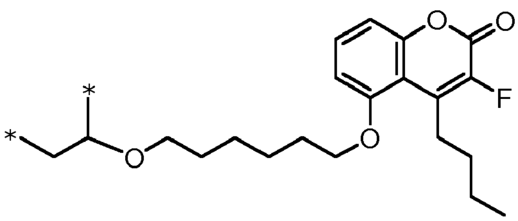 | $M^0$-063 |
| 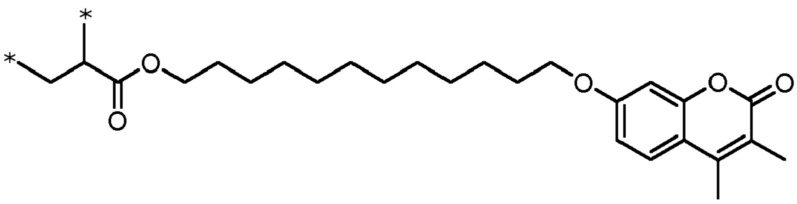 | $M^0$-064 |
| 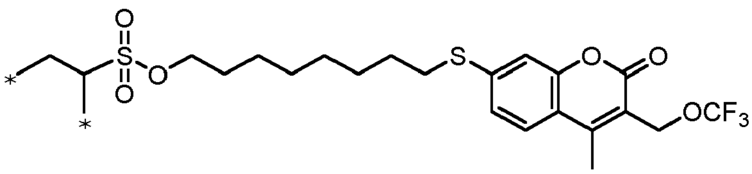 | $M^0$-065 |

(continued)

| | |
|---|---|
| 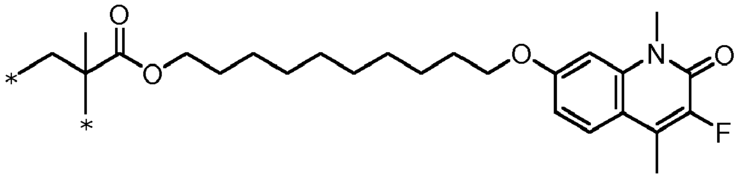 | $M^0$-066 |
| 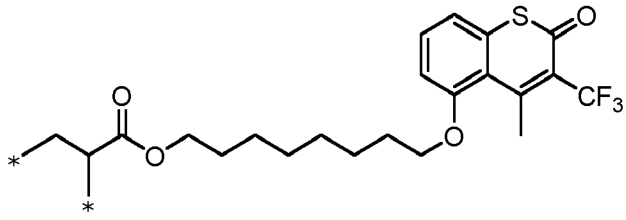 | $M^0$-067 |
| 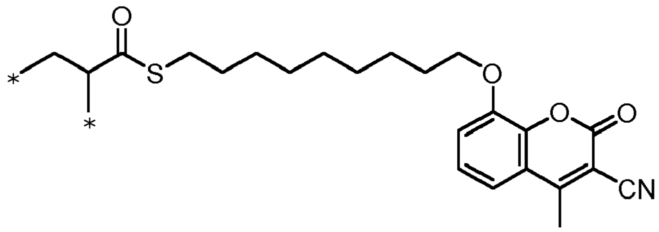 | $M^0$-068 |
| 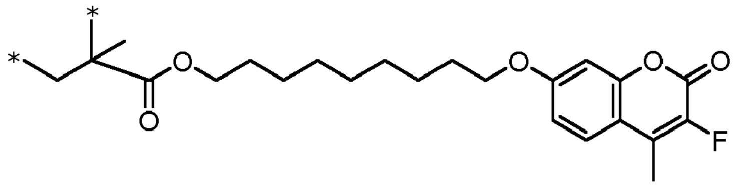 | $M^0$-069 |
| 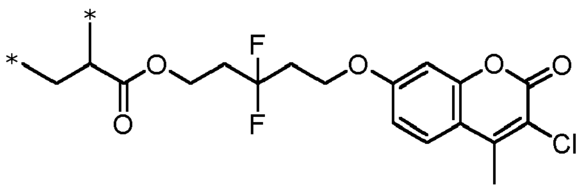 | $M^0$-070 |
| 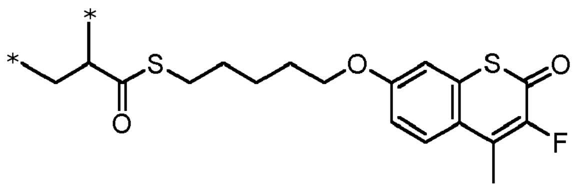 | $M^0$-071 |
| 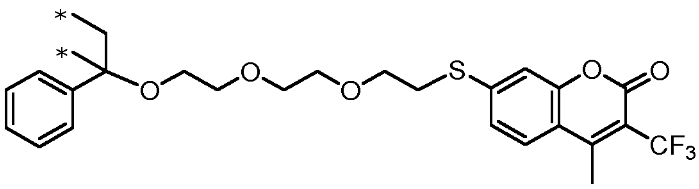 | $M^0$-072 |

(continued)

| | |
|---|---|
| 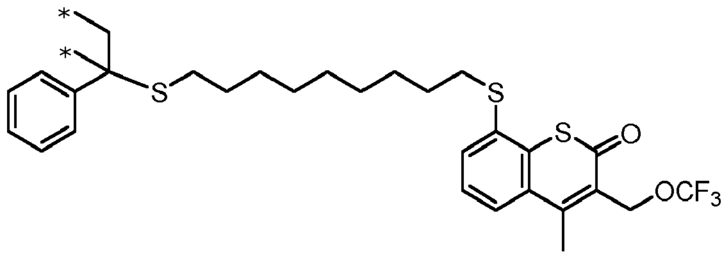 | $M^0$-073 |
| 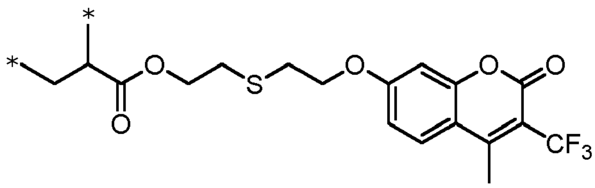 | $M^0$-074 |
| 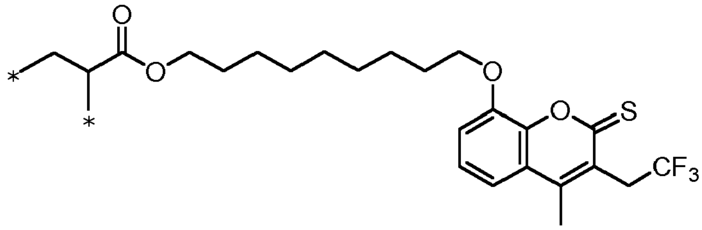 | $M^0$-075 |
| 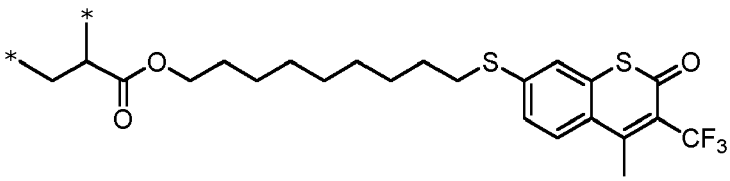 | $M^0$-076 |
| 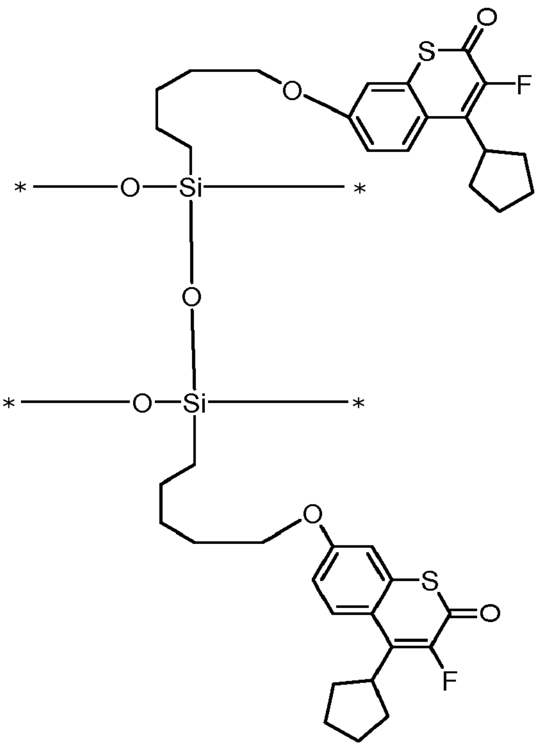 | $M^0$-077 |

(continued)

| | |
|---|---|
| 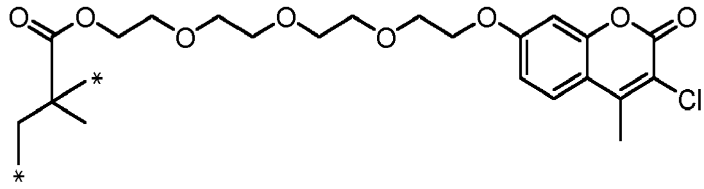 | M$^0$-078 |
| 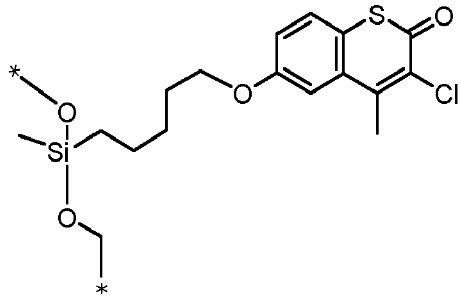 | M$^0$-079 |
| 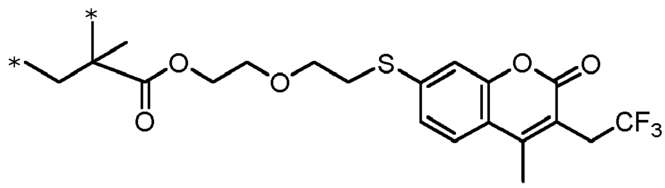 | M$^0$-080 |
| 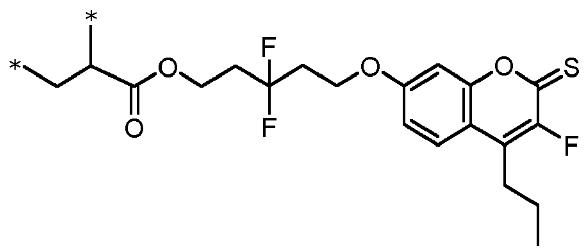 | M$^0$-081 |
| 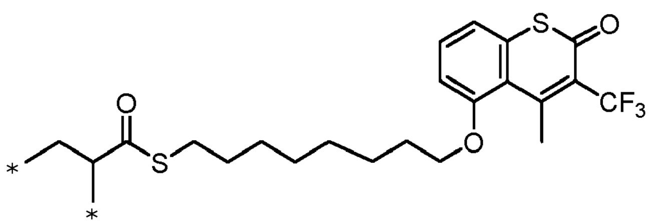 | M$^0$-082 |
| 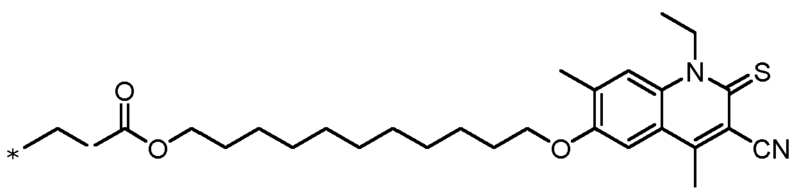 | M$^0$-083 |
| 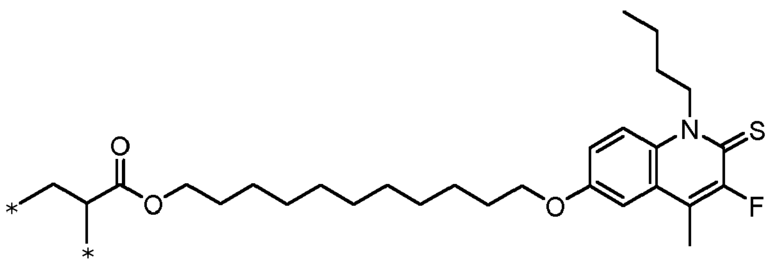 | M$^0$-084 |

(continued)

| | |
|---|---|
| 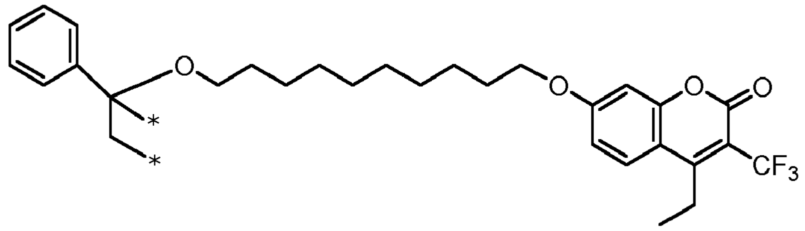 | $M^0$-085 |
| 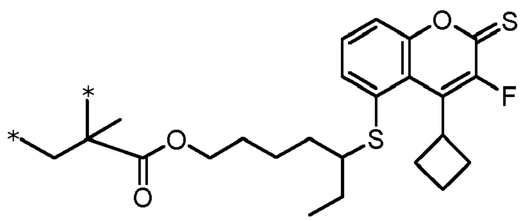 | $M^0$-086 |
| 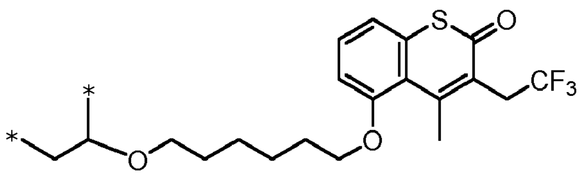 | $M^0$-087 |
| 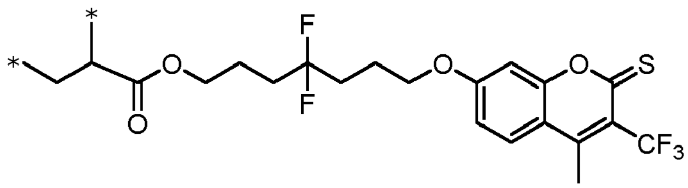 | $M^0$-088 |
| 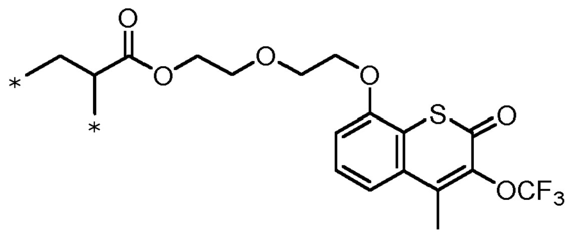 | $M^0$-089 |
| 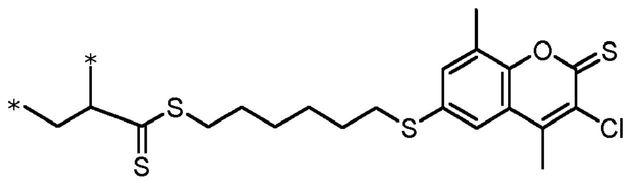 | $M^0$-090 |
| 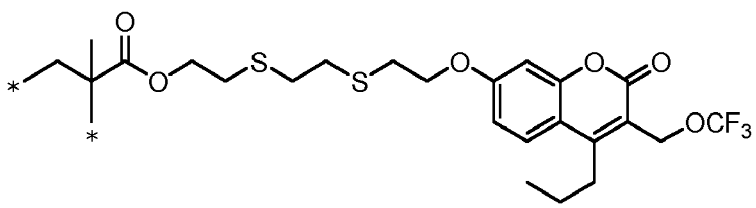 | $M^0$-091 |

(continued)

| | |
|---|---|
| 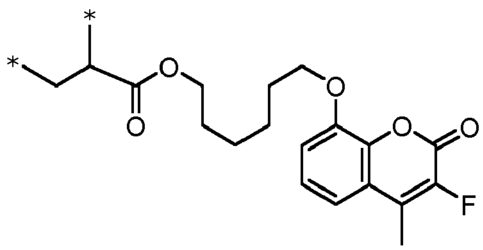 | $M^0$-092 |
| 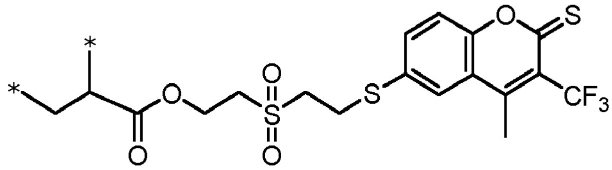 | $M^0$-093 |
| 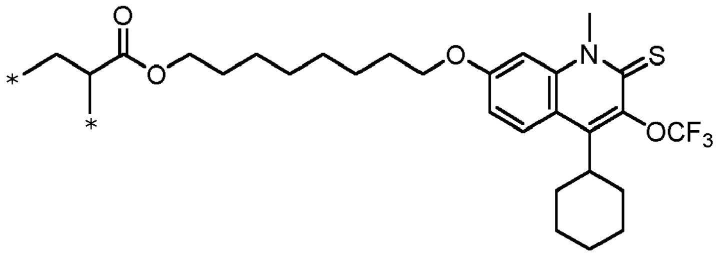 | $M^0$-094 |
| 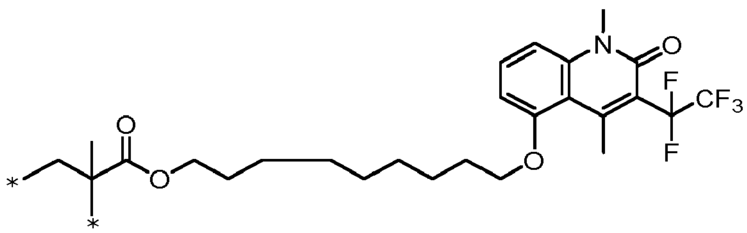 | $M^0$-095 |
| 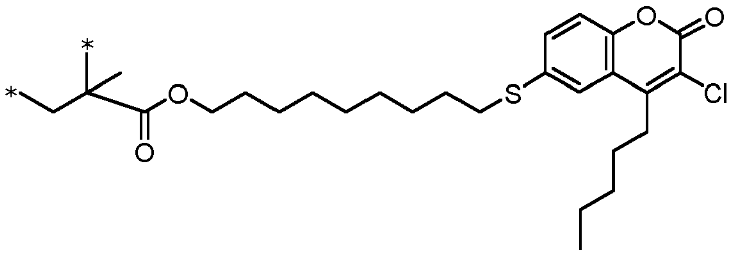 | $M^0$-096 |
| 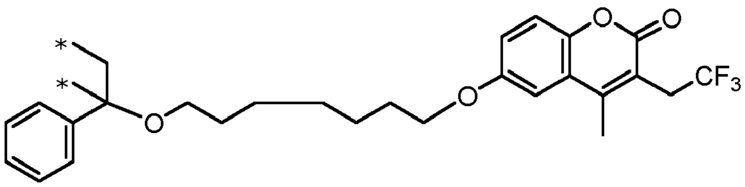 | $M^0$-097 |

(continued)

| | |
|---|---|
| 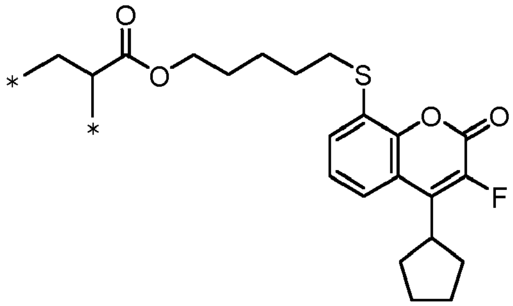 | $M^0$-098 |
| 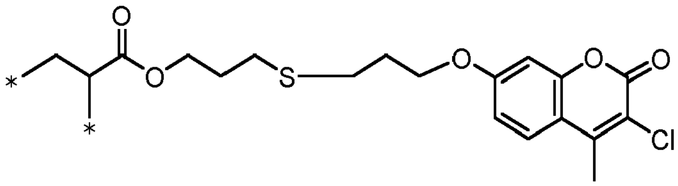 | $M^0$-099 |
| 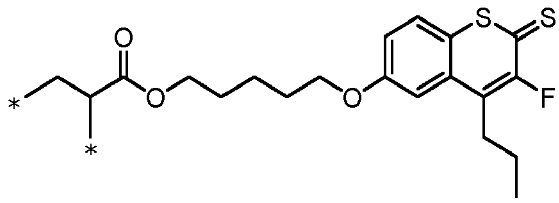 | $M^0$-100 |
| 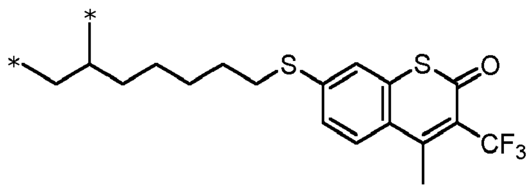 | $M^0$-101 |
| 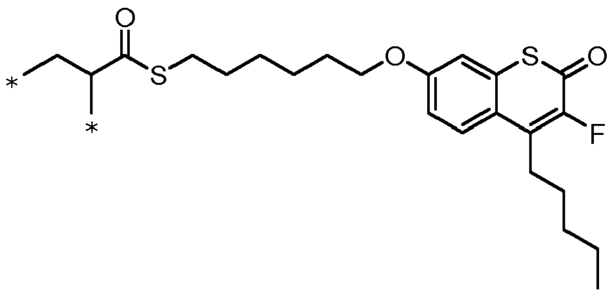 | $M^0$-102 |
| 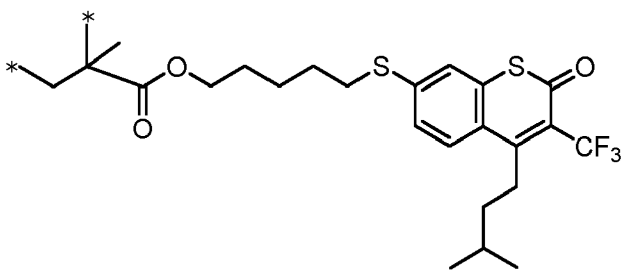 | $M^0$-103 |

(continued)

| | |
|---|---|
| 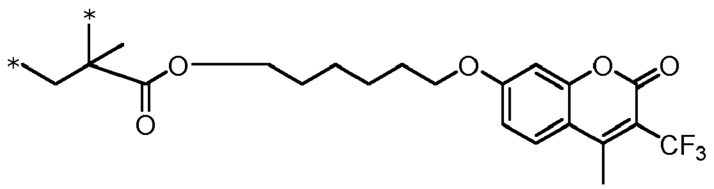 | $M^0$-104 |
| 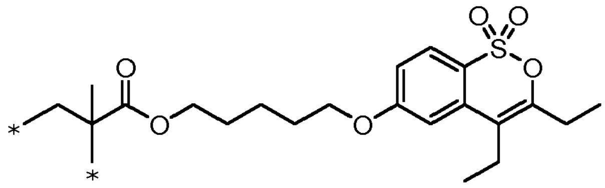 | $M^0$-105 |
| 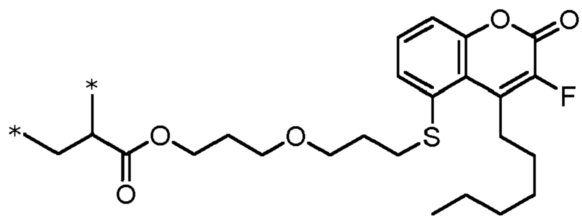 | $M^0$-106 |

Preferably a copolymer within the ophthalmic device or precursor material for an ophthalmic device according to the invention or the copolymer according to the invention as described before or preferably described before comprises the one or more constitutional units $M^0$ as described before with substituents as described before or preferably described before in a molar ratio m1 and the one or more constitutional units $M^2$ as described before or preferably described before in a molar ratio m2, wherein the ratio m1 : m2 is at least 0.01 and at most 100. Particularly preferably, such copolymer is comprised in the ophthalmic device or precursor article for an ophthalmic device according to the invention.

Preferably a copolymer within the ophthalmic device or precursor material for an ophthalmic device according to the invention or the copolymer according to the invention as described before or preferably described before comprises the one or more constitutional units $M^0$ as described before with substituents as described before or preferably described before in a concentration of at least 12 wt% to 96 wt%, preferably in a concentration of at least 20 wt% to 75 wt% or at least 25 wt% to 50 wt%.

Particularly preferably, such copolymer is comprised in the ophthalmic device or precursor article for an ophthalmic device according to the invention.

The invention therefore relates further to an ophthalmic device or a precursor article for the ophthalmic device as described or preferably described before wherein the total amount of photoactive chromophores of the polymerized compounds of formulae (I), (Ia), (Ib) or (Ic) or the total amount of constitutional unit $M^0$ of formulae ($M^0$-I), ($M^0$-Ia), ($M^0$-Ib) and/or ($M^0$-Ic) as described before or preferably described before or the total amount of the constitutional units ($M^0$-001) to ($M^0$-106) as described below is at least 12 wt% to 96 wt%, preferably at least 20 wt% to 75 wt%, particularly preferably or at least 25 wt% to 50 wt%.

The invention therefore relates further to an ophthalmic device or a precursor article for the ophthalmic device as described or preferably described before wherein the total amount of photoactive chromophores of the polymerized compounds of formulae (I), (Ia), (Ib) or (Ic) or the total amount of constitutional unit $M^0$ of formulae ($M^0$-I), ($M^0$-Ia), ($M^0$-Ib) and/or ($M^0$-Ic) as described before or preferably described before or the total amount of the constitutional units ($M^0$-001) to ($M^0$-106) as described below is at least 12 wt% to 96 wt%, preferably at least 20 wt% to 75 wt%, particularly preferably at least 25 wt% to 50 wt% and where the total amount of polymerized hydrophilic monomer as described before or preferably described before is at least 1 wt% to 80 wt%, preferably 5 wt% to 55 wt%, particularly preferably 8 wt% to 40 wt%, very particularly preferably 10 to 30 wt%.

The oligomers, polymers or copolymers, preferably polymers or copolymers, within the ophthalmic device or precursor material for an ophthalmic device according to the invention or the oligomers, polymers or copolymer according to the invention as described before or preferably described may be cross-linked. Particularly preferably, such polymer or copolymer is comprised in the ophthalmic device or precursor article for an ophthalmic device according to the invention.

The oligomers, polymers or copolymers within the ophthalmic device or precursor material for an ophthalmic device according to the invention or the oligomer, polymer or copolymer of the present invention may be made by any suitable method. It is, however, preferred that the present oligomers, polymers and copolymers are made by radical

polymerization, wherein the polymerization reaction is started by means of a suitable radical polymerization initiator. For the purposes of the present invention the type of radical polymerization initiator is not particularly limited and may be any suitable radical generating compound. Such compounds are well known to the skilled person. Suitable polymerization initiators may be selected from thermal initiators or photoinitiators, i.e. compounds that generate radicals by exposure to heat or irradiation with light of a suitable wavelength. Examples of suitable thermal polymerization initiators may be selected from the groups of compounds comprising one or more peroxide groups, i.e. compounds comprising a group -O-O-, and/or compounds comprising one or more azo groups, i.e. compounds comprising a group -NΞN-.

Suitable polymerization initiators comprising one or more peroxide groups may, for example, be selected from the groups consisting of t-butyl(peroxy-2-ethylhexanoate), di-(tert-butylcyclohexyl)peroxydicarbonate and benzoylperoxide.

Suitable polymerization initiators comprising one or more azo groups may, for example, be selected from the group consisting of 1,1'-azobis(cyclohexancarbonitrile) and 2,2'azobis(cyclohexanecarbonitrile) (AIBN).

Suitable examples of a photoinitiator are dimethylaminobenzoate /camphorquinone, diphenyl(2,4,6-trimethylbenzoyl)phosphine oxide (TPO) or phenylbis(2,4,6-trimethylbenzoyl)phosphine oxide (BAPO).

If a photoinitiator is used as polymerization initiator, it is preferred that the wavelength required to decompose said photoinitiator is different from the wavelength needed to irradiate the compound of the present application so as to change its optical properties.

Preferably, the radical initiators are used in an amount of at least 0.0001 eq and of at most 0.1 eq of the main monomer. Such radical initiators could be thermal initiators, e.g. azobisisobutyronitrile (AIBN) or photochemical initiators like dimethylaminobenzoate/camphorquinone, diphenyl(2,4,6-trimethylbenzoyl)phosphine oxide (TPO) or phenylbis(2,4,6-trimethylbenzoyl)phosphine oxide (BAPO).

Preferred ophthalmic devices are optically active ophthalmic devices. Examples of such ophthalmic devices or eye-implants include lenses, keratoprostheses, and corneal inlays or rings. More preferably, said ophthalmic device or eye-implant is a lens article. Most preferably, such ophthalmic device is a lens. The type of lens is not restricted and may comprise a contact lens or an intraocular lens. Most preferably, such ophthalmic device is an intraocular lens, which may, for example, be a posterior chamber intraocular lens or an anterior chamber intraocular lens.

A blank of this invention may be produced as a step in the manufacturing process used to create an ophthalmic device as described before, preferably an intraocular lens. For example, without limitation, a manufacturing process may include the steps of polymer synthesis, polymer sheet casting, blank cutting, optic lathe cutting, optic milling, haptic milling or attachment, polishing, solvent extraction, sterilization and packaging while the term polymer is used as described before or preferably described before.

The present ophthalmic devices or precursor articles for an ophthalmic device according to the invention as described before or preferably described before may be formed by a process comprising the steps of

- providing a composition comprising at least one compound of formulae (I), (Ia), (Ib) or (Ic) or the compounds (A-001) to (A-106) as described herein or preferably described herein and/or an oligomer or polymer as described herein or preferably described herein but having at least one reactive group left for polymerization and optionally further monomers different from compounds of formulae (I), (Ia), (Ib) and (Ic) or the compounds (A-001) to (A-106) as described herein or preferably described herein and crosslinking agents and/or UV absorbers and/or radical initiators; and
- subsequently forming the ophthalmic device or precursor article of said composition.

The present invention is also directed to a composition preferably for polymerization comprising a compound of formula (I)

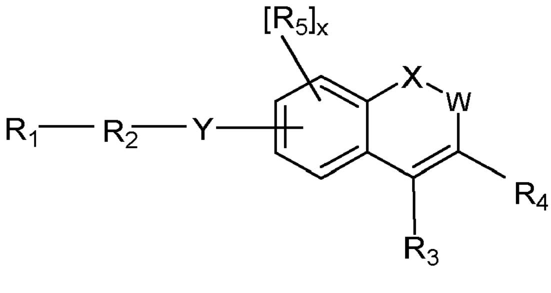

(I)

,

wherein

X-W is O-C(=O), O-C(=S), O-$SO_2$, $SO_2$-O, $SO_2$-C(=S), S-C(=O), S-C(=S), $NR_B$-C(=O) or $NR_B$-C(=S);

$R_B$ is at each occurrence independently H, a linear or branched alkyl group having 1 to 6 C atoms or a linear or branched partially or fully fluorinated alkyl group having 1 to 10 C atoms;

$R_3$ is a linear or branched alkyl group having 1 to 8 C atoms or a cycloalkyl group having 3 to 7 C atoms;

$R_4$ is F, Cl, a linear or branched alkyl group having 1 to 8 C atoms or a partially or fully fluorinated or partially or fully chlorinated alkyl group having 1 to 8 C atoms, a linear or branched partially or fully fluorinated alkoxy group having 1 to 8 C atoms, a linear or branched optionally partially or fully fluorinated alkoxyalkyl group having 2 to 8 C atoms or a cycloalkyl group having 3 to 6 C atoms which may be partially or fully fluorinated;

$R_5$ is at each occurrence independently a linear or branched alkyl group having 1 to 8 C atoms or a linear or branched alkoxy group having 1 to 8 C atoms;

x is 0 or 1;

Y is O, S, SO, $SO_2$, Se, $NR_0$ or a bond;

$R_0$ is at each occurrence independently selected from the group consisting of a linear or branched alkyl group having 1 to 4 C atoms;

$R_1$ is a trialkoxysilyl group or a dialkoxyalkylsilyl group where the alkyl and/or alkoxy groups are each independently linear or branched having 1 to 6 C atoms, or a silyl group of formula (1), (2) or (3) or a polymerizable group of formula (4),

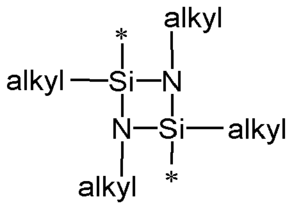 (1) , 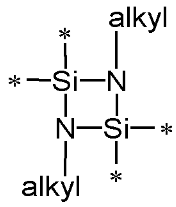 (2) ,

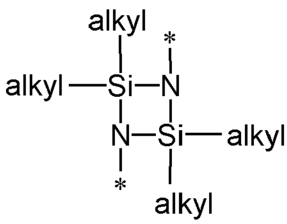 (3) , 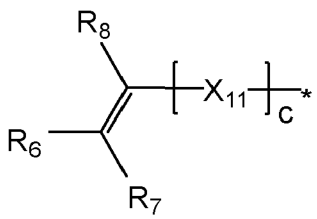 (4) ,

where alkyl means at each occurrence independently of each other a linear or branched alkyl group having 1 to 6 C atoms and the asterisk "*" denotes at each occurrence independently of each other a linkage to the linker $-R_2$-Y-;

and wherein

$X_{11}$ is selected from the group consisting of O, S, O-$SO_2$, $SO_2$-O, C(=O), OC(=O), C(=O)O, S(C=O) and (C=O)S,

$R_6$, $R_7$, $R_8$ are at each occurrence independently of each other selected from the group consisting of H, F, a linear or branched, non-fluorinated, partially or completely fluorinated alkyl group having 1 to 20 C atoms and aryl with 6 to 14 C atoms and

c is 0 or 1;

$-R_2-$ is $-(C(R)_2)_o-$;

R is at each occurrence independently selected from the group consisting of H, F, a linear or branched alkyl group having 1 to 4 C atoms or a linear or branched partially or fully fluorinated alkyl group having 1 to 4 C atoms; and

o is selected from the group consisting of 5 to 20

or comprising polymerized compounds of formulae (I), (Ia), (Ib) or (Ic) with substituents as described before or preferably described before and/or an oligomer or polymer derived from said compounds of formulae (I), (Ia), (Ib) or (Ic) having at least one reactive group left for polymerization and optionally further monomers different from compounds of formulae (I), (Ia), (Ib) or (Ic) .

Said composition preferably comprises a radical initiator and further monomers different from compounds of formula (I), (Ia), (Ib) or (Ic).

Depending upon the intended manufacturing process of the ophthalmic device or precursor article of said ophthalmic device such composition for polymerization as described or preferably described before may comprise further

different components. Such further components may, for example, be selected from the group consisting of UV absorbers, antioxidants and cross-linkers. Cross-linkers (crosslinkers) may also be referred to as crosslinking agents.

The present application is also directed to a composition for polymerization comprising at least one compound of formulae (I), (Ia), (Ib) or (Ic) or compounds (A-001) to (A-106) as described or preferably described before and/or an oligomer or polymer as described before or preferably described before but having at least one reactive group left for polymerization and a crosslinking agent and/or a UV absorber and/or a radical initiator and optionally further monomers different from compounds of formulae (I), (Ia), (Ib) and (Ic) or the compounds (A-001) to (A-106).

A composition comprising at least an oligomer or polymer comprising polymerized compounds of formulae (I), (Ia), (Ib) or (Ic) or polymerized compounds (A-001) to (A-106) as described or preferably described before is primarily used for the synthesis of block copolymers with the condition that the oligomer or polymer has at least one reactive group left which may react with the monomers.

The described compositions may include or comprise, essentially consist of or consist of the said requisite or optional constituents. All compounds or components which can be used in the compositions are either known and commercially available or can by synthesized by known processes or as described herein.

The components of the composition as described before are combined in such amounts that at least 12 wt% to 96 wt%, preferably at least 20 wt% to 75 wt%, particularly preferably at least 25 wt% to 50 wt% of photoactive chromophores of polymerized formulae (I), (Ia), (Ib) or (Ic) are comprised in the resulting oligomers, polymers or copolymers.

The components of the composition as described before are preferably combined in such amounts that at least 12 wt% to 96 wt%, preferably at least 20 wt% to 75 wt%, particularly preferably at least 25 wt% to 50 wt% of photoactive chromophores of polymerized formulae (I), (Ia), (Ib) or (Ic) are comprised in the resulting oligomers, polymers or copolymers and that at least 1 wt% to 80 wt%, preferably 5 wt% to 55 wt%, particularly preferably 8 wt% to 40 wt%, very particularly preferably 10 to 30 wt% of polymerized hydrophilic monomer as described before or preferably described before is comprised in the resulting oligomers, polymers or copolymers.

Besides one or more compounds of the formulae (I), (Ia), (Ib) or (Ic), as described above or preferably described or the compounds (A-001) to (A-106) and the hydrophilic monomers, as described above, in the said molar ratios, the composition according to the invention may comprise at least one further non-hydrophilic co-monomer which does not conform to the formula (I) and optionally at least one blue absorber.

Said non-hydrophilic monomers are preferably selected from styrene, methyl methacrylate (MMA), methyl acrylate, *n*-alkyl acrylates (the *n*-alkyl group comprising 2-20 C-atoms), *n*-alkyl methacrylates (the *n*-alkyl group comprising 2-20 C-atoms), *i*-alkyl acrylates (the *i*-alkyl group comprising 3-20 C-atoms), *i*-alkyl methacrylates (the *i*-alkyl group comprising 3-20 C-atoms), tetrahydrofuryl methacrylate (THFMA), heptafluorobutyl acrylate, heptafluorobutyl methacrylate, hexafluorobutyl acrylate, hexafluorobutyl methacrylate, hexafluoroisopropyl acrylate, hexafluoroisopropyle methacrylate, octafluoropentyl acrylate, octafluoropentyl methacrylate, petanfluoropropyl acrylate, pentafluoropropyl methacrylate, tetrafluoropropyl methacrylate, trifluoroethyl acrylate, trifluoroethyl methacrylate, trimethylcyclohexyl acrylate, trimethylcyclohexyl methacrylate, 2-(4-bromophenyl)ethyl methacrylate, 4-phenylbutyl methacrylate, 4-methylphenyl methacrylate, phenyl methacrylate, 4-methylphenyl acrylate, benzyl acrylate, benzyl methacrylate, 2-benzyloxyethyl methacrylate, 3-benzyloxypropyl methacrylate, phenylethyl acrylate, phenylethyl methacrylate, 3-phenylpropyl methacrylate, 2-phenoxyethyl acrylate, 2-benzyloxyethyl acrylate, 3-phenylpropyl acrylate, 4-methylbenzyl acrylate, phenyl acrylate, 2-(phenylthio)ethyl methacrylate, 4-phenylbutyl acrylate, 5-phenylpentyl acrylate, 3-benzyloxypropyl acrylate, 2-(phenylthio)propyl acrylate, 2-(phenylthio)ethyl acrylate, 4-phenylbutyl acrylate, 4-methylbenzyl methacrylate, 2-(2-methylphenyl)ethyl acrylate, 2-(2-methylphenyl)ethyl methacrylate, 2-(3-methylphenyl)ethyl acrylate, 2-(3-methylphenyl)ethyl methacrylate, 2-(4-methylphenyl)ethyl acrylate, 2-(4-methylphenyl)ethyl methacrylate, 2-(4-propylphenyl)ethyl acrylate, 2-(4-propylphenyl)ethyl methacrylate, 2-(4-(1-methylethyl)phenyl)ethyl acrylate, 2-(4-(1-methylethyl)phenyl)ethyl methacrylate, 2-(4-methoxyphenyl)ethyl methacrylate, 2-(4-methoxyphenyl)ethyl acrylate, 2-(4-cyclohexylphenyl)ethyl acrylate, 2-(4-cyclohexylphenyl)ethyl methacrylate, 2-(4-chlorophenyl)ethyl methacrylate, 2-(4-chlorophenyl)ethyl acrylate, 2-(3-chlorophenyl)ethyl methacrylate, 2-(3-chlorophenyl)ethyl acrylate, 2-(2-chlorophenyl)ethyl methacrylate, 2-(2-chlorophenyl)ethyl acrylate, 2-(4-bromophenyl)ethyl acrylate, 2-(4-phenylphenyl)ethyl methacrylate, 2-(4-phenylphenyl)ethyl acrylate, 2-(3-phenylphenyl)ethyl methacrylate, 2-(3-phenylphenyl)ethyl acrylate, 2-(4-benzylphenyl)ethyl methacrylate, 2-(4-benzylphenyl)ethyl acrylate, 2-(phenylthio)ethyl acrylate, 3-benzyloxypropyl acrylate, 2-[2-(benzyloxy)ethoxy]ethyl acrylate, 2-[2-(benzyloxy)ethoxy]ethyl methacrylate, pentafluorophenyl methacrylate, heptadecafluorodecyl methacrylate, dodecafluoroheptyl methacrylate, heptadecafluorodecyl acrylate, pentafluorophenyl acrylate.

Suitable blue absorbers are substances which exhibit absorption in the blue wavelength region of visible light. A blue absorber which is likewise an acrylate or a methacrylate and is available as further monomer during the polymerisation is preferably selected. Suitable blue absorbers are known from the literature, for example from WO 2012/167124. A particularly preferred blue absorber is N-2-[3-(2'-methylphenylazo)-4-hydroxyphenylethyl]ethylmethacrylamide. They can be added to the composition as described in order that the polymerised composition is also able to filter short-wave visible light in addition to the UV light in order thus to protect the retina better if the material is used for the production

of an ophthalmological product.

The UV absorber that may be used in the composition as described before is not particularly limited and can easily be selected from those generally known to the skilled person. Generally suitable UV absorbers are characterized by being unsaturated compounds, preferably compounds comprising one or more selected from group consisting of olefinic groups, aryl groups and heteroaryl groups; these groups may be present in any combination.

Suitable UV-absorber for use in the composition for polymerization as described before may, for example, be selected from those comprising a group selected from benzotriazole, benzophenone and triazine. Suitable UV-absorbers are, for example, disclosed in U.S. Pat. Nos. 5,290,892; 5,331,073 and 5,693,095.

Suitable UV-absorber are 2-(3-(t-butyl)-4-hydroxy-5-(5-methoxy-2-benzotriazolyl)phenoxy)ethyl methacrylate, 3-(3-(t-butyl)-4-hydroxy-5-(5-methoxy-2-benzotriazolyl)phenoxy)propyl methacrylate, 3-(3-t-butyl-5-(5-chlorobenzotriazol-2-yl)-4-hydroxyphenyl)propyl methacrylate, 3-(3-(tert-butyl)-4-hydroxy-5-(5-methoxy-2H-benzo[d][1,2,3]triazol-2-yl)phenoxy)propylmethacrylate, 2-(2-hydroxy-5-vinylphenyl)-2H-benzotriazol, allyl-2-hydroxybenzophenon, 2-allyl-6-(2*H*-benzotriazol-2-yl)-*p*-cresol, 4-methacryloxy-2-hydroxybenzophenon, 2-(2'-hydroxy-3'-methallyl-5'-methylphenyl)benzotriazol, 2-hydroxy-4-methacryloyloxybenzophenon, 4-acryloylethoxy-2-hydroxybenzophenon, 2-[3-(2H-benzotriazol-2-yl)-4-hydroxyphenyl]ethylmethacrylate, 2-(2'-hydroxy-5'-methacrylamidophenyl)-5-methoxybenzotriazol, 2-(2'-hydroxy-5'-methacrylamidophenyl)-5-chlorobenzotriazol, 2-(2'-hydroxy-5'-methacryloxypropylphenyl)benzotriazol, 2-(2'-hydroxy-5'-methacryloylpropyl-3'-tert-butyl-phenyl)-5-methoxy-2H-benzotriazol, 2-(3-(tert-butyl)-4-hydroxy-5-(5-methoxy-2H-benzo[d][1,2,3]triazol-2-yl)phenoxy)ethylmethacrylat, 2-[3'-tert-butyl-2'-hydroxy-5'-(3"-methacryloyloxypropyl)phenyl]-5-chlorbenzotriazol, 2-{2'-hydroxy-3'-tert-butyl-5'-[3'-methacryloyloxypropoxy]phenyl}-5-methoxy-2H-benzotriazol, 2-[3'tert-butyl-5'-(3"-dimethylvinylsilylpropoxy)-2'-hydroxyphenyl]-5-methoxybenzotriazol, 2-(tert-butyl)-6-(5-chloro-2H-benzo[d][1,2,3]triazol-2-yl)-4-vinylphenol, 2-(2H-1,2,3-benzotriazol-2-yl)-4-methyl-6-(2-methylprop-2-enyl)phenol, 2-(3-acetyl-2-aminophenoxy)ethyl methacrylat, 2-(4-benzoyl-3-hydroxyphenoxy)ethyl acrylate, 3-[3-(2H-1,2,3-benzotriazol-2-yl)-5-tert-butyl-4-hydroxyphenyl]propyl methacrylate or a combination of this compounds.

Preferred UV-Absorber are selected from the group of 2-[3'-2'H-benzotriazol- 2'-yl)-4'-hydroxyphenyl]ethyl methacrylate (BTPEM), 2-(3-(t-butyl)-4-hydroxy-5-(5-methoxy-2-benzotriazolyl)phenoxy)ethyl methacrylate, 3-(3-(t-butyl)-4-hydroxy-5-(5-methoxy-2-benzotriazolyl)phenoxy)propyl methacrylate, 3-(3-t-Butyl-5-(5-chlorobenzotriazol-2-yl)-4-hydroxyphenyl)propyl methacrylate, 3-[3-(2H-1,2,3-benzotriazol-2-yl)-5-tert-butyl-4-hydroxyphenyl]propyl methacrylate which may be polymerized together with the monomers as described or preferably described before.

A crosslinker is a monomer containing at least two polymerizable groups. The crosslinker preferably has two polymerizable groups. The crosslinker may optionally also contain functional groups which are capable of coordinating water, such as, for example, OH or $NH_2$ groups. Crosslinkers functionalised in this way are likewise suitable hydrophilic monomers in the sense of the invention and are preferably employed in combination with the hydrophilic co-monomers described above and below.

Suitable cross-linker may be used to impart elastomeric properties to the present composition and the ophthalmic devices or precursor articles produced therewith. Typically any suitable di- or tri-functional monomer may be used as crosslinker. Such monomers are generally well known to the skilled person and may be selected from the group of para-divinylbenzene, allyl acrylate, ethylene glycol divinyl ether, divinyl sulfone, allyl methacrylate, N,N'-methylene-bis-acrylamide, ethylene glycol diacrylate, ethyleneglycoldimethacrylate (EGDMA), N,N'-methylene-bis-methacrylamide, 1,3-propanediol diacrylate, 2,3-propanediol diacrylate, 1,4-butanediol diacrylate, 1,3-butanediol diacrylate, 1,5-pentanediol diacrylate, 1,6-hexanediol diacrylate, 1,7-heptanediol diacrylate, 1,8-octanediol diacrylate, 1,9-nonanediol diacrylate, 1,10-decanediol diacrylate, 1,11-undecanediol diacrylate, 1,12-dodecanediol diacrylate, 1,13-tridecanediol diacrylate, 1,14-tetradecanediol diacrylate, 1,15-pentadecanediol diacrylate, 1,16-hexadecanediol diacrylate, 1,17-heptadecanediol diacrylate, 1,18-octadecanediol diacrylate, 1,19-nonadecanediol diacrylate, 1,20-eicosanediol diacrylate, 1,21-heneicosanediol diacrylate, 1,22-docosanediol diacrylate, 1,23-tricosanediol diacrylate, 1,24-tetracosanediol diacrylate, ethylene glycol dimethacrylate, N,N'-dihydroxyethylene-bisacrylamide, thiodiethylene glycol diacrylate, 1,3-propanediol dimethacrylate, 2,3-propanediol dimethacrylate, 1,3-butanediol dimethacrylate, 1,4-butanediol dimethacrylate, 1,5-pentanediol dimethacrylate, 1,6-hexanediol dimethacrylate, 1,7-heptanediol dimethacrylate, 1,8-octanediol dimethacrylate, 1,9-nonanediol dimethacrylate, 1,10-decanediol dimethacrylate, 1,11-undecanediol dimethacrylate, 1,12-dodecanediol dimethacrylate, 1,13-tridecanediol dimethacrylate, 1,14-tetradecanediol dimethacrylate, 1,15-pentadecanediol dimethacrylate, 1,16-hexadecanediol dimethacrylate, 1,17-heptadecanediol dimethacrylate, 1,18-octadecanediol dimethacrylate, 1,19-nonadecanediol dimethacrylate, 1,20-eicosanediol dimethacrylate, 1,21-heneicosanediol dimethacrylate, 1,22-docosanediol dimethacrylate, 1,23-tricosanediol dimethacrylate, 1,24-tetracosanediol dimethacrylate, 2-(acryloyloxy)ethyl methacrylate, 2-(acryloyloxy)propyl methacrylate, 3-(acryloyloxy)propyl methacrylate, 4-(acryloyloxy)butyl methacrylate, 5-(acryloyloxy)pentyl methacrylate, 6-(acryloyloxy)hexyl methacrylate, 7-(acryloyloxy)heptyl methacrylate, 8-(acryloyloxy)octyl methacrylate, 9-(acryloyloxy)nonyl methacrylate, 10-(acryloyloxy)decyl methacrylate, 11-(acryloyloxy)undecyl methacrylate, 12-(acryloyloxy)dodecyl methacrylate, 13-(acryloyloxy)tridecyl methacrylate, 14-(acryloyloxy)tetradecyl methacrylate, 15-(acryloyloxy)pentadecyl methacrylate, 16-(acryloyloxy)hexadecyl methacrylate, 17-(acryloyloxy)heptadecyl methacrylate, 18-(acryloyloxy)octadecyl methacrylate, 19-(acryloyloxy)nonadecyl methacrylate, 20-(acryloyloxy)eicosanyl methacrylate, 21-(acryloyloxy)heneicosanyl methacrylate, 22-(acryloyloxy)-docosanyl methacrylate, 23-(acryloyloxy)tricosanyl methacrylate, 24-(acryloyloxy)-tetracosanyl methacrylate, neopentyl glycol diacrylate, di(ethylene glycol) diacrylate, N,N'-hexamethylenebisacrylamide, thiodiethylene glycol diacrylate, thiodiethylene glycol dimethacrylate, neopentyl glycol dimethacrylate, diethylene glycol dimethacrylate, diallyl phthalate, triallyl cyanurate, glyceryl 1,3-dimethacrylate, N,N'-hexamethylenebismethacrylamide, tri(ethylene glycol) diacrylate, tri(ethylene glycol) dimethacrylate (e.g. $M_n$ 286) tetra(ethylene glycol) diacrylate, tetra(ethylene glycol) dimethacrylate, penta(ethylene glycol) diacrylate, penta(ethylene glycol) dimethacrylate, hexa(ethylene glycol) diacrylate, hexa(ethylene glycol) dimethacrylate, poly(ethylene glycol) diacrylate (e.g. $M_n$ 250 to 750), poly(ethyleneglycol) dimethacrylate (e.g. $M_n$ 250 to 750).

Preferred crosslinkers are ethylene glycol dimethacrylate, 1,3-propanediol diacrylate, 2,3-propanediol diacrylate, 1,4-butanediol diacrylate, 1,3-butanediol diacrylate, 1,5-pentanediol diacrylate, 1,6-hexanediol diacrylate, 1,7-heptanediol diacrylate, 1,8-octanediol diacrylate, 1,9-nonanediol diacrylate, 1,10-decanediol diacrylate, 1,11-undecanediol diacrylate, 1,12-dodecanediol diacrylate, 1,13-tridecanediol diacrylate, 1,14-tetradecanediol diacrylate, 1,15-pentadecanediol diacrylate, 1,16-hexadecanediol diacrylate, 1,17-heptadecanediol diacrylate, 1,18-octadecanediol diacrylate, 1,19-nonadecanediol diacrylate, 1,20-eicosanediol diacrylate, 1,21-heneicosanediol diacrylate, 1,22-docosanediol diacrylate, 1,23-tricosanediol diacrylate, 1,24-tetracosanediol diacrylate, 1,3-propanediol dimethacrylate, 2,3-propanediol dimethacrylate, 1,3-butanediol dimethacrylate, 1,4-butanediol dimethacrylate, 1,5-pentanediol dimethacrylate, 1,6-hexanediol dimethacrylate, 1,7-heptanediol dimethacrylate, 1,8-octanediol dimethacrylate, 1,9-nonanediol dimethacrylate, 1,10-decanediol dimethacrylate, 1,11-undecanediol dimethacrylate, 1,12-dodecanediol dimethacrylate, 1,13-tridecanediol dimethacrylate, 1,14-tetradecanediol dimethacrylate, 1,15-pentadecanediol dimethacrylate, 1,16-hexadecanediol dimethacrylate, 1,17-heptadecanediol dimethacrylate, 1,18-octadecanediol dimethacrylate, 1,19-nonadecanediol dimethacrylate, 1,20-eicosanediol dimethacrylate, 1,21-heneicosanediol dimethacrylate, 1,22-docosanediol dimethacrylate, 1,23-tricosanediol dimethacrylate, 1,24-tetracosanediol dimethacrylate, glyceryl 1,3-dimethacrylate and diallyl phthalate.

Particularly preferred crosslinkers are ethylene glycol dimethacrylate (EGDMA), poly(ethyleneglycol) diacrylate (e.g. $M_n$ 250 to 750), poly(ethyleneglycol) dimethacrylate (e.g. $M_n$ 250 to 750), tri(ethyleneglycol) dimethacrylate ($M_n$ 286), alkylene dimethacrylate (the alkylene group is linear or branched and comprises 2 to 18 C-atoms), alkylene diacrylate (the alkylene group is linear or branched and comprises 2 to 18 C-atoms), glyceryl 1,3-dimethacrylate and diallyl phthalate.

By using alkylene dimethacrylate as crosslinker, the alkylene group is preferably linear and comprises 2 to 18 C-atoms, preferably 14 to 18 C-atoms.
By using alkylene acrylate as crosslinker, the alkylene group is preferably linear and comprises 2 to 18 C-atoms, preferably 14 to 18 C-atoms.

Glyceryl 1,3- dimethacrylate (GDMA), ethylene glycol dimethacrylate (EGDMA), penta(ethylene glycol) dimethacrylate, hexa(ethylene glycol) dimethacrylate, polyethyleneglycol diacrylate (e.g. $M_n$ 250 to 750), polyethyleneglycol dimethacrylate (e.g. $M_n$ 250 to 750), triethyleneglycol dimethacrylate ($M_n$ 286), alkylene dimethacrylate or alkylene diacrylate are particularly preferred.

Ethylene glycol dimethacrylate (EGDMA), polyethyleneglycol diacrylate (e.g. $M_n$ 250 to 750), polyethyleneglycol dimethacrylate (e.g. $M_n$ 250 to 750) or a combination of these compounds is very particularly preferably selected in accordance with the invention.

Suitable antioxidants are phenyl acrylate derivatives bearing a hindered phenol moiety. A preferred antioxidant is

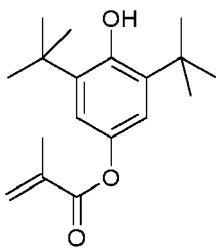

] .

The compounds of formulae (I), (Ia), (Ib) or (Ic) or the compounds (A-001) to (A-106) as described or preferably described before and their oligomers, polymers or copolymers as described before or preferably described before comprising one or more constitutional units $M^0$ of formulae ($M^0$-I), ($M^0$-Ia), ($M^0$-Ib) or ($M^0$-Ic) or one or more constitutional units ($M^0$-001) to ($M^0$-106) as described before or preferably described before are particularly well suited for use in optically active devices e.g. ophthalmic devices as described before.

The compounds of formulae (I), (Ia), (Ib) or (Ic) or the compounds (A-001) to (A-106) as described or preferably

described before and their oligomers, polymers or copolymers as described before or preferably described before comprising one or more constitutional units $M^0$ of formulae ($M^0$-I), ($M^0$-Ia), ($M^0$-Ib) or ($M^0$-Ic) or one or more constitutional units ($M^0$-001) to ($M^0$-106) as described before or preferably described before are particularly sensitive to two-photon or multiphoton absorption. Hence the ophthalmic device and the precursor article for the ophthalmic device are sensitive to two-photon or multiphoton absorption.

The system for two-photon or multi-photon irradiating of the ophthalmic device according to the invention, preferably of an intraocular lens preferably arranged within an eye of a patient is not restricted. Some examples are described below.

Hence the present invention is also directed to precursor articles wherein said precursor article is a blank which may be transformed into optically active ophthalmic devices comprising at least one oligomer, polymer or copolymer as described before or preferably comprising one or more constitutional units $M^0$ of formulae ($M^0$-I), ($M^0$-Ia), ($M^0$-Ib) or ($M^0$-Ic) or one or more constitutional units ($M^0$-001) to ($M^0$-106) as described before or preferably described before.

The type of intraocular lens is not limited in any way. It may, for example, be a pseudo-phakic intraocular lens or a phakic intraocular lens. The former type replaces the eye's natural, crystalline lens, usually to replace a cataractous lens that has been removed. The latter type is used to supplement an existing lens and functions as a permanent corrective lens, which is implanted in the anterior or posterior chamber to correct refractive errors of the eye. It may, for example, comprise one or more optic and one or more haptic components, wherein the one or more optic components serve as lens and the one or more haptic components are attached to the one or more optic components and hold the one or more optic components in place in the eye. The present intraocular lens may be of a one-piece design or of multi-piece design, depending on whether the one or more optic components and the one or more haptic components are formed from a single piece of material (one-piece design) or are made separately and then combined (multi-piece design). The present intraocular lens is also designed in such a way that it allows to be, for example, rolled up or folded small enough so that it fits through an incision in the eye, said incision being as small as possible, for example, at most 3 mm in length.

Additionally, intraocular lenses in accordance with the present invention allow for the non-invasive adjustment of the optical properties, particularly the polarizability or the refractive power, after implantation of the lens into the eye, thus reducing the need for post-surgery vision aids or reducing or totally avoiding follow-up surgery.

In order to change the optical properties and particularly the polarizability or refractive power of the ophthalmic device according to the invention e.g. an intraocular lens it is exposed to irradiation having a wavelength of at least 200 nm and of at most 1500 nm. Said irradiation is not limited and may be a based on a single-photon or two- or multi-photon process.

Hence, the present invention is also directed to a process of changing the optical properties of an ophthalmic device or a precursor article for an ophthalmic device as defined or preferably defined herein, said process comprising the steps of

- providing an ophthalmic device or a precursor article for an ophthalmic device as defined herein; and
- subsequently exposing said ophthalmic device or precursor article to irradiation having a wavelength of at least 200 nm and at most 1500 nm.

Preferably, said irradiation has a wavelength of at least 250 nm or 300 nm, more preferably of at least 350 nm, even more preferably of at least 400 nm, still even more preferably of at least 450 nm, and most preferably of at least 500 nm. Preferably, said irradiation has a wavelength of at most 1400 nm or 1300 nm or 1200 nm or 1100 nm or 1000 nm, more preferably of at most 950 nm or 900 nm, even more preferably of at most 850 nm, still even more preferably of at most 800 nm and most preferably of at most 750 nm.

Hence, the present invention is also directed to an ophthalmic device or precursor article for an ophthalmic device obtainable by said irradiation process as described before or preferably described before and below.

Alternatively, you may describe the change of refractive power as a modification of the index of refraction of said ophthalmic device as described before or preferably described before. Alternatively, you may describe the change of refractive power as a modification of the index of refraction of said intraocular lens as described before or preferably described before. Irradiation within the focal volume results in refractive optical structures characterized by a change in refractive index relative to the index of refraction of the bulk of said ophthalmic device or alternatively the non-irradiated portion of said ophthalmic device. Irradiation within the focal volume results in refractive optical structures characterized by a change in refractive index relative to the index of refraction of the bulk of said ophthalmic device or intraocular lens or alternatively the non-irradiated portion of said ophthalmic device or intraocular lens. The change in polarizability or refractive index can in other words be used to form patterned desired refractive structures in the optical ophthalmic device as described or preferably described before, preferably in the intraocular lens as described or preferably described before.

Hence, the present invention is also directed to an ophthalmic device obtainable by said irradiation process as

described before or preferably described before and below having refractive optical structures characterized by a change in refractive index relative to the index of refraction of the bulk of said ophthalmic device or alternatively the non-irradiated portion of said ophthalmic device.

It is preferred to provide refractive structures that exhibit a change in refractive index, and exhibit little or no scattering loss in such a way that ablation or removal of the optical ophthalmic device, preferably the intraocular lens article, is not observed in the irradiated region.

In such processes, the irradiated regions of the ophthalmic device as described before or preferably described before can take the form of two- or three-dimensional, area or volume filled refractive structures that can provide spherical, aspherical, toroidal, or cylindrical correction. In fact, any optical structure can be formed to yield power correction in both physical directions. Moreover, the optical structures can be stacked vertically or written in separate planes in the ophthalmic device as described before or preferably described before to act as a single lens element.

The invention is therefore further related to a method for locally adjusting a polarizability and/or a refractive index of an ophthalmic device according to the invention, preferably an intraocular lens, preferably arranged within an eye of a patient. The method relates in particular to fabrication of optical profiles by adjusting polarizability through two- or multi-photon processes in a non-destructive manner. Said two- or multi-photon processes allow for different optical profiles compared to single-photon processes and can be advantageously used for the manufacture of the ophthalmic devices according to the invention containing optical profiles.

The system to be used for said two- or multi-photon process advantageously allows for post-operative and non-invasive adjustment of optical properties/profiles of an implanted intraocular lens (IOL) to remove visual impairments such as refractive errors. Furthermore, when manufacturing the ophthalmic device according to the invention, the system advantageously allows for a gentle preparation of the ophthalmic device so as to in particular allow for refractive structures that can provide spherical, aspherical, toroidal, or cylindrical correction and/or maintaining flexibility of the ophthalmic device once preparation of the ophthalmic device is completed. The polarizability of the ophthalmic device is modified based on a two-photon (or generally multi-photon) process which allows adjustment of optical properties/profiles of said ophthalmic device or which allows adjustment of optical properties in different planes of the ophthalmic device. Furthermore, the modification of polarizability based on a two-photon or multi-photon process allows for improved maintaining of flexibility of the ophthalmic device when treated with wavelength of 400 nm to 590 nm.

Therefore, the invention further relates to a process for adjusting a polarizability of an ophthalmic device according to the invention based on a two- or multi-photon absorption process, the process comprising the steps of:

providing said ophthalmic device as described before or preferably described before; and
adjusting the polarizability of said ophthalmic device through irradiation of said ophthalmic device by using a system, said system comprising:

> one or more irradiation sources for two-photon or multi-photon irradiating a said ophthalmic device with an irradiation beam focused with an optic and of a first wavelength and/or a second wavelength different from the first wavelength,
> a scanner coupled to the one or more irradiation sources and configured to scan a said irradiation beam across said ophthalmic device, and
> an input unit coupled to the one or more irradiation sources and the scanner, wherein the input unit is configured to input data for treating said ophthalmic device by scanning a said irradiation beam across said ophthalmic device based on the input data, and
> wherein the first wavelength is between 600 nm and 800 nm for locally decreasing, based on said treating of said ophthalmic device, a polarizability of said ophthalmic device, and wherein the second wavelength is between 400 nm and 590 nm for locally increasing, based on said treating of said ophthalmic device, the polarizability of said ophthalmic device and thereupon changing the polymeric optical material of said ophthalmic device, preferably with significant differences in the UV/Vis spectrum with respect to the non-irradiated polymeric optical material of the ophthalmic device.

Ultraviolet-visible spectroscopy or ultraviolet-visible spectrophotometry (UV-Vis or UV/Vis) is known to a person skilled in the art. It refers to absorption spectroscopy or reflectance spectroscopy in part of the ultraviolet and the full, adjacent visible spectral regions. Suitable UV/Vis spectrometers are commercially available. The choice of the UV/Vis spectrometer is not critical for the comparison of the UV/Vis spectrum of the initial ophthalmic device and the UV/Vis spectrum of said irradiated ophthalmic device to be made according to the present invention. As long as both measurements are made under comparable conditions so that the results can be compared which is known to the person skilled in the art. A suitable spectrometer is the UV/Vis spectrometer Lambda 900 from Perkin Elmer.

The polarizability may hereby be locally changed particularly precisely.

The invention is furthermore related to a method for correcting vision in a patient by modifying the refractive

index of an intraocular lens within the eye of said patient comprising

identifying and measuring the degree of vision correction of the patient;
determining the position and type of refractive structures to be written into said intraocular lens to correct the patient's vision; and
subsequently exposing said intraocular lens to two-photon or multi-photon irradiation having a wavelength between 600 nm and 800 nm to locally decrease the polarizability of the intraocular lens or exposing said intraocular lens or subsequently exposing said intraocular lens to two-photon or multi-photon irradiation having a wavelength between 400 nm and 590 nm to locally increase the polarizability of the intraocular lens.

In the present application, input data are all kinds of data used for creating the treatment plan which is defined as the translation of the ophthalmic need into control commands for the writing process of the ophthalmic device according to the invention. During the writing process the optical pattern is written by irradiation in said ophthalmic device.
The term "control commands" refers to commands directly controlling the process of writing as defined before. A control command may control e.g. the movement of the scanner.
The term "scanner" used within the description is not part of the input unit according to the invention. The "scanner" as described herein is a component of the system to be used in the process for adjusting a polarizability of an ophthalmic device according to the invention which controls the movement of the irradiation beam.
The ophthalmic need refers to the desired optical profile which has to be created in the ophthalmic device through the system as described.
The optical profile is the needed change defined by the surgeon according to the patient's examination results before or after the ophthalmic device, preferably the intraocular lens is implanted; for example but not limiting to a spherical full diopter change, a toric profile, an EDOF profile or a bi-, tri- or multifocal profile. Alternatively, the optical profile is the optical property adjustment of the ophthalmic device.
The optical pattern is the necessary change of polarizability resulting in change of refractive index in every voxel of the ophthalmic device.
The term "optic" as used herein as a part of the system to be used in the process for adjusting a polarizability of an ophthalmic device according to the invention includes all optical equipment necessary to control the spatial distribution of the irradiation source (focus) on the ophthalmic device. Critical parameters of the focus include the lateral focus size (or beam waist) and the focus length (or Rayleigh range). The optic comprises all elements along the optical beam path that determine the focus, such as beam expanders, aperture stops, shutter and in particular the focusing optics, such as a microscope objective or a single aspherical lens.
Multi-photon excitation occurs only in the vicinity of the focal point and preferably by employing ultra-short laser pulses. The average power is limited by the sample damage threshold such threshold being part of the input data as defined before.

Criteria for the selection and optimization of system parameters:

One ultimate purpose is to generate localized refractive-index modification of IOLs post-implantation as prescribed by the physician to improve the visual acuity of the patient. A crucial criterion for the procedure of refractive-index modification is the total treatment time required to obtain the desired result. It is generally recognized that such procedure should not take more than a few minutes in order to be recognized as viable. The systems capable of localized refractive-index modifications of the state of the art do not include an approach to obtain practical treatment times for IOL applications.

Discussion of system tradeoffs and limitations:

For a practical high-performing system capable of adjusting ophthalmic devices in general or specifically IOLs after implantation it is recognized that its subcomponents have to be treated as a system and must therefore be optimized jointly as many interdependencies and tradeoffs between the subcomponents exist. The subcomponents include the irradiation source, the optic, the scanner and treatment plan.
A key requirement of any system/parameter optimization is to stay within safe limits for the ophthalmic device material in case of the treatment or the material and the eye with its components (e.g. retina) in case of the treatment of an ophthalmic device being an IOL. Such requirements build the basis for input data as described before. In particular, two main damage mechanisms for radiation from an irradiation source, preferably a pulsed laser source can be distinguished: Single-pulse damage (dielectric breakdown and avalanche breakdown), and thermal damage, where the temperature of the lens material and/or the eye is heated up subsequently for repeated pulses to the same volume. For example: the average power of a pulsed irradiation source relates to the heating and therefore to the potential damage of the lens

material and/or the eye. Therefore, while keeping the average power of the irradiation source below the threshold of overheating the lens material and/or eye, pulse energy and pulse repetition rate are inversely related product of pulse energy and number of pulses per second (= inverse of repetition rate) is equal to the average power.

Average power is defined as pulse energy multiplied by number of pulses per second) and is characterized by Watt (W).

Irradiance is equal to flux density) ($W/cm^2$).

Radiant exposure is equal to fluence) ($J/cm^2$).

One overall objective is to minimize the treatment time for an IOL adjustment after implantation. In theory, higher and higher pulse energies with more frequent pulses (= higher repetition rate) could be applied, however, above an average power of typically 1 Watt, overheating starts creating unsafe conditions for IOL material and retina. Therefore, in order to stay within safe operating limits, while completing a treatment of the full IOL volume in a few minutes, one can define a preferred radiant exposure. The preferred radiant exposure is $\leq$ 5 $kJ/cm^2$, particular preferably < 1 $kJ/cm^2$ and very particular preferably <0.3 $kJ/cm^2$. This described radiant exposure applies additionally to the processes and methods according to the invention as further described below.

For the case a treatment plan is too extensive and would exceed the limits of laser safety concerning overheating, it is possible to interrupt the treatment to allow a cool down of all by the treatment affected ophthalmic device material and tissues. After the cool-down the locating system can compare the treated voxel in the ophthalmic device with the optical pattern and the treatment can be continued.

The process of adjustment of optical properties/profiles of said ophthalmic device according to the invention through the system and with requirements as described before will be done according to a treatment plan as described before. According to a treatment plan, profiles for e.g. toric, spheric, multifocal or EDOF (extended depth of focus) can be written into the ophthalmic device according to the invention. An algorithm may be utilized to write in profiles for e.g. toric, spheric, multifocal or EDOF (extended depth of focus) profiles.

By combining the information of the desired optical profile together with the input data, the needed optical pattern and the control commands for irradiation source, optics and scanner of the system as described before can be calculated. Further input data are for example lens data as such as the needed laser-energy for a certain refractive index change per voxel of said ophthalmic device material, and further patient data as the exact position and orientation of the ophthalmic device in the patient's eye being part of the treatment plan data.

The control commands can be updated and modified during the writing process by in-process input data such as temperature data of the patient's eye by e.g. IR-temperature measurements, in-process positioning data of the irradiation beam, the ophthalmic device or the eye acquired for example by OCT (optical coherence tomography) and/or refractive data acquired from Scheimpflug images.

In a further embodiment of input data, the input data comprises lens data of said ophthalmic device preferably of said intraocular lens and/or treatment plan data relating to a treatment plan for said treating of said ophthalmic device. For example, the lens data may comprise data relating to one or more of the polarizability and/or refractive index of the ophthalmic device as a function of the location of a respective volume or part of the ophthalmic device, shape, diopter, cylinder and sphere and/or its individual aberrations in said dimensions. The polarizability may therefore be increased or decreased at a particular location or volume in one or more planes of the ophthalmic device depending on the current polarizability (or refractive index) and the polarizability (or refractive index) to be obtained via the treatment.

The treatment plan calculation may, in some examples, generate control commands resulting in one or more of treatment plan data comprising: scan strategy control command data of a scan strategy (for example a scanning pattern and/or a scanning sequence and/or a scanning speed and/or a scanning duration of the scanning pattern and/or a scanning duration of the scanning sequence and/or a pulse duration of a pulse of the irradiation beam of the first and/or second wavelength (for example, nanosecond or picosecond or femtosecond pulses) and/or an irradiation beam profile of the irradiation beam of the first and/or second wavelength and/or a radiation (photon) density and/or a radiation intensity and/or a radiation power and/or radiation wavelength) for said scanning of the irradiation beam of the first and/or second wavelength across the ophthalmic device, in-process input data such as temperature data of a current and/or predicted temperature of the ophthalmic device during said exposure, refractive index/polarizability data of a refractive index/polarizability of the ophthalmic device to be obtained based on said exposure, the refractive index/polarizability to be obtained in particular relating to a mapping of the refractive index/polarizability to be obtained to a specific location/coordinates of the ophthalmic device, rhexis dimension data of a dimension of a rhexis, and input data such as eye data relating to a dimension and/or a shape of the eye of the patient, positioning data relating to a position and/or orientation of the ophthalmic device relative to the eye, and registration data relating to an identification of the patient and/or the specific eye of the patient.

Preferably, the scan strategy control command data of a scan strategy are a scanning pattern and/or a scanning speed and/or a pulse duration of a pulse and/or radiation intensity as described further below.

The parameters of the irradiation beam(s) may then be adjusted according to the lens data and/or the treatment plan data as defined herein in order to precisely (locally) change the polarizability/refractive index of the ophthalmic

device, where desired.

Preferably, the parameters of the irradiation beam(s) are adjusted according to the lens data and/or the treatment plan data as described before or preferably described herein.

The skilled artisan is well aware in this regard that optimum irradiation focus conditions are reached when the depth-of-field (Rayleigh range) of the irradiation beam is matched to the desired thickness of the optical structure to be written into the ophthalmic device.

The skilled artisan is well aware in this regard that optimum irradiation focus conditions are reached when the depth-of-field (Rayleigh range) of the irradiation beam is matched adapted to the local thickness of the ophthalmic device.

In a further embodiment, the lens data comprises data relating to a radiation absorption property (for example an absorption and/or light attenuation coefficient, which may be dependent from the wavelength of light) of a said ophthalmic device, and wherein the system is configured to adjust the first wavelength and/or the second wave-length for said ophthalmic device to locally change the polarizability based on a multi-photon absorption process. For example, based on the material used for the ophthalmic device, a particular wavelength or wavelength ranges may be input for a precise local change of the polarizability of the ophthalmic device.

The one or more irradiation sources as part of the system to be used in the process for adjusting a polarizability of an ophthalmic device according to the invention may comprise one or more pulsed lasers which may be utilized to generate nano-second pulses, preferably pico-second pulses and more preferably femto-second pulses. Preferably, one irradiation source is used. Particular preferably, the one or more irradiation sources comprise one or more pulsed lasers which are used to generate femto-second pulses. Particular preferably, one pulsed laser is used to generate femto-second pulses is used as irradiation for the system according to the invention or for the processes and methods according to the invention.

In one embodiment, the one or more irradiation sources comprise a laser which is tunable to emit a laser beam having the first and second wavelengths, respectively. This may be particularly advantageous as a single laser may be used to (locally) increase or decrease the polarizability/refractive index of the ophthalmic device or intraocular lens, as desired.

Different pulsed laser types are suitable for said irradiation sources within the system to be used in the process for adjusting a polarizability of an ophthalmic device according to the invention. MHz laser as well as kHz laser are suitable and have their particular merits. While a MHz laser system, for example, operates at lower pulse energy, the focused laser spots can be kept at μm-scale (<1 μm to several μm) and thus be used for precise local index modifications in all three dimensions, for example, to generate diffractive structures. A preferred MHz-irradiation source is an 80 MHz laser with a pulse energy ranging from 0.1 to 10 nJ.

A kHz-laser on the other hand operates at higher pulse energy of typically 0.1 to 10 μJ and thus requires a larger spot size of, for example, 10 to 100 μm in order to not damage the lens material. A larger laser spot size, however, implies a large depth of field (= long Rayleigh range) that can be equal to or even exceed the thickness of the ophthalmic device material. For such long Rayleigh ranges, it might not be possible to modify the refractive index layer by layer in the IOL but only uniformly along a line around the focus. A preferred kHz-irradiation source is a laser with a repetition rate of 100 to 500 kHz.

The average power of the irradiation source as described before or preferably described before is preferably between 300 and 600 mW, particular preferably between 400 and 500 mW.

The irradiation source as part of the system to be used in the process for adjusting a polarizability of an ophthalmic device according to the invention preferably comprises a tunable laser that can provide a variable wavelength in the range of approximately 680-1080 nm, such as a Ti:Sapphire laser (for example Chameleon Ultra II by Coherent, Santa Clara, CA, USA). The system may also comprise an optical parametric oscillator (for example frequency doubled Chameleon Compact OPO-Vis by Coherent, Santa Clara, CA, USA).

The irradiation source as part of the system to be used in the process for adjusting a polarizability of an ophthalmic device according to the invention particularly preferably comprises a femtosecond pump laser along with an optical parametric amplifier. Said pump laser emits irradiation >10 Watt average power at 1030 nm in <350 fs pulses with a repetition rate of 0.1 to 700 kHz. The radiation of said pump laser is directed to an optical parametric amplifier, where the pump laser output is frequency-doubled and optically mixed, resulting in a final tunable output in a wavelength range of 600 nm to 800 nm. A preferred repetition rate is between 50 and 600 kHz. A particular preferred repetition rate is between 100 and 500 kHz.

The irradiation source as part of the system to be used in the process for adjusting a polarizability of an ophthalmic device according to the invention particularly preferably comprises a femto-second pump laser >10 Watt average power at 1030 nm in combination with an optical parametric amplifier, which is emitting irradiation pulses <350 fs at a repetition rate of 1 to 700 kHz. The radiation of said pump laser is directed to an optical parametric amplifier with one or multiple second-harmonic stages, resulting in a final optical output in a wavelength range of 400 nm to 590 nm. A preferred repetition rate is between 50 and 600 kHz. A particular preferred repetition rate is between 100 and 500 kHz.

The laser types as described before or preferably described before generate a collimated optical beam of a

few millimeter in diameter, which is then directed to optics and scanner. The optical beam quality (characterized through the beam quality factor or beam propagation factor) is ideally between 1.0 and 1.5, more ideally between 1.0 and 1.3. According to DIN EN ISO 11146, the optical beam quality is given in the dimension of $M^2$.

The first wavelength of the irradiation beam within the system to be used in the process for adjusting a polarizability of an ophthalmic device according to the invention is between 600 nm and 800 nm, preferably between 620 nm and 750 nm, in order to (locally) decrease the polarizability (and hence the refractive index) of the IOL.

The second wavelength of the irradiation beam within the system to be used in the process for adjusting a polarizability of an ophthalmic device according to the invention is between 400 nm and 590 nm, preferably between 500 nm and 580 nm, in order to (locally) increase the polarizability (and hence the refractive index) of the IOL. The polarizability may hereby be locally changed particularly precisely.

Optics within the system to be used in the process for adjusting a polarizability of an ophthalmic device according to the invention:

The main function of the optics is to focus the irradiation beam, which is emitted from the irradiation source and controlled by the scanner, onto the ophthalmic device. Key considerations as described before are spot size and depth of focus in order to minimize treatment time while staying within limits given by laser safety requirements and material damage as described before as part of common input data. The most important characteristics of the optic is given by its numerical aperture (NA), along with its effective focal length (EFL) and the diameter of the irradiation beam at the entry aperture of the focusing optics. Additionally, all optical elements within the system to be used in the process for adjusting a polarizability of an ophthalmic device according to the invention should be selected for diffraction- or near-diffraction-limited properties, in order to not substantially degrade the optical beam quality.

Different ophthalmic needs will require different spot sizes as the spot size determines the obtainable spatial resolution. Ideally, the spot size is between 1 and 100 μm, more ideally between 50 and 100 μm in order to minimize treatment time while also keeping the potential for material damage low.

Scanner within the system to be used in the process for adjusting a polarizability of an ophthalmic device according to the invention:

The scanner to be used within the system to be used in the process for adjusting a polarizability of an ophthalmic device according to the invention may comprise a Galvano-scanner, a piezo scanner, a rotational scanner or an acousto optic modulator or it may be digital such as a spatial light modulator, a digital micromirror device or stereolithography apparatus. Preferably, the scanner as part of the inventive system according to the description is selected from a Galvano-scanner, a piezo scanner, a rotational scanner, an acousto optic modulator, a spatial light modulator, a digital micromirror device or stereolithography apparatus. A preferred Galvano-scanner is a single Pivot-Point-Scanner.

Preferably, the scanner is configured to operate at a scanning speed of more than 50 mm/s. This may allow for keeping the treatment time short. As a general rule, the treatment time should not exceed several minutes and is preferably less than 10 minutes, preferably less than 5 minutes, particularly preferably less than 3 minutes per treatment session.

The treatment area may be defined as the ophthalmic devices volume and size. Typically, the optic of said ophthalmic device or intraocular lens is 5 mm to 7 mm in diameter and typically between 0.2 mm and 2.0 mm thick.

The optimum radiation exposure is <1 kJ/cm$^2$ and more ideally <0.3 kJ/cm$^2$ to keep the overall irradiation exposure low and treatment time short, while addressing the full volume of the ophthalmic device.

Particularly preferably, random scan patterns or interleaved scanning lines are used to spread out the irradiation energy of the irradiation beam.

The scanning can be performed with three modes. In the bottom-up scanning, the laser may travel from spot-to-spot with a specific dwell time on each spot ("bottom-up, spot-to-spot"). Alternatively, in the bottom-up scanning, the laser may dwell on spots that overlap with one another ("bottom-up, spot overlay"). Alternatively, the laser can travel with a fixed velocity without dwelling on any spots ("fly by, constant velocity").

In one embodiment of a scan pattern, the IOL is scanned with the irradiation source as described before or preferably described before by shining through the pupil. The IOL, contained in the capsular bag at the time of scanning, is previously inserted through an incision in the cornea using conventional operation procedures. In this embodiment, the full volume of the IOL is scanned and the scanning is performed in a bottom-up manner (i.e. parts of the IOL which are further away from the cornea are scanned first), this way the optical profile is created in order to avoid unnecessary changes in refractive index in the light path.

As described before a key consideration when selecting the scanning program is to minimize local heating of the ophthalmic device and/or the eye of the patient and therefore various variables are used in the scanning program. Taking into account anatomical features such as Rhexis and pupil size as well as optical features such as numerical aperture and laser pulse characteristics, a laser program is created with a specific scanning speed and sequence. The relation between lens coordinate and eye coordinate system are, in this example, automatically taken into account.

The parameters for the scanning program and/or treatment plan are preferably the first and second wavelengths, the scanning speed and sequence, the positioning (e.g. in Cartesian coordinates) of the lens relative to the eye, the

scan strategy, the refractive index change which is to be obtained (optical pattern), the numerical aperture of the objective, the Rhexis, the optical diameter (in some examples approximately 6 mm) of the pupil and/or the lens, the pulse duration (shape, intensity and x-y positioning) of the laser beam, laser safety when operating the laser, and centration with respect to the positioning of the lens and the eye.

The photons generated in the laser are in one embodiment of the system to be used in the process for adjusting a polarizability of an ophthalmic device according to the invention preferably guided through mirrors (e.g. as optic 1) to a e.g. a beam expander, which prepares the beam for the subsequent scanner and focusing optic. After passing through the beam expander, the photons are directed toward the scanner (e.g. Galvano-scanner or piezo scanner or rotational scanner or acousto optic modulator or digitally with a spatial light modulator or digital micromirror device or stereolithography apparatus).

After having gone through the scanner, the laser beam travels through another optic such as a divider mirror. In this embodiment, the divider mirror splits up the beam into the main imaging beam for the ophthalmic device irradiation and a beam for monitoring beam properties as well as for positioning feedback. After the divider mirror, the optical beam is focused onto the ophthalmic device by imaging group or focusing optic. In one embodiment, the imaging group comprise a microscope objective to obtain high numerical apertures (for μm-level spatial resolution) or low-NA optics to allow higher pulse energy of μJ-level.

The system as described before or preferably described before may further comprise a microscope objective coupled to the scanner for focusing, by the microscope objective, a said irradiation beam onto said ophthalmic device, wherein the microscope objective has a numerical aperture of between 0.1 and 0.8, preferably between 0.2 and 0.5, and more preferably between 0.2 and 0.4. Providing a microscope objective having such numerical aperture may allow for high irradiation beam quality in particular in terms of focusing and resolution characteristics of the beam used for treating the intraocular lens.

The microscope objective comprises of a typical lens configuration to allow for e.g. correction of chromatic aberration. The microscope objective is preferably linked to an eye interface system, typically a suction system that keeps the eye of the patient in a fixed position as further described below.

In a further embodiment of an objective to be used within the system as described before, the objective is an Olympus LUCPLFLN objective in order to focus the irradiation beam onto the ophthalmic device.

An alternative focusing optic/imaging group is configured with a single aspherical lens with an effective focal length preferably within 50 to 150 mm and a numerical aperture of preferably 0.025 to 0.1.

The system as described before or preferably described before may further comprise a positioning system for determining a position of a said focus of said irradiation beam within a said eye of a said patient, wherein the positioning system is coupled to the scanner and wherein the scanning, by the scanner, of said irradiation beam across said intraocular lens is based on the position of said focus of said irradiation beam within the eye.

The positioning system may comprise a locating system such as an optical coherence tomography system, a confocal microscope or a Scheimpflug camera. The positioning system may be directly or indirectly coupled to the scanner. In some examples in which a confocal microscope is used, the confocal microscope may be directly coupled to the scanner.

The locating system as described before is used to provide topographic data of the eye to the positioning system for determination of the position of the laser focus in dependence of the eye and said intraocular lens.

For confocal microscopy, a partially transparent mirror is used to allow for video imaging.

The system as described before or preferably described before is preferably configured further to determine a location and/or orientation of said intraocular lens relative to the eye and the outlet of the irradiation beam, and wherein the scanning, by the scanner, of said irradiation beam across said intraocular lens is based on the location and/or orientation of said intraocular lens relative to the eye. This may be particularly advantageous since the position of the intraocular lens may not be centered relative to the eye, which misalignment may be taken into account when treating the intraocular lens with the irradiation beam(s).

With respect to the position of the IOL, at least 2 coordinate systems may be considered relevant: coordinates-system of the eye and coordinates-system of the lens within the eye, as both may not be centered with respect to each other.

With respect to the position of the IOL, at least 2 coordinate systems may be considered relevant: x,y,z coordinates of the eye and x,y,z coordinates of the lens within the eye, as both may not be centered with respect to each other.

In one embodiment, the locating system creates input data. These input data contain for example data concerning lens position and/or orientation of the ophthalmic device within the eye and relative to the laser beam outlet, and/or an optical power mapping of the eye and/or the ophthalmic device. These data are used for the calculation of the optical pattern or a continuation of a treatment.

Additionally, it is possible that the locating system creates input data during the writing process. These in-process input data contain for example data concerning lens position and/or orientation of the ophthalmic device within the eye and relative to the laser beam outlet, and/or an optical power mapping of the eye and/or the ophthalmic device.

These data are used for in-process modification of the control commands used to generate the optical pattern.

The system as described before or preferably described before may further comprise a temperature management unit coupled to one or both of (i) the one or more irradiation sources and (ii) the scanner, wherein the temperature management unit is configured to determine, based on an irradiation beam property of a said irradiation beam and an ophthalmic device property of a said ophthalmic device, a temperature of a part of said ophthalmic device during said treating of said ophthalmic device by said scanning, and wherein the system is configured to control, based on said determination of the temperature, one or both of (i) the one or more irradiation sources and (ii) the scanner. This may allow for ensuring that the eye and/or the ophthalmic device may not be detrimentally affected based on the treatment with an irradiation beam.

Additionally, the temperature management unit is preferably configured to predict said temperature during said treating of said ophthalmic device, and wherein said input data comprises the predicted temperature. This may allow for taking preventative measures to ensure that the eye and/or the ophthalmic device may not be detrimentally affected based on the treatment with an irradiation beam.

Alternatively, the temperature management unit is an infrared camera logging the temperature of the eye and correlating the measured data with common data bearing calibration data to calculate the real temperature in the eye. In another embodiment, the temperature dependence of refractive index is used for temperature controlling. In these examples, the system comprises a refractive power mapping device. Based on the deviation of the measured refractive power map and the progress of the writing predicted refractive power map, temperatures in the lens can be calculated in process.

In another embodiment, the temperature dependence of the emission spectrum is used for temperature controlling. In these examples, the system comprises a UV-Vis spectrometer. Based on the deviation of the measured emission peak wavelength and/or peak width, temperatures in the focal spot can be calculated in process.

The system as described before or preferably described before may further comprise an eye interface system configured to keep a said eye of a said patient in a fixed position. The eye interface system may comprise a suction system for fixing the position of the eye of the patient during treatment.

The patient may be "docked" to the system in a lie flat or upright position.

The system as described before or preferably described before may further comprise a wireless or wired receiver and/or transceiver for one or more of (i) sending control commands to the one or more irradiation sources, (ii) sending control commands to the scanner, and (iii) inputting the control command data needed for creating the optical pattern into the scanner.

The one or more irradiation sources and/or the scanner may therefore be controlled remotely. Additionally or alternatively, the data relating to one or both of the lens data and the treatment plan data may be stored externally from the system and may be provided to the system as and when desired. In some examples, it may be preferable to provide a wired receiver or transceiver at least for controlling the one or more irradiation sources and/or for controlling the scanner in order to reduce (or avoid) any delay when sending a control signal to the one or more irradiation sources and/or the scanner.

In another example, the receiver/transceiver sends treatment plan data and lens data to a central computing unit which calculates the optical pattern and sends this as input data back to the receiver which provides it to the system.

The system as described before or preferably described before may further comprise a device for locally measuring the refractive power of said ophthalmic device during said treating of the ophthalmic device. Adjustments to one or more of the irradiation source(s), the scanner and the input data may hereby be made during the treatment process.

The system as described before or preferably described before may further comprise a refractometer for locally measuring the refractive index of said ophthalmic device during said treating of the ophthalmic device. Adjustments to one or more of the irradiation source(s), the scanner and the input data may hereby be made during the treatment process.

Further components of the system providing the photons are optionally a cover in which all the equipment is built in, a power unit to provide the system and all sub-systems with sufficient energy, and sub-systems like a suction system and/or chiller.

In addition to the above mentioned components, controller, firmware and a graphics user interface (GUI) as well as treatment algorithms may be provided. To connect to the system, connectivity may be established via Bluetooth, Wi-Fi or other ports like RS-232.

The invention further relates to a method for locally adjusting a polarizability of an intraocular lens according to the invention arranged within an eye of a patient, wherein the treatment plan data comprises one or more of:

scan strategy control command data of a scan strategy (for example a scanning pattern and/or a scanning sequence and/or a scanning speed and/or a scanning duration of the scanning pattern and/or a scanning duration of the scanning sequence and/or a pulse duration of a pulse of the irradiation beam of the first and/or second wavelength and/or an irradiation beam profile of a said irradiation beam and/or a radiation (photon) density and/or a radiation intensity and/or a radiation power and/or radiation wavelength) for said scanning of a said irradiation beam across

the intraocular lens,
temperature data of a current and/or predicted temperature of the intraocular lens during said exposure,
refractive index data of a refractive index of the intraocular lens to be obtained based on said exposure, the refractive index to be obtained in particular relating to a mapping of the refractive index to be obtained to a specific location/coordinates of the intraocular lens,
rhexis dimension data of a dimension of a rhexis,
eye data relating to a dimension and/or a shape of the eye of the patient,
positioning data relating to a position and/or orientation of the intraocular lens relative to the eye, and
registration data relating to an identification of the patient and/or the specific eye of the patient.

The invention further relates to a method for locally adjusting a polarizability of an intraocular lens according to the invention arranged within an eye of a patient, wherein said exposing of the intraocular lens to a said irradiation beam comprises exposing a first volume of the intraocular lens prior to exposing a second volume of the intraocular lens, wherein the first volume is further away from the cornea of the eye of the patient than the second volume.

In the above-stated clauses, an initial step of the methods may be to provide a said intraocular lens.

In examples, in which said exposing of the intraocular lens to a said irradiation beam comprises exposing a first volume and/or plane and/or location of the intraocular lens prior to exposing a second volume and/or plane and/or location of the intraocular lens, wherein the first volume and/or plane and/or location is further away from the cornea of the eye of the patient than the second volume and/or plane and/or location, volumes and/or planes and/or locations irradiated at later time points in the irradiation sequence may be closer to the cornea than volumes and/or planes and/or locations irradiated at earlier time points. A said volume may hereby relate to one or more planes of the intraocular lens.

The invention is furthermore related to a method for correcting vision in a patient by modifying the refractive index of an intraocular lens according to the invention within the eye of said patient comprising

identifying and measuring the degree of vision correction of the patient;
determining the position and type of refractive structures to be written into said intraocular lens to correct the patient's vision; and
subsequently exposing said intraocular lens to two-photon or multi-photon irradiation having a wavelength between 600 nm and 800 nm to locally decrease the polarizability of the intraocular lens and/or
subsequently exposing said intraocular lens to two-photon or multi-photon irradiation having a wavelength between 400 nm and 590 nm to locally increase the polarizability of the intraocular lens, preferably by using the system and/or the process as described before for exposing said intraocular lens to said irradiation.

As outlined above, the change of polarizability results in a change of refractive index.

It should be pointed out that variations of the embodiments described in the present invention are covered by the scope of this invention. Any feature disclosed in the present invention may, unless this is explicitly ruled out, be exchanged for alternative features which serve the same purpose or an equivalent or similar purpose. Thus, any feature disclosed in the present invention, unless stated otherwise, should be considered as an example of a generic series or as an equivalent or similar feature.

All features of the present invention may be combined with one another in any manner, unless particular features and/or steps are mutually exclusive. This is especially true of preferred features of the present invention. Equally, features of non-essential combinations may be used separately (and not in combination).

It should also be pointed out that many of the features, and especially those of the preferred embodiments of the present invention, are themselves inventive and should not be regarded merely as some of the embodiments of the present invention. For these features, independent protection may be sought in addition to or as an alternative to any currently claimed invention.

The technical teaching disclosed with the present invention may be abstracted and combined with other examples.

No doubt many other effective alternatives will occur to the skilled person. It will be understood that the invention is not limited to the described embodiments and encompasses modifications apparent to those skilled in the art and lying within the scope of the claims appended hereto.

Examples

The following examples are intended to show the advantages of the present compounds in a non-limiting way. Unless indicated otherwise, all syntheses are carried out under an inert atmosphere using dried (i.e. water-free) solvents. Solvents and reagents are purchased from commercial suppliers.

DCM is used to denote dichloromethane. DMF is used to denote dimethylformamide. EE or EtOAc is used to

denote ethyl acetate. THF is used to denote tetrahydrofuran. RT means room temperature. Cyhex is used to denote cyclohexane.
Melting points are measured with a TA Instruments Q2000 differential scanning calorimeter during heating in the first heating run with 20 K/min from -100 °C to 200 °C in a hermetic aluminium pans.
Copolymer-properties can be investigated on blanks, prepared by bulk polymerization of the monomers. Co-monomers, cross-linkers and initiators therefore can be purchased from commercial sources. All chemicals are of highest purity available and can be used as received.

Synthesis of precursor materials and compounds according to the invention:

Example 1a:

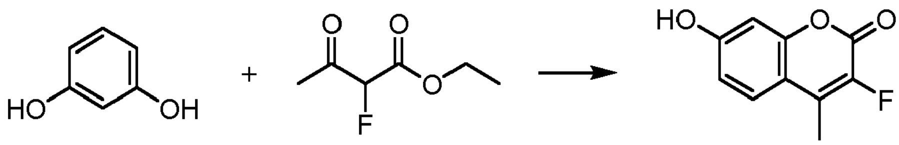

To a solution of resorcin (40 g; 0.363 mol) and ethyl 2-fluoroacetoacetate (49.7 ml; 0.628 mol, 1.0 equiv.) in 1,4-dioxane (20.5mL, 0.24 mol) is added dropwise sulfuric acid [98%] (97 ml, 1.82 mol, 5.0 equiv.) at 0 °C. The reaction mixture is stirred overnight at r.t., while the temperature is increased to 25 °C. The mixture is poured on a mixture of ice and water and the reaction mixture is slowly stirred for 3h. Then the resulting precipitate is collected by suction filtration, recrystallized from methanol/water, dried in vacuo, affording 3-fluoro-7-hydroxy-4-methyl-2H-chromen-2-one as a colourless powder 44,23 g; 0.228 mol; 63 % of theory).
**$^1$H NMR** (500 MHz, DMSO-$d_6$) δ 10.49 (s, 1H), 7.61 (d, *J* = 8.7 Hz, 1H), 6.87 (dd, *J* = 8.8, 2.3 Hz, 1H), 6.76 (d, *J* = 2.3 Hz, 1H), 2.33 (d, *J* = 2.8 Hz, 3H).
**$^{19}$F NMR** (470 MHz, DMSO-$d_6$) δ -142.9.
Analogously, other derivatives are prepared in the same manner: R1 means reactant, R2 means reactant 2, [P] means product.

| No. | | | Yield [%] |
|---|---|---|---|
| **1aa** | R1 | 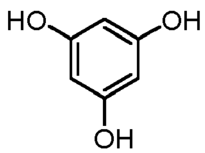 | |
| | R2 | 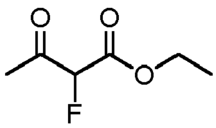 | |
| | [P] | 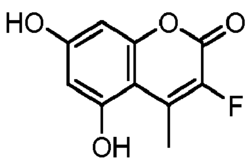 | 69 |
| **1ab** | R1 | 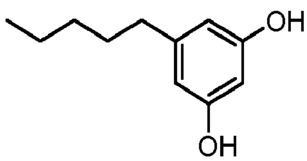 | |

(continued)

| No. | | | Yield [%] |
|---|---|---|---|
| | R2 | 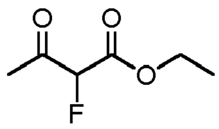 | |
| | [P] | 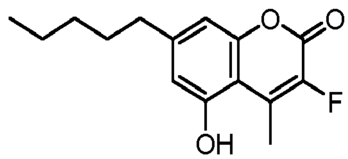 | quant. |

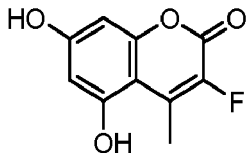

**$^1$H NMR** (500 MHz, DMSO) δ 10.58 (s, 1H), 10.27 (s, 1H), 6.32 (d, *J* = 2.3 Hz, 1H), 6.21 (d, *J* = 2.3 Hz, 1H), 2.47 (d, *J* = 3.3 Hz, 3H).

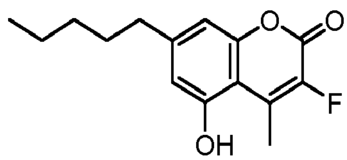

**$^1$H NMR** (500 MHz, $CDCl_3$) δ 10.54 (s, 1H), 6.76 (d, *J* = 1.5 Hz, 1H), 6.57 (d, *J* = 1.5 Hz, 1H), 2.70 (dd, *J* = 8.8, 6.8 Hz, 2H), 2.50 (d, *J* = 3.3 Hz, 3H), 1.72 - 1.61 (m, 2H), 1.36 (d, *J* = 9.1 Hz, 4H), 0.96 - 0.89 (m, 3H).

Example 1b:

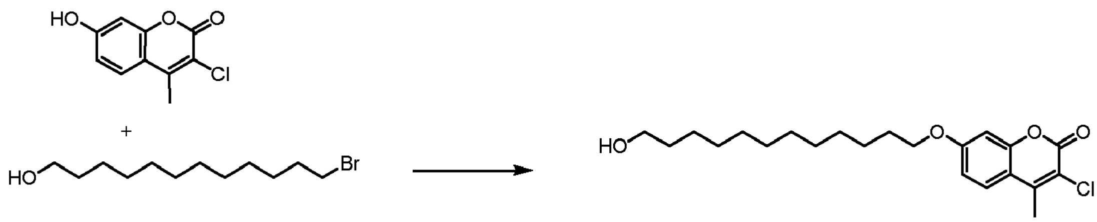

To a solution of 3-chloro-7-hydroxy-4-methylcoumarin (5.00 g; 23.03 mmol) in 60 ml *N*,*N*-dimethylformamide (anhydrous), potassium carbonate (3.18 g; 23.03 mmol) and 12-bromododecan-1-ol (6.437; 24.18 mmol) are added. The reaction mixture is heated to 90 °C overnight, then quenched with HCl (1M) and extracted with 2-methyl-THF. Afterwards, the organic layer is dried over anhydrous $Na_2SO_4$ and then concentrated under reduced pressure. The residue is purified by silica gel column chromatography using cyclohexane/tetrahydrofuran to get 3-chloro-7-[(12-hydroxydodecyl)oxy]-4-methyl-2H-chromen-2-one (7.72 g; 19.54 mmol; 71 % of theory).
**$^1$H NMR** (500 MHz, DMSO-$d_6$) δ 7.76 (d, *J* = 8.9 Hz, 1H), 7.03 (d, *J* = 2.5 Hz, 1H), 7.00 (dd, *J* = 8.9, 2.5 Hz, 1H), 4.30 (t, *J* = 5.2 Hz, 1H), 4.08 (t, *J* = 6.5 Hz, 2H), 3.36 (td, *J* = 6.5, 5.1 Hz, 2H), 2.54 (s, 3H), 1.77 -1.68 (m, 2H), 1.44 - 1.21 (m, 18H).
Analogously, other derivatives are prepared in the same manner: R1 means reactant, R2 means reactant 2, [P] means product.

| No. | | | Yield [%] |
|---|---|---|---|
| **1ba** | R1 | 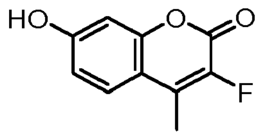 | |
| | R2 | 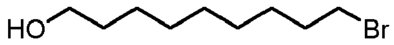 | |
| | [P] | 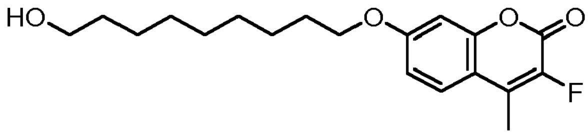 | 43 |
| **1bb** | R1 | 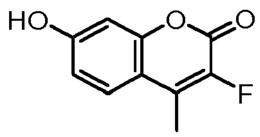 | |
| | R2 | 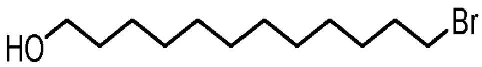 | |
| | [P] | 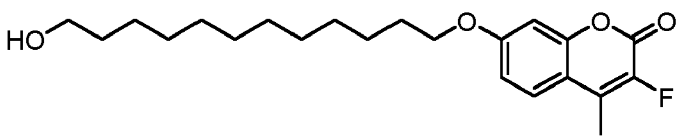 | 30 |
| **1bc** | R1 | 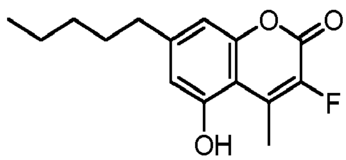 | |
| | R2 | 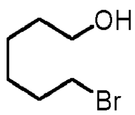 | |
| | [P] | 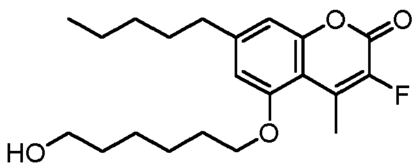 | 81 |
| **1bd** | R1 | 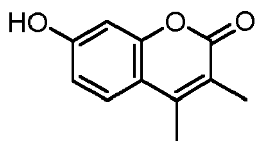 | |
| | R2 | 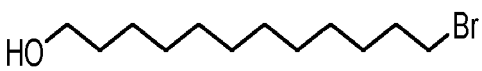 | |
| | [P] | 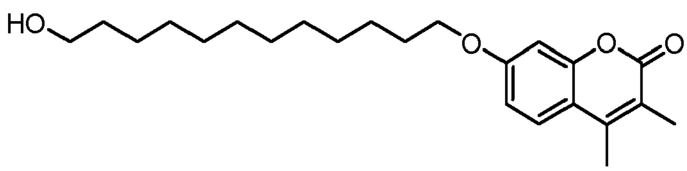 | 88 |

(continued)

| No. | | | Yield [%] |
|---|---|---|---|
| **1be** | | 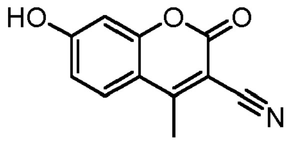 | |
| | | 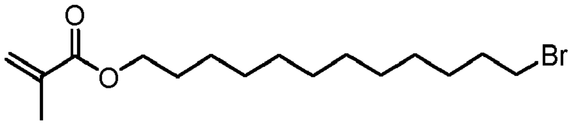 | |
| | | 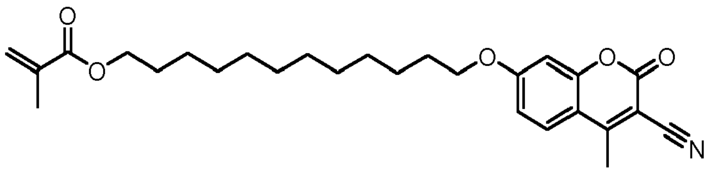 | 5 |
| **1bf** | R1 | 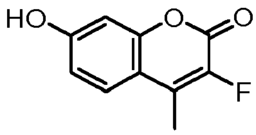 | |
| | R2 | 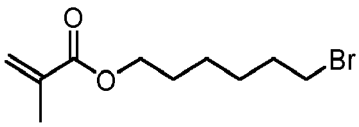 | |
| | [P] | 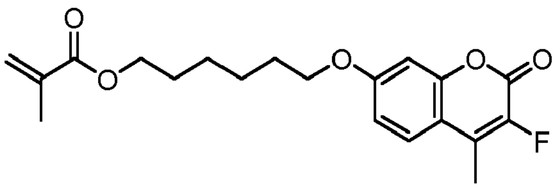 | 20 |
| **1bg** | R1 | 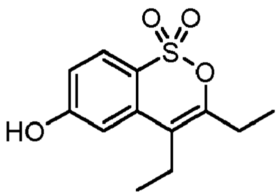 | |
| | R2 | 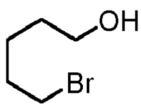 | |
| | [P] | 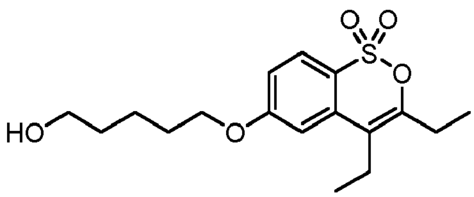 | 88 |

(continued)

| No. | | | Yield [%] |
|---|---|---|---|
| **1bh** | R1 | 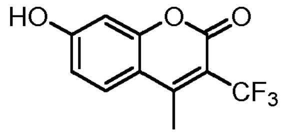 | |
| | R2 | 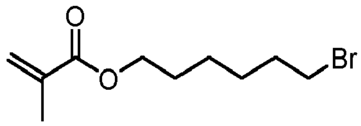 | |
| | [P] | 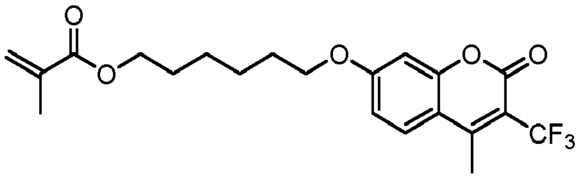 | 3 |

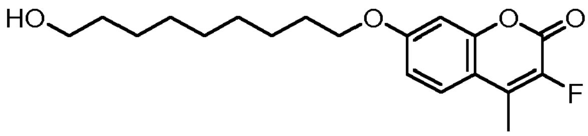

**$^1$H NMR** (500 MHz, DMSO-$d_6$) δ 7.69 (d, *J* = 8.7 Hz, 1H), 7.07 - 7.00 (m, 2H), 4.31 (t, *J* = 5.1 Hz, 1H), 4.07 (t, *J* = 6.5 Hz, 2H), 3.37 (td, *J* = 6.5, 5.1 Hz, 2H), 2.36 (d, *J* = 2.9 Hz, 3H), 1.77 - 1.68 (m, 2H), 1.46 - 1.35 (m, 4H), 1.35 - 1.24 (m, 8H).

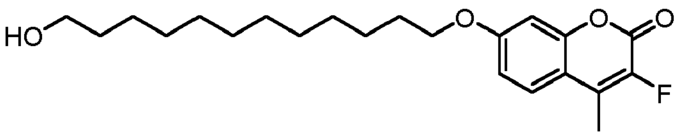

**$^1$H NMR** (500 MHz, DMSO) δ 7.68 (d, *J* = 8.6 Hz, 1H), 7.04 - 7.00 (m, 2H), 4.30 (t, *J* = 5.1 Hz, 1H), 4.06 (t, *J* = 6.5 Hz, 2H), 3.36 (td, *J* = 6.6, 5.1 Hz, 2H), 2.35 (d, *J* = 2.8 Hz, 3H), 1.77 - 1.68 (m, 2H), 1.40 (dp, *J* = 13.5, 6.6 Hz, 4H), 1.34-1.22 (m, 14H).
**$^{19}$F NMR** (470 MHz, DMSO-$d_6$) δ -141.6.

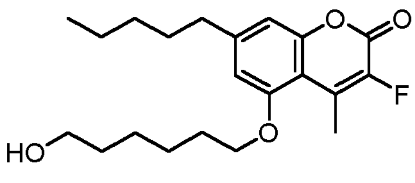

**$^1$H NMR** (500 MHz, $CDCl_3$) δ 6.76 (d, *J* = 1.5 Hz, 1H), 6.57 (d, *J* = 1.5 Hz, 1H), 4.04 (t, *J* = 6.5 Hz, 2H), 3.68 (t, *J* = 6.5 Hz, 2H), 2.64 - 2.59 (m, 2H), 2.57 (d, *J* = 3.3 Hz, 3H), 1.93 - 1.84 (m, 2H), 1.63 (p, *J* = 6.8 Hz, 4H), 1.52 - 1.40 (m, 4H), 1.33 (pt, *J* = 9.4, 4.6 Hz, 6H), 0.90 (t, *J* = 6.9 Hz, 3H).

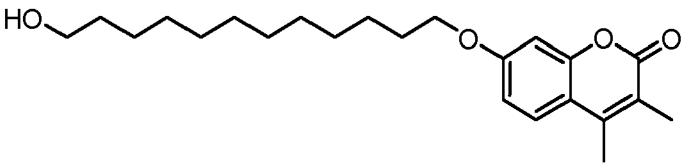

**$^1$H NMR** (500 MHz, DMSO) δ 7.70 - 7.65 (m, 1H), 6.95 - 6.89 (m, 2H), 4.31 (t, *J* = 5.1 Hz, 1H), 4.04 (t, *J* = 6.5 Hz, 2H), 3.36 (td, *J* = 6.6, 5.1 Hz, 2H), 2.36 (d, *J* = 1.0 Hz, 3H), 2.07 (d, *J* = 0.9 Hz, 3H), 1.75 - 1.69 (m, 2H), 1.39 (q, *J* = 7.7, 6.8 Hz, 4H), 1.24 (d, *J* = 5.5 Hz, 14H).

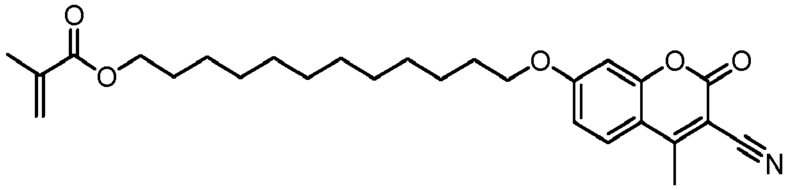
(A-014)

**$^1$H NMR** (500 MHz, $CDCl_3$) δ 7.62 (d, *J* = 8.8 Hz, 1H), 6.90 (d, *J* = 2.2 Hz, 1H), 6.73 (d, *J* = 2.2 Hz, 1H), 6.09 (s, 1H), 5.54 (s, 1H), 4.13 (t, *J* = 6.6 Hz, 2H), 1.94 (s, 3H), 1.77 (dq, *J* = 15.2, 8.9, 7.1 Hz, 2H), 1.67 (h, *J* = 6.7 Hz, 4H), 1.54 (s, 3H), 1.45 - 1.24 (m, 16H).
**m.p.** (optical): 58.0 °C

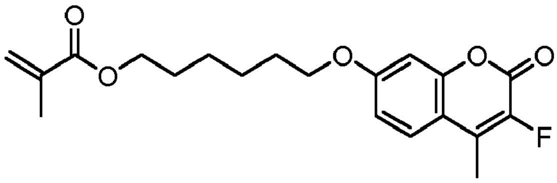
(A-011)

**$^1$H NMR** (500 MHz, $CDCl_3$) δ 7.45 (d, *J* = 8.9 Hz, 1H), 6.91 (dd, *J* = 8.8, 2.4 Hz, 1H), 6.82 (d, *J* = 2.4 Hz, 1H), 6.10 (s, 1H), 5.55 (p, *J* = 1.5 Hz, 1H), 4.16 (t, *J* = 6.6 Hz, 2H), 4.01 (t, *J* = 6.4 Hz, 2H), 2.38 (d, *J* = 2.8 Hz, 3H), 1.94 (t, *J* = 1.3 Hz, 3H), 1.87 - 1.80 (m, 2H), 1.73 (p, *J* = 6.9 Hz, 2H), 1.54 - 1.44 (m, 4H).
**m.p.** (DSC): 47.8 °C

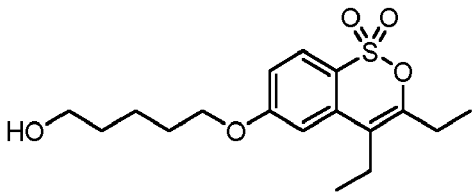

**$^1$H NMR** (500 MHz, $CDCl_3$) δ 7.84 - 7.78 (m, 1H), 6.97 - 6.91 (m, 2H), 4.05 (t, *J* = 6.4 Hz, 2H), 3.70 (q, *J* = 6.0 Hz, 2H), 2.54 (dq, *J* = 22.2, 7.5 Hz, 4H), 1.87 (p, *J* = 6.6 Hz, 2H), 1.70 - 1.63 (m, 2H), 1.59 (qd, *J* = 7.4, 3.1 Hz, 1H), 1.26 (td, *J* = 7.2, 3.0 Hz, 5H), 1.22 (t, 3H, *J* = 7.8 Hz).

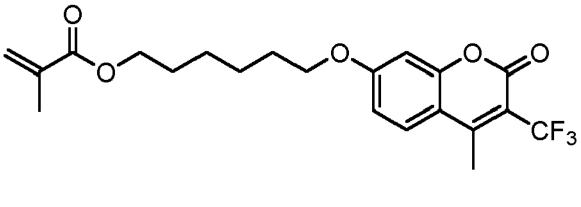
(A-104)

**$^1$H NMR** (500 MHz, $CDCl_3$) δ 7.68 (d, *J* = 9.1 Hz, 1H), 6.90 (dd, *J* = 9.0, 2.5 Hz, 1H), 6.78 (d, *J* = 2.5 Hz, 1H), 6.10 (dt, *J* = 2.0, 1.0 Hz, 1H), 5.55 (t, *J* = 1.6 Hz, 1H), 4.17 (t, *J* = 6.6 Hz, 2H), 4.04 (t, *J* = 6.4 Hz, 2H), 2.63 (q, *J* = 2.2 Hz, 3H), 1.94 (dd, *J* = 1.6, 1.0 Hz, 3H), 1.90 - 1.79 (m, 2H), 1.73 (dt, *J* = 14.0, 6.8 Hz, 2H), 1.56 - 1.40 (m, 4H).
**$^{19}$F NMR** (471 MHz, $CDCl_3$) δ -56.30.
**m. p.** (DSC): 58.5 °C

Example 1c:

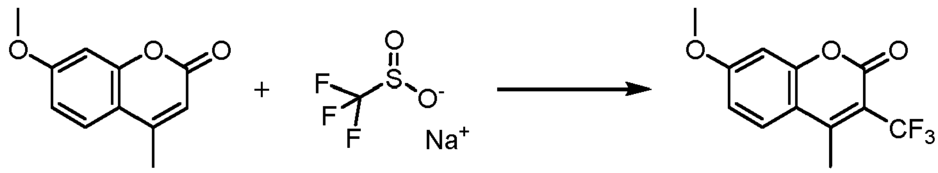

7-Methoxy-4-methyl-2H-chromen-2-one (5,26 mmol; 1,0 eq.; 1,00 g) and sodium triflate (15,77 mmol; 3,0 eq.; 2,46 g) are dissolved in acetic acid (1.748 mol, 332.56 eq.; 100ml). Manganese(III) acetate dihydrate (21,03 mmol; 4,0 eq.; 5,81 g) is added portionwise at r.t. and the reaction mixture is stirred overnight. The solvent is removed from the reaction mixture and the residue is taken up in $H_2O$ and extracted 3x with EtOAc, washed with $NaHCO_3$ and brine and dried over $Na_2SO_4$ The crude product is purified by column chromatography on silica using cyclohexane/EtOAc. 7-Methoxy-4-methyl-3-(trifluoromethyl)-2H-chromen-2-one is isolated as a yellow solid (0,91 g; 3,52 mmol; 67,0 % of theory).

**$^1$H NMR** (500 MHz, $CDCl_3$) δ 7.70 (d, *J* = 9.0 Hz, 1H), 6.92 (dd, *J* = 9.1, 2.6 Hz, 1H), 6.81 (d, *J* = 2.5 Hz, 1H), 3.90 (s, 3H), 2.63 (q, *J* = 2.2 Hz, 3H).

**$^{19}$F NMR** (471 MHz, $CDCl_3$) δ -56.33.

Example 1d:

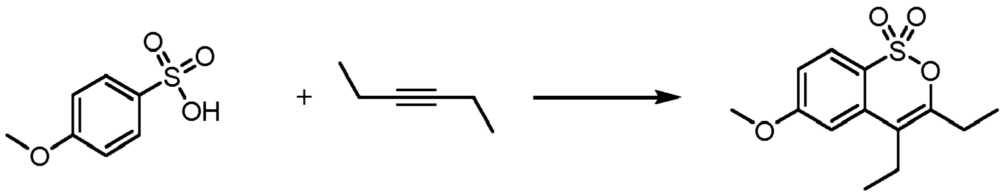

4-Methoxybenzene-1-sulfonic acid (38.73 mmol; 1.0 eq.; 7.289g), pentamethyl cyclopentadienyl rhodium (I) (0.704 mmol, 0.02 eq.; 0.435g), $AgSbF_6$ (2.82 mmol, 0.08 eq.; 0.988g) and silver(I) acetate are dissolved in 170ml 1,4-dioxane under argon atmosphere. Then, hex-3-yne (35.21 mmol, 1.00 eq.; 4ml) is added to the reaction mixture which is heated up to 100 °C overnight. Again, hex-3-yne (35.21 mmol, 1.00 eq.; 4ml) is added and the mixture is stirred at 100 °C for another day. Then, methanol is added, and the reaction mixture is filtered over a pad of celite and is rinsed with ethyl acetate. The solvent is removed in vacuo, and the crude residue is dissolved in ethyl acetate and washed with $NaHCO_3$-solution. Organics are dried over $Na_2SO_4$ and the organic phase is again filtered over silica and rinsed with cyclohexane/ethyl acetate 4:1. Organic volatiles are removed and 1.17g of crude 3,4-diethyl-6-methoxybenzo[c][1,2]oxathiine 1,1-dioxide (4.36 mmol, 12% of theory) is isolated as a brown oil.

**$^1$H NMR** (500 MHz, $CDCl_3$) δ 7.86 - 7.81 (m, 1H), 6.96 (d, *J* = 8.3 Hz, 2H), 3.90 (s, 3H), 2.55 (dq, *J* = 22.6, 7.5 Hz, 4H), 1.25 (t, *J* = 7.5 Hz, 3H), 1.20 (t, *J* = 7.5 Hz, 3H).

Example 1e:

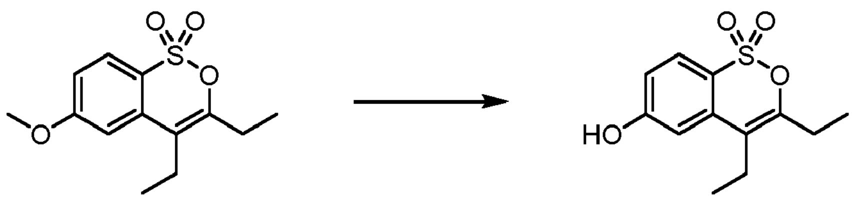

3,4-Diethyl-6-methoxybenzo[c][1,2]oxathiine 1,1-dioxide (6,22 mmol; 1,00 eq.; 1,67 g) is suspended in 22ml dry dichloromethane and the solution is cooled with an ice-bath. Then, borontribromide (6,85 mmol; 1,10 eq.; 1,72 g; 0,65 ml), dissolved in 10ml DCM, is added dropwise. The solution is warmed overnight to r.t. and the reaction mixture is quenched with water. The reaction mixture is extracted three times with ethyl acetate and the organic phase is washed

with sat. $NaHCO_3$ solution. The crude product is purified by column chromatography using cyclohexane/ethyl acetate as the eluent. 1.27g of 3,4-diethyl-6-hydroxybenzo[c][1,2]oxathiine 1,1-dioxide is isolated as brown crystals (80% yield of theory).

**$^1$H NMR** (500 MHz, $CDCl_3$) δ 7.76 (d, *J* = 8.4 Hz, 1H), 6.91 (d, *J* = 2.4 Hz, 1H), 6.88 (dd, *J* = 8.5, 2.3 Hz, 1H), 5.55 (br s, 1H), 2.53 (dq, *J* = 11.3, 7.6 Hz, 4H), 1.25 (t, *J* = 7.5 Hz, 3H), 1.18 (t, *J* = 7.6 Hz, 3H).

Analogously, other derivatives are prepared in the same manner:

| No. | Reactant | Product | Yield [%] |
|---|---|---|---|
| 1fa | 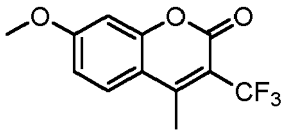 | 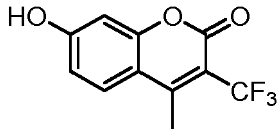 | 83 |

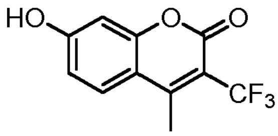

**$^1$H NMR** (500 MHz, $CDCl_3$) δ 7.69 (d, *J* = 8.9 Hz, 1H), 6.87 (dd, *J* = 8.9, 2.5 Hz, 1H), 6.82 (d, *J* = 2.5 Hz, 1H), 5.95 (s, 1H), 2.63 (q, *J* = 2.2 Hz, 3H).
**$^{19}$F NMR** (471 MHz, $CDCl_3$) δ -56.34.

Example 2:

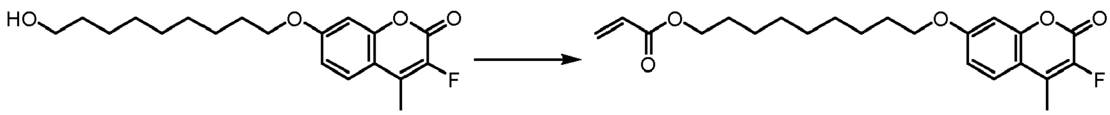

3-Fluoro-7-[(9-hydroxynonyl)oxy]-4-methyl-coumarin (0.7g, 2.08 mmol, 1.00 eq.) is dissolved in THF (7 mL) and triethylamine (1.15 mL, 8.00 mmol, 4.00 eq.) and acryloyl chloride (209.15 μL, 2.49 mmol, 1.2 eq.) are added at 0 °C. After stirring at r.t. overnight the suspension is filtered. The solvent of the filtrate is evaporated, and the crude product is cleaned by column chromatography (ethyl acetate/heptane). The synthesis yield 0.427 g 9-[(3-fluoro-4-methylcoumarin-7-yl)oxy]nonyl acrylate (A-031) (1.08 mmol, 52% of theory).

**$^1$H NMR** (500 MHz, $CDCl_3$) δ 7.45 (d, *J* = 8.8 Hz, 1H), 6.91 (dd, *J* = 8.9, 2.4 Hz, 1H), 6.83 (d, *J* = 2.5 Hz, 1H), 6.40 (dd, *J* = 17.4, 1.5 Hz, 1H), 6.12 (dd, *J* = 17.4, 10.4 Hz, 1H), 5.81 (dd, *J* = 10.3, 1.5 Hz, 1H), 4.15 (t, *J* = 6.7 Hz, 2H), 4.00 (t, *J* = 6.5 Hz, 2H), 2.38 (d, *J* = 2.8 Hz, 3H), 1.85 - 1.76 (m, 2H), 1.67 (p, *J* = 6.8 Hz, 2H), 1.51 - 1.41 (m, 2H), 1.36 (d, *J* = 11.7 Hz, 8H).
**m. p.** (DSC): 75.3 °C.

Analogously, other derivatives are prepared in the same manner by reaction with acryloyl chloride:

| No. | Reactant | Product | Yield [%] |
|---|---|---|---|
| 2a | 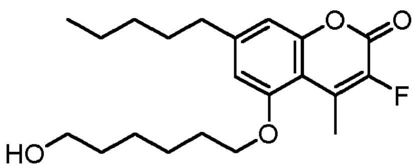 | 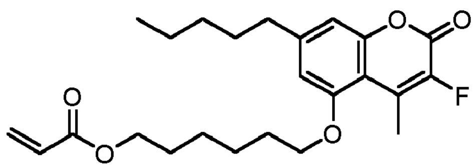 | 71 |
| 2c | 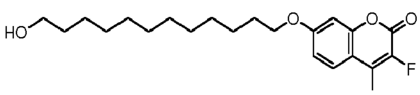 | 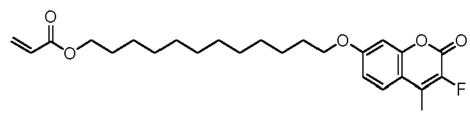 | 78 |

(continued)

| No. | Reactant | Product | Yield [%] |
|---|---|---|---|
| 2d | 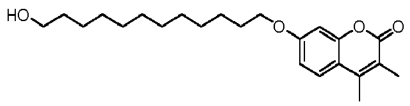 | 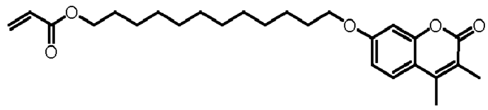 | 42 |

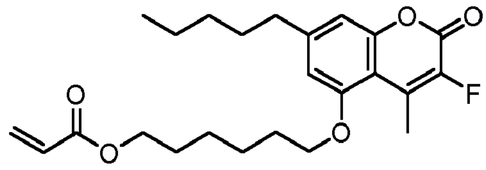

(A-022)

**$^1$H NMR** (500 MHz, $CDCl_3$) δ 6.76 (s, 1H), 6.57 (d, *J* = 1.5 Hz, 1H), 6.40 (dd, *J* = 17.4, 1.4 Hz, 1H), 6.12 (dd, *J* = 17.3, 10.4 Hz, 1H), 5.82 (dd, *J* = 10.4, 1.4 Hz, 1H), 4.18 (t, *J* = 6.6 Hz, 2H), 4.04 (t, *J* = 6.4 Hz, 2H), 2.65 - 2.59 (m, 2H), 2.57 (d, *J* = 3.3 Hz, 3H), 1.93 - 1.84 (m, 2H), 1.73 (p, *J* = 6.8 Hz, 2H), 1.63 (q, *J* = 7.6 Hz, 2H), 1.56 - 1.44 (m, 4H), 1.32 (dqt, *J* = 8.6, 5.7, 3.1 Hz, 4H), 0.90 (t, *J* = 6.8 Hz, 3H).
**m. p.** (DSC): 55.9 °C

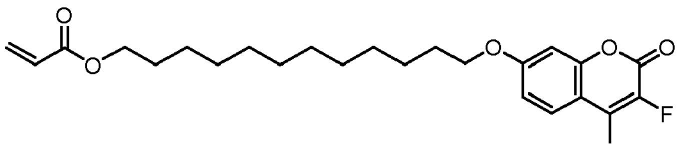

(A-051)

**$^1$H NMR** (500 MHz, DMSO) δ 7.68 (d, *J* = 8.6 Hz, 1H), 7.04 (d, *J* = 2.4 Hz, 2H), 6.31 (dd, *J* = 17.3, 1.5 Hz, 1H), 6.16 (dd, *J* = 17.3, 10.3 Hz, 1H), 5.92 (dd, *J* = 10.3, 1.6 Hz, 1H), 4.07 (dt, *J* = 11.4, 6.5 Hz, 4H), 2.35 (d, *J* = 2.8 Hz, 3H), 1.77 - 1.68 (m, 2H), 1.59 (p, *J* = 6.6 Hz, 2H), 1.40 (d, *J* = 4.8 Hz, 4H), 1.27 (d, *J* = 20.3 Hz, 12H).
**$^{19}$F NMR** (470 MHz, DMSO-$d_6$) δ -141.6.

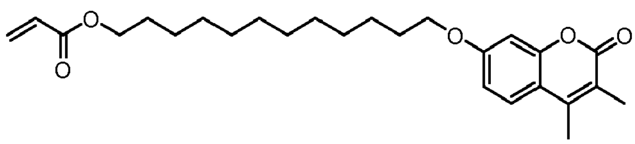

(A-064)

**$^1$H NMR** (500 MHz, $CDCl_3$) δ 7.51 (d, *J* = 8.8 Hz, 1H), 6.86 (dd, *J* = 8.8, 2.5 Hz, 1H), 6.81 (d, *J* = 2.5 Hz, 1H), 6.42 (dd, *J* = 17.3, 1.5 Hz, 1H), 6.14 (dd, *J* = 17.3, 10.4 Hz, 1H), 5.84 (dd, *J* = 10.4, 1.5 Hz, 1H), 4.17 (t, *J* = 6.7 Hz, 2H), 4.02 (t, *J* = 6.6 Hz, 2H), 2.39 (s, 3H), 2.21 (s, 3H), 1.87 - 1.78 (m, 2H), 1.73 - 1.64 (m, 2H), 1.48 (ddd, *J* = 11.8, 8.3, 6.2 Hz, 2H), 1.42-1.29 (m, 14H).
**m. p.** (DSC): 84.6 °C

Example 3:

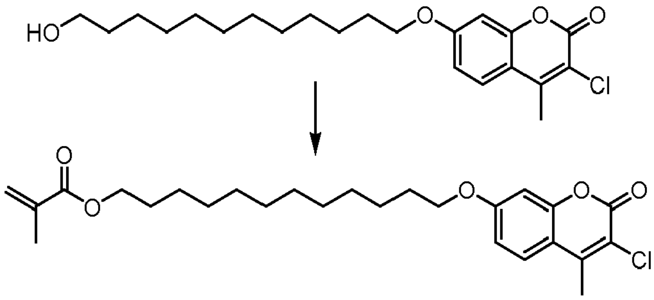

3-Chloro-7-[(12-hydroxydodecyl)oxy]-4-methyl-2H-chromen-2-one (7.72 g, 19.54 mmol, 1.00 eq.) is dissolved in DCM (94 mL) and triethylamine (10.83 mL, 78.14 mmol, 4.00 eq.). (Dimethylamino)pyridine (0.239 g, 1.95 mmol, 0.10 eq.) and methacrylic anhydride (3.79 ml, 25.40 mmol, 1.30 eq.) are added at 0 °C. After stirring at r.t. overnight, the reaction mixture is quenched with 1M aq. HCl solution and extracted with DCM three times. Combined organic extracts are washed with $NaHCO_3$ solution and brine and are dried over $Na_2SO_4$. The solvent of the filtrate is evaporated and the crude product is cleaned by column chromatography (0-20% ethyl acetate/cyclohexane). The synthesis yield 7.263 g 12-[(3-chloro-4-methyl-2-oxo-2H-chromen-7-yl)oxy]dodecyl 2-methylprop-2-enoate (A-045) (15.67 mmol, 80% of theory).

**$^1$H NMR** (500 MHz, $CDCl_3$) δ 7.51 (d, *J* = 8.9 Hz, 1H), 6.89 (dd, *J* = 8.9, 2.5 Hz, 1H), 6.81 (d, *J* = 2.5 Hz, 1H), 6.09 (t, *J* = 1.4 Hz, 1H), 5.54 (p, *J* = 1.6 Hz, 1H), 4.13 (t, *J* = 6.7 Hz, 2H), 4.01 (t, *J* = 6.5 Hz, 2H), 2.55 (s, 3H), 1.94 (d, *J* = 1.3 Hz, 3H), 1.86 - 1.76 (m, 2H), 1.71 - 1.62 (m, 2H), 1.50 - 1.42 (m, 2H), 1.39 - 1.26 (m, 14H).

**m. p.** (DSC): 63.89 °C

Analogously, other derivatives are prepared in the same manner by reaction with methacrylic anhydride; R1 means reactant and [P] means product

| No. | | | Yield [%] |
|---|---|---|---|
| **3a** | R1 | 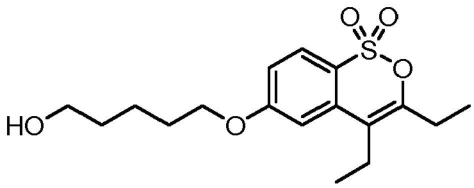 | |
| | [P] | 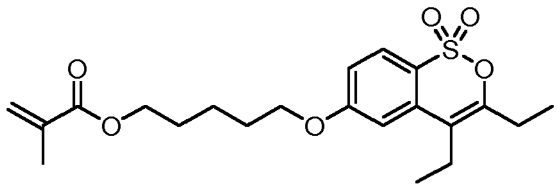 | 69 |
| **3b** | R1 | 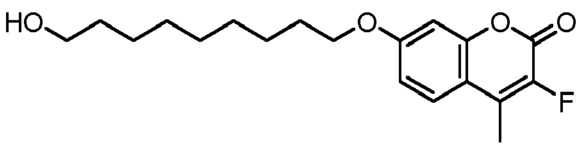 | |
| | [P] | 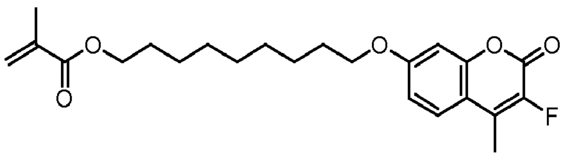 | 66 |

(continued)

| No. | | | Yield [%] |
|---|---|---|---|
| **3c** | R1 | 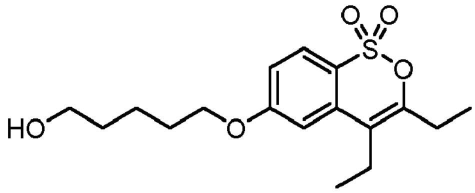 | |
| | [P] | 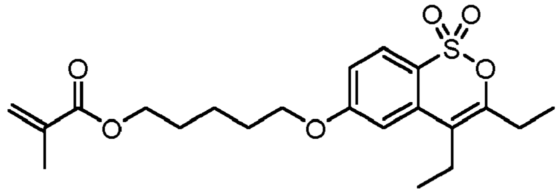 | 69 |

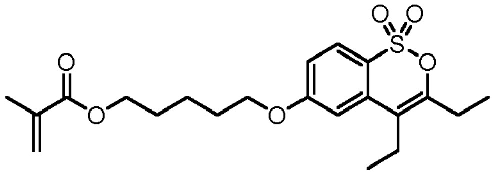

(A-105)

**$^1$H NMR** (500 MHz, $CDCl_3$) δ 7.84 - 7.78 (m, 1H), 6.97-6.91 (m, 2H), 6.10 (t, *J* = 1.3 Hz, 1H), 5.56 (p, *J* = 1.6 Hz, 1H), 4.19 (t, *J* = 6.5 Hz, 2H), 4.05 (t, *J* = 6.3 Hz, 2H), 2.54 (dq, *J* = 22.4, 7.5 Hz, 4H), 1.95 (t, *J* = 1.3 Hz, 3H), 1.88 (dq, *J* = 8.1, 6.4 Hz, 2H), 1.78 (dt, *J* = 14.8, 6.7 Hz, 2H), 1.64-1.54 (m, 2H), 1.25 (t, *J* = 7.5 Hz, 3H), 1.20 (t, *J* = 7.5 Hz, 3H).
**m. p.** (DSC): not observed

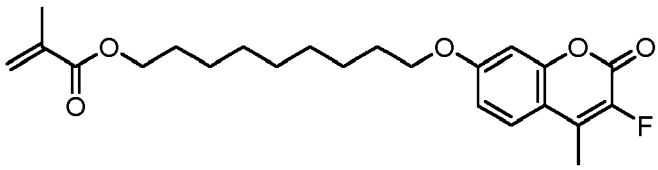

(A-069)

**$^1$H NMR** (500 MHz, $CDCl_3$) δ 7.45 (d, *J* = 8.8 Hz, 1H), 6.91 (dd, *J* = 8.8, 2.4 Hz, 1H), 6.82 (d, *J* = 2.4 Hz, 1H), 6.09 (t, *J* = 1.4 Hz, 1H), 5.54 (p, *J* = 1.6 Hz, 1H), 4.14 (t, *J* = 6.7 Hz, 2H), 4.00 (t, *J* = 6.5 Hz, 2H), 2.37 (d, *J* = 2.8 Hz, 3H), 1.94 (t, *J* = 1.3 Hz, 3H), 1.85 - 1.76 (m, 2H), 1.67 (p, *J* = 6.9 Hz, 2H), 1.46 (td, *J* = 10.4, 9.1, 4.6 Hz, 2H), 1.40 - 1.32 (m, 8H).
**m. p.** (DSC): 84.7 °C.

Example 4: Synthesis of prior art materials

Example 4a: Synthesis of prior art material Ref-[1]

A)

Analogously to example 1b, the precursor compound of compound Ref-[1] is prepared in the same manner by reaction of the reactants as indicated.

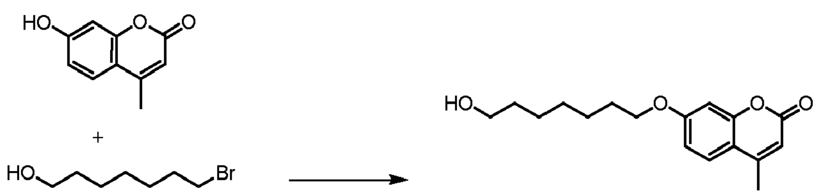

B)

Analogously to example 2, compound Ref-[1] is prepared in the same manner by reaction of the reactant R1 with acryloyl chloride; [P] means product

| **No.** | | | |
|---|---|---|---|
| **4a** | R1 | 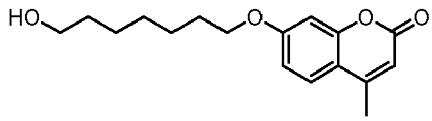 | |
| | [P] | 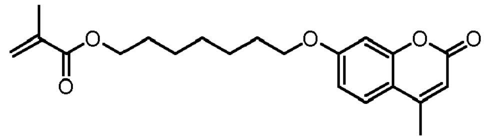 | 46 |

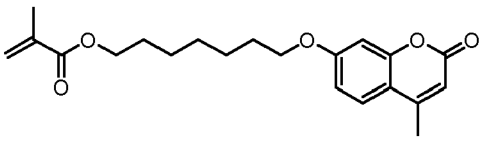

Ref-[1]

**$^1$H NMR** (500 MHz, $CDCl_3$) δ 7.42 (d, *J* = 8.8 Hz, 1H), 6.78 (dd, *J* = 8.8, 2.5 Hz, 1H), 6.73 (d, *J* = 2.5 Hz, 1H), 6.06 (q, *J* = 1.2 Hz, 1H), 6.03 (t, *J* = 1.4 Hz, 1H), 5.48 (p, *J* = 1.6 Hz, 1H), 4.08 (t, *J* = 6.6 Hz, 2H), 3.94 (t, *J* = 6.5 Hz, 2H), 2.33 (d, *J* = 1.3 Hz, 3H), 1.88 (t, *J* = 1.3 Hz, 3H), 1.75 (p, *J* = 6.6 Hz, 2H), 1.63 (dq, *J* = 10.4, 6.8, 5.2 Hz, 2H), 1.42 (h, *J* = 6.4, 5.9 Hz, 2H), 1.35 (p, *J* = 3.6 Hz, 4H).
**m. p.** (DSC): 58.7 °C

Example 4b: Synthesis of prior art materials Ref-[3] and Ref-[4] as described in M. Schraub and N. Hampp, Proc. Of SPIE, 2011, 7885 (Ophthalmic Technologies XXI), 78851Z-1-78851Z-11

A)

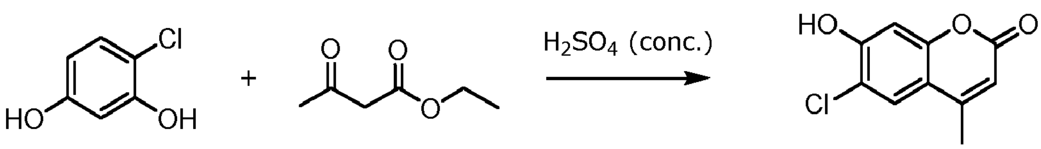


4-Chlororesorcinol (3.5 g, 1.0 eq., 23.0 mmol) and ethyl acetoacetate (2.94 ml, 1.0 eq, 23.0 mmol) are placed in a round bottomed flask and conc. sulfuric acid (25 ml) is carefully added under stirring. The solution is stirred at room temperature for 24 hrs. and then poured on ice. The precipitate is collected, washed thoroughly with dist. $H_2O$ until pH neutrality and dried in an oven to yield the product as beige solid (4.32 g, 20.51 mmol, 89% of theory).
**$^1$H NMR** (500 MHz, DMSO) δ 11.33 (s, 1H), 7.75 (s, 1H), 6.89 (s, 1H), 6.20 (d, *J* = 1.4 Hz, 1H), 2.37 (d, *J* = 1.3 Hz, 3H).

Analogously, other derivatives are prepared in the same manner: R1 means reactant, R2 means reactant 2, [P] means product.

| | | | |
|---|---|---|---|
| **4ba** | R1 | 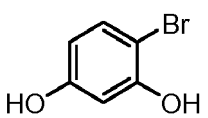 | |

(continued)

| | | | |
|---|---|---|---|
| | R2 | 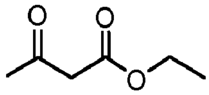 | |
| | [P] | 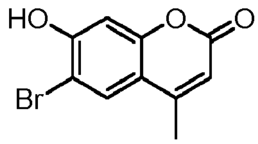 | 20 |

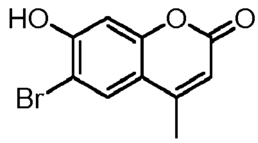

**$^1$H NMR** (500 MHz, DMSO) δ 11.39 (s, 1H), 7.87 (s, 1H), 6.87 (s, 1H), 6.18 (q, *J* = 1.3 Hz, 1H), 2.37 (d, *J* = 1.3 Hz, 3H).

B)

Analogously to example 3, compounds Ref-[3] and Ref-[4] are prepared in the same manner by reaction of the reactant R1 with methacrylic anhydride; [P] means product

| **No.** | | | **Yield [%]** |
|---|---|---|---|
| **4bb** | R1 | 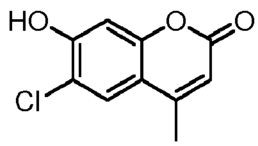 | |
| | [P] | 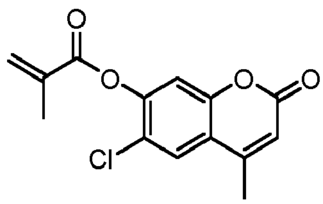 | 57 |
| **4bc** | R1 | 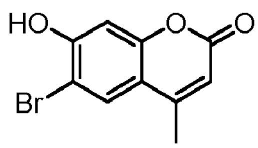 | |
| | [P] | 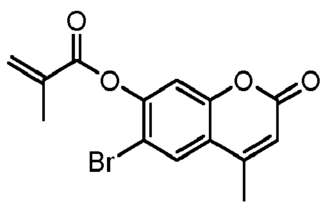 | 80 |

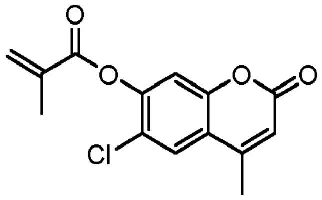

(Ref-[3])

**$^1$H NMR** (500 MHz, Aceton-$d_6$) δ 7.93 (s, 1H), 7.39 (s, 1H), 6.41 (t, *J* = 1.2 Hz, 1H), 6.38 (q, *J* = 1.4 Hz, 1H), 5.96 (p, *J* = 1.5 Hz, 1H), 2.53 (d, *J* = 1.3 Hz, 3H), 2.08 (t, *J* = 1.3 Hz, 3H).
**m. p.** (DSC): 184.7 °C

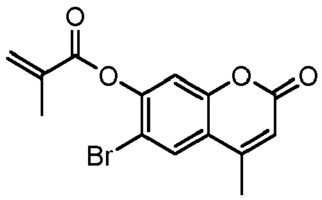

(Ref-[4])

**$^1$H NMR** (500 MHz, $CDCl_3$) δ 7.83 (s, 1H), 7.23 (s, 1H), 6.47 (d, *J* = 1.1 Hz, 1H), 6.30 (d, *J* = 1.5 Hz, 1H), 5.89 - 5.84 (m, 1H), 2.43 (d, *J* = 1.3 Hz, 3H), 2.10 (t, *J* = 1.3 Hz, 3H).
**m. p.** (DSC): 188.4 °C

Examples of application:

Example 5 - General polymerization procedure to produce bulk copolymer

For production of bulk polymer blanks, the monomers are melted under vacuum and additional components in respective amounts are added as indicated in table 3 below.

Exemplarily for application example 5-4, a composition of 9-[(3-fluoro-4-methylcoumarin-7-yl)oxy]nonyl acrylate (A-031) (0.199 g, 0.51 mmol, 42.2 mol%), 2,2,3,3,4,4,5,5-Octafluoropentyl acrylate (0.183 g, 0.619 mmol, 51.1 mol%) and ethylene glycol dimethacrylate (0.0144 g, 0.071 mmol, 5.9 mol%) as crosslinker is well mixed under stirring using gentle heat and degassed by three freeze pump-thaw cycles. Appropriate amounts (0.02 - 0.12 equiv.) of a radical initiator (e.g. 1,1'-(3,3,5-trimethylcyclohexylidene)bis[2-(1,1-dimethylethyl)-peroxide [Luperox® 231], 2-[(E)-2-(1-cyano-1-methylethyl)diazen-1-yl]-2-methylpropanenitrile) or tert-Butyl peroxide are added. The compositions shown in table 3 are manufactured in the same way as described for application example 4-4. A heating bath for the stirring is used, where necessary.

Polymerization:

Two glass plates are coated with a polyethylene terephthalate sheet and a 1 mm thick cell is created between the polyethylene terephthalate sheets using a silicone rubber gasket. The coated faces of the glass sheets are clipped together using spring clips with a syringe needle being placed between the gasket and the polyethylene terephthalate sheets. The cavity is then filled with the above manufactured compositions through the needle using a gastight syringe. Once the cavity is filled the syringe needle is removed, a final clip is used to seal the mould and the assembly is placed in an oven. The polymerization temperature is between 60 °C and 180 °C and the individual polymerization conditions are choosen for the respective initiators. The moulds are allowed to cool to room temperature before the polymer plate is removed from the mould.

Refractive index change is induced by irradiation at 275-340 nm. The refractive indices (n) of the polymer films and blanks at 589 nm are measured on Schmidt+Haensch ATR-L before and after irradiation. The refractive index **nD,** 35 °C is measured before irradiation. The difference of refractive indices before and after irradiation is **Δn.** The following table shows the refractive indices **nD,** 35 °C as well as the change in refractive index after irradiation **(Δn).**

The phase transition temperatures are determined with a TA Instruments Q2000 differential scanning calorimeter during heating in the second heating run with 20 K/min from -100 °C to 200 °C in a hermetic aluminium pans.

Ref-[1] is an example of a monomer embraced by the general disclosure of US2013033975 e.g. page 6.
Ref-[2] is described in M. Schraub et al, European Polymer Journal 51 (2014) 21-27.

Ref-[3] and Ref-[4] are examples of monomers embraced by the general disclosure of M. Schraub and N. Hampp, Proc. Of SPIE, 2011, 7885 (Ophthalmic Technologies XXI), 78851Z-1-78851Z-11.

| | m.p. [°C] |
|---|---|
| 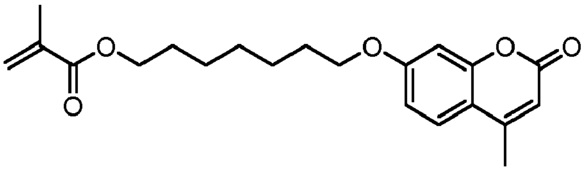 Ref-[1] | 58.7 |
| 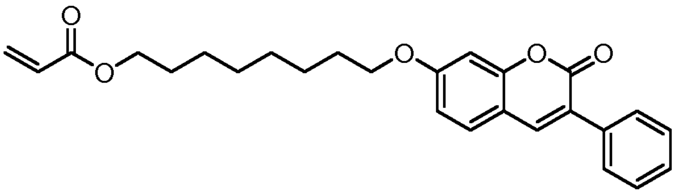 Ref-[2] | 100 |
| 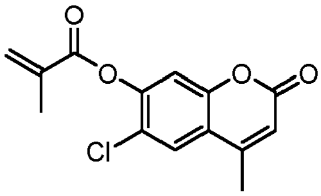 Ref-[3] | 184.7[a] |
| 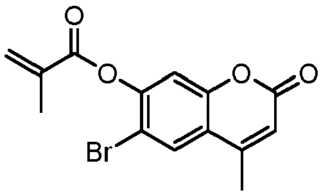 Ref-[4] | 188.4[a] |
| 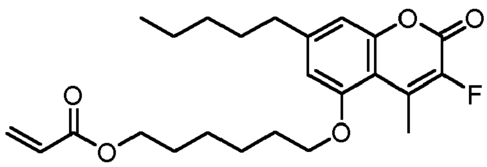 (A-022) | 55.9 |
| 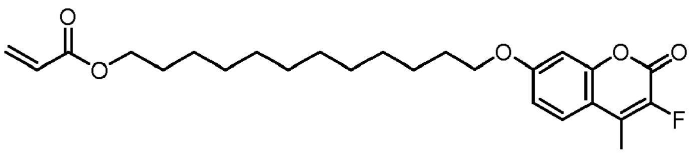 (A-051) | 69.6 |
| 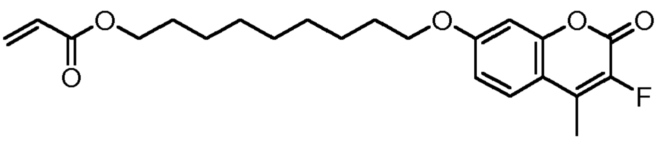 (A-031) | 75.3 |

(continued)

| | m.p. [°C] |
|---|---|
| 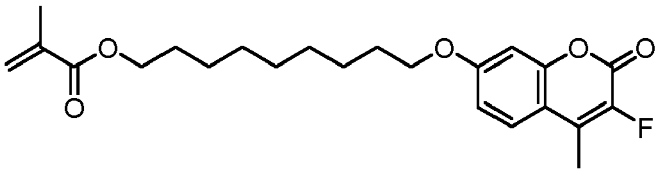 (A-069) | 84.7 |
| 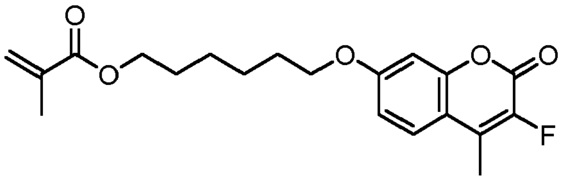 (A-011) | 47.8 |
| 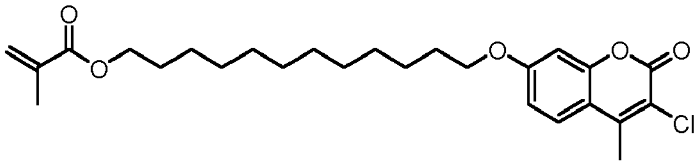 (A-045) | 63.9 |
| 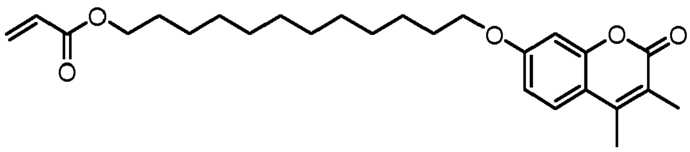 (A-064) | 84.6 |
| 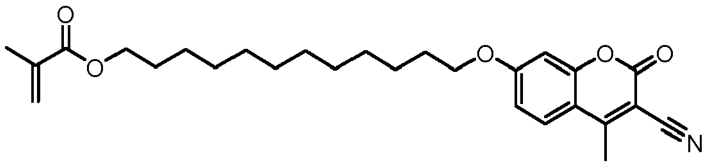 (A-014) | 58.0 |
| 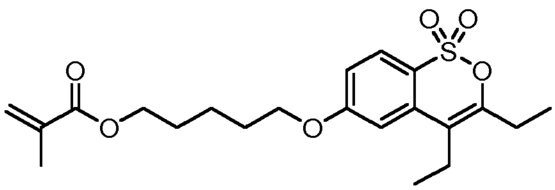 | not observed |
| (A-105) | |

(continued)

| | m.p. [°C] |
|---|---|
| 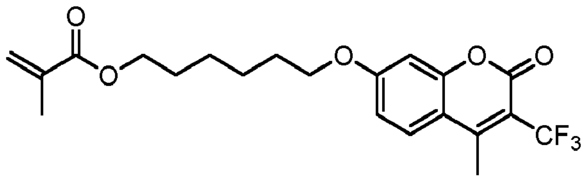 (A-104) | 58.5 |
| [a] As shown in the above overview, monomers Ref-[3] and Ref-[4] are characterized by very high melting points over 180 °C. Formulation mixtures with such high melting monomers are in general critical to process. In this case, no clear homogenous solutions and suspensions with a co-monomer such as *iso*decyl methacrylate at 80 °C or with *n*-butylacrylate at 35 °C are obtained. Monomers Ref-[3] and Ref-[4] are therefore not suitable for bulk polymerization. | |

Table 3: Compositions - Amount of components is given in mol-%, the amount of the respective chosen radical initiator adds to 100 mol%:

| applicatio n example | monomer | mol% monomer | mol% *n*-BuAc | mol% EGDMA | mol% HEMA | mol% UV-Abs. | mol% EtMAc |
|---|---|---|---|---|---|---|---|
| Reference example 1 | Ref-[1 ] | 94.4 | 0 | 0.9 | 0 | 0 | 0 |
| Reference example 2 | Ref-[2] | 18.4 | 65.8 | 0.9 | 14.1 | 0.6 | 0 |
| 5-1 | A-031 | 36.0 | 59.0[b] | 4.0[C] | 0 | 0 | 0 |
| 5-2 | A-031 | 91.9 | 0 | 6.3 | 0 | 0 | 0 |
| 5-3 | A-069 | 25.5 | 67.6 | 5.2 | 0 | 0.7 | 0 |
| 5-4 | A-031 | 42.2 | 51.1[d] | 5.9 | 0 | 0 | 0 |
| 5-5 | A-064 | 22.2 | 74.3 | 2.4 | 0 | 0 | 0 |
| 5-6 | A-051 | 13.5 | 63.7[e] | 2.5 | 19.1 | 0.8 | 0 |
| 5-7 | A-051 | 23.2 | 69.7 | 5.4 | 0 | 0.7 | 0 |
| 5-8 | A-045 | 32.2 | 62.6[b] | 4.2[c] | 0 | 0 | 0 |
| 5-9 | A-022 | 34.3 | 60.6[b] | 4.1[c] | 0 | 0 | 0 |
| 5-10 | A-014 | 32.7 | 62.2 | 4.2[c] | 0 | 0 | 0 |
| 5-11 | A-011 | 37.8 | 57.5[b] | 3.8[c] | 0 | 0 | 0 |
| 5-12 | A-105 | 35.0 | 60.1[b] | 4.0[C] | 0 | 0 | 0 |
| *n*-BuAc = *n*-butylacrylate; EGDMA = ethylene glycol dimethacrylate; HEMA = hydroxyethylmethacrylate; EtMAc = ethylmethacrylate. [b]*iso*-decyl methacrylate is used instead of n-butylacrylate. [c]polyethyleneglycol diacrylate (Mn 250) is used instead of EGDMA. [d]2,2,3,3,4,4,5,5-octafluoropentyl acrylate is used instead of *n*-butylacrylate. [e]2,2,2-trifluoroethyl methacrylate is used instead of *n*-butylacrylate. | | | | | | | |

Table 4: Polymer properties (refractive index $n_{D,\ 35\ °C}$ and Abbe number $v_0$) and refractive index change after irradiation

| Application Example | $T_g$ [°C] | $n_{D,\ 35\ °C}$ | $\Delta n$ | $v_0$ |
|---|---|---|---|---|
| Reference example 1 | 38.3 | 1.580 | 0.014 | 23.6 |
| Reference example 2 | 8.0 | 1.536 | 0.013 | 24.5 |
| 5-1 | -5.69 | 1.511 | 0.007 | 35.3 |

(continued)

| Application Example | $T_g$ [°C] | $n_{D, 35 °C}$ | Δn | $v_0$ |
|---|---|---|---|---|
| 5-2 | *not determined* | 1.554 | 0.017 | 25.5 |
| 5-3 | 8.06 | 1.520 | 0.006 | 33.3 |
| 5-4 | 14.25 | 1.513 | 0.014 | 25.6 |
| 5-5 | -15.0 | 1.514 | 0.005 | 33.9 |
| 5-6 | 46.5 | 1.482 | 0.006 | 42.2 |
| 5-7 | -0.05 | 1.525 | 0.007 | 30.7 |
| 5-8 | 3.47 | 1.518 | 0.007 | 33.9 |
| 5-9 | 1.31 | 1.505 | 0.003 | 38.8 |
| 5-10 | -21.4 | 1.508 | 0.005 | 30.9 |
| 5-11 | 12.3 | 1.518 | 0.008 | 34.8 |
| 5-12 | 11.2 | 1.517 | <0.001 | 36.1 |

The results of the application examples 5-1 to 5-12 show a refractive index change after irradiation and high Abbe numbers.

The refractive index change versus Abbe numbers of the application examples 5-1 to 5-12 compared to application examples for prior art reference compounds Ref-[1] and Ref-[2] is visualized in Figure 1.

Figure 1 clearly shows the advantage of the described polymers over prior art references.

## Claims

1. An ophthalmic device or a precursor article for an ophthalmic device comprising at least one polymerized compound of formula (I)

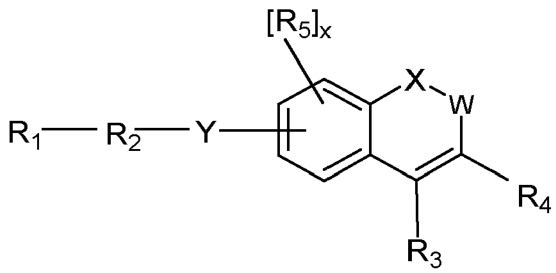

(I)

,

wherein

X-W is O-C(=O), O-C(=S), O-$SO_2$, $SO_2$-O, $SO_2$-C(=S), S-C(=O), S-C(=S), $NR_B$-C(=O) or $NR_B$-C(=S);
$R_B$ is at each occurrence independently H, a linear or branched alkyl group having 1 to 6 C atoms or a linear or branched partially or fully fluorinated alkyl group having 1 to 10 C atoms;
$R_3$ is a linear or branched alkyl group having 1 to 8 C atoms or a cycloalkyl group having 3 to 7 C atoms;
$R_4$ F, Cl, CN, a linear or branched alkyl group having 1 to 8 C atoms or a partially or fully fluorinated or partially or fully chlorinated alkyl group having 1 to 8 C atoms, a linear or branched partially or fully fluorinated alkoxy group having 1 to 8 C atoms, a linear or branched optionally partially or fully fluorinated alkoxyalkyl group having 2 to 8 C atoms or a cycloalkyl group having 3 to 6 C atoms which may be partially or fully fluorinated;
$R_5$ is at each occurrence independently a linear or branched alkyl group having 1 to 8 C atoms or a linear or branched alkoxy group having 1 to 8 C atoms;
x is 0 0r 1;
Y is O, S, SO, $SO_2$, Se, $NR_0$ or a bond;

$R_0$ is at each occurrence independently selected from the group consisting of a linear or branched alkyl group having 1 to 4 C atoms;

$R_1$ is a trialkoxysilyl group or a dialkoxyalkylsilyl group where the alkyl and/or alkoxy groups are each independently linear or branched having 1 to 6 C atoms, or a silyl group of formula (1), (2) or (3) or a polymerizable group of formula (4),

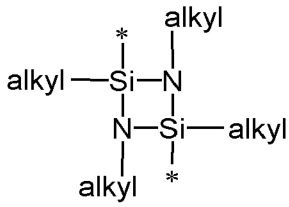 (1) , 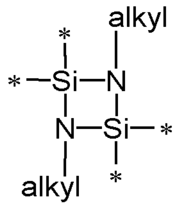 (2) ,

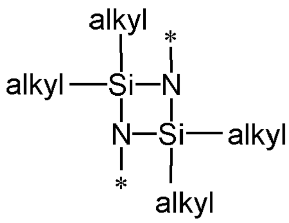 (3) , 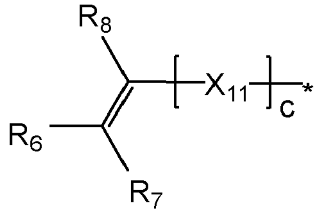 (4) ,

where alkyl means at each occurrence independently of each other a linear or branched alkyl group having 1 to 6 C atoms and the asterisk "*" denotes at each occurrence independently of each other a linkage to the linker $-R_2-Y-$;

and wherein

$X_{11}$ is selected from the group consisting of O, S, O-$SO_2$, $SO_2$-O, C(=O), OC(=O), C(=O)O, S(C=O) and (C=O)S,

$R_6$, $R_7$, $R_8$ are at each occurrence independently of each other selected from the group consisting of H, F, a linear or branched, non-fluorinated, partially or completely fluorinated alkyl group having 1 to 20 C atoms and aryl with 6 to 14 C atoms and

c is 0 0r 1;

$-R_2-$ is $-(C(R)_2)_o-$, or $-(C(R)_2)_p-X_8-(C(R)_2)_q-(X_9)_s-(C(R)_2)_r-(X_{10})_t-(C(R)_2)_u-$; R is at each occurrence independently selected from the group consisting of H, F, a linear or branched alkyl group having 1 to 4 C atoms or a linear or branched partially or fully fluorinated alkyl group having 1 to 4 C atoms;

o is selected from the group consisting of 5 to 20,

$X_8$, $X_9$, $X_{10}$ are at each occurrence independently O, S, $SO_2$, or $NR_B$,

s, t is 0 or 1,

p, q are at each occurrence independently selected from the group consisting of 1 to 10,

r, u are at each occurrence independently selected from the group consisting of 0 to 10, wherein the overall number of atoms for $-(C(R)_2)_p-X_8-(C(R)_2)_q-(X_9)_s-(C(R)_2)_r-(X_{10})_t-(C(R)_2)_u-$ is up to 20 atoms.

**2.** The ophthalmic device or the precursor article for an ophthalmic device according to claim 1 comprising at least one polymerized compound of formulae (Ia), (Ib) or (Ic),

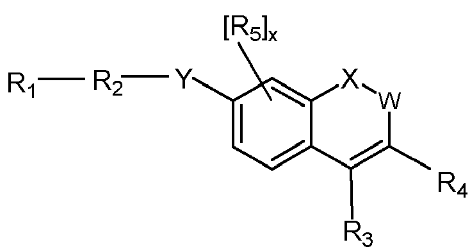 (Ia) , 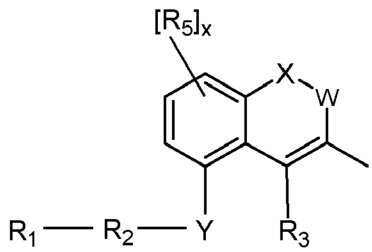 (Ib) ,

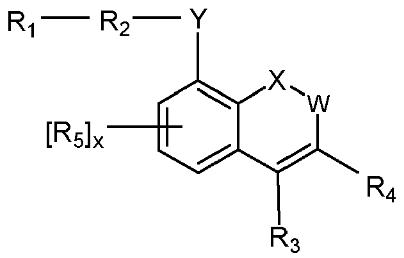

(Ic)

,

wherein $R_1$, $-R_2-$, Y, $R_3$, $R_4$, X-W, x and $R_5$ have a meaning as indicated in claim 1.

3. The ophthalmic device or the precursor article for an ophthalmic device according to claim 1 or 2 wherein X-W is O-C(=O), $O-SO_2$, $SO_2-O$, $SO_2-C(=S)$, S-C(=O) or $NR_B-C(=O)$.

4. The ophthalmic device or the precursor article for an ophthalmic device according to one or more of claims 1 to 3 comprising an oligomer, polymer or copolymer comprising a constitutional unit $M^0$ based on formulae (I), (Ia), (Ib) or (Ic) where $R_1$ on each occurrence is polymerized, $R_1$ thus forms the regioregular, alternated, regiorandom, statistical, block or random oligomer or polymer backbone or is part of the copolymer backbone.

5. The ophthalmic device or the precursor article for an ophthalmic device according to one or more of claims 1 to 4 where said polymerized group $R_1$ is of formulae (1-p), (2-p), (3-p) or (4-p)

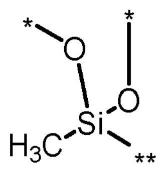

(1-p) ,

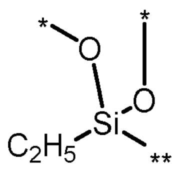

(2-p) ,

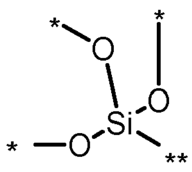

(3-p) ,

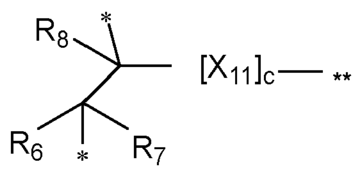

(4-p) ,

where the asterisk "*" within formulae (1-p) to (4-p) denotes a linkage to the adjacent repeating unit in the polymer chain or oligomer chain or to a terminal end group, the asterisk "**" within formulae (1-p) to (4-p) denotes the linkage to the remainder of formulae (I), (Ia), (Ib) or (Ic) and $R_6$, $R_7$, $R_8$, $X_{11}$ and c have a meaning according to claim 1.

6. The ophthalmic device or the precursor article for an ophthalmic device according to one or more of claims 1 to 5 wherein the constitutional unit $M^0$ is of formulae, ($M^0$-I), ($M^0$-Ia), ($M^0$-Ib) or ($M^0$-Ic),

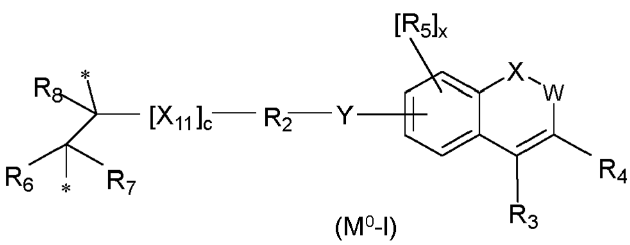

($M^0$-I) ,

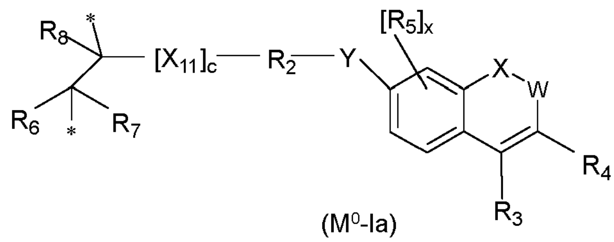

($M^0$-Ia) ,

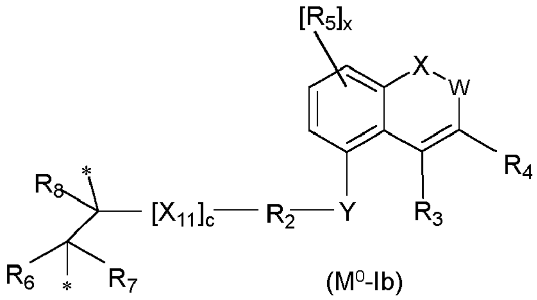

($M^0$-Ib)

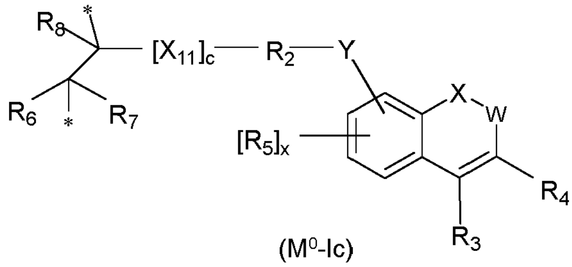

($M^0$-Ic) ,

where $R_1$, $-R_2-$, X-W, Y, $R_3$, $R_4$, $R_5$, x, $R_6$, $R_7$, $R_8$, $X_{11}$ and c have a meaning according to claim 1 and where the asterisk "*" denotes at each occurrence a linkage to the adjacent repeating unit in the polymer chain or oligomer chain or to a terminal end group.

**7.** The ophthalmic device or the precursor article for an ophthalmic device according to one or more of claims 1 to 6 comprising beside of the at least one polymerized compound of formulae (I), (Ia), (Ib) or (Ic) or the constitutional unit $M^0$ of formulae ($M^0$-I), ($M^0$-Ia), ($M^0$-Ib), ($M^0$-Ic) at least one further polymerized co-monomer selected from the group consisting of styrene, ethoxyethyl methacrylate (EOEMA), methyl methacrylate (MMA), methyl acrylate, *n*-alkyl acrylates (the *n*-alkyl group comprising 2-20 C-atoms), *n*-alkyl methacrylates (the *n*-alkyl group comprising 2-20 C-atoms), *i*-alkyl acrylates (the *i*-alkyl group comprising 3-20 C-atoms), *i*-alkyl methacrylates (the *i*-alkyl group comprising 3-20 C-atoms), ethoxyethoxy ethylacrylate (EEEA), *n*-hydroxalkyl acrylate (the n-alkyl group comprising 2 to 10 C-atoms), *n*-hydroxalkyl methacrylate (the *n*-alkyl group comprising 2 to 10 C-atoms), tetrahydrofuryl methacrylate (THFMA), glycidyl methacrylate (GMA), 16-hydroxyhexadecyl acrylate, 16-hydroxyhexadecyl methacrylate, 18-hydroxyoctadecyl acrylate, 18-hydroxyoctadecyl methacrylate, 2-phenoxyethyl acrylate (EGPEA), heptafluorobutyl acrylate, heptafluorobutyl methacrylate, hexafluorobutyl acrylate, hexafluorobutyl methacrylate, hexafluoroisopropyl acrylate, hexafluoroisopropyle methacrylate, octafluoropentyl acrylate, octafluoropentyl methacrylate, petanfluoropropyl acrylate, pentafluoropropyl methacrylate, tetrafluoropropyl methacrylate, trifluoroethyl acrylate, trifluoroethyl methacrylate, trimethylcyclohexyl acrylate, trimethylcyclohexyl methacrylate, 2-(4-bromophenyl)ethyl methacrylate, 4-phenylbutyl methacrylate, 4-methylphenyl methacrylate, phenyl methacrylate, 4-methylphenyl acrylate, benzyl acrylate, benzyl methacrylate, 2-benzyloxyethyl methacrylate, 3-benzyloxypropyl methacrylate, phenylethyl acrylate, phenylethyl methacrylate, 3-phenylpropyl methacrylate, 2-phenoxyethyl acrylate, 2-benzyloxyethyl acrylate, 3-phenylpropyl acrylate, 4-methylbenzyl acrylate, phenyl acrylate, 2-(phenylthio)ethyl methacrylate, 4-phenylbutyl acrylate, 5-phenylpentyl acrylate, 3-benzyloxypropyl acrylate, 2-(phenylthio)-propyl acrylate, 2-(phenylthio)ethyl acrylate, 4-phenylbutyl acrylate, 4-methylbenzyl methacrylate, 2-(2-methylphenyl)ethyl acrylate, 2-(2-methylphenyl)ethyl methacrylate, 2-(3-methylphenyl)ethyl acrylate, 2-(3-methylphenyl)ethyl methacrylate, 2-(4-methylphenyl)ethyl acrylate, 2-(4-methylphenyl)ethyl methacrylate, 2-(4-propylphenyl)ethyl acrylate, 2-(4-propylphenyl)ethyl methacrylate, 2-(4-(1-methylethyl)phenyl)ethyl acrylate, 2-(4-(1-methylethyl)phenyl)ethyl methacrylate, 2-(4-methoxyphenyl)ethyl methacrylate, 2-(4-methoxyphenyl)ethyl acrylate, 2-(4-cyclohexylphenyl)ethyl acrylate, 2-(4-cyclohexylphenyl)ethyl methacrylate, 2-(4-chlorophenyl)ethyl methacrylate, 2-(4-chlorophenyl)ethyl acrylate, 2-(3-chlorophenyl)ethyl methacrylate, 2-(3-chlorophenyl)ethyl acrylate, 2-(2-chlorophenyl)ethyl methacrylate, 2-(2-chlorophenyl)ethyl acrylate, 2-(4-bromophenyl)ethyl acrylate, 2-(4-phenylphenyl)ethyl methacrylate, 2-(4-phenylphenyl)ethyl acrylate, 2-(3-phenylphenyl)ethyl methacrylate, 2-(3-phenylphenyl)ethyl acrylate, 2-(4-benzylphenyl)ethyl methacrylate, 2-(4-benzylphenyl)ethyl acrylate, 2-(phenylthio)ethyl acrylate, 3-benzyloxypropyl acrylate, 2-[2-(benzyloxy)ethoxy]ethyl acrylate, 2-[2-(benzyloxy)ethoxy]ethyl methacrylate, pentafluorophenyl methacrylate, heptadecafluorodecyl methacrylate, dodecafluoroheptyl methacrylate, heptadecafluorodecyl acrylate, pentafluorophenyl acrylate.

**8.** The ophthalmic device or the precursor article for an ophthalmic device according to claim 7 wherein the at least one further polymerized co-monomer is selected from methyl methacrylate, *n*-alkyl acrylates (the *n*-alkyl group comprising 2-20 C-atoms), fluorinated *n*-alkyl acrylates (the *n*-alkyl group comprising 2-20 C-atoms), *n*-alkyl methacrylates (the *n*-alkyl group comprising 2-20 C-atoms), fluorinated *n*-alkyl methacrylates (the *n*-alkyl group comprising 2-20 C-atoms), *i*-alkyl acrylates (the *i*-alkyl group comprising 3-20 C-atoms), fluorinated *i*-alkyl acrylates (the *i*-alkyl group comprising 3-20 C-atoms), *i*-alkyl methacrylates (the *i*-alkyl group comprising 3-20 C-atoms), fluorinated *i*-alkyl methacrylates, *n*-hydroxyalkyl acrylates (the *n*-alkyl group comprising 2-10 C-atoms), *n*-hydroxyalkyl methacrylates (the *n*-alkyl group comprising 2-10 C-atoms) or a mixture thereof.

**9.** The ophthalmic device or the precursor article for an ophthalmic device according to one or more of claims 1 to 8 wherein $-R_2-$ is at each occurrence independently $-(C(R)_2)_o-$ and R and o have a meaning as indicated in claim 1.

**10.** The ophthalmic device or the precursor article for an ophthalmic device according to one or more of claims 1 to 9 wherein polymerized $R_1$ is at each occurrence independently derived from an acryl or methacryl radical.

**11.** The precursor article for an ophthalmic device according to one or more of claims 1 to 10 wherein said precursor article is a blank which may be transformed into an eye implant, preferably an intraocular lens.

**12.** Process of forming an ophthalmic device or a precursor article for an ophthalmic device according to one or more of claims 1 to 11, said process comprising the steps of

- providing a composition comprising at least one compound of formulae (I), (Ia), (Ib) or (Ic) as described in one or more of claims 1 to 3, 9 and 10 and/or an oligomer or polymer comprising at least one compound of formulae (I), (Ia), (Ib) or (Ic) as described in one or more of claims 1 to 3, 9 and 10 and having at least one reactive group left for polymerization and optionally further monomers different from compounds of formulae (I), (Ia), (Ib) or (Ic) and/or crosslinking agents and/or UV absorbers and/or radical initiators;
- subsequently forming the ophthalmic device or precursor article of said composition.

**13.** Process of changing the optical properties of an ophthalmic device or a precursor article for an ophthalmic device according to one or more of claims 1 to 11, said process comprising the steps of

- providing an ophthalmic device or a precursor article according to claim 1, and
- subsequently exposing said ophthalmic device or precursor article to irradiation having a wavelength of at least 200 nm and at most 1500 nm.

**14.** Ophthalmic device or precursor article for an ophthalmic device obtainable by the process according to claim 13.

**15.** Compounds of formula (I)

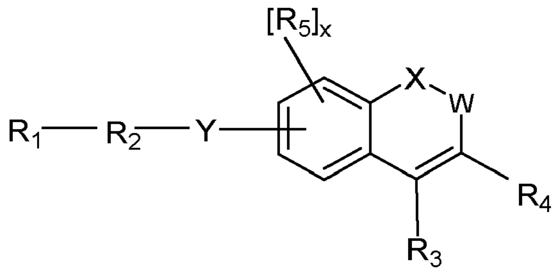

(I)

,

wherein

X-W is O-C(=O), O-C(=S), O-$SO_2$, $SO_2$-O, $SO_2$-C(=S), S-C(=O), S-C(=S), $NR_B$-C(=O) or $NR_B$-C(=S);
$R_B$ is at each occurrence independently H, a linear or branched alkyl group having 1 to 6 C atoms or a linear or branched partially or fully fluorinated alkyl group having 1 to 10 C atoms;
$R_3$ is a linear or branched alkyl group having 1 to 8 C atoms or a cycloalkyl group having 3 to 7 C atoms;
$R_4$ is F, Cl, a linear or branched alkyl group having 1 to 8 C atoms or a partially or fully fluorinated or partially or fully chlorinated alkyl group having 1 to 8 C atoms, a linear or branched partially or fully fluorinated alkoxy

group having 1 to 8 C atoms, a linear or branched optionally partially or fully fluorinated alkoxyalkyl group having 2 to 8 C atoms or a cycloalkyl group having 3 to 7 C atoms which may be partially or fully fluorinated;
$R_5$ is at each occurrence independently a linear or branched alkyl group having 1 to 8 C atoms or a linear or branched alkoxy group having 1 to 8 C atoms;
x is 0 0r 1;
Y is O, S, SO, $SO_2$, Se, $NR_0$ or a bond;
$R_0$ is at each occurrence independently selected from the group consisting of a linear or branched alkyl group having 1 to 4 C atoms;
$R_1$ is a trialkoxysilyl group or a dialkoxyalkylsilyl group where the alkyl and/or alkoxy groups are each independently linear or branched having 1 to 6 C atoms, or a silyl group of formula (1), (2) or (3) or a polymerizable group of formula (4),

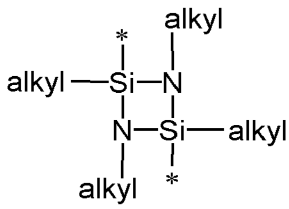 (1) ,
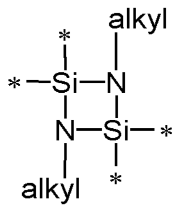 (2) ,
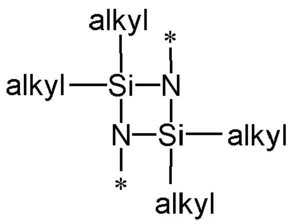 (3) ,
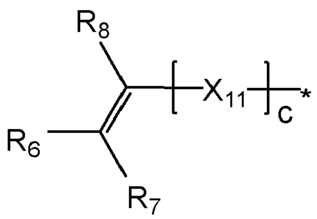 (4) ,

where alkyl means at each occurrence independently of each other a linear or branched alkyl group having 1 to 6 C atoms and the asterisk "*" denotes at each occurrence independently of each other a linkage to the linker $-R_2$-Y-;
and wherein
$X_{11}$ is selected from the group consisting of O, S, O-$SO_2$, $SO_2$-O, C(=O), OC(=O), C(=O)O, S(C=O) and (C=O)S,
$R_6$, $R_7$, $R_8$ are at each occurrence independently of each other selected from the group consisting of H, F, a linear or branched, non-fluorinated, partially or completely fluorinated alkyl group having 1 to 20 C atoms and aryl with 6 to 14 C atoms and
c is 0 0r 1;
$-R_2$- is $-(C(R)_2)_o$-;
R is at each occurrence independently selected from the group consisting of H, F, a linear or branched alkyl group having 1 to 4 C atoms or a linear or branched partially or fully fluorinated alkyl group having 1 to 4 C atoms; and
o is selected from the group consisting of 5 to 20.

**16.** Oligomer, polymer or copolymer comprising at least one polymerized compound of formula (I) as described in claim 15.

**17.** Polymer according to claim 16 comprising beside of the polymerized compounds of formula (I) at least one further polymerized monomer selected from the group consisting of styrene, ethoxyethyl methacrylate (EOEMA), methyl methacrylate (MMA), methyl acrylate, *n*-alkyl acrylates (the *n*-alkyl group comprising 2-20 C-atoms), *n*-alkyl methacrylates (the *n*-alkyl group comprising 2-20 C-atoms), *i*-alkyl acrylates (the *i*-alkyl group comprising 3-20 C-atoms), *i*-alkyl methacrylates (the *i*-alkyl group comprising 3-20 C-atoms), ethoxyethoxy ethylacrylate (EEEA), *n*-hydroxalkyl acrylate (the *n*-alkyl group comprising 2 to 10 C-atoms), *n*-hydroxalkyl methacrylate (the *n*-alkyl group comprising 2 to 10 C-atoms), tetrahydrofuryl methacrylate (THFMA), glycidyl methacrylate (GMA), 16-hydroxyhexadecyl acrylate, 16-hydroxyhexadecyl methacrylate, 18-hydroxyoctadecyl acrylate, 18-hydroxyoctadecyl methacrylate, 2-phenoxyethyl acrylate (EGPEA), heptafluorobutyl acrylate, heptafluorobutyl methacrylate, hexafluorobutyl acrylate, hexafluorobutyl methacrylate, hexafluoroisopropyl acrylate, hexafluoroisopropyle methacrylate, octafluoropentyl acrylate, octafluoropentyl methacrylate, petanfluoropropyl acrylate, pentafluoropropyl methacrylate, tetrafluoropropyl

methacrylate, trifluoroethyl acrylate, trifluoroethyl methacrylate, trimethylcyclohexyl acrylate, trimethylcyclohexyl methacrylate, 2-(4-bromophenyl)ethyl methacrylate, 4-phenylbutyl methacrylate, 4-methylphenyl methacrylate, phenyl methacrylate, 4-methylphenyl acrylate, benzyl acrylate, benzyl methacrylate, 2-benzyloxyethyl methacrylate, 3-benzyloxypropyl methacrylate, phenylethyl acrylate, phenylethyl methacrylate, 3-phenylpropyl methacrylate, 2-phenoxyethyl acrylate, 2-benzyloxyethyl acrylate, 3-phenylpropyl acrylate, 4-methylbenzyl acrylate, phenyl acrylate, 2-(phenylthio)ethyl methacrylate, 4-phenylbutyl acrylate, 5-phenylpentyl acrylate, 3-benzyloxypropyl acrylate, 2-(phenylthio)-propyl acrylate, 2-(phenylthio)ethyl acrylate, 4-phenylbutyl acrylate, 4-methylbenzyl methacrylate, 2-(2-methylphenyl)ethyl acrylate, 2-(2-methylphenyl)ethyl methacrylate, 2-(3-methylphenyl)ethyl acrylate, 2-(3-methylphenyl)ethyl methacrylate, 2-(4-methylphenyl)ethyl acrylate, 2-(4-methylphenyl)ethyl methacrylate, 2-(4-propylphenyl)ethyl acrylate, 2-(4-propylphenyl)ethyl methacrylate, 2-(4-(1-methylethyl)phenyl)ethyl acrylate, 2-(4-(1-methylethyl)phenyl)ethyl methacrylate, 2-(4-methoxyphenyl)ethyl methacrylate, 2-(4-methoxyphenyl)ethyl acrylate, 2-(4-cyclohexylphenyl)ethyl acrylate, 2-(4-cyclohexylphenyl)ethyl methacrylate, 2-(4-chlorophenyl)ethyl methacrylate, 2-(4-chlorophenyl)ethyl acrylate, 2-(3-chlorophenyl)ethyl methacrylate, 2-(3-chlorophenyl)ethyl acrylate, 2-(2-chlorophenyl)ethyl methacrylate, 2-(2-chlorophenyl)ethyl acrylate, 2-(4-bromophenyl)ethyl acrylate, 2-(4-phenylphenyl)ethyl methacrylate, 2-(4-phenylphenyl)ethyl acrylate, 2-(3-phenylphenyl)ethyl methacrylate, 2-(3-phenylphenyl)ethyl acrylate, 2-(4-benzylphenyl)ethyl methacrylate, 2-(4-benzylphenyl)ethyl acrylate, 2-(phenylthio)ethyl acrylate, 3-benzyloxypropyl acrylate, 2-[2-(benzyloxy)ethoxy]ethyl acrylate, 2-[2-(benzyloxy)ethoxy]ethyl methacrylate, pentafluorophenyl methacrylate, heptadecafluorodecyl methacrylate, dodecafluoroheptyl methacrylate, heptadecafluorodecyl acrylate, pentafluorophenyl acrylate.

**18.** Composition comprising at least one compound of formula (I) as described in claim 15 and/or an oligomer or polymer according to claim 16 or 17 having at least one reactive group left for polymerization.

Figure 1

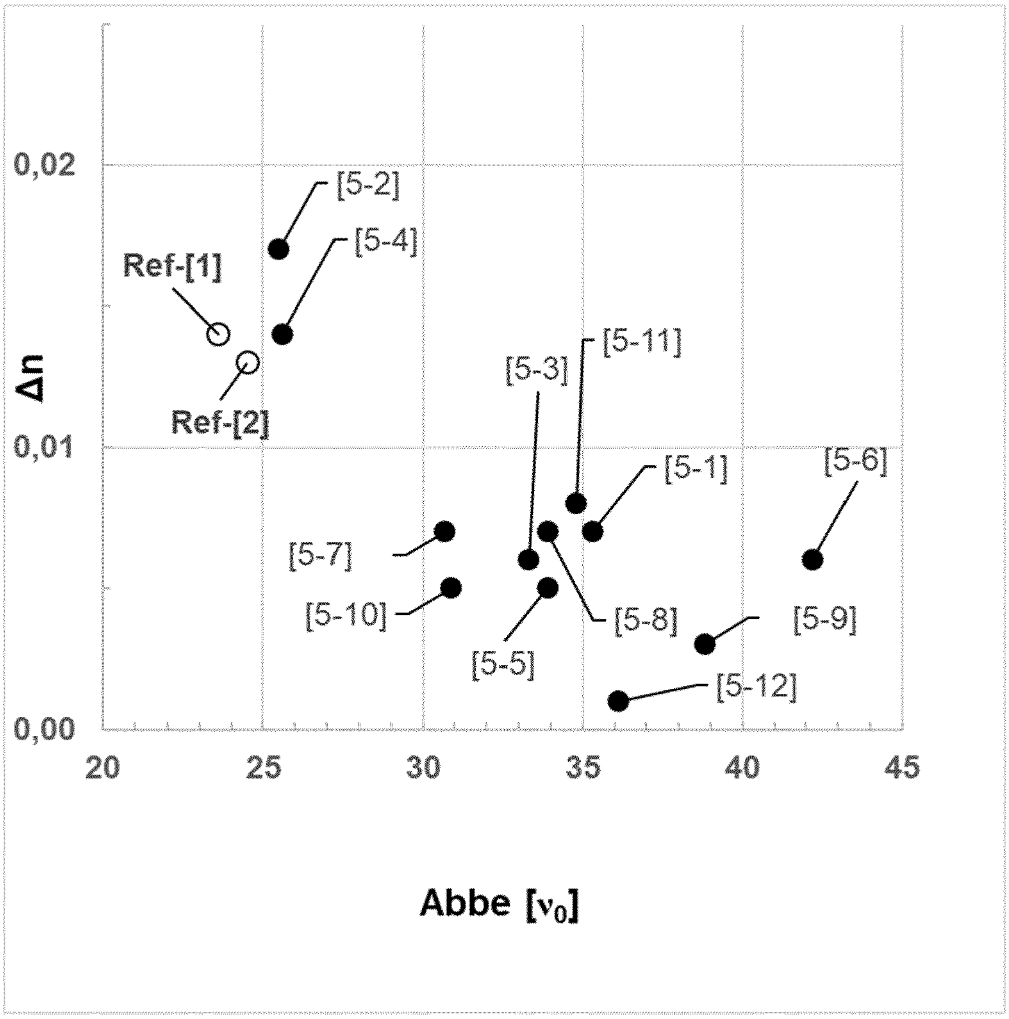
0,02
0,01
0,00
Δn
20
25
30
35
40
45
Abbe [ν0]
Ref-[1]
Ref-[2]
[5-1]
[5-2]
[5-3]
[5-4]
[5-5]
[5-6]
[5-7]
[5-8]
[5-9]
[5-10]
[5-11]
[5-12]

Europäisches Patentamt
European Patent Office
Office européen des brevets

# EUROPEAN SEARCH REPORT

Application Number
EP 21 15 3841

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | SKOWRONSKI L. ET AL: "Optical properties of coumarins containing copolymers", OPTICAL MATERIALS, vol. 47, 1 September 2015 (2015-09-01), pages 18-23, XP55812203, NL ISSN: 0925-3467, DOI: 10.1016/j.optmat.2015.06.047 * figure 1 * | 1-18 | INV. C07D311/16 A61P27/02 A61L27/16 C07D311/42 C08F220/30 C07F7/18 |
| A | KORCHIA LAETITIA ET AL: "UV-responsive amphiphilic graft copolymers based on coumarin and polyoxazoline", SOFT MATTER, vol. 13, no. 25, 1 January 2017 (2017-01-01), pages 4507-4519, XP55812195, ISSN: 1744-683X, DOI: 10.1039/C7SM00682A * figure 1 * | 1-18 | |
| A,D | WO 2017/032444 A1 (MERCK PATENT GMBH [DE]) 2 March 2017 (2017-03-02) * claims 1-24 * | 1-18 | TECHNICAL FIELDS SEARCHED (IPC) |
| A,D | WO 2009/074520 A2 (ACRI TEC GMBH [DE]; RITTER HELMUT [DE] ET AL.) 18 June 2009 (2009-06-18) * claims 1-24 * | 1-18 | C07D A61P A61L C08F C07F |

The present search report has been drawn up for all claims

1

| Place of search | Date of completion of the search | Examiner |
|---|---|---|
| The Hague | 10 June 2021 | Voyiazoglou, D |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 21 15 3841

This annex lists the patent family members relating to the patent documents cited in the above-mentioned European search report.
The members are as contained in the European Patent Office EDP file on
The European Patent Office is in no way liable for these particulars which are merely given for the purpose of information.

10-06-2021

| Patent document cited in search report | Publication date | Patent family member(s) | Publication date |
|---|---|---|---|
| WO 2017032444 A1 | 02-03-2017 | CN 107922370 A | 17-04-2018 |
| | | EP 3133066 A1 | 22-02-2017 |
| | | EP 3337794 A1 | 27-06-2018 |
| | | JP 6862449 B2 | 21-04-2021 |
| | | JP 2018525444 A | 06-09-2018 |
| | | US 2018237410 A1 | 23-08-2018 |
| | | US 2020262805 A1 | 20-08-2020 |
| | | WO 2017032444 A1 | 02-03-2017 |
| WO 2009074520 A2 | 18-06-2009 | AT 544791 T | 15-02-2012 |
| | | EP 2225293 A2 | 08-09-2010 |
| | | ES 2382255 T3 | 06-06-2012 |
| | | JP 2011507987 A | 10-03-2011 |
| | | US 2011028667 A1 | 03-02-2011 |
| | | WO 2009074520 A2 | 18-06-2009 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2007033831 A
- WO 2009074520 A
- US 20100324165 A
- WO 2017032442 A
- WO 2017032443 A
- WO 2017032444 A
- WO 2018149850 A
- WO 2018149852 A
- WO 2018149853 A
- WO 2018149855 A
- WO 2018149856 A
- WO 2018149857 A
- WO 2019121642 A
- US 5587444 A
- WO 9610049 A
- JP H1174077 B
- JP 2009099253 B
- WO 2009074521 A
- WO 2010049044 A
- WO 2012097858 A
- US 2013033975 A
- US 20140356788 A
- US 2016018737 A
- US 2017081532 A
- US 20170306121 A
- WO 2012167124 A
- US 5290892 A
- US 5331073 A
- US 5693095 A
- US 2013033975 E


### Non-patent literature cited in the description


- **M. SCHRAUB et al.** *European Polymer Journal,* 2014, vol. 51, 21-27
- **PATEL, M.P. et al.** *Biomaterials,* 1987, vol. 8, 53-56
- **S. HELMSTETTER et al.** *J Polym Res,* 2016, vol. 23, 249
- **C. E. WHEELOCK.** *J. Am. Chem. Soc.,* 1959, vol. 81 (6), 1348-1352
- **K. MUTHURAMU ; V. RAMAMURTHY.** *Ind. J. Chem. B,* 1984, vol. 23B (6), 502-508
- **J. TRÄGER et al.** *Macromolecular Bioscience,* 2008, vol. 8 (2), 177-183
- **M. SCHRAUB ; AND N. HAMPP.** Ophthalmic Technologies XXI. *Proc. Of SPIE,* 2011, vol. 7885, 78851Z-1, 78851Z-11
- **K. TIETZ et al.** *Macromol. Chem. Phys.,* 2014, vol. 215 (16), 1563-1572
- **M. SCHRAUB ; N. HAMPP.** Ophthalmic Technologies XXI. *Proc. Of SPIE,* 2011, vol. 7885, 78851Z-1, 78851Z-11
- *PAC,* 1996, vol. 68, 2291
- **J. M. G. COWIE.** Polymers: Chemistry & Physics of Modern Materials. Blackie, 1991
- **A. MIYATA ; S. YAGUCHI.** *J Cataract Refract Surg,* 2004, vol. 30, 1768-1772
- **BOZUKOVA D ; PAGNOULLE C ; JÊROME R ; JÊROME C.** Polymers in modern ophthalmic implants -Historical background and recent advances. *Material Science and Engineering R,* 2010, vol. 69, 63-83